(19) Europäisches Patentamt
European Patent Office
Office européen des brevets

(11) EP 2 128 163 A1

(12) **EUROPEAN PATENT APPLICATION**
published in accordance with Art. 153(4) EPC

(43) Date of publication:
**02.12.2009 Bulletin 2009/49**

(21) Application number: **08703778.4**

(22) Date of filing: **24.01.2008**

(51) Int Cl.:
***C07D 519/00*** *(2006.01)* ***A61K 31/4545*** *(2006.01)*
***A61K 31/695*** *(2006.01)* ***A61P 1/00*** *(2006.01)*
***A61P 1/04*** *(2006.01)* ***A61P 1/12*** *(2006.01)*
***A61P 1/14*** *(2006.01)* ***A61P 1/16*** *(2006.01)*
***A61P 1/18*** *(2006.01)* ***A61P 3/04*** *(2006.01)*
***A61P 3/06*** *(2006.01)* ***A61P 3/10*** *(2006.01)*
***A61P 7/00*** *(2006.01)* ***A61P 7/02*** *(2006.01)*
***A61P 7/10*** *(2006.01)* ***A61P 9/04*** *(2006.01)*
***A61P 9/10*** *(2006.01)* ***A61P 9/12*** *(2006.01)*
***A61P 11/00*** *(2006.01)* ***A61P 11/04*** *(2006.01)*
***A61P 13/10*** *(2006.01)*

(86) International application number:
**PCT/JP2008/050949**

(87) International publication number:
**WO 2008/090944 (31.07.2008 Gazette 2008/31)**

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **25.01.2007 JP 2007015593**

(71) Applicant: **Takeda Pharmaceutical Company Limited**
**Osaka 541-0045 (JP)**

(72) Inventors:
- **FUKATSU, Kohji**
**Osaka-shi**
**Osaka 540-8645 (JP)**
- **KAMATA, Makoto**
**Osaka-shi**
**Osaka 532-8686 (JP)**
- **YAMASHITA, Tohru**
**Osaka-shi**
**Osaka 532-8686 (JP)**

(74) Representative: **Jones, Nicholas Andrew et al**
**Withers & Rogers LLP**
**Goldings House**
**2 Hays Lane**
**GB-London SE1 2HW (GB)**

(54) **SPIRO-RING COMPOUND**

(57) The present invention aims to provide a compound having an acetyl-CoA carboxylase (ACC) inhibitory action, which is useful for the prophylaxis or treatment of obesity, diabetes, hypertension, hyperlipidemia, cardiac failure, diabetic complications, metabolic syndrome, sarcopenia, cancer and the like, and has superior efficacy.
The present invention provides a compound represented by the formula (I):

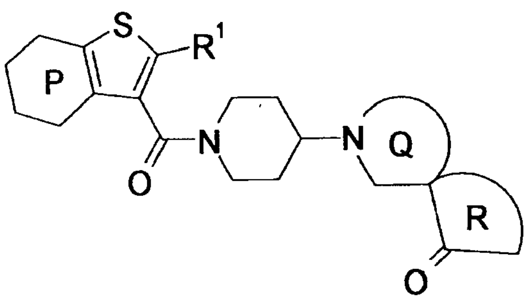

(I)

EP 2 128 163 A1

wherein
$R^1$ is a hydrogen atom or a substituent;
ring P is an optionally substituted 6-membered nitrogen-containing aromatic heterocycle;
ring Q is an optionally further substituted 5- to 7-membered nitrogen-containing non-aromatic heterocycle; and
ring R is an optionally fused 5- to 7-membered non-aromatic ring, which is further optionally substituted,
or a salt thereof.

## Description

## Technical Field

The present invention relates to a spiro ring compound having an acetyl-CoA carboxylase (sometimes to be abbreviated as ACC in the present specification) inhibitory action, which is useful for the prophylaxis or treatment of obesity, diabetes, hypertension, hyperlipidemia, cardiac failure, diabetic complications, metabolic syndrome, sarcopenia, cancer and the like.

## Background Art

ACC is an enzyme that converts acetyl-CoA to malonyl-CoA, and catalyzes a rate determining reaction in fatty acid metabolism. Malonyl-CoA, which is produced by an ACC catalyst reaction, inhibits fatty acid oxidation in mitochondria based on the feedback inhibition of carnitine palmitoyl transferase-1 (CPT-1). Accordingly, ACC plays a key role in controlling the balance between use of carbohydrate and fatty acid in the liver and skeletal muscle, and further, controlling insulin sensitivity in the liver, skeletal muscle and adipose tissue.

A reduced level of malonyl-CoA by ACC inhibition can promote an increase in fatty acid oxidation, decreased secretion of triglyceride (TG)-rich lipoprotein (VLDL) in the liver, regulation of insulin secretion in the pancreas, and further, improvement in the insulin sensitivity in the liver, skeletal muscle and adipose tissue.

In addition, long-term administration of a compound having an ACC inhibitory action can strikingly decrease the TG content of the liver and adipose tissues and selectively decrease body fat in obese test subjects taking low fat diet, by promoting fatty acid oxidation and suppressing de novo synthesis of fatty acid.

Accordingly, a compound having an ACC inhibitory action is extremely useful for the prophylaxis or treatment of metabolic syndrome, obesity, hypertension, diabetes, cardiovascular diseases associated with atherosclerosis and the like.

As a spiro ring compound, the following compound has been reported.

(1) A compound represented by the formula:

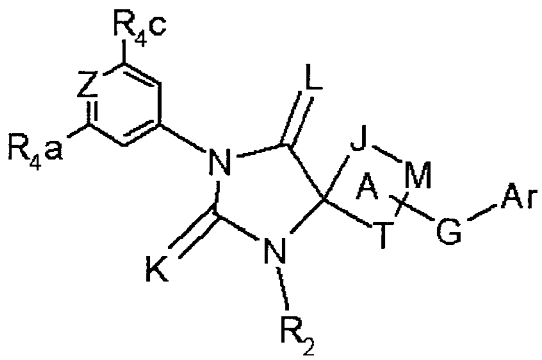

wherein

L and K are each independently O or S;

Z is N or $CR_{4b}$;

G is a linker or a bond, each of which is bonded to T or M;

Ar is aryl or heteroaryl, each of which is optionally substituted;

J, M and T are selected such that they form a 3- to 6-membered saturated or partially unsaturated cycloalkyl or heterocycle; and

$R_2$, $R_{4a}$, $R_{4b}$ and $R_{4c}$ are each a hydrogen atom or the like,

which is useful as a LFA-1/ICAM (Lymphocyte Function-associated Antigen-1/Intercellular adhesion molecule) inhibitor (see patent document 1).

In addition, as a compound having an ACC inhibitory action, the following compounds have been reported.

(2) A compound represented by the formula:

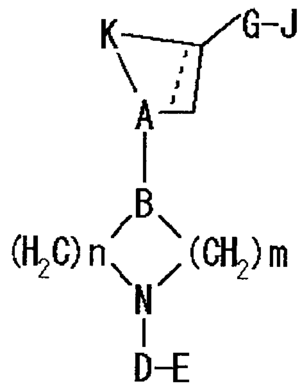

wherein
A-B is N-CH or CH-N;
K is $(CH_2)_r$ wherein r is an integer of 2-4;
m and n are each an integer of 1 to 3;
D is CO or $SO_2$;
E is an optionally substituted bi- to tetra-cyclic ring or the like;
G is CO, $SO_2$ or $CR^7R^8$ wherein $R^7$ and $R^8$ are each a hydrogen atom or the like; and
J is $OR^1$, $NR^2R^3$ or $CR^4R^5R^6$ wherein $R^1$, $R^2$, $R^3$, $R^4$, $R^5$ and $R^6$ are each a hydrogen atom or the like
(see patent document 2).

(3) A compound represented by the formula:

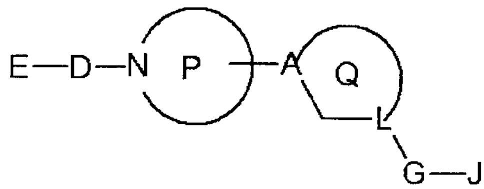

wherein
E is an optionally substituted cyclic group;
D and G are each independently CO or $SO_2$;
ring P is an optionally substituted nitrogen-containing 5- or 6-membered non-aromatic heterocycle;
ring Q is an optionally substituted aromatic ring or an optionally substituted non-aromatic heterocycle;
A and L are each independently C, CH or N; and
J is an optionally substituted hydrocarbon group, an optionally substituted hydroxy group, an optionally substituted heterocyclic group or an optionally substituted amino group
(see patent document 3).

However, the compound of the present invention is not reported.

patent document 1: WO03/029245
patent document 2: WO03/072197
patent document 3: JP-A-2006-131559

## Disclosure of the Invention

### Problems to be Solved by the Invention

There is a demand for the development of a compound having an ACC inhibitory action, which is useful for the prophylaxis or treatment of obesity, diabetes, hypertension, hyperlipidemia, cardiac failure, diabetic complications, metabolic syndrome, sarcopenia, cancer and the like, and has superior efficacy.

**Means of Solving the Problems**

The present inventors have found that a compound represented by the formula (I):
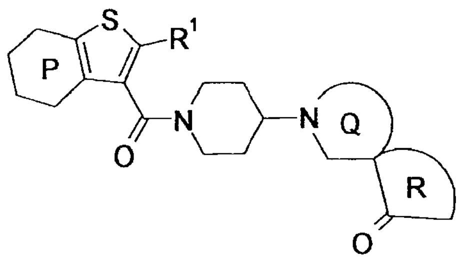

(I)

wherein
$R^1$ is a hydrogen atom or a substituent;
ring P is an optionally substituted 6-membered nitrogen-containing aromatic heterocycle;
ring Q is an optionally further substituted 5- to 7-membered nitrogen-containing non-aromatic heterocycle; and
ring R is an optionally fused 5- to 7-membered non-aromatic ring, which is further optionally substituted,
or a salt thereof [hereinafter sometimes to be referred to as compound (I)] has a superior ACC inhibitory action and is useful for the prophylaxis or treatment of obesity, diabetes, hypertension, hyperlipidemia, cardiac failure, diabetic complications, metabolic syndrome, sarcopenia, cancer and the like, and has superior efficacy. Based on this finding, the present inventors have conducted intensive studies and completed the present invention.
Accordingly, the present invention relates to

(1) compound (I);
(2) compound (I) wherein $R^1$ is an optionally substituted amino group;
(3) compound (I) wherein ring P is an optionally substituted pyridine ring;
(4) compound (I) wherein ring Q is an optionally further substituted 6-membered monocyclic nitrogen-containing non-aromatic heterocycle;
(5) compound (I) wherein ring R is an optionally further substituted 5-membered monocyclic non-aromatic heterocycle;
(6) N-(3-{[4-(3,3-dimethyl-1-oxo-2-oxa-7-azaspiro[4.5]dec-7-yl)piperidin-1-yl]carbonyl}thieno[2,3-b]pyridin-2-yl)-N'-methylurea,
N-(3-{[4-(2-isopropyl-1,3-dioxo-2,7-diazaspiro[4.5]dec-7-yl)piperidin-1-yl]carbonyl}-6-methylthieno[2,3-b]pyridin-2-yl)-N'-methylurea,
N-ethyl-N'-(3-{[4-(3-isopropyl-2-methyl-4-oxo-1,3,7-triazaspiro[4.5]dec-1-en-7-yl)piperidin-1-yl]carbonyl}thieno[2,3-b]pyridin-2-yl)urea, or
N-ethyl-N'-(3-{[4-(2-isopropyl-1-oxo-2,7-diazaspiro[4.5]dec-7-yl)piperidin-1-yl]carbonyl}-6-methylthieno[2,3-b]pyridin-2-yl)urea,
or a salt thereof;
(7) a prodrug of compound (I);
(8) a pharmaceutical agent comprising compound (I) or a prodrug thereof;
(9) the pharmaceutical agent of the above-mentioned (8), which is an acetyl-CoA carboxylase inhibitor;
(10) the pharmaceutical agent of the above-mentioned (8), which is an agent for the prophylaxis or treatment of obesity, diabetes, hypertension, hyperlipidemia, cardiac failure, diabetic complications, metabolic syndrome, sarcopenia or cancer;
(11) a method of inhibiting acetyl-CoA carboxylase in a mammal, which comprises administering compound (I) or a prodrug thereof to the mammal;
(12) a method for the prophylaxis or treatment of obesity, diabetes, hypertension, hyperlipidemia, cardiac failure, diabetic complications, metabolic syndrome, sarcopenia or cancer in a mammal, which comprises administering compound (I) or a prodrug thereof to the mammal;
(13) use of compound (I) or a prodrug thereof, for the production of an acetyl-CoA carboxylase inhibitor;
(14) use of compound (I) or a prodrug thereof, for the production of an agent for the prophylaxis or treatment of obesity, diabetes, hypertension, hyperlipidemia, cardiac failure, diabetic complications, metabolic syndrome, sarcopenia or cancer;

and the like.

## Effect of the Invention

The compound of the present invention has an ACC inhibitory action, which is useful for the prophylaxis or treatment of obesity, diabetes, hypertension, hyperlipidemia, cardiac failure, diabetic complications, metabolic syndrome, sarcopenia, cancer and the like, and has superior efficacy.

[Detailed Description of the Invention]

The definition of each symbol in the formula (I) is described in detail in the following.
The "halogen atom" in the present specification means, unless otherwise specified, fluorine, chlorine, bromine or iodine.
The "$C_{1-3}$ alkylenedioxy group" in the present specification means, unless otherwise specified, methylenedioxy, ethylenedioxy or the like.
The "$C_{1-6}$ alkyl group" in the present specification means, unless otherwise specified, methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl or the like.
The "$C_{1-6}$ alkoxy group" in the present specification means, unless otherwise specified, methoxy, ethoxy, propoxy, isopropoxy, butoxy, isobutoxy, sec-butoxy, tert-butoxy or the like.
The "$C_{1-6}$ alkoxy-carbonyl group" in the present specification means, unless otherwise specified, methoxycarbonyl, ethoxycarbonyl, propoxycarbonyl, tert-butoxycarbonyl or the like.
The "$C_{1-6}$ alkyl-carbonyl group" in the present specification means, unless otherwise specified, acetyl, propanoyl, butanoyl, isobutanoyl, pentanoyl, isopentanoyl, hexanoyl or the like.
$R^1$ is a hydrogen atom or a substituent.
Examples of the "substituent" for $R^1$ include an "optionally substituted hydrocarbon group", an "optionally substituted heterocyclic group", an "optionally substituted hydroxy group", an "optionally substituted mercapto group", an "optionally substituted amino group", a "cyano group", a "nitro group", an "acyl group", a "halogen atom" and the like.
Examples of the "hydrocarbon group" of the above-mentioned "optionally substituted hydrocarbon group" include a $C_{1-10}$ alkyl group, a $C_{2-10}$ alkenyl group, a $C_{2-10}$ alkynyl group, a $C_{3-10}$ cycloalkyl group, a $C_{3-10}$ cycloalkenyl group, a $C_{4-10}$ cycloalkadienyl group, a $C_{6-14}$ aryl group, a $C_{7-13}$ aralkyl group, $C_{8-13}$ arylalkenyl group and the like.
Examples of the $C_{1-10}$ alkyl group include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, heptyl, octyl, nonyl, decyl and the like.
Examples of the $C_{2-10}$ alkenyl group include ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, 5-hexenyl, 1-heptenyl, 1-octenyl and the like.
Examples of the $C_{2-10}$ alkynyl group include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, 1-heptynyl, 1-octynyl and the like.
Examples of the $C_{3-10}$ cycloalkyl group include cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl, bicyclo[3.2.2]nonyl, bicyclo[3.3.1]nonyl, bicyclo[4.2.1]nonyl, bicyclo[4.3.1]decyl, adamantyl and the like.
Examples of the $C_{3-10}$ cycloalkenyl group include 2-cyclopenten-1-yl, 3-cyclopenten-1-yl, 2-cyclohexen-1-yl, 3-cyclohexen-1-yl and the like.
Examples of the $C_{4-10}$ cycloalkadienyl group include 2,4-cyclopentadien-1-yl, 2,4-cyclohexadien-1-yl, 2,5-cyclohexadien-1-yl and the like.
The above-mentioned $C_{3-10}$ cycloalkyl group, $C_{3-10}$ cycloalkenyl group and $C_{4-10}$ cycloalkadienyl group are each optionally condensed with a benzene ring to form a fused ring group. Examples of the fused ring group include indanyl, dihydronaphthyl, tetrahydronaphthyl, fluorenyl and the like.
Examples of the $C_{6-14}$ aryl group include phenyl, naphthyl, anthryl, phenanthryl, acenaphthyl, biphenylyl and the like.
Examples of the $C_{7-13}$ aralkyl group include benzyl, phenethyl, naphthylmethyl, biphenylylmethyl and the like.
Examples of the $C_{8-13}$ arylalkenyl group include styryl and the like.
The $C_{1-10}$ alkyl group, $C_{2-10}$ alkenyl group and $C_{2-10}$ alkynyl group which are exemplified as the above-mentioned "hydrocarbon group" optionally have 1 to 3 substituents at substitutable positions.
Examples of the substituent include

(1) a $C_{3-10}$ cycloalkyl group (e.g., cyclopropyl, cyclohexyl);

(2) a $C_{6-14}$ aryl group (e.g., phenyl, naphthyl) optionally substituted by 1 to 3 substituents selected from

(a) a $C_{1-6}$ alkyl group optionally substituted by 1 to 3 halogen atoms,
(b) a hydroxy group,
(c) a $C_{1-6}$ alkoxy group optionally substituted by 1 to 3 halogen atoms, and
(d) a halogen atom;

(3) an aromatic heterocyclic group (e.g., thienyl, furyl, pyridyl, pyrazolyl, imidazolyl, tetrazolyl, oxazolyl, thiazolyl, oxadiazolyl, thiadiazolyl) optionally substituted by 1 to 3 substituents selected from

(a) a $C_{1-6}$ alkyl group optionally substituted by 1 to 3 halogen atoms,
(b) a hydroxy group,
(c) a $C_{1-6}$ alkoxy group optionally substituted by 1 to 3 halogen atoms, and
(d) a halogen atom;

(4) a non-aromatic heterocyclic group (e.g., tetrahydrofuryl, morpholinyl, thiomorpholinyl, piperidinyl, pyrrolidinyl, piperazinyl) optionally substituted by 1 to 3 substituents selected from

(a) a $C_{1-6}$ alkyl group optionally substituted by 1 to 3 halogen atoms,
(b) a hydroxy group,
(c) a $C_{1-6}$ alkoxy group optionally substituted by 1 to 3 halogen atoms,
(d) a halogen atom, and
(e) an oxo group;

(5) an amino group optionally mono- or di-substituted by substituent(s) selected from

(a) a $C_{1-6}$ alkyl group optionally substituted by 1 to 3 halogen atoms,
(b) a $C_{1-6}$ alkyl-carbonyl group optionally substituted by 1 to 3 halogen atoms,
(c) a $C_{1-6}$ alkoxy-carbonyl group optionally substituted by 1 to 3 halogen atoms,
(d) a $C_{1-6}$ alkylsulfonyl group (e.g., methylsulfonyl) optionally substituted by 1 to 3 halogen atoms,
(e) a carbamoyl group optionally mono- or di-substituted by $C_{1-6}$ alkyl group(s) optionally substituted by 1 to 3 halogen atoms, and
(f) an aromatic heterocyclic group (e.g., thiazolyl, oxazolyl, pyridyl, imidazolyl);

(6) a $C_{1-6}$ alkyl-carbonyl group optionally substituted by 1 to 3 halogen atoms;
(7) a $C_{1-6}$ alkoxy-carbonyl group optionally substituted by 1 to 3 substituents selected from

(a) a halogen atom,
(b) a $C_{1-6}$ alkoxy group, and
(c) a $C_{6-14}$ aryl group (e.g., phenyl);

(8) a $C_{1-6}$ alkylsulfonyl group (e.g., methylsulfonyl, ethylsulfonyl, isopropylsulfonyl) optionally substituted by 1 to 3 halogen atoms;
(9) a carbamoyl group optionally mono- or di-substituted by $C_{1-6}$ alkyl group(s) optionally substituted by 1 to 3 halogen atoms;
(10) a thiocarbamoyl group optionally mono- or di-substituted by $C_{1-6}$ alkyl group(s) optionally substituted by 1 to 3 halogen atoms;
(11) a sulfamoyl group optionally mono- or di-substituted by $C_{1-6}$ alkyl group(s) optionally substituted by 1 to 3 halogen atoms;
(12) a carboxy group;
(13) a hydroxy group;
(14) a $C_{1-6}$ alkoxy group optionally substituted by 1 to 3 substituents selected from

(a) a halogen atom,
(b) a carboxy group,
(c) a $C_{1-6}$ alkoxy group,
(d) a $C_{1-6}$ alkoxy-carbonyl group optionally substituted by 1 to 3 $C_{6-14}$ aryl groups (e.g., phenyl), and
(e) an amino group optionally mono- or di-substituted by substituent(s) selected from a $C_{1-6}$ alkyl group and a

$C_{1-6}$ alkoxy-carbonyl group;

(15) a $C_{2-6}$ alkenyloxy group (e.g., ethenyloxy) optionally substituted by 1 to 3 halogen atoms;
(16) a $C_{7-13}$ aralkyloxy group (e.g., benzyloxy);
(17) a $C_{6-14}$ aryloxy group (e.g., phenyloxy, naphthyloxy);
(18) a $C_{1-6}$ alkyl-carbonyloxy group (e.g., acetyloxy, tert-butylcarbonyloxy);
(19) a $C_{6-14}$ aryl-carbonyl group (e.g., benzoyl) optionally substituted by 1 to 3 substituents selected from

(a) a halogen atom, and
(b) a $C_{1-6}$ alkyl group optionally substituted by 1 to 3 halogen atoms;

(20) a non-aromatic heterocyclylcarbonyl group (e.g., pyrrolidinylcarbonyl, morpholinylcarbonyl, 1,1-dioxidothiomorpholinylcarbonyl) optionally substituted by 1 to 3 substituents selected from a $C_{1-6}$ alkyl group optionally substituted by 1 to 3 halogen atoms;
(21) a mercapto group;
(22) a $C_{1-6}$ alkylthio group (e.g., methylthio, ethylthio) optionally substituted by 1 to 3 substituents selected from

(a) a halogen atom, and
(b) a $C_{1-6}$ alkoxycarbonyl;

(23) a $C_{7-13}$ aralkylthio group (e.g., benzylthio);
(24) a $C_{6-14}$ arylthio group (e.g., phenylthio, naphthylthio);
(25) a cyano group;
(26) a nitro group;
(27) a halogen atom;
(28) a $C_{1-3}$ alkylenedioxy group;
(29) an aromatic heterocyclylcarbonyl group (e.g., pyrazolylcarbonyl, pyrazinylcarbonyl, isoxazolylcarbonyl, pyridylcarbonyl, thiazolylcarbonyl) optionally substituted by 1 to 3 $C_{1-6}$ alkyl groups optionally substituted by 1 to 3 halogen atoms;
and the like. When the number of the substituents is not less than 2, the respective substituents may be the same or different.

The $C_{3-10}$ cycloalkyl group, $C_{3-10}$ cycloalkenyl group, $C_{4-10}$ cycloalkadienyl group, $C_{6-14}$ aryl group, $C_{7-13}$ aralkyl group and $C_{8-13}$ arylalkenyl group which are exemplified as the above-mentioned "hydrocarbon group" optionally have 1 to 3 substituents at substitutable positions.
Examples of the substituent include

(1) the groups exemplified as the substituents for the above-mentioned $C_{1-10}$ alkyl group and the like;
(2) a $C_{1-6}$ alkyl group optionally substituted by 1 to 3 substituents selected from

(a) a halogen atom,
(b) a carboxy group,
(c) a hydroxy group,
(d) a $C_{1-6}$ alkoxy-carbonyl group,
(e) a $C_{1-6}$ alkoxy group, and
(f) an amino group optionally mono- or di-substituted by $C_{1-6}$ alkyl group(s);

(3) a $C_{2-6}$ alkenyl group (e.g., ethenyl, 1-propenyl) optionally substituted by 1 to 3 substituents selected from

(a) a halogen atom,
(b) a carboxy group,
(c) a hydroxy group,
(d) a $C_{1-6}$ alkoxy-carbonyl group,
(e) a $C_{1-6}$ alkoxy group, and
(f) an amino group optionally mono- or di-substituted by $C_{1-6}$ alkyl group(s);

(4) a $C_{7-13}$ aralkyl group (e.g., benzyl) optionally substituted by 1 to 3 substituents selected from

(a) a $C_{1-6}$ alkyl group optionally substituted by 1 to 3 halogen atoms,
(b) a hydroxy group,
(c) a $C_{1-6}$ alkoxy group, and
(d) a halogen atom;

and the like. When the number of the substituents is not less than 2, the respective substituents may be the same or different.

Examples of the "heterocyclic group" of the above-mentioned "optionally substituted heterocyclic group" include an "aromatic heterocyclic group" and a "non-aromatic heterocyclic group".

Examples of the aromatic heterocyclic group include a 4-to 7-membered (preferably 5- or 6-menbered) monocyclic aromatic heterocyclic group containing, as a ring-constituting atom besides carbon atoms, 1 to 4 hetero atoms selected from an oxygen atom, a sulfur atom and a nitrogen atom, and a fused aromatic heterocyclic group. Examples of the fused aromatic heterocyclic group include a group derived from a fused ring wherein a ring corresponding to the 4- to 7-membered monocyclic aromatic heterocyclic group and 1 or 2 rings selected from a 5- or 6-membered aromatic heterocycle containing 1 or 2 nitrogen atoms (e.g., pyrrole, imidazole, pyrazole, pyrazine, pyridine, pyrimidine), a 5-membered aromatic heterocycle containing one sulfur atom (e.g., thiophene) and a benzene ring are condensed, and the like.

Preferable examples of the aromatic heterocyclic group include monocyclic aromatic heterocyclic groups such as furyl (e.g., 2-furyl, 3-furyl), thienyl (e.g., 2-thienyl, 3-thienyl), pyridyl (e.g., 2-pyridyl, 3-pyridyl, 4-pyridyl), pyrimidinyl (e.g., 2-pyrimidinyl, 4-pyrimidinyl, 5-pyrimidinyl), pyridazinyl (e.g., 3-pyridazinyl, 4-pyridazinyl), pyrazinyl (e.g., 2-pyrazinyl), pyrrolyl (e.g., 1-pyrrolyl, 2-pyrrolyl, 3-pyrrolyl), imidazolyl (e.g., 1-imidazolyl, 2-imidazolyl, 4-imidazolyl, 5-imidazolyl), pyrazolyl (e.g., 1-pyrazolyl, 3-pyrazolyl, 4-pyrazolyl), thiazolyl (e.g., 2-thiazolyl, 4-thiazolyl, 5-thiazolyl), isothiazolyl (e.g., 3-isothiazolyl, 4-isothiazolyl, 5-isothiazolyl), oxazolyl (e.g., 2-oxazolyl, 4-oxazolyl, 5-oxazolyl), isoxazolyl (e.g., 3-isoxazolyl, 4-isoxazolyl, 5-isoxazolyl), oxadiazolyl (e.g., 1,2,4-oxadiazol-5-yl, 1,3,4-oxadiazol-2-yl), thiadiazolyl (e.g., 1,3,4-thiadiazol-2-yl), triazolyl (e.g., 1,2,4-triazol-1-yl, 1,2,4-triazol-3-yl, 1,2,3-triazol-1-yl, 1,2,3-triazol-2-yl, 1,2,3-triazol-4-yl), tetrazolyl (e.g., tetrazol-1-yl, tetrazol-5-yl), triazinyl (e.g., 1,2,4-triazin-1-yl, 1,2,4-triazin-3-yl) and the like; fused aromatic heterocyclic groups such as quinolyl (e.g., 2-quinolyl, 3-quinolyl, 4-quinolyl, 6-quinolyl), isoquinolyl (e.g., 3-isoquinolyl), quinazolyl (e.g., 2-quinazolyl, 4-quinazolyl), quinoxalyl (e.g., 2-quinoxalyl, 6-quinoxalyl), benzofuranyl (e.g., 2-benzofuranyl, 3-benzofuranyl), benzothienyl (e.g., 2-benzothienyl, 3-benzothienyl), benzoxazolyl (e.g., 2-benzoxazolyl), benzisoxazolyl (e.g., 7-benzisoxazolyl), benzothiazolyl (e.g., 2-benzothiazolyl), benzimidazolyl (e.g., benzimidazol-1-yl, benzimidazol-2-yl, benzimidazol-5-yl), benzotriazolyl (e.g., 1H-1,2,3-benzotriazol-5-yl), indolyl (e.g., indol-1-yl, indol-2-yl, indol-3-yl, indol-5-yl), indazolyl (e.g., 1H-indazol-3-yl), pyrrolopyrazinyl (e.g., 1H-pyrrolo[2,3-b]pyrazin-2-yl, 1H-pyrrolo [2,3-b]pyrazin-6-yl), imidazopyridinyl (e.g., 1H-imidazo[4,5-b]pyridin-2-yl, 1H-imidazo[4,5-c]pyridin-2-yl, 2H-imidazo[1,2-a]pyridin-3-yl), imidazopyrazinyl (e.g., 1H-imidazo[4,5-b]pyrazin-2-yl), pyrazolopyridinyl (e.g., 1H-pyrazolo[4,3-c]pyridin-3-yl), pyrazolothienyl (e.g., 2H-pyrazolo[3,4-b]thiophen-2-yl), pyrazolotriazinyl (e.g., pyrazolo[5,1-c][1,2,4]triazin-3-yl) and the like; and the like.

Examples of the non-aromatic heterocyclic group include a 4- to 7-membered (preferably 5- or 6-membered) monocyclic non-aromatic heterocyclic group containing, as a ring-constituting atom besides carbon atoms, 1 to 4 hetero atoms selected from an oxygen atom, a sulfur atom (the sulfur atom is optionally oxidized) and a nitrogen atom, and a fused non-aromatic heterocyclic group. Examples of the fused non-aromatic heterocyclic group include a group derived from a fused ring wherein a ring corresponding to the 4- to 7-membered monocyclic non-aromatic heterocyclic group and 1 or 2 rings selected from a 5- or 6-membered aromatic or non-aromatic heterocycle containing 1 or 2 nitrogen atoms (e.g., pyrrole, imidazole, pyrazole, pyrazine, pyridine, pyrimidine), a 5-membered aromatic or non-aromatic heterocycle containing one sulfur atom (e.g., thiophene) and a benzene ring are condensed, a group wherein the above-mentioned group is partially saturated, and the like.

Preferable examples of the non-aromatic heterocyclic group include
monocyclic non-aromatic heterocyclic groups such as pyrrolidinyl (e.g., 1-pyrrolidinyl, 2-pyrrolidinyl), piperidinyl (e.g., piperidino, 2-piperidinyl, 3-piperidinyl, 4-piperidinyl), morpholinyl (e.g., morpholino), thiomorpholinyl (e.g., thiomorpholino), piperazinyl (e.g., 1-piperazinyl, 2-piperazinyl, 3-piperazinyl), hexamethyleniminyl (e.g., hexamethylenimin-1-yl), oxazolidinyl (e.g., oxazolidin-2-yl), thiazolidinyl (e.g., thiazolidin-2-yl), imidazolidinyl (e.g., imidazolidin-2-yl, imidazolidin-3-yl), oxazolinyl (e.g., oxazolin-2-yl), thiazolinyl (e.g., thiazolin-2-yl), imidazolinyl (e.g., imidazolin-2-yl, imidazolin-3-yl), dioxolyl (e.g., 1,3-dioxol-4-yl), dioxolanyl (e.g., 1,3-dioxolan-4-yl), dihydrooxadiazolyl (e.g., 4,5-dihydro-1,2,4-oxadiazol-3-yl), thioxooxazolidinyl (e.g., 2-thioxo-1,3-oxazolidin-5-yl), pyranyl (e.g., 4-pyranyl), tetrahydropyranyl (e.g., 2-tetrahydropyranyl, 3-tetrahydropyranyl, 4-tetrahydropyranyl), thiopyranyl (e.g., 4-thiopyranyl), tetrahydrothiopyranyl (e.g., 2-tetrahydrothiopyranyl, 3-tetrahydrothiopyranyl, 4-tetrahydrothiopyranyl), 1-oxidotetrahydrothiopyranyl (e.g., 1-oxidotetrahydrothiopyran-4-yl), 1,1-dioxidotetrahydrothiopyranyl (e.g., 1,1-dioxidotetrahydrothiopyran-4-yl), tetrahydrofuryl (e.g., tetrahydrofuran-3-yl, tetrahydrofuran-2-yl), pyrazolidinyl (e.g., pyrazolidin-1-yl, pyrazolidin-3-yl), pyrazolinyl

(e.g., pyrazolin-1-yl), tetrahydropyrimidinyl (e.g., tetrahydropyrimidin-1-yl), dihydrotriazolyl (e.g., 2,3-dihydro-1H-1,2,3-triazol-1-yl), tetrahydrotriazolyl (e.g., 2,3,4,5-tetrahydro-1H-1,2,3-triazol-1-yl) and the like;
fused non-aromatic heterocyclic groups such as dihydroindolyl (e.g., 2,3-dihydro-1H-indol-1-yl), dihydroisoindolyl (e.g., 1,3-dihydro-2H-isoindol-2-yl), dihydrobenzofuranyl (e.g., 2,3-dihydro-1-benzofuran-5-yl), dihydrobenzodioxinyl (e.g., 2,3-dihydro-1,4-benzodioxinyl), dihydrobenzodioxepinyl (e.g., 3,4-dihydro-2H-1,5-benzodioxepinyl), tetrahydrobenzofuranyl (e.g., 4,5,6,7-tetrahydro-1-benzofuran-3-yl), chromenyl (e.g., 4H-chromen-2-yl, 2H-chromen-3-yl), dihydroquinolinyl (e.g., 1,2-dihydroquinolin-4-yl), tetrahydroquinolinyl (e.g., 1,2,3,4-tetrahydroquinolin-4-yl), dihydroisoquinolinyl (e.g., 1,2-dihydroisoquinolin-4-yl), tetrahydroisoquinolinyl (e.g., 1,2,3,4-tetrahydroisoquinolin-4-yl), dihydrophthalazinyl (e.g., 1,4-dihydrophthalazin-4-yl) and the like; and the like.

The "heterocyclic group" of the above-mentioned "optionally substituted heterocyclic group" optionally has 1 to 3 substituents at substitutable positions. Examples of the substituent include those similar to the substituent that the $C_{3-10}$ cycloalkyl group and the like exemplified as the "hydrocarbon group" of the above-mentioned "optionally substituted hydrocarbon group" optionally has. When the heterocyclic group is a "non-aromatic heterocyclic group", the substituent further includes an oxo group. When the number of the substituents is not less than 2, the respective substituents may be the same or different.

Examples of the above-mentioned "optionally substituted hydroxy group" include a hydroxy group optionally substituted by a substituent selected from a $C_{1-10}$ alkyl group, a $C_{2-10}$ alkenyl group, a $C_{3-10}$ cycloalkyl group, a $C_{3-10}$ cycloalkenyl group, a $C_{6-14}$ aryl group, a $C_{7-13}$ aralkyl group, a $C_{8-13}$ arylalkenyl group, a $C_{1-6}$ alkyl-carbonyl group, a heterocyclic group and the like, each of which is optionally substituted.

Examples of the $C_{1-10}$ alkyl group, $C_{2-10}$ alkenyl group, $C_{3-10}$ cycloalkyl group, $C_{3-10}$ cycloalkenyl group, $C_{6-14}$ aryl group, $C_{7-13}$ aralkyl group and $C_{8-13}$ arylalkenyl group include those exemplified as the "hydrocarbon group" of the above-mentioned "optionally substituted hydrocarbon group".

Examples of the heterocyclic group include those similar to the "heterocyclic group" of the above-mentioned "optionally substituted heterocyclic group".

The above-mentioned $C_{1-10}$ alkyl group, $C_{2-10}$ alkenyl group, $C_{3-10}$ cycloalkyl group, $C_{3-10}$ cycloalkenyl group, $C_{6-14}$ aryl group, $C_{7-13}$ aralkyl group, $C_{8-13}$ arylalkenyl group, $C_{1-6}$ alkyl-carbonyl group and heterocyclic group optionally have 1 to 3 substituents at substitutable positions. When the number of the substituents is not less than 2, the respective substituents may be the same or different.

Examples of the substituent for the $C_{1-10}$ alkyl group, $C_{2-10}$ alkenyl group and $C_{1-6}$ alkyl-carbonyl group include those similar to the substituent that the $C_{1-10}$ alkyl group and the like exemplified as the "hydrocarbon group" of the above-mentioned "optionally substituted hydrocarbon group" optionally has.

Examples of the substituent for the $C_{3-10}$ cycloalkyl group, $C_{3-10}$ cycloalkenyl group, $C_{6-14}$ aryl group, $C_{7-13}$ aralkyl group and $C_{8-13}$ arylalkenyl group include those similar to the substituent that the $C_{3-10}$ cycloalkyl group and the like exemplified as the "hydrocarbon group" of the above-mentioned "optionally substituted hydrocarbon group" optionally has. Examples of the substituent for the heterocyclic group include those similar to the substituent of the above-mentioned "optionally substituted heterocyclic group".

Examples of the above-mentioned "optionally substituted mercapto group" include a mercapto group optionally substituted by a substituent selected from a $C_{1-10}$ alkyl group, a $C_{2-10}$ alkenyl group, a $C_{3-10}$ cycloalkyl group, a $C_{3-10}$ cycloalkenyl group, a $C_{6-14}$ aryl group, a $C_{7-13}$ aralkyl group, a $C_{8-13}$ arylalkenyl group, a $C_{1-6}$ alkyl-carbonyl group, a heterocyclic group and the like, each of which is optionally substituted.

Examples of the substituent include those exemplified as the substituents of the above-mentioned "optionally substituted hydroxy group".

Examples of the above-mentioned "optionally substituted amino group" include an amino group optionally mono- or di-substituted by substituent(s) selected from a $C_{1-10}$ alkyl group, a $C_{2-10}$ alkenyl group, a $C_{3-10}$ cycloalkyl group, a $C_{3-10}$ cycloalkenyl group, a $C_{6-14}$ aryl group, a $C_{7-13}$ aralkyl group, a $C_{8-13}$ arylalkenyl group and a heterocyclic group, each of which is optionally substituted; an acyl group and the like.

Examples of the $C_{1-10}$ alkyl group, $C_{2-10}$ alkenyl group, $C_{3-10}$ cycloalkyl group, $C_{3-10}$ cycloalkenyl group, $C_{6-14}$ aryl group, $C_{7-13}$ aralkyl group and $C_{8-13}$ arylalkenyl group include those exemplified as the "hydrocarbon group" of the above-mentioned "optionally substituted hydrocarbon group".

Examples of the heterocyclic group include those similar to the "heterocyclic group" of the above-mentioned "optionally substituted heterocyclic group". Of these, a 5- to 7-membered monocyclic aromatic heterocyclic group is preferable.

The $C_{1-10}$ alkyl group, $C_{2-10}$ alkenyl group, $C_{3-10}$ cycloalkyl group, $C_{3-10}$ cycloalkenyl group, $C_{6-14}$ aryl group, $C_{7-13}$ aralkyl group, $C_{8-13}$ arylalkenyl group and heterocyclic group optionally have 1 to 3 substituents at substitutable positions. When the number of the substituents is not less than 2, the respective substituents may be the same or different.

Examples of the substituent for the $C_{1-10}$ alkyl group and $C_{2-10}$ alkenyl group include those similar to the substituent that the $C_{1-10}$ alkyl group and the like exemplified as the "hydrocarbon group" of the above-mentioned "optionally substituted hydrocarbon group" optionally has.

Examples of the substituent for the $C_{3-10}$ cycloalkyl group, $C_{3-10}$ cycloalkenyl group, $C_{6-14}$ aryl group, $C_{7-13}$ aralkyl group and $C_{8-13}$ arylalkenyl group include those similar to the substituent that the $C_{3-10}$ cycloalkyl group and the like exemplified as the "hydrocarbon group" of the above-mentioned "optionally substituted hydrocarbon group" optionally has. Examples of the substituent for the heterocyclic group include those similar to the substituent of the above-mentioned "optionally substituted heterocyclic group".

Examples of the "acyl group" exemplified as the substituent for the "optionally substituted amino group" include those similar to the "acyl group" below, which is exemplified as the "substituent" for $R^1$.

Examples of the "acyl group" which is exemplified as the "substituent" for $R^1$ include a group represented by the formula: $-COR^a$, $-CO-OR^a$, $-SO_3R^a$, $-SO_2R^a$, $-SOR^a$, $-CO-NR^{a'}R^{b'}$, $-CS-NR^{a'}R^{b'}$ or $-SO_2NR^{a'}R^{b'}$ wherein $R^a$ is a hydrogen atom, an optionally substituted hydrocarbon group or an optionally substituted heterocyclic group, and $R^{a'}$ and $R^{b'}$ are the same or different and each is a hydrogen atom, an optionally substituted hydrocarbon group or an optionally substituted heterocyclic group, or $R^{a'}$ and $R^{b'}$ optionally form, together with the adjacent nitrogen atom, an optionally substituted nitrogen-containing heterocycle, and the like.

Examples of the "optionally substituted hydrocarbon group" and "optionally substituted heterocyclic group" for $R^a$, $R^{a'}$ or $R^{b'}$ include those similar to the "optionally substituted hydrocarbon group" and "optionally substituted heterocyclic group", which are exemplified as the "substituent" for $R^1$.

Examples of the "nitrogen-containing heterocycle" of the "optionally substituted nitrogen-containing heterocycle" formed by $R^{a'}$ and $R^{b'}$ together with the adjacent nitrogen atom include a 5- to 7-membered nitrogen-containing heterocycle containing, as a ring-constituting atom besides carbon atoms, at least one nitrogen atom and optionally further containing one or two hetero atoms selected from an oxygen atom, a sulfur atom and a nitrogen atom. Preferable examples of the nitrogen-containing heterocycle include pyrrolidine, imidazolidine, pyrazolidine, piperidine, piperazine, morpholine, thiomorpholine and the like.

The nitrogen-containing heterocycle optionally has 1 to 3 substituents at substitutable positions. Examples of the substituent include those similar to the substituent of the above-mentioned "optionally substituted heterocyclic group". When the number of the substituents is not less than 2, the respective substituents may be the same or different.

Preferable examples of the "acyl group" include

(1) a formyl group;
(2) a carboxy group;
(3) a $C_{1-6}$ alkyl-carbonyl group optionally substituted by 1 to 3 substituents selected from

  (i) a halogen atom,
  (ii) a $C_{1-6}$ alkoxy-carbonyl group,
  (iii) a $C_{6-14}$ aryl group (e.g., phenyl), and
  (iv) a $C_{1-6}$ alkoxy group;

(4) a $C_{1-6}$ alkoxy-carbonyl group (e.g., methoxycarbonyl, ethoxycarbonyl, propoxycarbonyl, tert-butoxycarbonyl) optionally substituted by 1 to 3 substituents selected from

  (i) a halogen atom,
  (ii) a $C_{6-14}$ aryl group (e.g., phenyl), and
  (iii) a $C_{1-6}$ alkoxy group;

(5) a $C_{3-10}$ cycloalkyl-carbonyl group (e.g., cyclopropylcarbonyl, cyclopentylcarbonyl, cyclohexylcarbonyl);
(6) a $C_{6-14}$ aryl-carbonyl group (e.g., benzoyl) optionally substituted by 1 to 3 halogen atoms;
(7) a carbamoyl group optionally mono- or di-substituted by substituent(s) selected from

  (i) a $C_{1-6}$ alkyl group optionally substituted by 1 to 3 substituents selected from

    (a) a halogen atom,
    (b) a $C_{1-6}$ alkoxy-carbonyl group,
    (c) a $C_{6-14}$ aryl group (e.g., phenyl),
    (d) a $C_{1-6}$ alkoxy group, and
    (e) an aromatic heterocyclic group (e.g., furyl),

  (ii) a $C_{3-10}$ cycloalkyl group (e.g., cyclohexyl),
  (iii) a $C_{6-14}$ aryl group (e.g., phenyl) optionally substituted by 1 to 3 substituents selected from

(a) a halogen atom,
(b) a $C_{1-6}$ alkyl group optionally substituted by 1 to 3 halogen atoms, and
(c) a $C_{1-6}$ alkoxy group, and

(iv) an aromatic heterocyclic group (e.g., pyridyl);

(8) a $C_{1-6}$ alkylsulfonyl group (e.g., methylsulfonyl, ethylsulfonyl, isopropylsulfonyl) optionally substituted by 1 to 3 substituents selected from

(i) a halogen atom, and
(ii) a $C_{6-14}$ aryl group (e.g., phenyl);

(9) a $C_{6-14}$ arylsulfonyl group (e.g., benzenesulfonyl) optionally substituted by 1 to 3 halogen atoms;
(10) a sulfamoyl group optionally mono- or di-substituted by substituent(s) selected from

(i) a $C_{1-6}$ alkyl group optionally substituted by 1 to 3 substituents selected from

(a) a halogen atom, and
(b) a non-aromatic heterocyclic group (e.g., pyrrolidinyl) optionally substituted by oxo group(s);

(11) a thiocarbamoyl group optionally mono- or di-substituted by substituent(s) selected from a $C_{1-6}$ alkyl group optionally substituted by 1 to 3 halogen atoms;
(12) an aromatic heterocyclylcarbonyl group (e.g., furylcarbonyl, thienylcarbonyl) optionally substituted by 1 to 3 substituents selected from a $C_{1-6}$ alkyl group optionally substituted by 1 to 3 halogen atoms;
(13) a non-aromatic heterocyclylcarbonyl group (e.g., tetrahydrofurylcarbonyl) optionally substituted by 1 to 3 substituents selected from a $C_{1-6}$ alkyl group optionally substituted by 1 to 3 halogen atoms; and the like.

$R^1$ is preferably an optionally substituted amino group, more preferably an amino group optionally mono- or di-substituted by substituent(s) selected from

(1) a $C_{1-6}$ alkoxy-carbonyl group,
(2) a carbamoyl group optionally mono- or di-substituted by $C_{1-6}$ alkyl group(s),
and the like.

Ring P is an optionally substituted 6-membered nitrogen-containing aromatic heterocycle. Examples of the "6-membered nitrogen-containing aromatic heterocycle" of the "optionally substituted 6-membered nitrogen-containing aromatic heterocycle" include a ring corresponding to a 6-membered nitrogen-containing aromatic heterocyclic group, from among the "heterocyclic group" of the "optionally substituted heterocyclic group" exemplified as "substituent" for $R^1$. Specific examples of the "6-membered nitrogen-containing aromatic heterocycle" include pyridine, pyrazine, pyrimidine, pyridazine and the like. Of these, pyridine is preferable.
The "6-membered nitrogen-containing aromatic heterocycle" of the "optionally substituted 6-membered nitrogen-containing aromatic heterocycle" for ring P optionally has 1 to 3 substituents at substitutable positions, besides condensation with the thiophene ring. Examples of the substituent include those similar to the substituent that the $C_{3-10}$ cycloalkyl group and the like exemplified as the above-mentioned "substituent" for $R^1$ optionally has. When the number of the substituents is not less than 2, the respective substituents may be the same or different.
Preferable examples of the substituent for ring P include a $C_{1-6}$ alkyl group and the like.
Other preferable examples of the substituent for ring P include

(1) a $C_{1-6}$ alkyl group optionally substituted by 1 to 3 substituents selected from

(i) a halogen atom, and
(ii) a hydroxy group optionally substituted by a di($C_{6-14}$ aryl)($C_{1-6}$alkyl)silyl group,

and the like.

Ring P is preferably an optionally substituted pyridine ring, more preferably a pyridine ring optionally substituted by 1 to 3 $C_{1-6}$ alkyl groups and the like.
As another embodiment, ring P is more preferably a pyridine ring optionally substituted by 1 to 3 substituents selected from

(1) a $C_{1-6}$ alkyl group optionally substituted by 1 to 3 substituents selected from

(i) a halogen atom, and
(ii) a hydroxy group optionally substituted by a di($C_{6-14}$ aryl)($C_{1-6}$ alkyl)silyl group,

and the like.

Ring Q is an optionally further substituted 5- to 7-membered nitrogen-containing non-aromatic heterocycle. Examples of the "5- to 7-membered nitrogen-containing non-aromatic heterocycle" of the "optionally further substituted 5- to 7-membered nitrogen-containing non-aromatic heterocycle" include a ring corresponding to a 5- to 7-membered nitrogen-containing non-aromatic heterocyclic group, from among the "heterocyclic group" of the "optionally substituted heterocyclic group" exemplified as "substituent" for $R^1$. Specific examples of the "5- to 7-membered nitrogen-containing non-aromatic heterocycle" include pyrrolidine, piperidine, piperazine, morpholine, thiomorpholine, pyrazolidine, imidazolidine, oxazolidine, thiazolidine, isoxazolidine, isothiazolidine, oxadiazolidine, thiadiazolidine, hexamethylenimine and the like. Of these, a 6-membered monocyclic nitrogen-containing non-aromatic heterocycle (preferably piperidine, piperazine, morpholine, thiomorpholine) is preferable, and piperidine is more preferable.

The "5- to 7-membered nitrogen-containing non-aromatic heterocycle" of the "optionally further substituted 5- to 7-membered nitrogen-containing non-aromatic heterocycle" for ring Q optionally has 1 to 3 substituents at substitutable positions, besides condensation with the piperidine ring. Examples of the substituent include those similar to the substituent that the $C_{3-10\ 10}$ cycloalkyl group and the like exemplified as the above-mentioned "substituent" for $R^1$ optionally has, and an oxo group. When the number of the substituents is not less than 2, the respective substituents may be the same or different.

Preferable examples of the substituent for ring Q include

(1) a $C_{1-6}$ alkyl-carbonyl group optionally substituted by 1 to 3 halogen atoms;
(2) a $C_{1-6}$ alkyl group optionally substituted by 1 to 3 substituents selected from a carboxyl group and a $C_{1-6}$ alkoxy-carbonyl group;
(3) a $C_{1-6}$ alkoxy-carbonyl group optionally substituted by 1 to 3 $C_{6-14}$ aryl groups (e.g., phenyl); and the like.

Ring Q is preferably an optionally further substituted 6-membered monocyclic nitrogen-containing non-aromatic heterocycle (preferably piperidine, piperazine, morpholine, thiomorpholine, more preferably piperidine), more preferably an unsubstituted piperidine ring.

Ring R is an optionally fused 5- to 7-membered non-aromatic ring, which is further optionally substituted. Examples of the "optionally fused 5- to 7-membered non-aromatic ring" of the "optionally fused 5- to 7-membered non-aromatic ring, which is further optionally substituted" include a 5- to 7-membered non-aromatic ring selected from a $C_{5-7}$ cycloalkane, a $C_{5-7}$ cycloalkene, a $C_{5-7}$ cycloalkadiene and 5- to 7-membered monocyclic non-aromatic heterocycle, and a fused ring formed by the 5- to 7-membered non-aromatic ring and a ring selected from a 5- to 7-membered monocyclic non-aromatic heterocycle, a 5- to 7-membered monocyclic aromatic heterocycle, a benzene ring and a partially saturated ring thereof.
Examples of the $C_{5-7}$ cycloalkane, $C_{5-7}$ cycloalkene and $C_{5-7}$ cycloalkadiene include a 5- to 7-membered ring, from among the rings corresponding to the $C_{3-10}$ cycloalkyl group, $C_{3-10}$ cycloalkenyl group and $C_{4-10}$ cycloalkadienyl group, which are exemplified as the "hydrocarbon group" of the "optionally substituted hydrocarbon group" exemplified as the "substituent" for $R^1$. Examples of the 5- to 7-membered monocyclic non-aromatic heterocycle include a ring corresponding to a 5- to 7-membered monocyclic non-aromatic heterocyclic group, from among the "heterocyclic group" of the "optionally substituted heterocyclic group" exemplified as the "substituent" for $R^1$. Examples of the 5- to 7-membered monocyclic aromatic heterocycle include a ring corresponding to a 5- to 7-membered monocyclic aromatic heterocyclic group, from among the "heterocyclic group" of the "optionally substituted heterocyclic group" exemplified as the "substituent" for $R^1$. The "optionally fused 5- to 7-membered non-aromatic ring" is preferably a 5-membered monocyclic non-aromatic heterocycle (preferably pyrrolidine, tetrahydrofuran, imidazolidine, imidazoline, oxazolidine, oxazoline, thiazolidine, thiazoline).

The "optionally fused 5- to 7-membered non-aromatic ring" of the "optionally fused 5- to 7-membered non-aromatic ring, which is further optionally substituted" for ring R is substituted by an oxo group, and optionally has 1 to 3 substituents at substitutable positions. Examples of the substituent include those similar to the substituent that the $C_{3-10\ 10}$ cycloalkyl group and the like exemplified as the above-mentioned "substituent" for $R^1$ optionally has, and an oxo group. Of these, an oxo group, $C_{1-6}$ alkyl group and the like are preferable. When the number of the substituents is not less than 2, the respective substituents may be the same or different.

Ring R is preferably a 5-membered monocyclic non-aromatic heterocycle (preferably pyrrolidine, tetrahydrofuran, imidazolidine, imidazoline, oxazolidine, oxazoline, thiazolidine, thiazoline) substituted by an oxo group, and optionally further substituted, more preferably a 5-membered monocyclic non-aromatic heterocycle (preferably pyrrolidine, tetrahydrofuran, imidazolidine, imidazoline, oxazolidine, oxazoline, thiazolidine, thiazoline) substituted by an oxo group, and optionally further substituted by 1 to 3 substituents selected from

(1) an oxo group,
(2) a $C_{1-6}$ alkyl group,
and the like.

Preferable examples of compound (I) include the following compounds.

[Compound A]

Compound (I) wherein
$R^1$ is an optionally substituted amino group;
ring P is an optionally substituted pyridine ring;
ring Q is an optionally further substituted 6-membered monocyclic nitrogen-containing non-aromatic heterocycle (preferably piperidine, piperazine, morpholine, thiomorpholine); and
ring R is a 5-membered monocyclic non-aromatic heterocycle (preferably pyrrolidine, tetrahydrofuran, imidazolidine, imidazoline, oxazolidine, oxazoline, thiazolidine, thiazoline) substituted by an oxo group, and optionally further substituted.

[Compound B]

Compound (I) wherein
$R^1$ is an amino group optionally mono- or di-substituted by substituent(s) selected from

(1) a $C_{1-6}$ alkoxy-carbonyl group, and
(2) a carbamoyl group optionally mono- or di-substituted by $C_{1-6}$ alkyl group(s);

ring P is a pyridine ring optionally substituted by 1 to 3 $C_{1-6}$ alkyl groups;
ring Q is a 6-membered monocyclic nitrogen-containing non-aromatic heterocycle (preferably piperidine, piperazine, morpholine, thiomorpholine, more preferably piperidine); and
ring R is a 5-membered monocyclic non-aromatic heterocycle (preferably pyrrolidine, tetrahydrofuran, imidazolidine, imidazoline, oxazolidine, oxazoline, thiazolidine, thiazoline, more preferably pyrrolidine, tetrahydrofuran, imidazolidine, imidazoline, oxazolidine, thiazolidine) substituted by an oxo group, and optionally further substituted by 1 to 3 substituents selected from

(1) an oxo group, and
(2) a $C_{1-6}$ alkyl group.

[Compound C]

Compound (I) wherein
$R^1$ is an amino group optionally mono- or di-substituted by substituent(s) selected from

(1) a $C_{1-6}$ alkoxy-carbonyl group, and
(2) a carbamoyl group optionally mono- or di-substituted by $C_{1-6}$ alkyl group(s);

ring P is a pyridine ring optionally substituted by 1 to 3 substituents selected from

(1) a $C_{1-6}$ alkyl group optionally substituted by 1 to 3 substituents selected from

(i) a halogen atom, and
(ii) a hydroxy group optionally substituted by a di($C_{6-14}$ aryl)($C_{1-6}$ alkyl)silyl group;

ring Q is a 6-membered monocyclic nitrogen-containing non-aromatic heterocycle (preferably piperidine, piperazine, morpholine, thiomorpholine, more preferably piperidine); and
ring R is a 5-membered monocyclic non-aromatic heterocycle (preferably pyrrolidine, tetrahydrofuran, imidazolidine,

imidazoline, oxazolidine, oxazoline, thiazolidine, thiazoline, more preferably pyrrolidine, tetrahydrofuran, imidazolidine, imidazoline, oxazolidine, thiazolidine) substituted by an oxo group, and optionally further substituted by 1 to 3 substituents selected from

(1) an oxo group, and
(2) a $C_{1-6}$ alkyl group.

[Compound D]

N-(3-{[4-(3,3-dimethyl-1-oxo-2-oxa-7-azaspiro[4.5]dec-7-yl)piperidin-1-yl]carbonyl}thieno[2,3-b]pyridin-2-yl)-N'-methylurea,
N-(3-{[4-(2-isopropyl-1,3-dioxo-2,7-diazaspiro[4.5]dec-7-yl)piperidin-1-yl]carbonyl}-6-methylthieno[2,3-b]pyridin-2-yl)-N'-methylurea,
N-ethyl-N'-(3-{[4-(3-isopropyl-2-methyl-4-oxo-1,3,7-triazaspiro[4.5]dec-1-en-7-yl)piperidin-1-yl]carbonyl}thieno[2,3-b]pyridin-2-yl)urea, and
N-ethyl-N'-(3-{[4-(2-isopropyl-1-oxo-2,7-diazaspiro[4.5]dec-7-yl)piperidin-1-yl]carbonyl}-6-methylthieno[2,3-b]pyridin-2-yl)urea,
and a salt thereof.
As a salt of the compound represented by the formula (I), a pharmacologically acceptable salt is preferable. Examples of such salt include salts with inorganic base, salts with organic base, salts with inorganic acid, salts with organic acid, salts with basic or acidic amino acid, and the like.
Preferable examples of the salt with inorganic base include alkali metal salts such as sodium salt, potassium salt and the like; alkaline earth metal salts such as calcium salt, magnesium salt and the like; aluminum salt: ammonium salt and the like.
Preferable examples of the salt with organic base include salts with trimethylamine, triethylamine, pyridine, picoline, ethanolamine, diethanolamine, triethanolamine, tromethamine[tris(hydroxymethyl)methylamine], tert-butylamine, cyclohexylamine, benzylamine, dicyclohexylamine, N,N-dibenzylethylenediamine and the like.
Preferable examples of the salt with inorganic acid include salts with hydrochloric acid, hydrobromic acid, nitric acid, sulfuric acid, phosphoric acid and the like.
Preferable examples of the salt with organic acid include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like.
Preferable examples of the salt with basic amino acid include salts with arginine, lysine, ornithine and the like.
Preferable examples of the salt with acidic amino acid include salts with aspartic acid, glutamic acid and the like.
A prodrug of compound (I) means a compound which is converted to compound (I) with a reaction due to an enzyme, an gastric acid, etc. under the physiological condition in the living body, that is, a compound which is converted to compound (I) by oxidation, reduction, hydrolysis, etc. according to an enzyme; a compound which is converted to compound (I) by hydrolysis etc. due to gastric acid, etc.
Examples of the prodrug of compound (I) include a compound obtained by subjecting an amino group in compound (I) to an acylation, alkylation or phosphorylation (e.g., a compound obtained by subjecting an amino group in compound (I) to an eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation or tert-butylation); a compound obtained by subjecting a hydroxy group in compound (I) to an acylation, alkylation, phosphorylation or boration (e.g., a compound obtained by subjecting a hydroxy group in compound (I) to an acetylation, palmitoylation, propanoylation, pivaloylation, succinylation, fumarylation, alanylation or dimethylaminomethylcarbonylation); a compound obtained by subjecting a carboxyl group in compound (I) to an esterification or amidation (e.g., a compound obtained by subjecting a carboxyl group in compound (I) to an ethyl esterification, phenyl esterification, carboxymethyl esterification, dimethylaminomethyl esterification, pivaloyloxymethyl esterification, ethoxycarbonyloxyethyl esterification, phthalidyl esterification, (5-methyl-2-oxo-1,3-dioxolen-4-yl)methyl esterification, cyclohexyloxycarbonylethyl esterification or methyl amidation etc.) and the like. These compounds can be produced from compound (I) according to a method known per se.
A prodrug for compound (I) may also be one which is converted to compound (I) under a physiological condition, such as those described in IYAKUHIN no KAIHATSU, Development of Pharmaceuticals, Vol. 7, Design of Molecules, p. 163-198, Published by HIROKAWA SHOTEN, 1990.
In addition, compound (I) may be labeled with an isotope (e.g., $^{3}H$, $^{14}C$, $^{35}S$, $^{125}I$) and the like.
Moreover, compound (I) may be an anhydride or a hydrate.
Compound (I) or a prodrug thereof (hereinafter sometimes to be abbreviated simply as the compound of the present invention) has low toxicity, and can be used as an agent for the prophylaxis or treatment of various diseases mentioned below in a mammal (e.g., human, mouse, rat, rabbit, dog, cat, bovine, horse, swine, monkey) directly or in

the form of a pharmaceutical composition by admixing with a pharmacologically acceptable carrier and the like.

Here, examples of the pharmacologically acceptable carrier include various organic or inorganic carrier substances conventionally used as preparation materials, which are added as excipient, lubricant, binder or disintegrant for solid dosage forms; as solvent, solubilizing agent, suspending agent, isotonicity agent, buffer or soothing agent for liquid preparation, and the like. Where necessary, preparation additives such as preservative, antioxidant, colorant, sweetener and the like can also be used.

Preferable examples of the excipient include lactose, sucrose, D-mannitol, D-sorbitol, starch, pregelatinized starch, dextrin, crystalline cellulose, low-substituted hydroxypropylcellulose, sodium carboxymethylcellulose, gum arabic, pullulan, light anhydrous silicic acid, synthetic aluminum silicate and magnesium aluminometasilicate.

Preferable examples of the lubricant include magnesium stearate, calcium stearate, talc and colloidal silica.

Preferable examples of the binder include pregelatinized starch, sucrose, gelatin, gum arabic, methylcellulose, carboxymethylcellulose, sodium carboxymethylcellulose, crystalline cellulose, sucrose, D-mannitol, trehalose, dextrin, pullulan, hydroxypropylcellulose, hydroxypropylmethylcellulose and polyvinylpyrrolidone.

Preferable examples of the disintegrant include lactose, sucrose, starch, carboxymethylcellulose, calcium carboxymethylcellulose, sodium croscarmellose, sodium carboxymethylstarch, light anhydrous silicic acid and low-substituted hydroxypropylcellulose.

Preferable examples of the solvent include water for injection, physiological brine, Ringer's solution, alcohol, propylene glycol, polyethylene glycol, sesame oil, corn oil, olive oil and cottonseed oil.

Preferable examples of the solubilizing agent include polyethylene glycol, propylene glycol, D-mannitol, trehalose, benzyl benzoate, ethanol, trisaminomethane, cholesterol, triethanolamine, sodium carbonate, sodium citrate, sodium salicylate and sodium acetate.

Preferable examples of the suspending agent include surfactants such as stearyltriethanolamine, sodium lauryl sulfate, lauryl aminopropionic acid, lecithin, benzalkonium chloride, benzethonium chloride, glyceryl monostearate and the like; hydrophilic polymers such as polyvinyl alcohol, polyvinylpyrrolidone, sodium carboxymethylcellulose, methylcellulose, hydroxymethylcellulose, hydroxyethylcellulose, hydroxypropylcellulose and the like; polysorbates and polyoxyethylene hydrogenated castor oil.

Preferable examples of the isotonicity agent include sodium chloride, glycerol, D-mannitol, D-sorbitol and glucose.

Preferable examples of the buffer include buffers such as phosphate, acetate, carbonate, citrate and the like. Preferable examples of the soothing agent include benzyl alcohol.

Preferable examples of the preservative include paraoxybenzoates, chlorobutanol, benzyl alcohol, phenethyl alcohol, dehydroacetic acid and sorbic acid.

Preferable examples of the antioxidant include sulfite, ascorbate and the like.

Preferable examples of the colorant include aqueous food tar colors (e.g., food colors such as Food Red No. 2 and No. 3, Food Yellow No. 4 and No. 5, Food Blue No. 1 and No. 2, etc.), water insoluble lake dye (e.g., aluminum salt of the above-mentioned aqueous food tar color) and natural dye (e.g., β-carotene, chlorophyll, red iron oxide).

Preferable examples of the sweetening agent include sodium saccharin, dipotassium glycyrrhizinate, aspartame and stevia.

Examples of the dosage form of the above-mentioned pharmaceutical composition include oral preparations such as tablets (inclusive of sugar-coated tablets, film-coated tablets, sublingual tablets, orally disintegrating tablets), capsules (inclusive of soft capsules, microcapsules), granules, powders, troches, syrups, emulsions, suspensions, films (e.g., orally disintegrable films) and the like; and parenteral agents such as injections (e.g., subcutaneous injections, intravenous injections, intramuscular injections, intraperitoneal injections, drip infusions), external preparations (e.g., dermal preparations, ointments), suppository (e.g., rectal suppositories, vaginal suppositories), pellets, nasal preparations, pulmonary preparations (inhalants), eye drops and the like. These may be safely administered orally or parenterally (e.g., topically, rectally, intravenously administered).

These preparations may be release control preparations (e.g., sustained-release microcapsule) such as immediate-release preparation, sustained-release preparation and the like.

A pharmaceutical composition can be produced by a method conventionally used in the technical field of pharmaceutical preparation, for example, the method described in the Japanese Pharmacopoeia and the like.

While the content of the compound of the present invention in the pharmaceutical composition varies depending on the dosage form, dose of the compound of the present invention, and the like, it is, for example, about 0.1 to 100 wt%.

During production of an oral preparation, coating may be applied as necessary for the purpose of masking of taste, enteric property or durability.

Examples of the coating base to be used for coating include sugar coating base, aqueous film coating base, enteric film coating base and sustained-release film coating base.

As the sugar coating base, sucrose is used. Moreover, one or more kinds selected from talc, precipitated calcium carbonate, gelatin, gum arabic, pullulan, carnauba wax and the like may be used in combination.

Examples of the aqueous film coating base include cellulose polymers such as hydroxypropyl cellulose, hydroxypropylmethyl cellulose, hydroxyethyl cellulose, methylhydroxyethyl cellulose etc.; synthetic polymers such as polyvinylacetal diethylaminoacetate, aminoalkyl methacrylate copolymer E [Eudragit E (trade name)], polyvinylpyrrolidone etc.; and polysaccharides such as pullulan etc.

Examples of the enteric film coating base include cellulose polymers such as hydroxypropylmethyl cellulose phthalate, hydroxypropylmethyl cellulose acetate succinate, carboxymethylethyl cellulose, cellulose acetate phthalate etc.; acrylic polymers such as methacrylic acid copolymer L [Eudragit L (trade name)], methacrylic acid copolymer LD [Eudragit L-30D55 (trade name)], methacrylic acid copolymer S [Eudragit S (trade name)] etc.; and naturally occurring substances such as shellac etc.

Examples of the sustained-release film coating base include cellulose polymers such as ethyl cellulose etc.; and acrylic polymers such as aminoalkyl methacrylate copolymer RS [Eudragit RS (trade name)], ethyl acrylate-methyl methacrylate copolymer suspension [Eudragit NE (trade name)] etc.

The above-mentioned coating bases may be used after mixing with two or more kinds thereof at appropriate ratios. For coating, for example, a light shielding agent such as titanium oxide, diiron trioxide and the like can be used.

The compound of the present invention shows low toxicity (e.g., acute toxicity, chronic toxicity, genetic toxicity, reproductive toxicity, cardiotoxicity, carcinogenicity and the like) and a few side effects. Therefore, it can be used as an agent for the prophylaxis or treatment or a diagnostic of various diseases in a mammal (e.g., human, bovine, horse, dog, cat, monkey, mouse, rat).

The compound of the present invention has a superior ACC (acetyl-CoA carboxylase) inhibitory action. Examples of ACC include liver, adipose tissue or pancreas-specific isozyme (ACC1); and muscle specific isozyme (ACC2). The compound of the present invention particularly has a selective inhibitory action on ACC2.

In addition, the compound of the present invention is superior in metabolic stability, and provides advantages such as long half-life of the compound, resistance to metabolism in the body and the like.

Moreover, the compound of the present invention is superior in kinetics (e.g., oral absorbability, bioavailability) in the body.

The compound of the present invention can be used as an agent for the prophylaxis or treatment of obesity, diabetes (e.g., type 1 diabetes, type 2 diabetes, gestational diabetes, obese diabetes), hyperlipidemia (e.g., hypertriglyceridemia, hypercholesterolemia, hypoHDL-emia, postprandial hyperlipemia), hypertension, cardiac failure, diabetic complications [e.g., neuropathy, nephropathy, retinopathy, diabetic cardiomyopathy, cataract, macroangiopathy, osteopenia, hyperosmolar diabetic coma, infections (e.g., respiratory infection, urinary tract infection, gastrointestinal infection, dermal soft tissue infections, inferior limb infection), diabetic gangrene, xerostomia, hypacusis, cerebrovascular disorder, peripheral blood circulation disorder], metabolic syndrome (pathology having three or more selected from hypertriglyceridemia (TG), low HDL cholesterol (HDL-C), hypertension, abdomen obesity and impaired glucose tolerance), sarcopenia, cancer and the like.

For diagnostic criteria of diabetes, Japan Diabetes Society reported new diagnostic criteria in 1999.

According to this report, diabetes is a condition showing any of a fasting blood glucose level (glucose concentration of intravenous plasma) of not less than 126 mg/dl, a 75 g oral glucose tolerance test (75 g OGTT) 2 hr level (glucose concentration of intravenous plasma) of not less than 200 mg/dl, and a non-fasting blood glucose level (glucose concentration of intravenous plasma) of not less than 200 mg/dl. A condition not falling under the above-mentioned diabetes and different from "a condition showing a fasting blood glucose level (glucose concentration of intravenous plasma) of less than 110 mg/dl or a 75 g oral glucose tolerance test (75 g OGTT) 2 hr level (glucose concentration of intravenous plasma) of less than 140 mg/dl" (normal type) is called a "borderline type".

In addition, ADA (American Diabetes Association) in 1997 and WHO in 1998 reported new diagnostic criteria of diabetes.

According to these reports, diabetes is a condition showing a fasting blood glucose level (glucose concentration of intravenous plasma) of not less than 126 mg/dl and a 75 g oral glucose tolerance test 2 hr level (glucose concentration of intravenous plasma) of not less than 200 mg/dl.

According to the above-mentioned reports, impaired glucose tolerance is a condition showing fasting blood sugar level (glucose concentration of intravenous plasma) of less than 126 mg/dl and a 75 g oral glucose tolerance test 2 hr level (glucose concentration of intravenous plasma) of not less than 140 mg/dl and less than 200 mg/dl. According to the report of ADA, a condition showing a fasting blood glucose level (glucose concentration of intravenous plasma) of not less than 110 mg/dl and less than 126 mg/dl is called IFG (Impaired Fasting Glucose). According to the report of WHO, among the IFG (Impaired Fasting Glucose), a condition showing a 75g oral glucose tolerance test 2 hr level (glucose concentration of intravenous plasma) of less than 140 mg/dl is called IFG (Impaired Fasting Glycemia).

The compound of the present invention can be also used as an agent for the prophylaxis or treatment of diabetes, borderline type, impaired glucose tolerance, IFG (Impaired Fasting Glucose) and IFG (Impaired Fasting Glycemia), as determined according to the above-mentioned new diagnostic criteria. Moreover, the compound of the present invention can prevent progress of borderline type, impaired glucose tolerance, IFG (Impaired Fasting Glucose) or IFG (Impaired Fasting Glycemia) into diabetes.

The compound of the present invention can also be used, for example, as an agent for the prophylaxis or treatment of osteoporosis, cachexia (e.g., carcinocachexia, tuberculous cachexia, diabetic cachexia, hemopathic cachexia, endocrinopathic cachexia, infectious cachexia or cachexia induced by acquired immunodeficiency syndrome), fatty liver, polycystic ovary syndrome, renal disease (e.g., diabetic nephropathy, glomerulonephritis, glomerulosclerosis, nephrosis syndrome, hypertensive nephrosclerosis, terminal renal disorder), muscular dystrophy, myocardial infarction, angina pectoris, cerebrovascular disorder (e.g., cerebral infarction, cerebral apoplexy), Alzheimer's disease, Parkinson's disease, anxiety, dementia, insulin resistance syndrome, syndrome X, hyperinsulinemia, sensory abnormality in hyperinsulinemia, irritable bowel syndrome, acute or chronic diarrhea, inflammatory disease (e.g., chronic rheumatoid arthritis, spondylitis deformans, osteoarthritis, lumbago, gout, postoperative or posttraumatic inflammation, swelling, neuralgia, pharyngolaryngitis, cystitis, hepatitis (including nonalcoholic steatohepatitis), pneumonia, pancreatitis, enteritis, inflammatory bowel disease (including inflammatory colitis), ulcerative colitis, stomach mucous membrane injury (including stomach mucous membrane injury caused by aspirin)), small intestine mucous membrane injury, malabsorption, testis dysfunction, visceral obesity syndrome or sarcopenia.

In addition, the compound of the present invention can also be used as an agent for the prophylaxis or treatment of various cancers (particularly breast cancer (e.g., invasive ductal carcinoma, non-invasive ductal carcinoma, inflammatory breast cancer and the like), prostate cancer (e.g., hormone-dependent prostate cancer, hormone-independent prostate cancer and the like), pancreatic cancer (e.g., pancreatic duct cancer and the like), gastric cancer (e.g., papillary adenocarcinoma, mucinous adenocarcinoma, adenosquamous carcinoma and the like), lung cancer (e.g., non-small cell lung cancer, small cell lung cancer, malignant mesothelioma and the like), colon cancer (e.g., gastrointestinal stromal tumor and the like), rectal cancer (e.g., gastrointestinal stromal tumor and the like), colorectal cancer (e.g., familial colorectal cancer, hereditary nonpolyposis colorectal cancer, gastrointestinal stromal tumor and the like), small intestinal cancer (e.g., non-Hodgkin lymphoma, gastrointestinal stromal tumor and the like), esophagus cancer, duodenal cancer, cancer of the tongue, pharyngeal cancer (e.g., nasopharyngeal cancer, mesopharyngeal cancer, hypopharyngeal cancer and the like), salivary gland cancer, brain tumor (e.g., pineal astrocytoma, pilocytic astrocytoma, diffuse astrocytoma, anaplastic astrocytoma and the like), neurinoma, liver cancer (e.g., primary liver cancer, Extrahepatic Bile Duct Cancer and the like), kidney cancer (e.g., renal cell carcinoma, transitional cell carcinoma of kidney pelvis and urinary duct, and the like), cancer of the bile duct, endometrial carcinoma, uterine cervical cancer, ovary cancer (e.g., ovarian epithelial carcinoma, extragonadal germ cell tumor, ovarian germ cell tumor, ovarian low malignant potential tumor and the like), urinary bladder cancer, urinary tract cancer, skin cancer (e.g., intraocular (ocular) melanoma, Merkel cell carcinoma and the like), Hemangioma, malignant lymphoma, malignant melanoma, thyroid cancer (e.g., medullary thyroid carcinoma and the like), parathyroid cancer, nasal cavity cancer, paranasal sinus cancer, bone tumor (e.g., osteosarcoma, Ewing's tumor, uterus sarcoma, soft tissue sarcoma and the like), vascular fibroma, retinoblastoma, penile cancer, testis tumor, solid cancer in childhood (e.g., Wilms' tumor, childhood kidney tumor and the like), Kaposi's sarcoma, Kaposi's sarcoma derived from AIDS, maxillary tumor, fibrous histiocytoma, leiomyosarcoma, rhabdomyosarcoma, leukemia (e.g., acute myelocytic leukemia, acute lymphoblastic leukemia and the like) etc.).

The compound of the present invention can also be used for secondary prevention or suppression of progression of the above-mentioned various diseases (e.g., cardiovascular events such as myocardial infarction and the like).

While the dose of the compound of the present invention varies depending on the subject of administration, administration route, target disease, symptom and the like, for example, for oral administration to an adult diabetic patient, it is generally about 0.01 to 100 mg/kg body weight, preferably 0.05 to 30 mg/kg body weight, more preferably 0.1 to 10 mg/kg body weight for one dose, which is desirably administered once to 3 times a day.

With the aim of enhancing the action of the compound of the present invention or decreasing the dose of the compound and the like, the compound can be used in combination with pharmaceutical agents such as therapeutic agents for diabetes, therapeutic agents for diabetic complications, therapeutic agents for hyperlipidemia, antihypertensive agents, antiobesity agents, diuretics, antithrombotic agents, anticancer agents and the like (hereinafter to be abbreviated as concomitant drug). The time of administration of the compound of the present invention and that of the concomitant drug are not limited, and they may be administered simultaneously or in a staggered manner to the administration subject. In addition, the compound of the present invention and the concomitant drug may be administered as two kinds of preparations containing respective active ingredients or a single preparation containing both active ingredients.

The dose of the concomitant drug can be appropriately determined based on the dose employed clinically. In addition, the mixing ratio of the compound of the present invention and the concomitant drug can be appropriately determined according to the administration subject, administration route, target disease, condition, combination, and the like. For example, when the administration subject is a human, the concomitant drug may be used in an amount of 0.01 to 100 parts by weight per 1 part by weight of the compound of the present invention.

Examples of the therapeutic agents for diabetes include insulin preparations (e.g., animal insulin preparations extracted from pancreas of bovine or swine; human insulin preparations genetically synthesized using *Escherichia coli* or yeast; zinc insulin; protamine zinc insulin; fragment or derivative of insulin (e.g., INS-1), oral insulin preparation), insulin sensitizers (e.g., pioglitazone or a salt thereof (preferably hydrochloride), rosiglitazone or a salt thereof (preferably

maleate), Tesaglitazar, Ragaglitazar, Muraglitazar, Edaglitazone, Metaglidasen, Naveglitazar, AMG-131, THR-0921), α-glucosidase inhibitors (e.g., voglibose, acarbose, miglitol, emiglitate), biguanides (e.g., metformin, buformin or a salt thereof (e.g., hydrochloride, fumarate, succinate)), insulin secretagogues [e.g., sulfonylurea (e.g., tolbutamide, glibenclamide, gliclazide, chlorpropamide, tolazamide, acetohexamide, glyclopyramide, glimepiride, glipizide, glybuzole), repaglinide, nateglinide, mitiglinide or a calcium salt hydrate thereof], dipeptidyl peptidase IV inhibitors (e.g., Vildagliptin, Sitagliptin, Saxagliptin, T-6666, TS-021), β3 agonists (e.g., AJ-9677), GPR40 agonists, GLP-1 receptor agonists [e.g., GLP-1, GLP-1MR agent, NN-2211, AC-2993 (exendin-4), BIM-51077, Aib(8,35)hGLP-1(7,37)NH2, CJC-1131], amylin agonists (e.g., pramlintide), phosphotyrosine phosphatase inhibitors (e.g., sodium vanadate), gluconeogenesis inhibitors (e.g., glycogen phosphorylase inhibitors, glucose-6-phosphatase inhibitors, glucagon antagonists), SGLUT (sodium-glucose cotransporter) inhibitors (e.g., T-1095), 11β-hydroxysteroid dehydrogenase inhibitors (e.g., BVT-3498), adiponectin or agonist thereof, IKK inhibitors (e.g., AS-2868), leptin resistance improving drugs, somatostatin receptor agonists, glucokinase activators (e.g., Ro-28-1675), GIP (Glucose-dependent insulinotropic peptide) and the like.

Examples of the therapeutic agent for diabetic complications include aldose reductase inhibitors (e.g., tolrestat, epalrestat, zenarestat, zopolrestat, minalrestat, fidarestat, CT-112), neurotrophic factors and increasing drugs thereof (e.g., NGF, NT-3, BDNF, neurotrophin production/secretion promoting agents (e.g., 4-(4-chlorophenyl)-2-(2-methyl-1-imidazolyl)-5-[3-(2-methylphenoxy)propyl]oxazole) described in WO01/14372), nerve regeneration promoters (e.g., Y-128), PKC inhibitors (e.g., ruboxistaurin mesylate), AGE inhibitors (e.g., ALT946, pimagedine, pyratoxanthine, N-phenacylthiazolium bromide (ALT766), ALT-711, EXO-226, pyridorin, pyridoxamine), active oxygen scavengers (e.g., thioctic acid), cerebral vasodilators (e.g., tiapuride, mexiletine), somatostatin receptor agonists (e.g., BIM23190) and apoptosis signal regulating kinase-1 (ASK-1) inhibitor.

Examples of the therapeutic agent for hyperlipidemia include statin compounds (e.g., pravastatin, simvastatin, lovastatin, atorvastatin, fluvastatin, rosuvastatin, pitavastatin or a salt thereof (e.g., sodium salt, calcium salt)), squalene synthase inhibitors (e.g., compounds described in WO97/10224, for example, N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid), fibrate compounds (e.g., bezafibrate, clofibrate, simfibrate, clinofibrate), ACAT inhibitors (e.g., Avasimibe, Eflucimibe), anion exchange resins (e.g., colestyramine), probucol, nicotinic acid drugs (e.g., nicomol, niceritrol), ethyl icosapentate and phytosterols (e.g., soysterol, γ-oryzanol).

Examples of the antihypertensive agent include angiotensin converting enzyme inhibitors (e.g., captopril, enalapril, delapril), angiotensin II antagonists (e.g., candesartan cilexetil, losartan, eprosartan, valsartan, telmisartan, irbesartan, tasosartan, 1-[[2'-(2,5-dihydro-5-oxo-4H-1,2,4-oxadiazol-3-yl)biphenyl-4-yl]methyl]-2-ethoxy-1H-benzimidazole-7-carboxylic acid), calcium antagonists (e.g., manidipine, nifedipine, amlodipine, efonidipine, nicardipine), potassium channel openers (e.g., levcromakalim, L-27152, AL 0671, NIP-121) and clonidine.

Examples of the antiobesity agent include central nervous system antiobesity drugs (e.g., dexfenfluramine, fenfluramine, phentermine, sibutramine, amfepramone, dexamphetamine, mazindol, phenylpropanolamine, clobenzorex; MCH receptor antagonists (e.g., SB-568849; SNAP-7941; compounds described in WO01/82925 and WO01/87834); neuropeptide Y antagonists (e.g., CP-422935); cannabinoid receptor antagonists (e.g., SR-141716, SR-147778); ghrelin antagonist; 11β-hydroxysteroid dehydrogenase inhibitors (e.g., BVT-3498)), pancreatic lipase inhibitors (e.g., orlistat, ATL-962), β3 agonists (e.g., AJ-9677, AZ40140), anorectic peptides (e.g., leptin, CNTF (ciliary neurotrophic factor)), cholecystokinin agonists (e.g., lintitript, FPL-15849) and feeding deterrents (e.g., P-57).

Examples of the diuretics include xanthine derivatives (e.g., theobromine sodium salicylate, theobromine calcium salicylate), thiazide preparations (e.g., ethiazide, cyclopenthiazide, trichloromethiazide, hydrochlorothiazide, hydroflumethiazide, benzylhydrochlorothiazide, penflutizide, polythiazide, methyclothiazide), antialdosterone preparations (e.g., spironolactone, triamterene), carbonic anhydrase inhibitors (e.g., acetazolamide), chlorobenzenesulfonamide agents (e.g., chlortalidone, mefruside, indapamide), azosemide, isosorbide, ethacrynic acid, piretanide, bumetanide and furosemide.

Examples of the antithrombotic agent include heparin (e.g., heparin sodium, heparin calcium, dalteparin sodium), warfarin (e.g., warfarin potassium), anti-thrombin drugs (e.g., aragatroban), thrombolytic agents (e.g., urokinase, tisokinase, alteplase, nateplase, monteplase, pamiteplase) and platelet aggregation inhibitors (e.g., ticlopidine hydrochloride, cilostazol, ethyl icosapentate, beraprost sodium, sarpogrelate hydrochloride).

The production method of compound (I) is explained in the following. Compound (I) can be produced by, for example, Reaction Scheme 1, 2 or 7 to be described in detail in the following or a method according thereto.
In the following Reaction Schemes 1 to 8, the compound used as a starting compound may be each in the form of a salt. Examples of the salt include those exemplified as the salt of compound (I).

In each reaction of the following Reaction Schemes 1 to 8, the product can be used for the next reaction as the reaction mixture or as a crude product, or can also be isolated according to a conventional method from the reaction mixture, and can also be easily purified according to a conventional separation means (e.g., recrystallization, distillation, chromatography).

When alkylation reaction, hydrolysis, amination reaction, esterification reaction, amidation reaction, esterification reaction, etherification reaction, oxidation reaction, reduction reaction and the like are to be performed in the following Reaction Schemes 1 to 8, these reactions are performed according to a method known per se. Examples of such method include the methods described in ORGANIC FUNCTIONAL GROUP PREPARATIONS, 2nd ed., ACADEMIC PRESS, INC., 1989; Comprehensive Organic Transformations, VCH Publishers Inc., 1989 and the like, and the like.

In the following Reaction Schemes 1 to 8, the deprotection can be carried out according to a method known per se, for example, the method described in Protective Groups in Organic Synthesis, John Wiley and Sons (1980), or the like.

Specific examples thereof include a method using acid, base, ultraviolet rays, hydrazine, phenylhydrazine, sodium N-methyldithiocarbamate, tetrabutylammonium fluoride, palladium acetate, trialkylsilyl halides (e.g., trimethylsilyl iodide, trimethylsilyl bromide) and the like, reduction method and the like.

The solvents used as a generic term for the reaction are explained in the following.

Examples of the "nitrile solvent" include acetonitrile, propionitrile and the like.

Examples of the "amide solvent" include N,N-dimethylformamide (DMF), N,N-dimethylacetamide, N-methylpyrrolidone and the like.

Examples of the "halogenated hydrocarbon solvent" include dichloromethane, chloroform, 1,2-dichloroethane, carbon tetrachloride and the like.

Examples of the "ether solvent" include diethyl ether, diisopropyl ether, tert-butyl methyl ether, tetrahydrofuran (THF), 1,4-dioxane, 1,2-dimethoxyethane and the like.

Examples of the "aromatic solvent" include benzene, toluene, xylene, pyridine and the like.

Examples of the "aliphatic hydrocarbon solvent" include hexane, pentane, cyclohexane and the like.

Examples of the "sulfoxide solvent" include dimethyl sulfoxide (DMSO) and the like.

Examples of the "alcohol solvent" include methanol, ethanol, propanol, 2-propanol, butanol, isobutanol, tert-butanol and the like.

Examples of the "ester solvent" include methyl acetate, ethyl acetate, n-butyl acetate, tert-butyl acetate and the like.

Examples of the "ketone solvent" include acetone, methyl ethyl ketone and the like.

Examples of the "organic acid solvent" include formic acid, acetic acid, propionic acid, trifluoroacetic acid, methanesulfonic acid and the like.

The bases used as a generic term for the reaction are explained in the following.

Examples of the "inorganic base" include sodium hydroxide, potassium hydroxide, lithium hydroxide, barium hydroxide and the like.

Examples of the "basic salt" include sodium carbonate, potassium carbonate, cesium carbonate, sodium hydrogen carbonate, potassium hydrogen carbonate and the like.

Examples of the "aromatic amine" include pyridine, imidazole, 2,6-lutidine and the like.

Examples of the "secondary amine" include pyrrolidine, piperidine, morpholine and the like.

Examples of the "tertiary amine" include triethylamine, diisopropylethylamine, N-methylmorpholine, DBU (1,8-diazabicyclo[5.4.0]undec-7-ene), DBN (1,5-diazabicyclo[4.3.0]non-5-ene) and the like.

Examples of the "hydride of alkali metal or alkaline earth metal" include lithium hydride, sodium hydride, potassium hydride, calcium hydride and the like.

Examples of the "metal amide" include lithium amide, sodium amide, lithium diisopropylamide, lithium dicyclohexylamide, lithium hexamethyldisilazide, sodium hexamethyldisilazide, potassium hexamethyldisilazide and the like.

Examples of the "alkyl metal" include n-butyllithium, sec-butyllithium, tert-butyllithium, methylmagnesium bromide and the like.

Examples of the "aryl metal" include phenyllithium, phenylmagnesium bromide and the like.

Examples of the "metal alkoxide" include sodium methoxide, sodium ethoxide, potassium tert-butoxide and the like.

The abbreviations in the reaction schemes are explained.

Ring P, ring Q, ring R and $R^1$ are as defined above.

Ra, Ra', Rb, Rc, Rd and Re are the same or different and each is a hydrogen atom or a substituent. Examples of the "substituent" include those exemplified as the above-mentioned "substituent" for $R^1$.

Hal is a halogen atom.

J is the below-mentioned hydroxyl-protecting group generally used in peptide chemistry and the like.

L is the below-mentioned mercapto-protecting group generally used in peptide chemistry and the like.

W is O or S.

X is the below-mentioned amino-protecting group generally used in peptide chemistry and the like.

X" is an oxo group, a halogen atom, a sulfonylated hydroxy group (e.g., toluenesulfonyloxy group, methanesulfonyloxy group, trifluoromethanesulfonyloxy group) or the like.

Y is an optionally substituted linear $C_{1-3}$ alkylene.

Examples of the "linear $C_{1-3}$ alkylene" include $-CH_2-$, $-CH_2CH_2-$ and $-CH_2CH_2CH_2-$. Examples of the substituent include those similar to the substituent that the $C_{1-10}$ alkyl group and the like exemplified as the "hydrocarbon group" of the

above-mentioned "optionally substituted hydrocarbon group" optionally has.

Y' is an optionally substituted linear $C_{1-3}$ alkylene.

Examples of the "linear $C_{1-3}$ alkylene" include $-CH_2-$, $-CH_2CH_2-$ and $-CH_2CH_2CH_2-$. Examples of the substituent include those similar to the substituent that the $C_{1-10}$ alkyl group and the like exemplified as the "hydrocarbon group" of the above-mentioned "optionally substituted hydrocarbon group" optionally has.

Z is a leaving group. Examples of the "leaving group" include a halogen atom, a sulfonylated hydroxy group (e.g., toluenesulfonyloxy group, methanesulfonyloxy group, trifluoromethanesulfonyloxy group) and the like.

<Reaction Scheme 1>

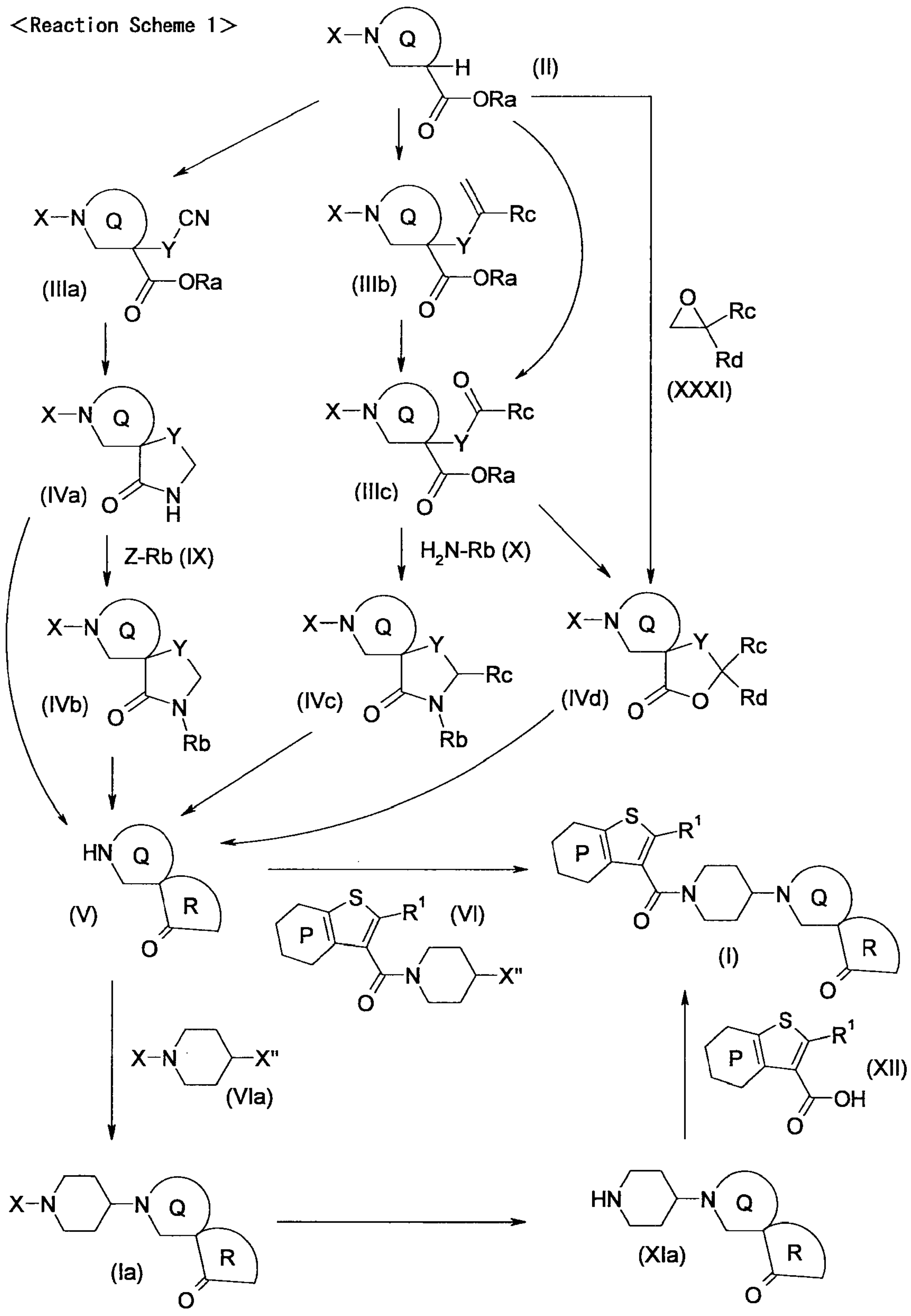


Compound (IIIa) can be produced, for example, by subjecting compound (II) to an alkylation reaction. The alkylation reaction can be carried out according to a method known per se, for example, the method described in Journal of Medicinal Chemistry (J. Med. Chem.) pages 2439-2441, 1998, or the like, or a method analogous thereto. Compound (II) may be commercially available, or can be produced according to a method known per se or a method

analogous thereto.
This reaction is carried out by reacting compound (II) with an alkylating agent in the presence of a base, in an inert solvent.
Examples of the above-mentioned "alkylating agent" include cyanoalkyl halides (e.g., bromoacetonitrile), alkenylnitriles (e.g., acrylonitrile) and the like. The amount of the "alkylating agent" to be used is generally 1 to 5 equivalents, preferably 1 to 1.5 equivalents, relative to compound (II).
Examples of the above-mentioned "inert solvent" include ether solvents, aromatic solvents, aliphatic hydrocarbon solvents and the like. These solvents may be used in a mixture of two or more kinds thereof at an appropriate ratio. Of these, ether solvents and the like are preferable.
Examples of the above-mentioned "base" include "hydrides of alkali metal or alkaline earth metal", "metal amides", "alkyl metals", "aryl metals" and the like. The amount of the "base" to be used is generally 1 to 10 equivalents, preferably 1 to 1.5 equivalents, relative to compound (II).
The reaction temperature is generally -100°C to 150°C, preferably -78°C to 100°C.
The reaction time is generally 5 min to 48 hr, preferably 30 min to 24 hr.

Compound (IIIb) can be produced, for example, by subjecting compound (II) to an alkylation reaction.
The alkylation reaction can be carried out according to a method known per se, for example, the method described in Journal of Medicinal Chemistry (J. Med. Chem.) pages 2439-2441, 1998, or the like, or a method analogous thereto.
This reaction is carried out by reacting compound (II) with an alkylating agent in the presence of a base, in an inert solvent.
Examples of the above-mentioned "alkylating agent" include alkenyl halides (e.g., allyl bromide) and the like. The amount of the "alkylating agent" to be used is generally 1 to 5 equivalents, preferably 1 to 1.5 equivalents, relative to compound (II).
Examples of the above-mentioned "inert solvent" include ether solvents, aromatic solvents, aliphatic hydrocarbon solvents and the like. These solvents may be used in a mixture of two or more kinds thereof at an appropriate ratio. Of these, ether solvents and the like are preferable.
Examples of the above-mentioned "base" include "hydrides of alkali metal or alkaline earth metal", "metal amides", "alkyl metals", "aryl metals" and the like. The amount of the "base" to be used is generally 1 to 10 equivalents, preferably 1 to 1.5 equivalents, relative to compound (II).
The reaction temperature is generally -100°C to 150°C, preferably -78°C to 100°C.
The reaction time is generally 5 min to 48 hr, preferably 30 min to 24 hr.

Compound (IIIc) can be produced, for example, by subjecting compound (II) to an alkylation reaction.
The alkylation reaction can be carried out according to a method known per se, for example, the method described in Journal of Medicinal Chemistry (J. Med. Chem.) pages 2439-2441, 1998, or the like, or a method analogous thereto.
This reaction is carried out by reacting compound (II) with an alkylating agent in the presence of a base, in an inert solvent.
Examples of the above-mentioned "alkylating agent" include alkenylaldehydes (e.g., acrolein), alkenyl ketones (e.g., methyl vinyl ketone), haloketones (e.g., bromoacetone) and the like. The amount of the "alkylating agent" to be used is generally 1 to 5 equivalents, preferably 1 to 1.5 equivalents, relative to compound (II).
Examples of the above-mentioned "inert solvent" include ether solvents, aromatic solvents, aliphatic hydrocarbon solvents and the like. These solvents may be used in a mixture of two or more kinds thereof at an appropriate ratio. Of these, ether solvents and the like are preferable.
Examples of the above-mentioned "base" include "hydrides of alkali metal or alkaline earth metal", "metal amides", "alkyl metals", "aryl metals" and the like. The amount of the "base" to be used is generally 1 to 10 equivalents, preferably 1 to 1.5 equivalents, relative to compound (II).
The reaction temperature is generally -100°C to 150°C, preferably -78°C to 100°C.
The reaction time is generally 5 min to 48 hr, preferably 30 min to 24 hr.

Compound (IIIc) can also be produced, for example, by subjecting compound (IIIb) to an oxidization reaction.
The oxidation reaction can be carried out according to a method known per se, for example, the method described in Heterocycles pages 2263-2267, 1992, or the like, or a method analogous thereto.
This reaction is carried out by reacting compound (IIIb) with an oxidant in an inert solvent.
Examples of the above-mentioned "oxidant" include ozone, potassium permanganate, sodium periodate, osmium tetraoxide and the like. The amount of the "oxidant" to be used is generally 1 to 5 equivalents, preferably 1 to 1.5 equivalents, relative to compound (IIIb).
Examples of the above-mentioned "inert solvent" include alcohol solvents, nitrile solvents, amide solvents, halogenated hydrocarbon solvents, ether solvents and the like. These solvents may be used in a mixture of two or more kinds thereof at an appropriate ratio. Of these, halogenated hydrocarbon solvents and the like are preferable.
The reaction temperature is generally -100°C to 50°C, preferably -78°C to 0°C.
The reaction time is generally 5 min to 48 hr, preferably 30 min to 24 hr.

Compound (IVa) can be produced, for example, by subjecting compound (IIIa) to a reduction reaction.
The reduction reaction can be carried out according to a method known per se, for example, the method described in Bioorganic and Medicinal Chemistry (Bioorg. Med. Chem.) pages 2945-2952, 1999, or the like, or a method analogous thereto.

This reaction is carried out by reacting compound (IIIa) with a reducing agent in an inert solvent.
Examples of the above-mentioned "reducing agent" include metal hydrogen compounds (e.g., sodium bis(2-methoxyethoxy)aluminum hydride, diisobutylaluminum hydride), metal hydrogen complex compounds (e.g., sodium borohydride, sodium cyanoborohydride, lithium aluminum hydride, sodium aluminum hydride) and the like. The amount of the "reducing agent" to be used is generally 0.1 to 20 equivalents, preferably 1 to 5 equivalents, relative to compound (IIIa).
Examples of the above-mentioned "inert solvent" include alcohol solvents, aromatic solvents, aliphatic hydrocarbon solvents, ether solvents, ester solvents, amide solvents and the like. These solvents may be used in a mixture of two or more kinds thereof at an appropriate ratio.
The reaction temperature is generally -70 to 150°C, preferably -20 to 100°C.
The reaction time is generally 0.1 to 100 hr, preferably 0.1 to 40 hr.
Alternatively, this reaction is also carried out by reacting compound (IIIa) in the presence of a metal catalyst and a hydrogen source, in an inert solvent.
Examples of the above-mentioned "metal catalyst" include palladium-carbon, palladium black, palladium chloride, platinum oxide, platinum black, platinum-palladium, Raney-nickel, Raney-cobalt and the like. The amount of the "metal catalyst" to be used is generally 0.001 to 1000 equivalents, preferably 0.01 to 100 equivalents, relative to compound (IIIa).
Examples of the above-mentioned "hydrogen source" include hydrogen gas, formic acid, amine salts of formic acid, phosphinates, hydrazine and the like.
Examples of the above-mentioned "inert solvent" include those exemplified in the above-mentioned reduction reaction of compound (IIIa).
The reaction temperature and reaction time are the same as in the above-mentioned reduction reaction of compound (IIIa).
Where necessary, this reaction may be carried out in the presence of ammonia (e.g., aqueous ammonia, ammonia-ethanol). By reacting compound (IIIa) in the presence of ammonia, side reaction is suppressed, and compound (IVa) can be produced in high yield.

Compound (IVb) can be produced, for example, by reacting compound (IVa) to an alkylation reaction.
The alkylation reaction can be carried out according to a method known per se, for example, the method described in Journal of Medicinal Chemistry (J. Med. Chem.) pages 2441-2450, 2004, or the like, or a method analogous thereto.
This reaction is carried out by reacting compound (IVa) with compound (IX) in the presence of a base, in an inert solvent.
Compound (IX) may be commercially available, or can be produced according to a method known per se or a method analogous thereto.
The amount of compound (IX) to be used is generally 1 to 5 equivalents, preferably 1 to 1.5 equivalents, relative to compound (IVa).
Examples of the above-mentioned "inert solvent" include nitrile solvents, amide solvents, halogenated hydrocarbon solvents, ether solvents and the like. These solvents may be used in a mixture of two or more kinds thereof at an appropriate ratio. Of these, THF, DMF and the like are preferable.
Examples of the above-mentioned "base" include "hydrides of alkali metal or alkaline earth metal" and the like. The amount of the "base" to be used is generally 1 to 10 equivalents, preferably 1 to 1.5 equivalents, relative to compound (IVa).
The reaction temperature is generally -100°C to 150°C, preferably 0°C to 100°C.
The reaction time is generally 5 min to 48 hr, preferably 30 min to 24 hr.

Compound (IVc) can be produced, for example, by subjecting compound (IIIc) to a reductive amination reaction.
The reductive amination reaction can be carried out according to a method known per se, for example, the method described in Tetrahedron Letters (Tetrahedron Lett.) pages 8345-8349, 2001, or the like, or a method analogous thereto.
This reaction is carried out by reacting compound (IIIc) with compound (X) in the presence of a reducing agent, in an inert solvent. Where necessary, the reaction may be carried out in the presence of 1 to 50 equivalents of an organic acid.
Compound (X) may be commercially available, or can be produced according to a method known per se or a method analogous thereto.
The amount of compound (X) to be used is generally 1 to 5 equivalents, preferably 2 to 4 equivalents, relative to compound (IIIc).
Examples of the above-mentioned "reducing agent" include metal hydrogen compounds (e.g., sodium bis(2-methoxyethoxy)aluminum hydride, diisobutylaluminum hydride), metal hydrogen complex compounds (e.g., sodium borohydride, sodium cyanoborohydride, sodium triacetoxyborohydride, lithium aluminum hydride, sodium aluminum hydride) and the like. The amount of the "reducing agent" to be used is generally 0.1 to 20 equivalents, preferably 1 to 5 equivalents, relative to compound (IIIc).
Examples of the above-mentioned "inert solvent" include alcohol solvents, nitrile solvents, amide solvents, halogenated hydrocarbon solvents, ether solvents and the like. These solvents may be used in a mixture of two or more kinds thereof at an appropriate ratio. Of these, THF, dichloromethane and the like are preferable.
Examples of the above-mentioned "organic acid" include acetic acid and the like.
The reaction temperature is generally -78°C to 100°C, preferably 0°C to 50°C.
The reaction time is generally 5 min to 48 hr, preferably 30 min to 24 hr.

Compound (IVd) wherein Rd is a hydrogen atom is can be produced, for example, by subjecting compound (IIIc) to a reduction reaction.
This reaction is carried out by reacting compound (IIIc) with a reducing agent in an inert solvent.
Examples of the above-mentioned "reducing agent" include metal hydrogen compounds (e.g., sodium bis(2-methoxyethoxy)aluminum hydride, diisobutylaluminum hydride), metal hydrogen complex compounds (e.g., sodium borohydride, sodium cyanoborohydride, lithium aluminum hydride, sodium aluminum hydride) and the like. The amount of the "reducing agent" to be used is generally 0.1 to 20 equivalents, preferably 1 to 5 equivalents, relative to compound (IIIc).
Examples of the above-mentioned "inert solvent" include nitrile solvents, aromatic solvents, aliphatic hydrocarbon solvents, ether solvents, amide solvents, halogenated hydrocarbon solvents and the like. These solvents may be used in a mixture of two or more kinds thereof at an appropriate ratio. Of these, THF, methanol and the like are preferable.
The reaction temperature is generally -78°C to 150°C, preferably -20°C to 100°C.
The reaction time is generally 5 min to 48 hr, preferably 30 min to 24 hr.
Compound (IVd) wherein Rd is other than a hydrogen atom can be produced, for example, by reacting compound (IIIc) with an organic metal reagent in an inert solvent.
Examples of the above-mentioned "organic metal reagent" include organic Grignard reagents (e.g., methylmagnesium bromide), organic lithium reagents (e.g., methyllithium) and the like. The amount of the "organic metal reagent" to be used is generally 0.1 to 20 equivalents, preferably 1 to 5 equivalents, relative to compound (IIIc).
Examples of the above-mentioned "inert solvent" include nitrile solvents, aromatic solvents, aliphatic hydrocarbon solvents, ether solvents, amide solvents, halogenated hydrocarbon solvents and the like. These solvents may be used in a mixture of two or more kinds thereof at an appropriate ratio. Of these, THF and the like are preferable.
The reaction temperature is generally -78°C to 150°C, preferably -20°C to 100°C.
The reaction time is generally 5 min to 48 hr, preferably 30 min to 24 hr.
Compound (IVd) can also be produced, for example, by reacting compound (II) with compound (XXXI).
Compound (XXXI) may be commercially available, or can be produced according to a method known per se or a method analogous thereto.
This reaction is carried out by reacting compound (II) with compound (XXXI) in the presence of a base, in an inert solvent.
Examples of the above-mentioned "base" include "hydrides of alkali metal or alkaline earth metal", "metal amides", "alkyl metals", "aryl metals" and the like. The amount of the "base" to be used is generally 1 to 10 equivalents, preferably 1 to 1.5 equivalents, relative to compound (II).
Examples of the above-mentioned "inert solvent" include nitrile solvents, aromatic solvents, aliphatic hydrocarbon solvents, ether solvents, amide solvents, halogenated hydrocarbon solvents and the like. These solvents may be used in a mixture of two or more kinds thereof at an appropriate ratio. Of these, THF and the like are preferable.
The reaction temperature is generally -78°C to 150°C, preferably -20°C to 100°C.
The reaction time is generally 5 min to 48 hr, preferably 30 min to 24 hr.
Compound (V) can be produced, for example, by subjecting compound (IVa), (IVb), (IVc) or (IVd) to deprotection.
Compound (Ia) can be produced, for example, by subjecting compound (V) to a coupling reaction with compound (VIa).
This reaction is carried out by reacting compound (V) with compound (VIa) in the presence of a reducing agent, in an inert solvent. Where necessary, the reaction may be carried out in the presence of 1 to 50 equivalents of an organic acid.
Compound (VIa) may be commercially available, or can be produced according to a method known per se or a method analogous thereto.
The amount of compound (VIa) to be used is generally 1 to 5 equivalents, preferably 1 to 4 equivalents, relative to compound (V).
Examples of the above-mentioned "reducing agent" include metal hydrogen compounds (e.g., sodium bis(2-methoxyethoxy)aluminum hydride, diisobutylaluminum hydride), metal hydrogen complex compounds (e.g., sodium borohydride, sodium cyanoborohydride, sodium triacetoxyborohydride, lithium aluminum hydride, sodium aluminum hydride) and the like. The amount of the "reducing agent" to be used is generally 0.1 to 20 equivalents, preferably 1 to 5 equivalents, relative to compound (V).
Examples of the above-mentioned "inert solvent" include alcohol solvents, nitrile solvents, amide solvents, halogenated hydrocarbon solvents, ether solvents and the like. These solvents may be used in a mixture of two or more kinds thereof at an appropriate ratio. Of these, THF, dichloromethane and the like are preferable.
Examples of the above-mentioned "organic acid" include acetic acid and the like.
The reaction temperature is generally -78°C to 50°C, preferably room temperature to 50°C.
The reaction time is generally 5 min to 48 hr, preferably 30 min to 24 hr.
Compound (Ia) can also be produced by reacting compound (V) with compound (VIa) wherein X" is a halogen atom, a sulfonylated hydroxy group or the like, in the presence of a base, in an inert solvent.
The amount of compound (VIa) to be used is generally 1 to 3 equivalents, preferably 1 to 1.5 equivalents, relative to compound (V).

Examples of the above-mentioned "base" include "inorganic bases", "basic salts", "tertiary amines" and the like. The amount of the "base" to be used is generally 0.1 to 20 equivalents, preferably 1 to 5 equivalents, relative to compound (V). Examples of the above-mentioned "inert solvent" include amide solvents, ether solvents, aromatic solvents and the like. These solvents may be used in a mixture of two or more kinds thereof at an appropriate ratio.
The reaction temperature is generally -78°C to 150°C, preferably 0°C to 50°C.
The reaction time is generally 5 min to 48 hr, preferably 30 min to 24 hr.
Compound (XIa) can be produced by subjecting compound (Ia) to deprotection.
Compound (I) can be produced by subjecting compound (XIa) to an amidation reaction.
Examples of the above-mentioned "amidation reaction" include the following "method using a dehydration-condensation agent", "method using a reactive derivative of carboxylic acid" and the like.

i) Method using a dehydration-condensation agent

This reaction is carried out by reacting compound (XIa) with compound (XII) in the presence of a dehydration-condensation agent, in an inert solvent. Where necessary, the reaction may be carried out in the presence of a catalytic amount to 5 equivalents of 1-hydroxybenzotriazole (HOBt), a catalytic amount to 5 equivalents of a base or the like.
Compound (XII) may be commercially available, or can be produced according to a method known per se or a method analogous thereto, or a method described in Reaction Scheme 7.
The amount of compound (XII) to be used is generally 0.5 to 5 equivalents, preferably 0.8 to 1.5 equivalents, relative to compound (XIa).
Examples of the above-mentioned "dehydration-condensation agent" include dicyclohexylcarbodiimide (DCC), 1-ethyl-3-(3-dimethylaminopropyl)carbodiimide hydrochloride (EDC·HCl) and the like. Of these, EDC·HCl is preferable. The amount of the "dehydration-condensation agent" to be used is generally 1 to 10 equivalents, preferably 1 to 5 equivalents, relative to compound (XIa).
Examples of the above-mentioned "inert solvent" include nitrile solvents, amide solvents, halogenated hydrocarbon solvents, ether solvents and the like. These solvents may be used in a mixture of two or more kinds thereof at an appropriate ratio. Of these, amide solvents are preferable.
Examples of the above-mentioned "base" include "aromatic amines", "tertiary amines" and the like.
The reaction temperature is generally -70 to 150°C, preferably -20 to 100°C.
The reaction time is generally 0.1 to 100 hr, preferably 1 to 48 hr.

ii) Method using a reactive derivative of carboxylic acid

This reaction is carried out by reacting a reactive derivative of compound (XII) with 0.5 to 5 equivalents (preferably 0.8 to 3 equivalents) of compound (XIa) in an inert solvent. Where necessary, the reaction may be carried out in the presence of 1 to 10 equivalents, preferably 1 to 3 equivalents of a base.
Examples of the "reactive derivative" of compound (XII) include acid halides (e.g., acid chloride, acid bromide), mixed acid anhydrides (e.g., acid anhydrides with a $C_{1-6}$ alkyl-carboxylic acid, a $C_{6-10}$ aryl-carboxylic acid or a $C_{1-6}$ alkylcarbonic acid), activated esters (e.g., esters with a phenol optionally having substituent(s), HOBt, N-hydroxysuccinimide or the like) and the like.
Examples of the "substituent" of the above-mentioned "phenol optionally having substituent(s)" include those similar to the substituent that the $C_{3-10}$ cycloalkyl group and the like exemplified as the "hydrocarbon group" of the above-mentioned "optionally substituted hydrocarbon group" optionally has.
Specific examples of the "phenol optionally having substituent(s)" include phenol, pentachlorophenol, pentafluorophenol, p-nitrophenol and the like.
The reactive derivative is preferably an acid halide.
Examples of the above-mentioned "inert solvent" include ether solvents, halogenated hydrocarbon solvents, aromatic solvents, aliphatic hydrocarbon solvents, nitrile solvents, amide solvents, ketone solvents, sulfoxide solvents, water and the like. These solvents may be used in a mixture of two or more kinds thereof at an appropriate ratio. Of these, acetonitrile, THF, dichloromethane, chloroform and the like are preferable.
Examples of the above-mentioned "base" include "aromatic amines", "tertiary amines" and the like.
The reaction temperature is generally -20°C to 100°C, preferably -20°C to 50°C.
The reaction time is generally 5 min to 40 hr, preferably 30 min to 18 hr.
Compound (I) can also be produced by reacting compound (V) with compound (VI), according to the same method as in the above-mentioned production method of compound (Ia) from compound (V) and compound (VIa).
Compound (VI) can be produced according to a method known per se or a method analogous thereto, or a method described in the below-mentioned Reaction Scheme 7.
<Reaction Scheme 2>

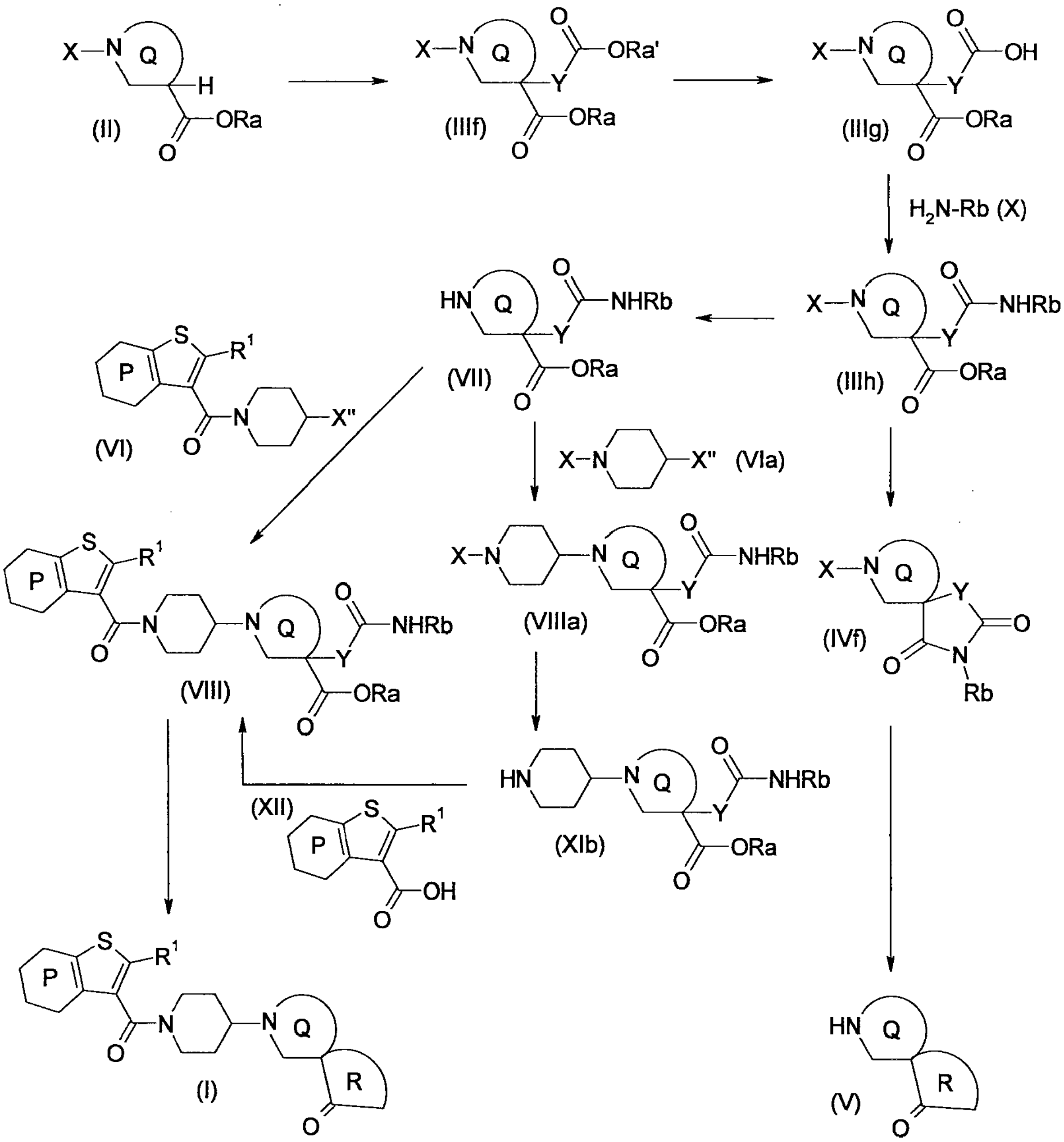


Compound (IIIf) can be produced, for example, by subjecting compound (II) to an alkylation reaction.
The alkylation reaction can be carried out according to a method known per se, for example, the method described in Journal of Medicinal Chemistry (J. Med. Chem.) pages 2439-2441, 1998, or the like, or a method analogous thereto. This reaction is carried out by reacting compound (II) with an alkylating agent in the presence of a base, in an inert solvent.
Examples of the above-mentioned "alkylating agent" include (alkoxycarbonyl)alkyl halides (e.g., ethyl bromoacetate), alkenyl esters (e.g., methyl acrylate) and the like. The amount of the "alkylating agent" to be used is generally 1 to 5 equivalents, preferably 1 to 1.5 equivalents, relative to compound (II).
Examples of the above-mentioned "inert solvent" include ether solvents, aromatic solvents, aliphatic hydrocarbon solvents and the like. These solvents may be used in a mixture of two or more kinds thereof at an appropriate ratio. Of these, ether solvents and the like are preferable.
Examples of the above-mentioned "base" include "hydrides of alkali metal or alkaline earth metal", "metal amides", "alkyl metals", "aryl metals" and the like. The amount of the "base" to be used is generally 1 to 10 equivalents, preferably 1 to

1.5 equivalents, relative to compound (II).
The reaction temperature is generally -100°C to 150°C, preferably -78°C to 100°C.
The reaction time is generally 5 min to 48 hr, preferably 30 min to 24 hr.

Compound (IIIg) can be produced, for example, by subjecting compound (IIIf) to hydrolysis.
This reaction is carried out by reacting compound (IIIf) with a base in an inert solvent.
Examples of the above-mentioned "base" include "inorganic bases" and the like. The amount of the "base" to be used is generally 1 to 10 equivalents, preferably 1 to 1.5 equivalents, relative to compound (IIIf).
Examples of the above-mentioned "inert solvent" include alcohol solvents, nitrile solvents, aromatic solvents, aliphatic hydrocarbon solvents, ether solvents, amide solvents, halogenated hydrocarbon solvents and the like. These solvents are preferably used in a mixture with water at an appropriate ratio. Of these, alcohol solvents containing water are preferable.
The reaction temperature is generally -78°C to 150°C, preferably -20°C to 100°C.
The reaction time is generally 5 min to 100 hr, preferably 30 min to 24 hr.
Alternatively, this reaction is also carried out by reacting compound (IIIf) in the presence of a metal catalyst and a hydrogen source, in an inert solvent.
Examples of the above-mentioned "metal catalyst" include palladium-carbon, palladium black, palladium chloride, platinum oxide, palladium black, platinum-palladium, Raney-nickel, Raney-cobalt and the like. The amount of the "metal catalyst" to be used is generally 0.001 to 1000 equivalents, preferably 0.01 to 100 equivalents, relative to compound (IIIf).
Examples of the above-mentioned "hydrogen source" include hydrogen gas, formic acid, amine salts of formic acid, phosphinates, hydrazine and the like.
Examples of the above-mentioned "inert solvent" include alcohol solvents, nitrile solvents, aromatic solvents, aliphatic hydrocarbon solvents, ether solvents, amide solvents, halogenated hydrocarbon solvents and the like. These solvents may be used in a mixture with water at an appropriate ratio. Of these, alcohol solvents are preferable.
The reaction temperature is generally -70 to 150°C, preferably -20 to 100°C.
The reaction time is generally 0.1 to 100 hr, preferably 0.1 to 40 hr.

In addition, the production method of compound (IIIg) by removing carboxyl-protecting group Ra' of compound (IIIf) can be carried out according to a method known per se, for example, the method described in Protective Groups in Organic Synthesis, John Wiley and Sons (1980), or the like.

Compound (IIIh) can be produced by subjecting compound (IIIg) to an amidation reaction with compound (X), according to the same method as in the amidation reaction of compound (XIa) in the above-mentioned Reaction Scheme 1.

Compound (VII) can be produced, for example, by subjecting compound (IIIh) to deprotection.

Compound (VIIIa) can be produced by reacting compound (VII) with compound (VIa), according to the same method as in the production method of compound (Ia) from compound (V) and compound (VIa) in the above-mentioned Reaction Scheme 1.

Compound (XIb) can be produced, for example, by subjecting compound (VIIIa) to deprotection.

Compound (VIII) can be produced by subjecting compound (XIb) to an amidation reaction with compound (XII), according to the same method as in the amidation reaction of compound (XIa) in the above-mentioned Reaction Scheme 1. Compound (XII) may be commercially available, or can be produced according to a method known per se or a method analogous thereto, or a method described in the below-mentioned Reaction Scheme 7.

Compound (VIII) can also be produced by reacting compound (VII) with compound (VI), according to the same method as in the production method of compound (Ia) from compound (V) and compound (VIa) in the above-mentioned Reaction Scheme 1.

Compound (IVf) can be produced, for example, by subjecting compound (IIIh) to a cyclization reaction.
The cyclization reaction can be carried out according to a method known per se, for example, the method described in Journal of Medicinal Chemistry (J. Med. Chem.) pages 4118-4129, 1998, or the like, or a method analogous thereto.
This reaction is carried out by reacting compound (IIIh) with a base in an inert solvent.
Examples of the above-mentioned "base" include "hydrides of alkali metal or alkaline earth metal" and the like. The amount of the "base" to be used is generally 1 to 10 equivalents, preferably 1 to 1.5 equivalents, relative to compound (IIIh).
Examples of the above-mentioned "inert solvent" include nitrile solvents, aromatic solvents, aliphatic hydrocarbon solvents, ether solvents, amide solvents, halogenated hydrocarbon solvents and the like. These solvents may be used in a mixture of two or more kinds thereof at an appropriate ratio. Of these, amide solvents are preferable.
The reaction temperature is generally -100°C to 150°C, preferably 0°C to 100°C.
The reaction time is generally 5 min to 48 hr, preferably 30 min to 24 hr.

Compound (V) can also be produced by subjecting compound (IVf) to deprotection.

Compound (I) can also be produced by subjecting compound (VIII) to cyclization reaction, according to the same method as in the above-mentioned cyclization reaction of compound (IIIh).

<Reaction Scheme 3>

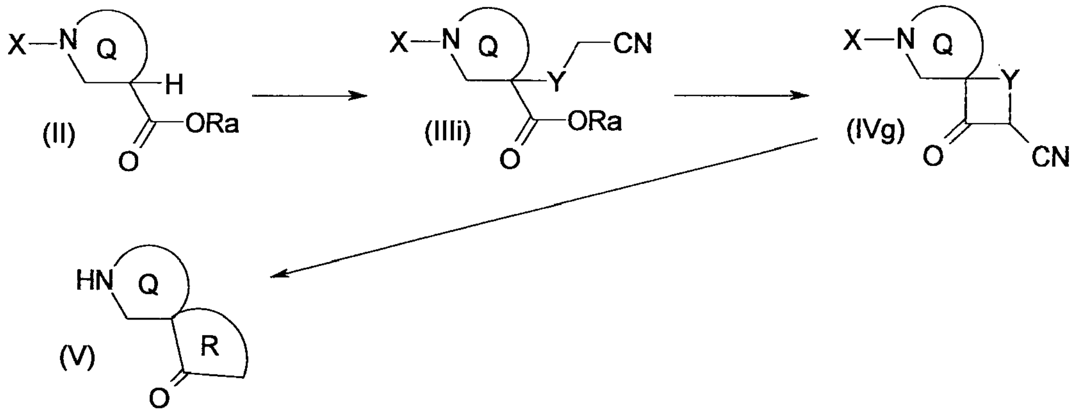

Compound (IIIi) can be produced by subjecting compound (II) to an alkylation reaction, according to the same method as in the production method of compound (IIIa) from compound (II) in the above-mentioned Reaction Scheme 1.

Compound (IVg) can be produced, for example, by subjecting compound (IIIi) to a cyclization reaction.
The cyclization reaction can be carried out according to a method known per se, for example, the method described in Journal of Medicinal Chemistry (J. Med. Chem.) pages 820-826, 1990, or the like, or a method analogous thereto.
This reaction is carried out by reacting compound (IIIi) with a base in an inert solvent.
Examples of the above-mentioned "base" include "hydrides of alkali metal or alkaline earth metal" and the like. The amount of the "base" to be used is generally 1 to 10 equivalents, preferably 1 to 1.5 equivalents, relative to compound (IIIi).
Examples of the above-mentioned "inert solvent" include nitrile solvents, aromatic solvents, aliphatic hydrocarbon solvents, ether solvents, amide solvents, halogenated hydrocarbon solvents and the like. These solvents may be used in a mixture of two or more kinds thereof at an appropriate ratio. Of these, amide solvents are preferable.
The reaction temperature is generally -100°C to 150°C, preferably 0°C to 100°C.
The reaction time is generally 5 min to 48 hr, preferably 30 min to 24 hr.

Compound (V) can also be produced, for example, by subjecting compound (IVg) to deprotection.

<Reaction Scheme 4>

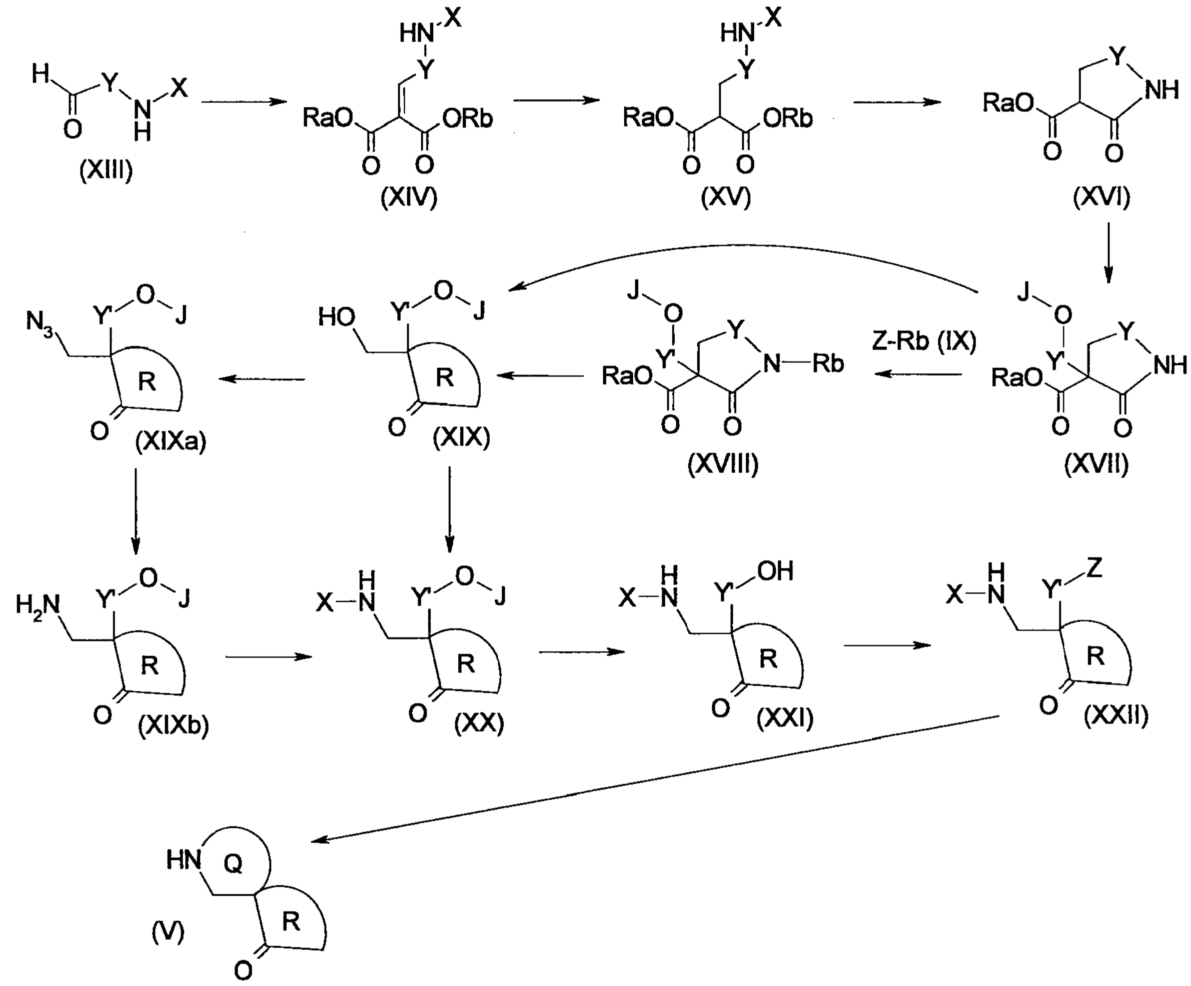


Compound (XIV) can be produced, for example, by subjecting compound (XIII) to a Knoevenagel reaction. This reaction can be carried out according to a method known per se, for example, the method described in Helvetica Chimica Acta (Hel. Chim. Acta) pages 450-465, 1983, or the like, or a method analogous thereto.

Compound (XIII) may be commercially available, or can be produced according to a method known per se or a method analogous thereto.

This reaction is carried out by reacting compound (XIII) with a malonic acid derivative in the presence of one or both of an acid and a base, in an inert solvent.

Examples of the above-mentioned "acid" include organic acids and Lewis acids. The amount of the "acid" to be used is generally 1 to 10 equivalents, preferably 1 to 1.5 equivalents, relative to compound (XIII).

Examples of the above-mentioned "organic acids" include acetic acid and the like.

Examples of the above-mentioned "Lewis acids" include titanium(IV) chloride and the like.

Preferable examples of the above-mentioned "base" include "aromatic amines", "secondary amines", "tertiary amines" and the like. The amount of the "base" to be used is generally 1 to 10 equivalents, preferably 1 to 1.5 equivalents, relative to compound (XIII).

Examples of the above-mentioned "inert solvent" include ether solvents, aromatic solvents, aliphatic hydrocarbon solvents and the like. These solvents may be used in a mixture of two or more kinds thereof at an appropriate ratio. Of these, ether solvents and the like are preferable.

The reaction temperature is generally -100°C to 150°C, preferably -78°C to 100°C.

The reaction time is generally 5 min to 48 hr, preferably 30 min to 24 hr.

Compound (XV) can be produced, for example, by subjecting compound (XIV) to a reduction reaction.

This reaction is carried out by reacting compound (XIV) in the presence of a metal catalyst and a hydrogen source, in an inert solvent.

Examples of the above-mentioned "metal catalyst" include palladium-carbon, palladium black, palladium chloride, platinum oxide, platinum black, platinum-palladium, Raney-nickel, Raney-cobalt and the like. The amount of the "metal catalyst" to be used is generally 0.001 to 1000 equivalents, preferably 0.01 to 100 equivalents, relative to compound (XIV). Examples of the above-mentioned "hydrogen source" include hydrogen gas, formic acid, amine salts of formic acid, phosphinates, hydrazine and the like.
Examples of the above-mentioned "inert solvent" include alcohol solvents, nitrile solvents, aromatic solvents, aliphatic hydrocarbon solvents, ether solvents, amide solvents, halogenated hydrocarbon solvents and the like. These solvents may be used in a mixture with water at an appropriate ratio. Of these, alcohol solvents are preferable.
The reaction temperature is generally -70 to 150°C, preferably -20 to 100°C.
The reaction time is generally 0.1 to 100 hr, preferably 0.1 to 40 hr.

Compound (XVI) can be produced, for example, by subjecting compound (XV) to deprotection to remove an amino-protecting group.

Compound (XVII) can be produced, for example, by subjecting compound (XVI) to an alkylation reaction.
The alkylation reaction can be carried out according to a method known per se, for example, the method described in Journal of Medicinal Chemistry (J. Med. Chem.) pages 2439-2441, 1998, or the like, or a method analogous thereto.
This reaction is carried out by reacting compound (XVI) with an alkylating agent in the presence of a base, in an inert solvent.
Examples of the above-mentioned "alkylating agent" include alkyl halides (e.g., benzyl 3-bromopropyl ether) and the like. The amount of the "alkylating agent" to be used is generally 1 to 5 equivalents, preferably 1 to 1.5 equivalents, relative to compound (XVI).
Examples of the above-mentioned "inert solvent" include ether solvents, aromatic solvents, aliphatic hydrocarbon solvents and the like. These solvents may be used in a mixture of two or more kinds thereof at an appropriate ratio. Of these, ether solvents and the like are preferable.
Examples of the above-mentioned "base" include "hydrides of alkali metal or alkaline earth metal", "metal amides", "alkyl metals", "aryl metals" and the like. The amount of the "base" to be used is generally 1 to 10 equivalents, preferably 1 to 1.5 equivalents, relative to compound (XVI).
The reaction temperature is generally -100°C to 150°C, preferably -78°C to 100°C.
The reaction time is generally 5 min to 48 hr, preferably 30 min to 24 hr.

Compound (XVIII) can be produced, for example, by subjecting compound (XVII) to an alkylation reaction.
The alkylation reaction can be carried out according to a method known per se, for example, the method described in Journal of Organic Chemistry (J. Org. Chem.) pages 2441-2450, 2004, or the like, or a method analogous thereto.
This reaction is carried out by reacting compound (XVII) with compound (IX) in the presence of a base, in an inert solvent.
The amount of compound (IX) to be used is generally 1 to 5 equivalents, preferably 1 to 1.5 equivalents, relative to compound (XVII).
Examples of the above-mentioned "inert solvent" include nitrile solvents, amide solvents, halogenated hydrocarbon solvents, ether solvents and the like. These solvents may be used in a mixture of two or more kinds thereof at an appropriate ratio. Of these, THF, DMF and the like are preferable.
Examples of the above-mentioned "base" include "hydrides of alkali metal or alkaline earth metal" and the like. The amount of the "base" to be used is generally 1 to 10 equivalents, preferably 1 to 1.5 equivalents, relative to compound (XVII).
The reaction temperature is generally -100°C to 150°C, preferably 0°C to 100°C.
The reaction time is generally 5 min to 48 hr, preferably 30 min to 24 hr.

Compound (XIX) can be produced, for example, by subjecting compound (XVII) to a reduction reaction.
This reaction is carried out by reacting compound (XVII) in the presence of a reducing agent, in an inert solvent.
Examples of the above-mentioned "reducing agent" include metal hydrogen compounds (e.g., sodium bis(2-methoxyethoxy)aluminum hydride, diisobutylaluminum hydride), metal hydrogen complex compounds (e.g., sodium borohydride, lithium borohydride, lithium aluminum hydride, sodium aluminum hydride) and the like. The amount of the "reducing agent" to be used is generally 0.1 to 20 equivalents, preferably 1 to 5 equivalents, relative to compound (XVII).
Examples of the above-mentioned "inert solvent" include alcohol solvents, aromatic solvents, aliphatic hydrocarbon solvents, ether solvents, ester solvents, amide solvents and the like. These solvents may be used in a mixture of two or more kinds thereof at an appropriate ratio.
The reaction temperature is generally -70 to 150°C, preferably -20 to 100°C.
The reaction time is generally 0.1 to 100 hr, preferably 0.1 to 40 hr.

Compound (XIX) can also be produced by subjecting compound (XVIII) to a reduction reaction, according to the same method as in the above-mentioned reduction reaction of compound (XVII).

Compound (XIXa) can be produced, for example, by subjecting compound (XIX) to a substitution reaction.
This reaction is carried out by converting compound (XIX) to an activated derivative thereof with a hydroxyl group-activator, and reacting the resulting compound with a nitrogen nucleophile in an inert solvent. Where necessary, the reaction may be carried out in the presence of 1 to 5 equivalents of a base and the like.

Examples of the above-mentioned "hydroxyl group-activator" include methanesulfonyl chloride, p-toluenesulfonyl chloride and the like. The amount of the hydroxyl group-activator to be used is generally 1 to 10 equivalents, preferably 1 to 1.5 equivalents, relative to compound (XIX).
Examples of the above-mentioned "nitrogen nucleophile" include sodium azide, lithium azide, diphenylphosphoryl azide and the like. The amount of the "nitrogen nucleophile" to be used is generally 1 to 10 equivalents, preferably 1 to 5 equivalents, relative to compound (XIX).
Examples of the above-mentioned "base" include "aromatic amines", "tertiary amines" and the like.
Examples of the above-mentioned "inert solvent" include aromatic solvents, aliphatic hydrocarbon solvents, ether solvents, ester solvents, amide solvents and the like. These solvents may be used in a mixture of two or more kinds thereof at an appropriate ratio.
The reaction temperature is generally -70 to 150°C, preferably -20 to 100°C.
The reaction time is generally 0.1 to 100 hr, preferably 0.1 to 40 hr.

Compound (XIXb) can be produced by subjecting compound (XIXa) to a reduction reaction, using the same method as in the reduction reaction of compound (IIIa) in the above-mentioned Reaction Scheme 1.

Compound (XX) can be produced, for example, by subjecting compound (XIX) to a substitution reaction.
This reaction is carried out by converting compound (XIX) to an activated derivative thereof with a hydroxyl group-activator, and reacting the resulting compound with a nitrogen nucleophile in an inert solvent. Where necessary, the reaction may be carried out in the presence of 1 to 5 equivalents of a base and the like.
Examples of the above-mentioned "hydroxyl group-activator" include cyanomethylene tri-n-butylphosphorane, a combination of diethyl azodicarboxylate and triphenylphosphine, and the like. The amount of the "hydroxyl group-activator" to be used is generally 1 to 10 equivalents, preferably 1 to 1.5 equivalents, relative to compound (XIX).
Examples of the above-mentioned "nitrogen nucleophile" include nitrobenzenesulfonamide, p-toluenesulfonamide and the like. The amount of the "nitrogen nucleophile" to be used is generally 1 to 10 equivalents, preferably 1 to 5 equivalents, relative to compound (XIX).
Examples of the above-mentioned "base" include "aromatic amines", "tertiary amines" and the like.
Examples of the above-mentioned "inert solvent" include aromatic solvents, aliphatic hydrocarbon solvents, ether solvents, ester solvents, amide solvents and the like. These solvents may be used in a mixture of two or more kinds thereof at an appropriate ratio.
The reaction temperature is generally -70 to 150°C, preferably -20 to 100°C.
The reaction time is generally 0.1 to 100 hr, preferably 0.1 to 40 hr.

Compound (XX) can also be produced, for example, by subjecting compound (XIXb) to a protection reaction.
The protection reaction can be carried out according to a method known per se, for example, the method described in Protective Groups in Organic Synthesis, John Wiley and Sons (1980), or the like.
Specifically, the protection reaction is carried out by a method using di-tert-butyl dicarbonate, a combination of benzyl chloroformate and triethylamine, a combination of acetic anhydride and pyridine, or the like.

Compound (XXI) can be produced, for example, by subjecting compound (XX) to deprotection.

Compound (XXII) can be produced, for example, by activating the hydroxyl group of compound (XXI).
This reaction is carried out by reacting compound (XXI) with a hydroxyl group-activator in the presence of a base, in an inert solvent.
Examples of the above-mentioned "hydroxyl group-activator" include methanesulfonyl chloride, p-toluenesulfonyl chloride and the like. The amount of the "hydroxyl group-activator" to be used is generally 1 to 10 equivalents, preferably 1 to 1.5 equivalents, relative to compound (XXI).
Preferable examples of the above-mentioned "base" include "aromatic amines", "tertiary amines" and the like. The amount of the "base" to be used is generally 1 to 10 equivalents, preferably 1 to 1.5 equivalents, relative to compound (XXI).
Examples of the above-mentioned "inert solvent" include aromatic solvents, aliphatic hydrocarbon solvents, ether solvents, ester solvents, amide solvents and the like. These solvents may be used in a mixture of two or more kinds thereof at an appropriate ratio.
The reaction temperature is generally -70 to 150°C, preferably -20 to 100°C.
The reaction time is generally 0.1 to 100 hr, preferably 0.1 to 40 hr.

Compound (V) can be produced by subjecting compound (XXII) to deprotection.
In this reaction condition, the ring closure reaction of ring Q simultaneously proceed.

<Reaction Scheme 5>

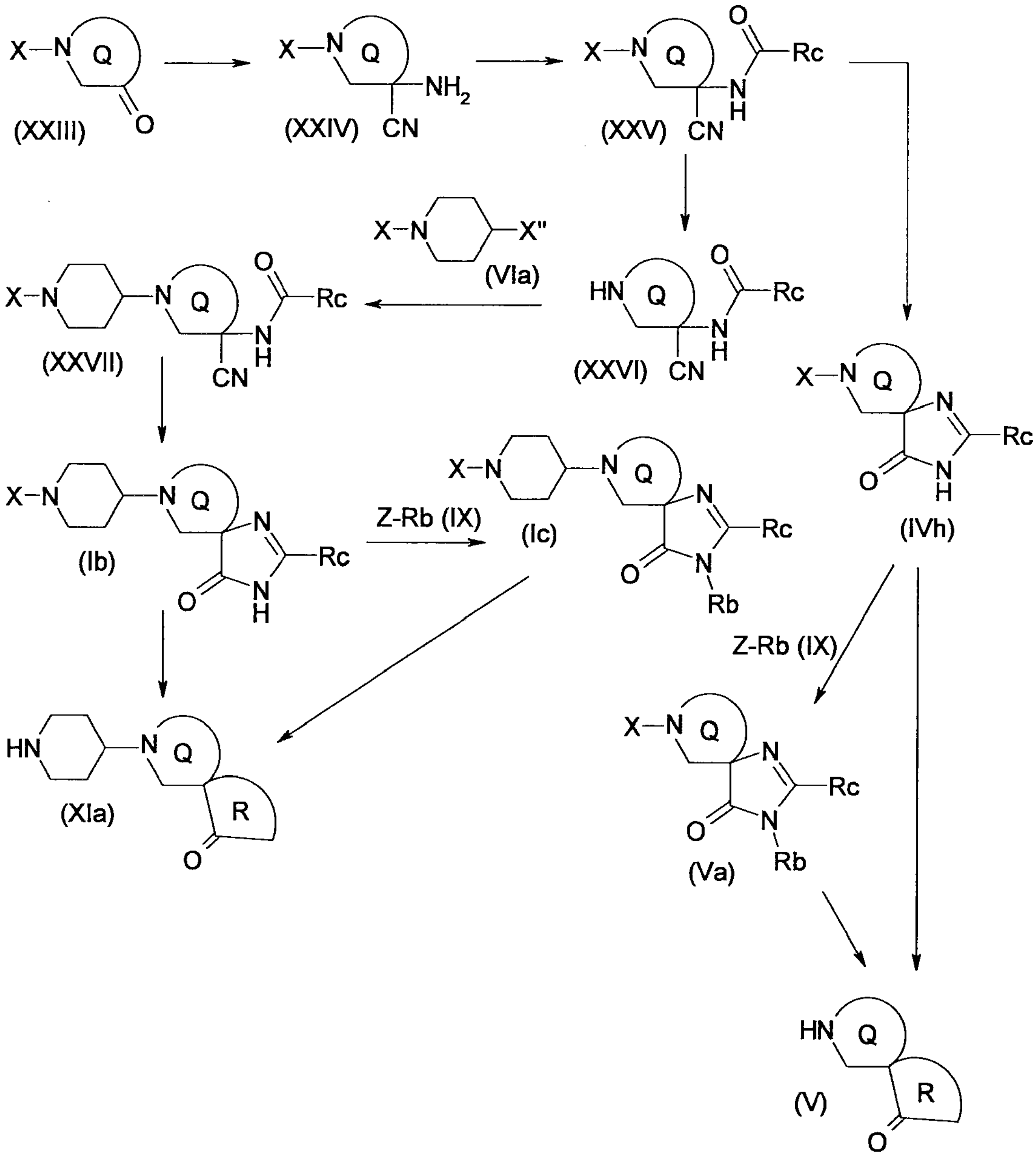


Compound (XXIV) can be produced, for example, by subjecting compound (XXIII) to the Strecker reaction. Compound (XXIII) may be commercially available, or can be produced according to a method known per se or a method analogous thereto.
The Strecker reaction can be carried out according to a method known per se, for example, the method described in Tetrahedron Letters (Tetrahedron Lett.) pages 3285-3288, 1986, or the like, or a method analogous thereto.
This reaction is carried out by reacting compound (XXIII) with ammonia and a cyanating agent in the presence of an acid, in an inert solvent.
Examples of the above-mentioned "cyanating agent" include sodium cyanide, potassium cyanide, trimethylsilyl cyanide and the like. The amount of the "cyanating agent" to be used is generally 1 to 10 equivalents, preferably 1 to 5 equivalents, relative to compound (XXIII).
Preferable examples of the above-mentioned "acid" include acetic acid, ammonium chloride and the like. The amount of the "acid" to be used is generally 1 to 10 equivalents, preferably 1 to 1.5 equivalents, relative to compound (XXIII).
Examples of the above-mentioned "inert solvent" include alcohol solvents, aromatic solvents, aliphatic hydrocarbon solvents, ether solvents, ester solvents, amide solvents and the like. These solvents may be used in a mixture of two or more kinds thereof at an appropriate ratio.

The reaction temperature is generally -70 to 150°C, preferably -20 to 100°C.
The reaction time is generally 0.1 to 100 hr, preferably 0.1 to 40 hr.

Compound (XXV) can be produced by subjecting compound (XXIV) to an amidation reaction, according to the same method as in the amidation reaction of compound (XIa) in the above-mentioned Reaction Scheme 1.

Compound (XXVI) can be produced, for example, by subjecting compound (XXV) to deprotection.

Compound (XXVII) can be produced by reacting compound (XXVI) with compound (VIa), according to the same method as in the production method of compound (Ia) from compound (V) and compound (VIa) in the above-mentioned Reaction Scheme 1.

Compound (Ib) can be produced, for example, by subjecting compound (XXVII) to a cyclization reaction. This reaction is carried out by reacting compound (XXVII) with a base in an inert solvent. Where necessary, the reaction may be carried out in the presence of a catalytic amount to 5 equivalents of a hydrogen peroxide.
Examples of the above-mentioned "base" include "inorganic bases" and the like. The amount of the "base" to be used is generally 1 to 10 equivalents, preferably 1 to 1.5 equivalents, relative to compound (XXVII).
Examples of the above-mentioned "inert solvent" include alcohol solvents, nitrile solvents, aromatic solvents, aliphatic hydrocarbon solvents, ether solvents, amide solvents, halogenated hydrocarbon solvents and the like. These solvents are preferably used in a mixture with water at an appropriate ratio. Of these, alcohol solvents containing water are preferable.
The reaction temperature is generally -78°C to 150°C, preferably -20°C to 100°C.
The reaction time is generally 5 min to 100 hr, preferably 30 min to 24 hr.

Compound (Ic) can be produced by subjecting compound (Ib) to an alkylation reaction, according to the same method as in the alkylation reaction of compound (IVa) in the above-mentioned Reaction Scheme 1.

Compound (XIa) can also be produced by subjecting compound (Ib) or compound (Ic) to deprotection.

Compound (IVh) can be produced by subjecting compound (XXV) to a cyclization reaction, according to the same method as in the above-mentioned cyclization reaction of compound (XXVII).

Compound (Va) can be produced by subjecting compound (IVh) to an alkylation reaction with compound (IX), according to the same method as in the alkylation reaction of compound (IVa) in the above-mentioned Reaction Scheme 1.

Compound (V) can be produced, for example, by subjecting compound (IVh) or compound (Va) to deprotection.

<Reaction Scheme 6>

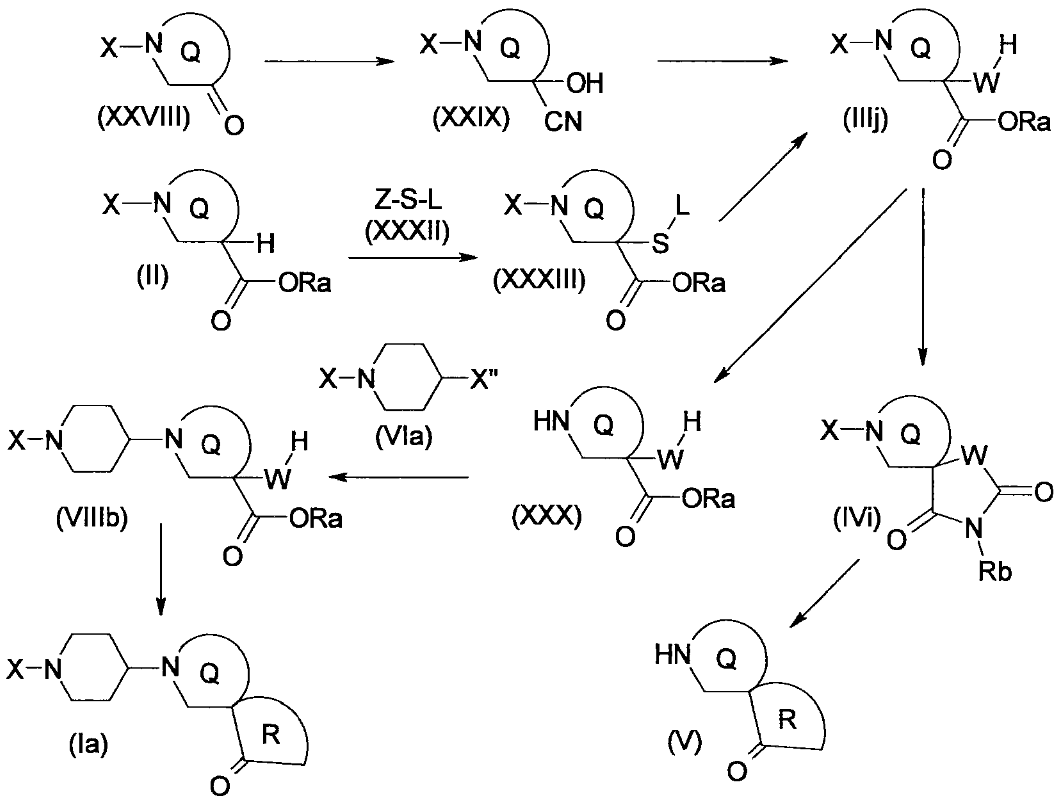


Compound (XXIX) can be produced, for example, by subjecting compound (XXVIII) to cyanation reaction. Compound (XXVIII) may be commercially available, or can be produced according to a method known per se or a method analogous thereto.
The cyanation reaction can be carried out according to a method known per se, for example, the method described in Journal of Medicinal Chemistry (J. Med. Chem.) pages 486-491, 1988, or the like, or a method analogous thereto.
This reaction is carried out by reacting compound (XXVIII) with a "cyanating agent" in an inert solvent. Where necessary, the reaction may be carried out in the presence of 1 to 10 equivalents of an acid.
Examples of the above-mentioned "cyanating agent" include sodium cyanide, potassium cyanide, trimethylsilyl cyanide and the like. The amount of the "cyanating agent" to be used is generally 1 to 10 equivalents, preferably 1 to 1.5 equivalents, relative to compound (XXVIII).
Examples of the above-mentioned "acid" include organic acids (e.g., formic acid, acetic acid), Lewis acids (e.g., aluminum chloride, boron trifluoride-diethyl etherate, zinc iodide) and the like.
Examples of the above-mentioned "inert solvent" include nitrile solvents, amide solvents, halogenated hydrocarbon solvents, ether solvents and the like. These solvents may be used in a mixture of two or more kinds thereof at an appropriate ratio. Of these, halogenated hydrocarbon solvents are preferable.
The reaction temperature is generally -100°C to 150°C, preferably 0°C to 100°C.
The reaction time is generally 5 min to 48 hr, preferably 30 min to 24 hr.
Compound (XXXIII) can be produced, for example, by reacting compound (II) with compound (XXXII). Compound (XXXII) may be commercially available, or can be produced according to a method known per se or a method analogous thereto.
This reaction is carried out by reacting compound (II) with compound (XXXII) in the presence of a base, in an inert solvent. The amount of compound (XXXII) to be used is generally 1 to 5 equivalents, preferably 1 to 1.5 equivalents, relative to compound (II).
Examples of the above-mentioned "inert solvent" include ether solvents, aromatic solvents, aliphatic hydrocarbon solvents and the like. These solvents may be used in a mixture of two or more kinds thereof at an appropriate ratio. Of these, ether solvents and the like are preferable.
Examples of the above-mentioned "base" include "hydrides of alkali metal or alkaline earth metal", "metal amides", "alkyl

metals", "aryl metals" and the like. The amount of the "base" to be used is generally 1 to 10 equivalents, preferably 1 to 1.5 equivalents, relative to compound (II).
The reaction temperature is generally -100°C to 150°C, preferably -78°C to 100°C.
The reaction time is generally 5 min to 48 hr, preferably 30 min to 24 hr.

Compound (IIIj) can be produced, for example, by subjecting compound (XXIX) to hydrolysis.
This reaction is carried out by reacting compound (XXIX) with a base or an acid, in an inert solvent.
Examples of the above-mentioned "base" include "inorganic bases" and the like. The amount of the "base" to be used is generally 1 to 10 equivalents, preferably 1 to 1.5 equivalents, relative to compound (XXIX).
Examples of the above-mentioned "acid" include organic acids, hydrochloric acid, sulfuric acid and the like. The amount of the "acid" to be used is generally 1 to 50 equivalents, preferably 1 to 10 equivalents, relative to compound (XXIX). These "acid" may be used as a solvent. Examples of the "organic acids" include formic acid, acetic acid and the like.
Examples of the above-mentioned "inert solvent" include alcohol solvents, nitrile solvents, aromatic solvents, aliphatic hydrocarbon solvents, ether solvents, amide solvents, halogenated hydrocarbon solvents and the like. These solvents are preferably used in a mixture with water at an appropriate ratio. Of these, alcohol solvents containing water are preferable.
The reaction temperature is generally -78°C to 150°C, preferably -20°C to 100°C.
The reaction time is generally 5 min to 100 hr, preferably 30 min to 24 hr.
When a solvent other than "alcohol solvent" is used as the above-mentioned "inert solvent", compound (IIIj) can be produced by subjecting the resulting compound to esterification according to a method known per se.

Compound (IIIj) can also be produced, for example, by subjecting compound (XXXIII) to deprotection.

Compound (IVi) can be produced by subjecting compound (IIIj) to a cyclization reaction.
This reaction is carried out by reacting compound (IIIj) with an isocyanate (hereinafter sometimes to be referred to as an ester of isocyanic acid; e.g., ethylisocyanate, isopropylisocyanate) in the presence of a base, in an inert solvent. The amount of the isocyanate to be used is generally 1 to 5 equivalents, preferably 1 to 2 equivalents, relative to compound (IIIj).
Examples of the above-mentioned "base" include "hydrides of alkali metal or alkaline earth metal" and the like. The amount of the "base" to be used is generally 0.5 to 10 equivalents, preferably 0.5 to 1.5 equivalents, relative to compound (IIIj).
Examples of the above-mentioned "inert solvent" include nitrile solvents, amide solvents, halogenated hydrocarbon solvents, ether solvents and the like. These solvents may be used in a mixture of two or more kinds thereof at an appropriate ratio. Of these, THF is preferable.
The reaction temperature is generally -78°C to 50°C, preferably room temperature to 50°C.
The reaction time is generally 5 min to 48 hr, preferably 30 min to 24 hr.

Compound (XXX) can be produced, for example, by subjecting compound (IIIj) to deprotection.

Compound (VIIIb) can be produced by reacting compound (XXX) with compound (VIa), according to the same method as in the production method of compound (Ia) from compound (V) and compound (VIa) in the above-mentioned Reaction Scheme 1.

Compound (V) can also be produced, for example, by compound (IVi) to deprotection.

Compound (Ia) can also be produced by subjecting compound (VIIIb) to a cyclization reaction, according to the same method as in the above-mentioned cyclization reaction of compound (IIIj).

<Reaction Scheme 7>

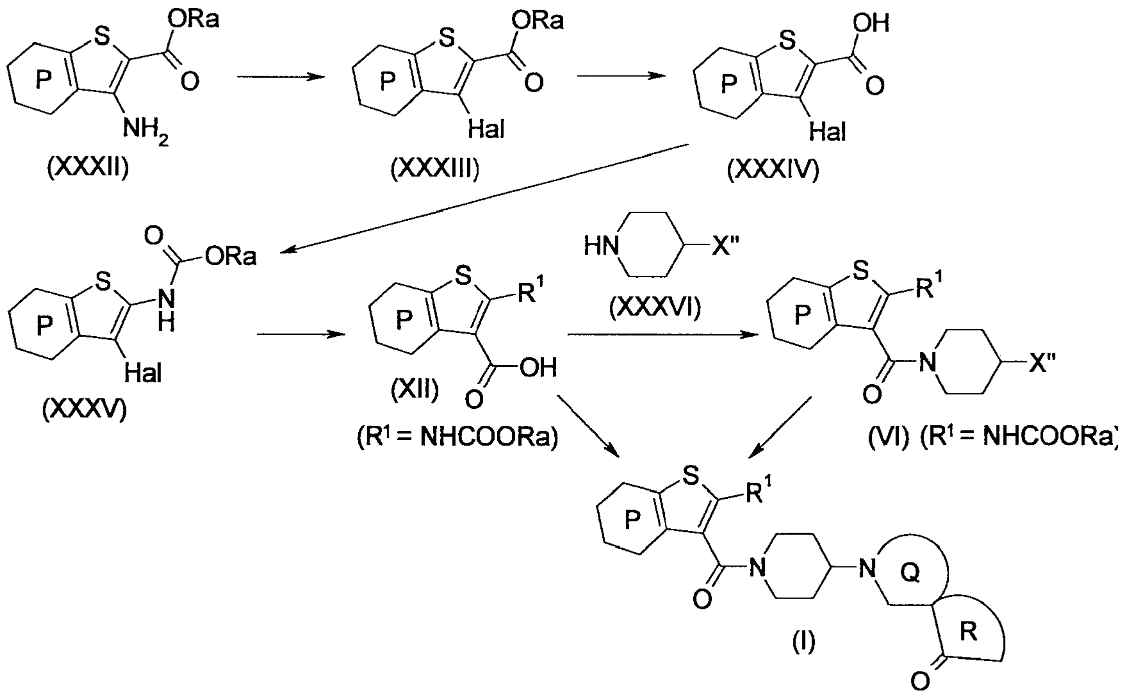

Compound (XXXIII) can be produced, for example, by compound (XXXII) to halogenation.
This reaction is carried out by reacting compound (XXXII) in the presence of a nitrite and a copper halide, in an inert solvent. Compound (XXXII) used for this reaction can be produced, for example, according to the method described in WO2004/000846, or a method analogous thereto.
Examples of the above-mentioned "nitrite" include sodium nitrite, potassium nitrite, tert-butyl nitrite, isoamyl nitrite and the like. The amount of the "nitrite" to be used is generally 1 to 3 equivalents, preferably 1 to 2 equivalents, relative to compound (XXXII).
Examples of the above-mentioned "copper halide" include copper(I) bromide, copper(II) bromide, copper(I) chloride and the like. The amount of the "copper halide" to be used is generally 0.5 to 3 equivalents, preferably 0.5 to 1.5 equivalents, relative to compound (XXXII).
Examples of the above-mentioned "inert solvent" include nitrile solvents, ether solvents, amide solvents, water, a mixture of two or more kinds thereof, and the like.
The reaction temperature is generally -78°C to 50°C, preferably -20°C to 10°C.
The reaction time is generally 5 min to 100 hr, preferably 30 min to 10 hr.

Compound (XXXIV) can be produced by subjecting compound (XXXIII) to hydrolysis and the like, using the same method as the hydrolysis of compound (IIIf) in the above-mentioned Reaction Scheme 2.

Compound (XXXV) can be produced, for example, by directly subjecting compound (XXXIV), or a reactive derivative thereof (e.g., an acid halide, an acid amide, an acid anhydride, an ester etc.) and the like, which are obtained by converting compound (XXXIV), to a rearrangement reaction.
Examples of the above-mentioned "rearrangement reaction" include Curtius rearrangement, Hofmann rearrangement, Schmidt rearrangement and the like.
The rearrangement reaction using diphenylphosphoryl azide is exemplified below.
The amount of the diphenylphosphoryl azide to be used is generally 1 to 3 equivalents, preferably 1 to 1.5 equivalents, relative to compound (XXXIV).
Where necessary, the reaction may be carried out in the presence of a base.
Preferable examples of the above-mentioned "base" include "aromatic amines", "tertiary amines" and the like.
This reaction is advantageously carried out in a solvent inert to the reaction. While the solvent is not particularly limited as long as the reaction proceeds, for example, alcohol solvents are preferable.
The reaction time is generally about 10 min to about 48 hr, preferably about 15 min to about 24 hr.
The reaction temperature is generally -20°C to 200°C, preferably 0°C to 150°C.
The method described in "Jikken Kagaku Kouza (The Chemical Society of Japan ed.), 4th Edition, Vol. 20, pages 304

and 477-479, or a method analogous thereto, or the like is used as conditions of the other reaction.

Compound (XII) can be produced by reacting compound (XXXV) with an "alkyl metals" or "aryl metals", and reacting the obtained activated compound with a carbon dioxide.

The amount of the "alkyl metals" or "aryl metals" to be used is generally 1 to 2 equivalents, preferably 1 to 1.5 equivalents, relative to compound (XXXV).

The carbon dioxide is generally used in an excess amount.

This reaction is advantageously carried out in a solvent inert to the reaction. While the solvent is not particularly limited as long as the reaction proceeds, for example, aromatic solvents, aliphatic hydrocarbon solvents, ether solvents, a mixture thereof and the like are preferable.

The reaction time is generally 10 min to 48 hr, preferably 15 min to 24 hr.

The reaction temperature is generally -78°C to 100°C, preferably -78°C to 50°C.

Compound (XII) can also be produced by reacting compound (XXXV) with a transition metal catalyst A in an inert solvent.

Examples of the above-mentioned "transition metal catalyst A" include palladium catalysts, nickel catalysts, iron catalysts, cobalt catalysts and the like. Examples of the "palladium catalyst" include dichlorobis(benzonitrile)palladium and the like. Examples of the "nickel catalyst" include tetracarbonylnickel (0) and the like. Examples of the "iron catalyst" include disodium tetracarbonylferrate and the like. Examples of the "cobalt catalyst" include dicobalt octacarbonyl and the like.

The amount of the "transition metal catalyst A" to be used is generally about 0.01 to 1 equivalents, preferably about 0.01 to 0.5 equivalents, relative to compound (XXXV).

This reaction can be carried out under carbon monoxide atmosphere.

Examples of the above-mentioned "inert solvent" include aromatic solvents, aliphatic hydrocarbon solvents, amide solvents, sulfoxide solvents and the like. These solvents may be used in a mixture of two or more kinds thereof at an appropriate ratio.

The reaction temperature is generally 0°C to 200°C, preferably 0°C to 150°C.

The reaction time is generally 1 hr to 48 hr, preferably 2 hr to 24 hr.

Compound (VI) can be produced by subjecting compound (XII) to an amidation reaction with compound (XXXVI), according to the same method as in the amidation reaction of compound (XIa) in the above-mentioned Reaction Scheme 1.

Compound (XXXVI) may be commercially available, or can be produced according to a method known per se or a method analogous thereto.

Compound (I) can be produced from compound (XII) or compound (VI), according to the above-mentioned Reaction Scheme 1.

<Reaction Scheme 8>

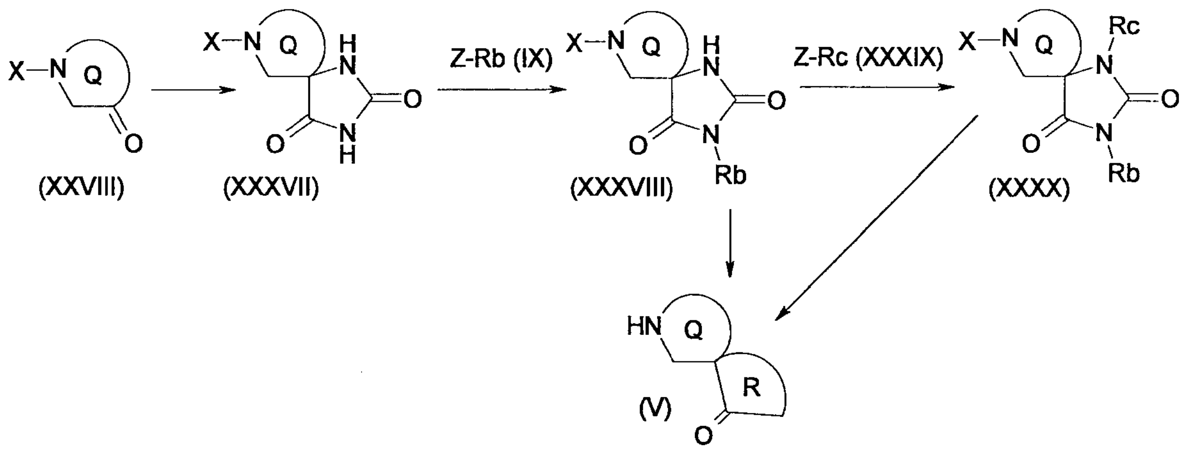


Compound (XXXVII) can be produced, for example, from compound (XXVIII) according to the method described in WO2006/053024A2 or a method analogous thereto.

Compound (XXXVIII) can be produced by subjecting compound (XXXVII) to an alkylation reaction with compound (IX), according to the same method as in the alkylation reaction of compound (IVa) in the above-mentioned Reaction Scheme 1.

Compound (XXXX) can be produced by subjecting compound (XXXVIII) to an alkylation reaction with compound (XXXIX), according to the same method as in the alkylation reaction of compound (IVa) in the above-mentioned Reaction

Scheme 1.
Compound (XXXIX) may be commercially available, or can be produced according to a method known per se or a method analogous thereto.
Compound (V) can also be produced, for example, by subjecting compound (XXXVIII) or compound (XXXX) to deprotection.
In compound (I) thus obtained, a functional group within a molecule can also be converted to a desired functional group by a combination of chemical reactions known per se. Examples of the chemical reaction here include oxidation reaction, reduction reaction, alkylation reaction, acylation reaction, ureation reaction, hydrolysis reaction, amination reaction, esterification reaction, aryl coupling reaction, deprotection reaction and the like.
In the above-mentioned production methods, when the starting compound has an amino group, a carboxyl group, a hydroxy group, a carbonyl group or a mercapto group as a substituent, a protecting group generally used in peptide chemistry and the like may be introduced into these groups. By removing the protecting group as necessary after the reaction, the object compound can be obtained.
Examples of the amino-protecting group include a formyl group, a $C_{1-6}$ alkyl-carbonyl group, a $C_{1-6}$ alkoxy-carbonyl group, a benzoyl group, a $C_{7-10}$ aralkyl-carbonyl group (e.g., benzylcarbonyl), a $C_{7-14}$ aralkyloxy-carbonyl group (e.g., benzyloxycarbonyl, 9-fluorenylmethoxycarbonyl), a trityl group, a phthaloyl group, a N,N-dimethylaminomethylene group, a substituted silyl group (e.g., trimethylsilyl, triethylsilyl, dimethylphenylsilyl, tert-butyldimethylsilyl, tert-butyldiethylsilyl), a $C_{2-6}$ alkenyl group (e.g., 1-allyl) and the like. These groups are optionally substituted by 1 to 3 substituents selected from a halogen atom, a $C_{1-6}$ alkoxy group and a nitro group.
Examples of the carboxyl-protecting group include a $C_{1-6}$ alkyl group, a $C_{7-10}$ aralkyl group (e.g., benzyl), a phenyl group, a trityl group, a substituted silyl group (e.g., trimethylsilyl, triethylsilyl, dimethylphenylsilyl, tert-butyldimethylsilyl, tert-butyldiethylsilyl), a $C_{2-6}$ alkenyl group (e.g., 1-allyl) and the like. These groups are optionally substituted by 1 to 3 substituents selected from a halogen atom, a $C_{1-6}$ alkoxy group and a nitro group.
Examples of the hydroxy-protecting group include a $C_{1-6}$ alkyl group, a phenyl group, a trityl group, a $C_{7-10}$ aralkyl group (e.g., benzyl), a formyl group, a $C_{1-6}$ alkyl-carbonyl group, a benzoyl group, a $C_{7-10}$ aralkyl-carbonyl group (e.g., benzylcarbonyl), a 2-tetrahydropyranyl group, a 2-tetrahydrofuranyl group, a substituted silyl group (e.g., trimethylsilyl, triethylsilyl, dimethylphenylsilyl, tert-butyldimethylsilyl, tert-butyldiethylsilyl), a $C_{2-6}$ alkenyl group (e.g., 1-allyl) and the like. These groups are optionally substituted by 1 to 3 substituents selected from a halogen atom, a $C_{1-6}$ alkyl group, a $C_{1-6}$ alkoxy group and a nitro group.
Examples of the carbonyl-protecting group include a cyclic acetal (e.g., 1,3-dioxane), a non-cyclic acetal (e.g., a di-$C_{1-6}$ alkylacetal) and the like.
Examples of the mercapto-protecting group include a $C_{1-6}$ alkyl group, a phenyl group, a trityl group, a $C_{7-10}$ aralkyl group (e.g., benzyl), a $C_{1-6}$ alkyl-carbonyl group, a benzoyl group, a $C_{7-10}$ aralkyl-carbonyl group (e.g., benzylcarbonyl), a $C_{1-6}$ alkoxy-carbonyl group, a $C_{6-14}$ aryloxy-carbonyl group (e.g., phenyloxycarbonyl), a $C_{7-14}$ aralkyloxy-carbonyl group (e.g., benzyloxycarbonyl, 9-fluorenylmethoxycarbonyl), a 2-tetrahydropyranyl group, a $C_{1-6}$ alkylamino-carbonyl group (e.g., methylaminocarbonyl, ethylaminocarbonyl), and the like. These groups are optionally substituted by 1 to 3 substituents selected from a halogen atom, a $C_{1-6}$ alkyl group, a $C_{1-6}$ alkoxy group and a nitro group.
The above-mentioned protecting group can be removed according to deprotection known per se.
Compound (I) obtained by the above-mentioned production methods can be isolated and purified by a known means, for example, solvent extraction, liquid conversion, phase transfer, crystallization, recrystallization, chromatography and the like.
When compound (I) contains an optical isomer, a stereoisomer, a regioisomer or a rotamer, these are also encompassed in compound (I), and can be obtained as a single product according to synthesis and separation methods known *per se.* For example, when compound (I) has an optical isomer, an optical isomer resolved from this compound is also encompassed in compound (I).
The optical isomer can be produced by a method known per se.
Compound (I) may be a crystal.
Crystals of compound (I) (hereinafter sometimes to be abbreviated as the crystals of the present invention) can be produced by crystallization according to crystallization methods known per *se.*
In the present specification, the melting point means that measured using, for example, a micromelting point apparatus (Yanako, MP-500D or Buchi, B-545), a DSC (differential scanning calorimetry) device (SEIKO, EXSTAR6000) or the like. In general, the melting points vary depending on the measurement apparatuses, the measurement conditions and the like. The crystal in the present specification may show different values from the melting point described in the present specification, as long as they are within each of a general error range.
The crystal of the present invention is superior in physicochemical properties (e.g., melting point, solubility, stability) and biological properties (e.g., pharmacokinetics (absorption, distribution, metabolism, excretion), efficacy expression), and thus it is extremely useful as a medicament.

## Examples

The present invention is explained in detail in the following by referring to Reference Examples, Examples, Experimental Examples and Preparation Examples, which are not to be construed as limitative. In addition, the present invention may be modified without departing from the scope of the invention.

Reference Example 1

1-tert-butyl 3-ethyl 3-(cyanomethyl)piperidine-1,3-dicarboxylate
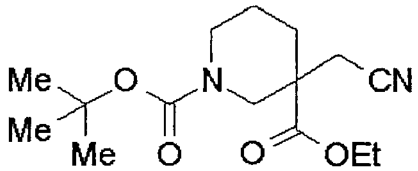

To a solution of 1-tert-butyl 3-ethyl piperidine-1,3-dicarboxylate (10.0 g, 38.9 mmol) in THF (100 mL) was added a 1.1 M solution (50.0 mL, 55.0 mmol) of lithium bis(trimethylsilyl)amide in THF at -78°C, and the mixture was stirred at the same temperature for 30 min. To this solution was added bromoacetonitrile (3.20 mL, 46.7 mmol) at -78°C, and the mixture was heated to room temperature and stirred for 30 min. The reaction mixture was dissolved in ethyl acetate, and the solution was washed with saturated aqueous ammonium chloride solution and saturated brine, and dried over anhydrous magnesium sulfate. The solvent was evaporated under reduced pressure, and the obtained residue was purified by silica gel column chromatography (hexane:ethyl acetate=4:1 to 1:1) to give the title compound (4.15 g, yield 36%) as an oil.

Reference Example 2

1'-tert-butyl 3-ethyl 3-(cyanomethyl)-1,4'-bipiperidine-1',3-dicarboxylate
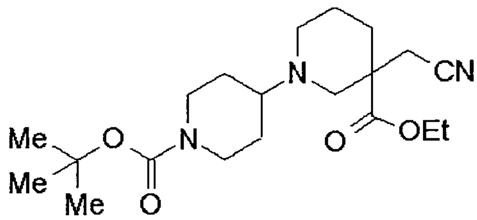

1-tert-Butyl 3-ethyl 3-(cyanomethyl)piperidine-1,3-dicarboxylate (3.09 g, 10.4 mmol) obtained in Reference Example 1 was dissolved in 4N hydrogen chloride-ethyl acetate (30 mL), and the solution was stirred at room temperature for 30 min. The reaction solution was concentrated under reduced pressure, and the obtained residue was dissolved in dichloromethane (30 mL). To this solution were added triethylamine (1.5 mL, 1.04 mmol), 1-(tert-butoxycarbonyl)-4-piperidone (2.08 g, 10.4 mmol) under ice-cooling, and then sodium triacetoxyborohydride (6.62 g, 31.2 mmol) after stirring for about 30 min, and the mixture was stirred at room temperature for 16 hr. The reaction mixture was diluted with ethyl acetate, washed successively with saturated aqueous sodium hydrogen carbonate solution and saturated brine, and dried over anhydrous sodium sulfate. The solvent was evaporated under reduced pressure and the obtained residue was purified by silica gel column chromatography (ethyl acetate:hexane=1:9 to 3:7) to give the title compound (2.64 g, yield 67%) as an oil.

Reference Example 3

optically active forms (two kinds) of 1'-tert-butyl 3-ethyl 3-(cyanomethyl)-1,4'-bipiperidine-1',3-dicarboxylate
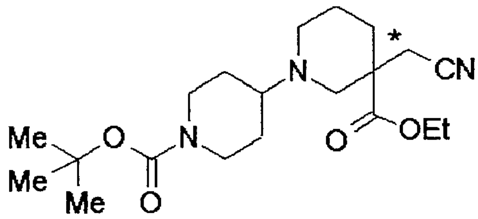

1'-tert-Butyl 3-ethyl 3-(cyanomethyl)-1,4'-bipiperidine-1',3-dicarboxylate (racemate) (6.20 g) obtained in Reference Example 2 was optically resolved by high performance liquid chromatography (HPLC) under the following conditions to give two kinds of optically active forms having "long retention time (3.03 g)" and "short retention time (3.03 g)".
specific optical rotation of "short retention time":
$[\alpha]_D^{20}$+21.1° (c 0.846, methanol).

<preparative HPLC conditions>

column: CHIRALPAK AD (50 mmID×500 mmL)
mobile phase: hexane/ethanol=9/1
flow rate: 80 mL/min
column temperature: 35°C
detection: UV 220 nm
compound injection volume: 2.6 g/220 mL (hexane:ethanol=9:1)

<HPLC analysis conditions>

column: CHIRALPAK AD-H (4.6 mmID×250 mmL)
mobile phase: hexane:ethanol=9:1
flow rate: 1.0 mL/min
column temperature: 30°C
detection: UV 220 nm
retention time of "long retention time": 13.75 min
retention time of "short retention time": 6.88 min

Reference Example 4

optically active form of tert-butyl 4-(1-oxo-2,7-diazaspiro[4.5]dec-7-yl)piperidine-1-carboxylate
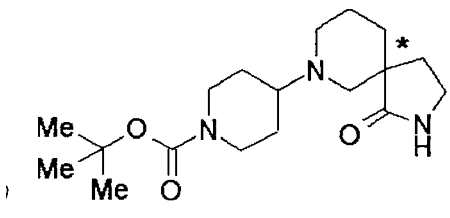

To a solution of 1'-tert-butyl 3-ethyl 3-(cyanomethyl)-1,4'-bipiperidine-1',3-dicarboxylate in an optically active form (long retention time, 3.83 g, 10.1 mmol) obtained in Reference Example 3 in isopropanol (30 mL) were added Raney-nickel (5 g, manufactured by Kawaken Fine Chemicals) and 25% aqueous ammonia solution (5 mL), and the mixture was stirred under a hydrogen atmosphere (0.5 MPa) at 50°C for 2 hr. The reaction mixture was filtered, and the filtrate was concentrated under reduced pressure. The obtained residue was purified by silica gel column chromatography (ethyl acetate to methanol:ethyl acetate=1:4) to give the title compound (3.00 g, yield 88%) as an oil.

Reference Example 5

optically active form of tert-butyl 4-(2-isopropyl-1-oxo-2,7-diazaspiro[4.5]dec-7-yl)piperidine-1-carboxylate

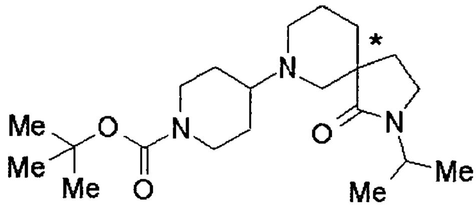

To a solution of tert-butyl 4-(1-oxo-2,7-diazaspiro[4.5]dec-7-yl)piperidine-1-carboxylate in an optically active form (4.22 g, 12.5 mmol) obtained in Reference Example 4 in DMF (30 mL) was added 60% sodium hydride (0.5 g, 12.5 mmol), and the mixture was stirred at room temperature for 15 min. 2-Iodopropane (1.38 mL, 13.8 mmol) was added to the reaction mixture and the mixture was heated at 90°C for 45 min. 60% Sodium hydride (0.5 g, 12.5 mmol) was added, and the mixture was stirred at the same temperature for 5 min. 2-Iodopropane (1.38 mL, 13.8 mmol) was added and the mixture was stirred for 30 min. This operation (sodium hydride and 2-iodopropane were added and the mixture was heated at 90°C for 30 min) was repeated twice, and the reaction mixture was diluted with ethyl acetate, washed 3 times with saturated brine, and dried over anhydrous sodium sulfate. The solvent was evaporated under reduced pressure, and the obtained residue was purified by silica gel column chromatography (ethyl acetate to methanol:ethyl acetate=1:4) to give the title compound (2.84 g, yield 60%) as a yellow solid.

Reference Example 6

1-tert-butyl 3-ethyl 3-[2-(benzyloxy)-2-oxoethyl]piperidine-1,3-dicarboxylate
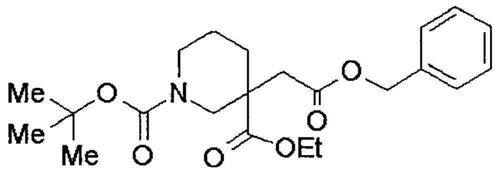

In the same manner as in Reference Example 1 and using 1-tert-butyl 3-ethyl piperidine-1,3-dicarboxylate (10.0 g, 38.9 mmol) and benzyl bromoacetate (7.40 mL, 46.6 mmol), the title compound (12.3 g, yield 78%) was obtained as an oil.
EI(pos) 428 $[M+Na]^+$

Reference Example 7

1'-tert-butyl 3-ethyl 3-[2-(benzyloxy)-2-oxoethyl]-1,4'-bipiperidine-1',3-dicarboxylate
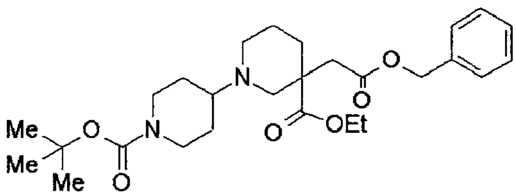

To 1-tert-butyl 3-ethyl 3-[2-(benzyloxy)-2-oxoethyl]piperidine-1,3-dicarboxylate (16.4 g, 40.4 mmol) obtained in Reference Example 6 was added 4N hydrogen chloride-ethyl acetate (200 mL), and the mixture was stirred for 1 hr. The solvent was evaporated under reduced pressure, toluene (50 mL) was added to the obtained residue, and the

mixture was concentrated under reduced pressure, which operation was performed 3 times. The obtained residue was dissolved in tetrahydrofuran (200 mL), acetic acid (20 mL) and triethylamine (8.44 mL, 60.7 mmol) were added thereto, tert-butyl 4-oxopiperidine-1-carboxylate (8.86 g, 44.5 mmol) and sodium triacetoxyborohydride (12.9 g, 60.7 mmol) were successively added under ice-cooling, and the mixture was stirred at room temperature for 16 hr. The solvent was evaporated under reduced pressure, and the residue was diluted with ethyl acetate, and the mixture was washed with aqueous potassium carbonate solution and saturated brine, and dried over anhydrous sodium sulfate. The solvent was evaporated under reduced pressure, and the obtained residue was purified by silica gel column chromatography (hexane: ethyl acetate=9:1 to 1:1) to give the title compound (13.8 g, yield 70%) as an oil.
$^1H$ NMR ($CDCl_3$) δ1.17 (3H, t, J = 6.9 Hz), 1.29-1.39 (2H, m), 1.45 (9H, s), 1.52-1.66 (5H, m), 1.82-1.87 (1H, m), 2.32-2.44 (2H, m), 2.53-2.95 (7H, m), 4.03-4.15 (4H, m), 5.05 (2H, m), 7.31 (5H, m).

Reference Example 8

optically active forms (two kinds) of 1'-tert-butyl 3-ethyl 3-[2-(benzyloxy)-2-oxoethyl]-1,4'-bipiperidine-1',3-dicarboxylate

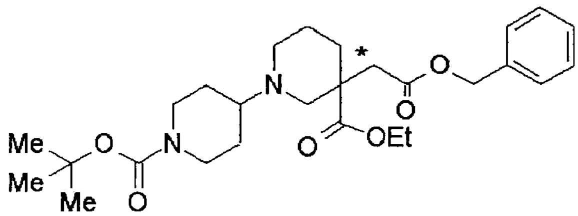

1'-tert-Butyl 3-ethyl 3-[2-(benzyloxy)-2-oxoethyl]-1,4'-bipiperidine-1',3-dicarboxylate (racemate) (10.3 g) obtained in Reference Example 7 was optically resolved by HPLC under the following conditions to give two kinds of optically active forms having "long retention time (4.80 g)" and "short retention time (4.50 g)".
specific optical rotation of "short retention time":
$[\alpha]_D^{20}$+10.7° (c 1. 150, methanol).

<preparative HPLC conditions>

column: CHIRALPAK AD (50 mmID×500 mmL)
mobile phase: hexane:ethanol=9:1
flow rate: 80 mL/min
column temperature: 35°C
detection: UV 220 nm
compound injection volume: 1.0 g/80 mL (hexane:ethanol=9:1)

<HPLC analysis conditions>

column: CHIRALPAK AD-H (4.6 mmID×250 mmL)
mobile phase: hexane:ethanol=9:1
flow rate: 1.0 mL/min
column temperature: 35°C
detection: UV 220 nm
retention time of "long retention time": 10.46 min
retention time of "short retention time": 6.64 min

Reference Example 9

optically active form of [1'-(tert-butoxycarbonyl)-3-(ethoxycarbonyl)-1,4'-bipiperidin-3-yl]acetic acid

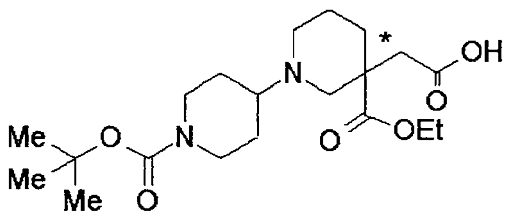

Ethanol (50 mL) was added to 1'-tert-butyl 3-ethyl 3-[2-(benzyloxy)-2-oxoethyl]-1,4'-bipiperidine-1',3-dicarboxylate in an optically active form (long retention time) (4.80 g, 9.82 mmol) obtained in Reference Example 8 and 10% palladium carbon (50% water-containing product, 3.0 g), and the mixture was stirred under a hydrogen atmosphere for 16 hr. The reaction mixture was filtered, and the filtrate was concentrated under reduced pressure to give the title compound (3.68 g, yield 94%) as an oil. The obtained compound was used in the next step without purification.

Reference Example 10

optically active form of 1'-tert-butyl 3-ethyl 3-[2-(isopropylamino)-2-oxoethyl]-1,4'-bipiperidine-1',3-dicarboxylate

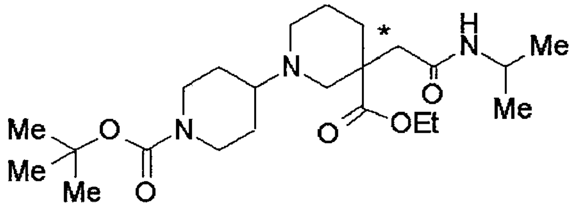

To a solution of [1'-(tert-butoxycarbonyl)-3-(ethoxycarbonyl)-1,4'-bipiperidin-3-yl]acetic acid in an optically active form (3.68 g, 9.23 mmol) obtained in Reference Example 9, isopropylamine (1.18 mL, 13.9 mmol) and 1-hydroxybenzotriazole (1.25 g, 9.23 mmol) in DMF (10 mL) was added 1-ethyl-3-(3'-dimethylaminopropyl)carbodiimide hydrochloride (1.77 g, 9.23 mmol), and the mixture was stirred at room temperature for 16 hr. The reaction mixture was diluted with ethyl acetate, and the mixture was washed with 10% aqueous potassium carbonate solution and saturated brine, and dried over anhydrous sodium sulfate. The solvent was evaporated under reduced pressure, and the obtained residue was purified by silica gel column chromatography (hexane:ethyl acetate=3:1 to ethyl acetate) to give the title compound (4.06 g, quantitatively) as an oil.

Reference Example 11

optically active form of tert-butyl 4-(2-isopropyl-1,3-dioxo-2,7-diazaspiro[4.5]dec-7-yl)piperidine-1-carboxylate

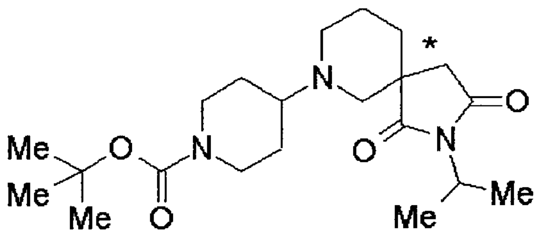

To a solution of 1'-tert-butyl 3-ethyl 3-[2-(isopropylamino)-2-oxoethyl]-1,4'-bipiperidine-1',3-dicarboxylate in an optically active form (4.06 g, 9.24 mmol) obtained in Reference Example 10 in DMF (20 mL) was added 60% sodium hydride (369 mg, 9.24 mmol) under ice-cooling, and the mixture was stirred at room temperature for 3 hr. The reaction

mixture was diluted with ethyl acetate, and the mixture was washed with saturated brine, and dried over anhydrous sodium sulfate. The solvent was evaporated under reduced pressure, and the obtained residue was purified by silica gel column chromatography (hexane:ethyl acetate=3:1 to ethyl acetate) to give the title compound (2.54 g, yield 70%) as an oil.

Reference Example 12

optically active form of 2-isopropyl-7-(piperidin-4-yl)-2,7-diazaspiro[4.5]decane-1,3-dione dihydrochloride
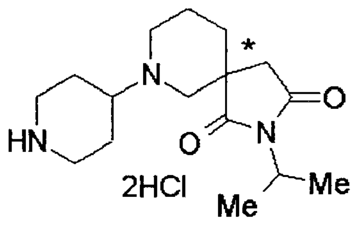

To tert-butyl 4-(2-isopropyl-1,3-dioxo-2,7-diazaspiro[4.5]dec-7-yl)piperidine-1-carboxylate in an optically active form (2.54 g, 6.45 mmol) obtained in Reference Example 11 was added 4N hydrogen chloride-ethyl acetate (25 mL), and the mixture was concentrated under reduced pressure 1 hr later. The obtained residue was triturated with diisopropyl ether to give the title compound (2.36 g, quantitatively).

Reference Example 13

1'-tert-butyl 3-methyl 3-hydroxy-1,4'-bipiperidine-1',3-dicarboxylate
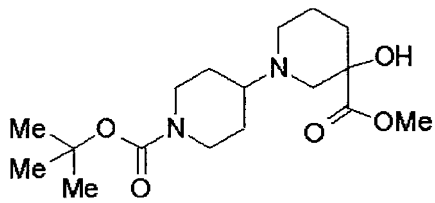

A suspension of methyl 1-benzyl-3-hydroxypiperidine-3-carboxylate (4.00 g, 16.0 mmol) and 20% palladium hydroxide carbon (1.13 g) in ethanol (50 mL) was stirred under a hydrogen atmosphere for 2 hr. After filtration, the filtrate was concentrated under reduced pressure. The obtained residue, 1-(tert-butoxycarbonyl)-4-piperidone (3.04 g, 15.3 mmol) and sodium triacetoxyborohydride (9.73 g, 45.9 mmol) were added under ice-cooling, and the mixture was stirred at room temperature for 1 day. Saturated aqueous sodium hydrogen carbonate solution was added to the reaction mixture, and the mixture was extracted with ethyl acetate. The organic layer was washed with saturated brine, and dried over anhydrous magnesium sulfate. The solvent was evaporated under reduced pressure and the obtained residue was purified by silica gel column chromatography (ethyl acetate to methanol:ethyl acetate=1:9) to give the title compound (2.73 g, yield 52%) as a yellow oil
EI(pos) 343 $[M+H]^+$

Reference Example 14

optically active forms (two kinds) of 1'-tert-butyl 3-methyl 3-hydroxy-1,4'-bipiperidine-1',3-dicarboxylate
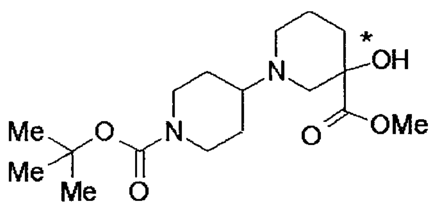

1'-tert-Butyl 3-methyl 3-hydroxy-1,4'-bipiperidine-1',3-dicarboxylate (racemate) (7.0 g) obtained in Reference Example 13 was optically resolved by HPLC under the following conditions to give two kinds of optically active forms having "long retention time (2.98 g)" and "short retention time (2.93 g)".

<preparative HPLC conditions>

column: CHIRALPAK AD (50 mmID×500 mmL)
mobile phase: hexane:ethanol=9:1
flow rate: 80 mL/min
column temperature: 30°C
detection: UV 220 nm
compound injection volume: 1.0 g/50 mL (hexane:ethanol=9:1)

<HPLC analysis conditions>

column: CHIRALPAK AD (4.6 mmIDx250 mmL)
mobile phase: hexane:ethanol=9:1
flow rate: 1.0 mL/min
column temperature: 30°C
detection: UV 220 nm
retention time of "long retention time": 11.55 min
retention time of "short retention time": 8.92 min

Reference Example 15

tert-butyl 4-(3-tert-butyl-2,4-dioxo-1-oxa-3,7-diazaspiro[4.5]dec-7-yl)piperidine-1-carboxylate
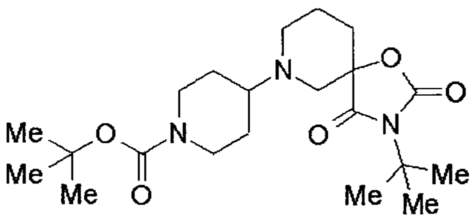

To a solution of 1'-tert-buty 3-methyl 3-hydroxy-1,4'-bipiperidine-1',3-dicarboxylate (5.00 g, 14.6 mmol) obtained in Reference Example 13 in THF (70 mL) were added tert-butyl isocyanate (2.51 mL, 21.9 mmol) and 60% sodium hydride (292 mg, 7.30 mmol), and the mixture was stirred at room temperature for 1 hr. The reaction mixture was diluted with ethyl acetate, and the mixture was washed with saturated brine, and dried over anhydrous sodium sulfate. The solution was applied to basic silica gel column chromatography (ethyl acetate), and the solvent was evaporated under reduced pressure. The obtained residue was purified by silica gel column chromatography (hexane to hexane:ethyl acetate=1:1) to give the title compound (5.98 g, quantitatively) as an oil.
EI(pos) 410 $[M+H]^+$

Reference Example 16

3-tert-butyl-7-(piperidin-4-yl)-1-oxa-3,7-diazaspiro[4.5]decane-2,4-dione dihydrochloride

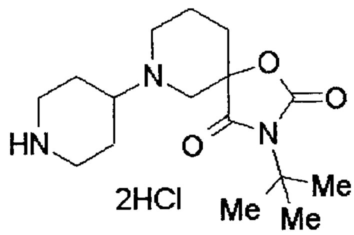

The title compound (389 mg, yield 79%) was obtained by an operation in the same manner as in Reference Example 12 and using tert-butyl 4-(3-tert-butyl-2,4-dioxo-1-oxa-3,7-diazaspiro[4.5]dec-7-yl)piperidine-1-carboxylate (525 mg, 1.28 mmol) obtained in Reference Example 15, followed by trituration with diisopropyl ether. The obtained compound was used in the next step without purification.

Reference Example 17

optically active form of tert-butyl 4-(3-isopropyl-2,4-dioxo-1-oxa-3,7-diazaspiro[4.5]dec-7-yl)piperidine-1-carboxylate

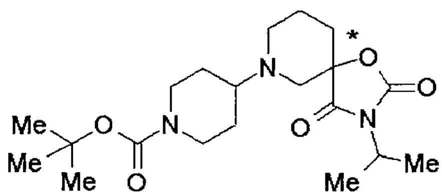

The title compound (6.46 g, yield 90%) as an oil was obtained in the same manner as in Reference Example 15 and using 1'-tert-butyl 3-methyl 3-hydroxy-1,4'-bipiperidine-1',3-dicarboxylate in an optically active form (long retention time) (6.25 g, 18.3 mmol) obtained in Reference Example 14 and isopropyl isocyanate (2.69 mL, 27.4 mmol).
EI(pos) 396 $[M+H]^+$

Reference Example 18

optically active form of 3-isopropyl-7-(piperidin-4-yl)-1-oxa-3,7-diazaspiro[4.5]decane-2,4-dione dihydrochloride

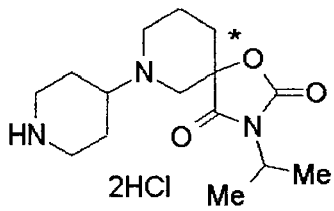

The title compound (5.34 g, yield 89%) was obtained by an operation in the same manner as in Reference Example 12 and using tert-butyl 4-(3-isopropyl-2,4-dioxo-1-oxa-3,7-diazaspiro[4.5]dec-7-yl)piperidine-1-carboxylate in an optically active form (6.46 g, 16.3 mmol) obtained in Reference Example 17, followed by trituration with diisopropyl

ether. The obtained compound was used in the next step without purification.

Reference Example 19

1-tert-butyl 3-ethyl 3-[(methoxycarbonyl)thio]piperidine-1,3-dicarboxylate

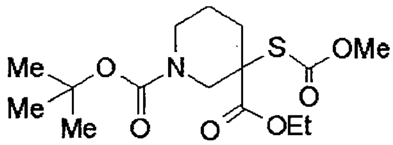

To a solution of 1-tert-butyl 3-ethyl piperidine-1,3-dicarboxylate (16.1 g, 55.3 mmol) in THF (250 mL) was added a 1.0 M solution (60.8 mL, 60.8 mmol) of lithium bis(trimethylsilyl)amide in THF at -78°C, and the mixture was stirred at the same temperature for 30 min. To this solution was added dropwise a solution of (chlorothio)(methoxy)oxomethane (6.0 mL, 66.3 mmol) in THF (25 mL) at -78°C over 10 min, and the mixture was heated to room temperature, and stirred for 30 min. The reaction mixture was dissolved in ethyl acetate, and the solution was washed with water, 0.2N hydrochloric acid and saturated brine, and dried over anhydrous sodium sulfate. The solvent was evaporated under reduced pressure, and the obtained residue was purified by silica gel column chromatography (hexane to hexane:ethyl acetate=3:1) to give the title compound (8.02 g, yield 38%) as an oil. The obtained compound was used in the next step without purification.

Reference Example 20

1-tert-butyl 3-ethyl 3-mercaptopiperidine-1,3-dicarboxylate

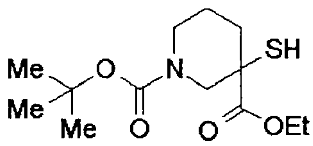

To a solution of 1-tert-butyl 3-ethyl 3-[(methoxycarbonyl)thio]piperidine-1,3-dicarboxylate (6.95 g, 20.0 mmol) obtained in Reference Example 19 in ethanol (90 mL) was added sodium ethoxide (1.91 g, 28.0 mmol), and the mixture was stirred at room temperature for 30 min. The solvent was evaporated under reduced pressure, ethyl acetate was added to the obtained residue, and the mixture was washed with 0.5N hydrochloric acid and saturated brine, and dried over anhydrous sodium sulfate. The solvent was evaporated under reduced pressure, and the obtained residue was purified by silica gel column chromatography (hexane to hexane:ethyl acetate=7:3) to give the title compound (5.46 g, yield 94%) as an oil.
EI(pos) 190 $[M+H-Boc]^+$

Reference Example 21

tert-butyl 3-isopropyl-2,4-dioxo-1-thia-3,7-diazaspiro[4.5]decane-7-carboxylate

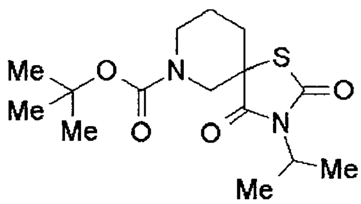

To a solution of 1-tert-butyl 3-ethyl 3-mercaptopiperidine-1,3-dicarboxylate (2.77 g, 9.57 mmol) obtained in Reference Example 20 and isopropyl isocyanate (1.88 mL, 19.1 mmol) in THF (30 mL) was added 60% sodium hydride (191 mg, 4.79 mmol), and the mixture was stirred at room temperature for 1 hr. The reaction mixture was diluted with ethyl acetate, and the mixture was washed with saturated brine, and dried over anhydrous sodium sulfate. The solvent was evaporated under reduced pressure, and the obtained residue was purified by silica gel column chromatography (hexane to ethyl acetate:hexane=3:7) to give the title compound (3.14 g, quantitatively) as an oil.
EI(pos) 228 $[M+H-Boc]^+$

Reference Example 22

tert-butyl 4-(3-isopropyl-2,4-dioxo-1-thia-3,7-diazaspiro[4.5]dec-7-yl)piperidine-1-carboxylate
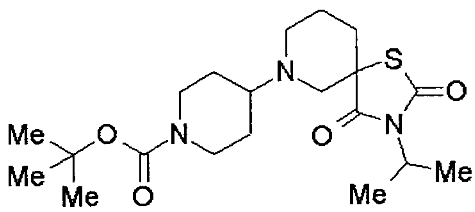

To tert-butyl 3-isopropyl-2,4-dioxo-1-thia-3,7-diazaspiro[4.5]decane-7-carboxylate (3.14 g, 9.56 mmol) obtained in Reference Example 21 was added 4N hydrogen chloride-ethyl acetate (30 mL) and, after stirring at room temperature for 1 hr, the mixture was concentrated under reduced pressure. Toluene was added and the mixture was concentrated, which operation was repeated twice. To a suspension of the obtained residue in THF (30 mL) were added triethylamine (1.33 mL, 9.56 mmol), acetic acid (3.00 mL, 52.4 mmol), 1-(tert-butoxycarbonyl)-4-piperidone (1.90 g, 9.56 mmol) and sodium triacetoxyborohydride (3.04 g, 14.3 mmol) in this order under ice-cooling, and the mixture was stirred at room temperature for 16 hr. 10% Aueous potassium carbonate solution was added to the reaction mixture, and the mixture was extracted with ethyl acetate. The organic layer was washed with saturated brine, and dried over anhydrous sodium sulfate. This liquid was passed through basic silica gel column chromatography (ethyl acetate). The solvent was evaporated under reduced pressure and the obtained residue was purified by silica gel column chromatography (hexane to ethyl acetate:hexane=3:7) to give the title compound (3.35 g, yield 85%) as an oil.
EI(pos) 412 $[M+H]^+$

Reference Example 23

3-isopropyl-7-(piperidin-4-yl)-1-thia-3,7-diazaspiro[4.5]decane-2,4-dione dihydrochloride
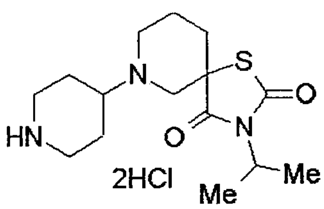

The title compound (2.71 g, yield 87%) was obtained by an operation in the same manner as in Reference Example 12 and using tert-butyl 4-(3-isopropyl-2,4-dioxo-1-thia-3,7-diazaspiro[4.5]dec-7-yl)piperidine-1-carboxylate (3.34 g, 8.12 mmol) obtained in Reference Example 22, followed by trituration with diisopropyl ether. The obtained compound was used in the next step without purification.

Reference Example 24

tert-butyl 3,3-dimethyl-1-oxo-2-oxa-7-azaspiro[4.5]decane-7-carboxylate
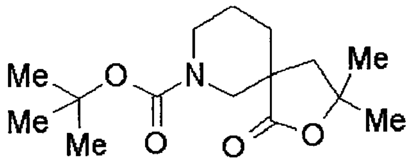

To a solution of 1-tert-butyl 3-ethyl piperidine-1,3-dicarboxylate (32.4 g, 126 mmol) in THF (150 mL) was added a 1.0 M solution (164 mL, 164 mmol) of lithium bis(trimethylsilyl)amide in THF at -78°C, and the mixture was stirred at the same temperature for 30 min. To this solution was added isobutylene oxide (16.3 mL, 189 mmol) at -78°C, and the mixture was heated to room temperature and stirred for 3.5 hr. The reaction mixture was dissolved in ethyl acetate, and the solution was washed with saturated aqueous ammonium chloride solution and saturated brine, and dried over anhydrous magnesium sulfate. The solvent was evaporated under reduced pressure, and the obtained residue was purified by silica gel column chromatography (hexane:ethyl acetate=4:1 to 1:1) to give the title compound (24.8 g, yield 69%) as an oil. EI(pos) 306 $[M+Na]^+$

Reference Example 25

tert-butyl 4-(3,3-dimethyl-1-oxo-2-oxa-7-azaspiro[4.5]dec-7-yl)piperidine-1-carboxylate
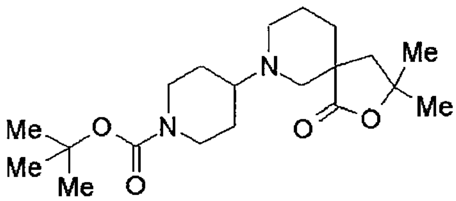

tert-Butyl 3,3-dimethyl-1-oxo-2-oxa-7-azaspiro[4.5]decane-7-carboxylate (19.1 g, 67.4 mmol) obtained in Reference Example 24 was dissolved in 4N hydrogen chloride-ethyl acetate (200 mL), and the solution was stirred at room temperature for 30 min. The reaction solution was concentrated under reduced pressure, and the obtained residue was dissolved in dichloromethane (200 mL). To this solution were added triethylamine (9.4 mL, 67.4 mmol), 1-(tert-butoxycarbonyl)-4-piperidone (13.5 g, 67.4 mmol) and sodium triacetoxyborohydride (42.9 g, 202 mmol) under ice-cooling, and the mixture was stirred at room temperature for 4 days. Saturated aqueous sodium hydrogen carbonate solution was added to the reaction mixture, and the mixture was extracted with ethyl acetate. The organic layer was washed with saturated brine, and dried over anhydrous magnesium sulfate. The solvent was evaporated under reduced pressure and the obtained residue was purified by basic silica gel column chromatography (hexane:ethyl acetate=4:1 to 3:2) and then silica gel column chromatography (hexane:ethyl acetate=1:1 to ethyl acetate) to give the title compound (15.2 g, yield 61%) as a white solid. EI(pos) 367 $[M+H]^+$

Reference Example 26

3,3-dimethyl-7-(piperidin-4-yl)-2-oxa-7-azaspiro[4.5]decan-1-one dihydrochloride
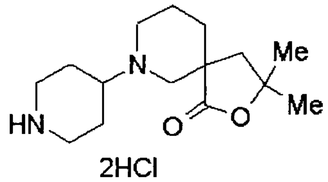

tert-Butyl 4-(3,3-dimethyl-1-oxo-2-oxa-7-azaspiro[4.5]dec-7-yl)piperidine-1-carboxylate (13.5 g, 36.9 mmol) obtained in Reference Example 25 was dissolved in 4N hydrogen chloride-ethyl acetate (250 mL), and the solution was

stirred at room temperature for 30 min. The precipitated solid was collected by filtration, and washed with diethyl ether to give the title compound (12.4 g, yield 99%) as a white solid. EI(pos) 267 $[M+H]^+$

Reference Example 27

benzyl 4-(3,3-dimethyl-1-oxo-2-oxa-7-azaspiro[4.5]dec-7-yl)piperidine-1-carboxylate

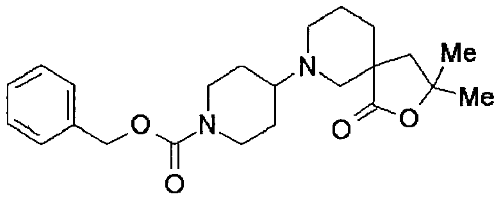

tert-Butyl 3,3-dimethyl-1-oxo-2-oxa-7-azaspiro[4.5]decane-7-carboxylate (13.2 g, 46.7 mmol) obtained in Reference Example 24 was dissolved in 4N hydrogen chloride-ethyl acetate (200 mL), and the solution was stirred at room temperature for 30 min. The reaction solution was concentrated under reduced pressure, and the obtained residue was dissolved in dichloromethane (150 mL). To this solution were added triethylamine (6.5 mL, 46.7 mmol), N-benzyloxycarbonyl-4-piperidone (10.9 g, 46.7 mmol) and sodium triacetoxyborohydride (29.7 g, 140 mmol) under ice-cooling, and the mixture was stirred at room temperature for 3 days. Saturated aqueous sodium hydrogen carbonate solution was added to the reaction mixture, and the mixture was extracted with ethyl acetate. The organic layer was washed with saturated brine, and dried over anhydrous magnesium sulfate. The solvent was evaporated under reduced pressure and the obtained residue was purified by basic silica gel column chromatography (eluent; hexane:ethyl acetate=4:1 to 3:2) to give the title compound (13.4 g, yield 71%) as a yellow oil
EI(pos) 401 $[M+H]^+$

Reference Example 28

optically active forms (two kinds) of benzyl 4-(3,3-dimethyl-1-oxo-2-oxa-7-azaspiro[4.5]dec-7-yl)piperidine-1-carboxylate

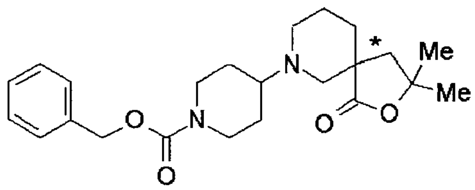

Benzyl 4-(3,3-dimethyl-1-oxo-2-oxa-7-azaspiro[4.5]dec-7-yl)piperidine-1-carboxylate (racemate) (5.90 g) obtained in Reference Example 27 was optically resolved using HPLC under the following conditions to give two kinds of optically active forms having "long retention time (2.89 g)" and "short retention time (2.82 g)".

<preparative HPLC conditions>

column: CHIRALPAK AS (50 mmID×500 mmL)
mobile phase: hexane:ethanol=9:1
flow rate: 60 mL/min
column temperature: 25°C
detection: UV 220 nm
compound injection volume: 1.5 g/60 mL (hexane:ethanol=9:1)

<HPLC analysis conditions>

column: CHIRALPAK AD-H (4.6 mmIDx250 mmL)
mobile phase: hexane:ethanol=9:1
flow rate: 1.0 mL/min
column temperature: 25°C
detection: UV 220 nm
retention time of "long retention time": 26.30 min
retention time of "short retention time": 18.71 min

Reference Example 29

optically active form of 3,3-dimethyl-7-(piperidin-4-yl)-2-oxa-7-azaspiro[4.5]decan-1-one dihydrochloride
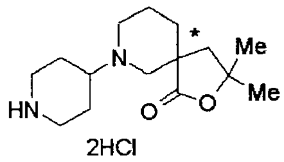

A suspension of benzyl 4-(3,3-dimethyl-1-oxo-2-oxa-7-azaspiro[4.5]dec-7-yl)piperidine-1-carboxylate in an optically active form (long retention time) (6.01 g, 15.0 mmol) obtained in Reference Example 28 and 10% palladium carbon (50% water-containing product, 1.60 g) in ethanol (50 mL) was stirred under a hydrogen atmosphere at room temperature for 1.5 hr. The reaction mixture was filtered, and the filtrate was concentrated under reduced pressure. The obtained residue was dissolved in 4N hydrogen chloride-ethyl acetate (20 mL), and the solution was stirred at room temperature for 30 min. The precipitated solid was collected by filtration and washed with diethyl ether to give the title compound (4.72 g, yield 93%) as a white solid.
EI(pos) 267 $[M+H]^+$

Reference Example 30

tert-butyl 3-isopropyl-2,4-dioxo-1,3,7-triazaspiro[4.5]decane-7-carboxylate
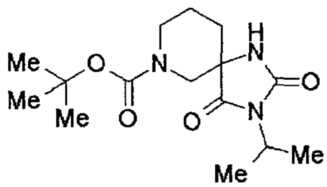

To a solution of tert-butyl 2,4-dioxo-1,3,7-triazaspiro[4.5]decane-7-carboxylate (5.00 g, 18.6 mmol) and potassium carbonate (5.15 g, 37.2 mmol) in DMF (50 mL) was added 2-iodopropane (2.8 mL, 27.9 mmol), and the mixture was stirred at room temperature for 1 hr. The reaction mixture was diluted with ethyl acetate, and the mixture was washed with aqueous sodium hydrogen carbonate solution and saturated brine, and dried over anhydrous sodium sulfate. The solvent was evaporated under reduced pressure, and the obtained solid was washed with ethyl acetate to give the title compound (2.35 g, yield 41%) as a white solid.
EI(pos) 212 $[M-Boc]^+$, 256 $[M-tert-Bu]^+$

Reference Example 31

tert-butyl 4-(3-isopropyl-2,4-dioxo-1,3,7-triazaspiro[4.5]dec-7-yl)piperidine-1-carboxylate
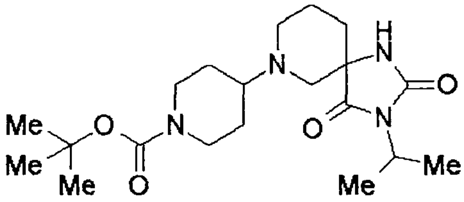

The title compound (1.48 g, yield 50%) as a white solid was obtained in the same manner as in Reference Example 2 and using tert-butyl 3-isopropyl-2,4-dioxo-1,3,7-triazaspiro[4.5]decane-7-carboxylate (2.35 g, 7.55 mmol) obtained in Reference Example 30.
EI(pos) 381 $[M+H]^+$

Reference Example 32

3-isopropyl-7-(piperidin-4-yl)-1,3,7-triazaspiro[4.5]decane-2,4-dione dihydrochloride
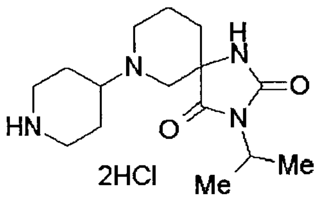

The title compound (1.38 g, yield 99%) as a white solid was obtained in the same manner as in Reference Example 12 and using tert-butyl 4-(3-isopropyl-2,4-dioxo-1,3,7-triazaspiro[4.5]dec-7-yl)piperidine-1-carboxylate (1.48 g, 3.76 mmol) obtained in Reference Example 31.
EI(pos) 295 $[M+H]^+$

Reference Example 33

tert-butyl 3-acetamido-3-cyanopiperidine-1-carboxylate
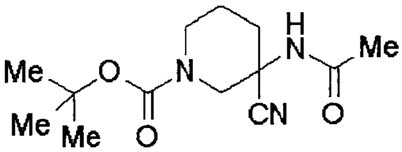

To a mixed solution of tert-butyl 3-oxopiperidine-1-carboxylate (5.00 g, 25.1 mmol) and ammonium chloride (5.35 g, 100 mmol) in isopropyl alcohol-25% aqueous ammonia (30 mL-62 mL) was added potassium cyanide (6.54 g, 100 mmol), and the mixture was stirred for 14 hr. The reaction mixture was extracted 3 times with ethyl acetate, and the organic layer was dried over anhydrous magnesium sulfate. To a solution of the residue obtained by evaporating the solvent under reduced pressure and triethylamine (5.3 mL, 37.7 mmol) in THF (100 mL) was added acetyl chloride (2.32 mL, 32.6 mmol), and the mixture was stirred at room temperature for 30 min. Saturated aqueous sodium hydrogen carbonate solution was added to the reaction mixture, and the mixture was extracted twice with ethyl acetate. The organic layer was dried over anhydrous magnesium sulfate. The solvent was evaporated under reduced pressure and the obtained residue was purified by silica gel column chromatography (ethyl acetate to methanol:ethyl acetate=1:9) to give the title compound (5.26 g, yield 78%) as an oil.
EI(pos) 168 $[M-Boc]^+$

Reference Example 34

tert-butyl 3-acetamido-3-cyano-1,4'-bipiperidine-1'-carboxylate

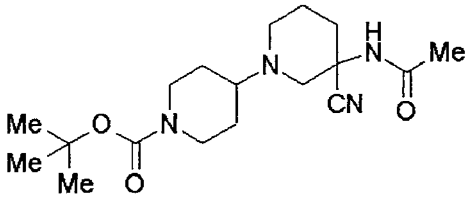

The title compound (6.04 g, yield 43%) as an oil was obtained in the same manner as in Reference Example 2 and using tert-butyl 3-acetamido-3-cyanopiperidine-1-carboxylate (10.7 g, 40.0 mmol) obtained in Reference Example 33.
EI(pos) 351 $[M+H]^+$

Reference Example 35

optically active forms (two kinds) of tert-butyl 3-acetamido-3-cyano-1,4'-bipiperidine-1'-carboxylate

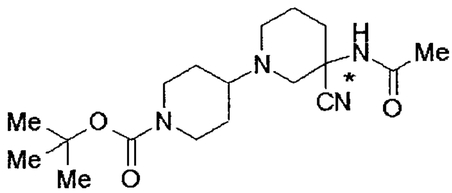

tert-Butyl 3-acetamido-3-cyano-1,4'-bipiperidine-1'-carboxylate (racemate) (4.50 g) obtained in Reference Example 34 was optically resolved using HPLC under the following conditions to give two kinds of optically active forms having "long retention time (2.13 g)" and "short retention time (2.11 g)".

<preparative HPLC conditions>

column: CHIRALPAK AD (50 mmID×500 mmL)
mobile phase: hexane:ethanol=9:1
flow rate: 80 mL/min
column temperature: 35°C
detection: UV 220 nm
compound injection volume: 400 mg/40 mL (hexane:ethanol=9:1)

<HPLC analysis conditions>

column: CHIRALPAK AD-H (4.6 mmIDx250 mmL)
mobile phase: hexane:ethanol=9:1
flow rate: 1.0 mL/min
column temperature: 30°C
detection: UV 220 nm
retention time of "long retention time": 11.71 min
retention time of "short retention time": 8.09 min

Reference Example 36

tert-butyl 4-(2-methyl-4-oxo-1,3,7-triazaspiro[4.5]dec-1-en-7-yl)piperidine-1-carboxylate

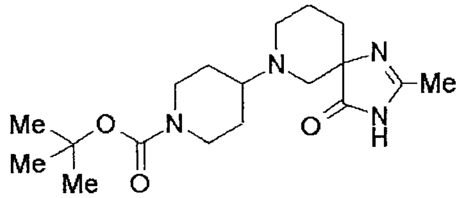

To a solution of tert-butyl 3-acetamido-3-cyano-1,4'-bipiperidine-1'-carboxylate (13.9 g, 39.7 mmol) obtained in Reference Example 34 in ethanol (100 mL) was added 30% aqueous hydrogen peroxide (2 mL), and the mixture was stirred at 70°C for 1.5 hr. 30% Aqueous hydrogen peroxide (2 mL) was further added, and the mixture was further stirred at 70°C for 1 hr. The reaction mixture was concentrated under reduced pressure, water was added, and the mixture was extracted 3 times with acetic acid - THF (1:1). The extract was dried over anhydrous magnesium sulfate and concentrated under reduced pressure. The residual solid was washed with diethyl ether, and dried to give the title compound (10.0 g, yield 72%).

EI(pos) 351 $[M+H]^+$

Reference Example 37

optically active form of tert-butyl 4-(2-methyl-4-oxo-1,3,7-triazaspiro[4.5]dec-1-en-7-yl)piperidine-1-carboxylate

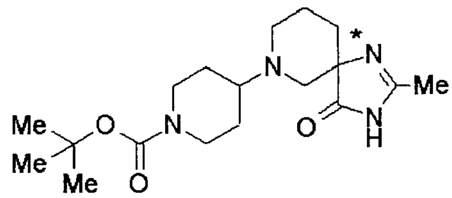

The title compound (16.5 g, yield 52%) was obtained by an operation in the same manner as in Reference Example 36 and using tert-butyl 3-acetamido-3-cyano-1,4'-bipiperidine-1'-carboxylate in an optically active form (long retention time) (31.5 g, 89.8 mmol) obtained in Reference Example 35, followed by trituration with hexane-diisopropyl ether.

EI(pos) 351.1 $[M+H]^+$

Reference Example 38

tert-butyl 4-(3-ethyl-2-methyl-4-oxo-1,3,7-triazaspiro[4.5]dec-1-en-7-yl)piperidine-1-carboxylate

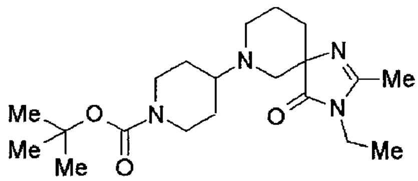

To a solution of tert-butyl 4-(2-methyl-4-oxo-1,3,7-triazaspiro[4.5]dec-1-en-7-yl)piperidine-1-carboxylate (1.23 g, 3.51 mmol) obtained in Reference Example 36 in DMF (20 mL) was added sodium hydride (0.18 g, 4.57 mmol), and

the mixture was stirred at room temperature for 30 min. To this solution was added iodoethane (0.42 mL, 5.27 mmol) and the mixture was stirred for 1 hr. Saturated brine was added to the reaction mixture, and the mixture was extracted with ethyl acetate. The organic layer was washed twice with saturated brine, and dried over anhydrous magnesium sulfate. The solvent was evaporated and the obtained residue was purified by basic silica gel column chromatography (ethyl acetate to ethyl acetate:methanol=9:1) to give the title compound (1.13 g, yield 85%) as an oil.
EI(pos) 379 $[M+H]^+$

Reference Example 39

tert-butyl 4-(3-isopropyl-2-methyl-4-oxo-1,3,7-triazaspiro[4.5]dec-1-en-7-yl)piperidine-1-carboxylate
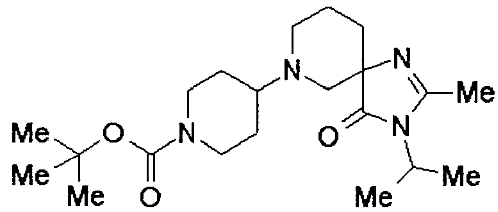

The title compound (210 mg, yield 13%) as an oil was obtained in the same manner as in Reference Example 38 and using tert-butyl 4-(2-methyl-4-oxo-1,3,7-triazaspiro[4.5]dec-1-en-7-yl)piperidine-1-carboxylate (1.42 g, 4.05 mmol) obtained in Reference Example 36 and 2-iodopropane.
EI(pos) 393 $[M+H]^+$

Reference Example 40

optically active form of tert-butyl 4-(3-isopropyl-2-methyl-4-oxo-1,3,7-triazaspiro[4.5]dec-1-en-7-yl)piperidine-1-carboxylate
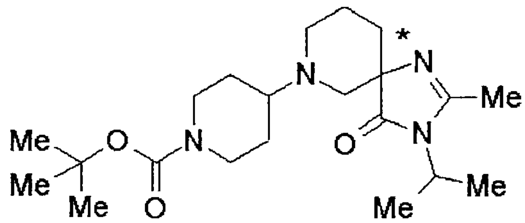

The title compound (6.86 g, yield 37%) was obtained by an operation in the same manner as in Reference Example 38 and using tert-butyl 4-(2-methyl-4-oxo-1,3,7-triazaspiro[4.5]dec-1-en-7-yl)piperidine-1-carboxylate in an optically active form (16.5 g, 47.0 mmol) obtained in Reference Example 37 and 2-iodopropane, followed by trituration with hexane.
EI(pos) 393.1 $[M+H]^+$

Reference Example 41

3-isopropyl-2-methyl-7-(piperidin-4-yl)-1,3,7-triazaspiro[4.5]dec-1-en-4-one dihydrochloride
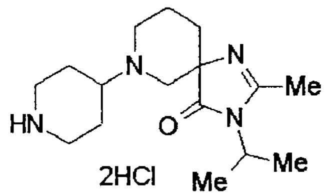

The title compound was quantitatively obtained in the same manner as in Reference Example 12 and using tert-butyl 4-(3-isopropyl-2-methyl-4-oxo-1,3,7-triazaspiro[4.5]dec-1-en-7-yl)piperidine-1-carboxylate obtained in Reference Example 39.
EI(pos) 293.2 $[M+H]^+$(free base)

Reference Example 42

optically active form of 3-isopropyl-2-methyl-7-(piperidin-4-yl)-1,3,7-triazaspiro[4.5]dec-1-en-4-one dihydrochloride
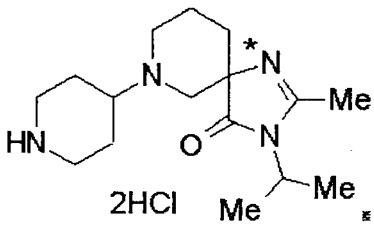

The title compound was quantitatively obtained by an operation in the same manner as in Reference Example 12 and using tert-butyl 4-(3-isopropyl-2-methyl-4-oxo-1,3,7-triazaspiro[4.5]dec-1-en-7-yl)piperidine-1-carboxylate in an optically active form obtained in Reference Example 40.

Reference Example 43

ethyl 3-amino-6-methylthieno[2,3-b]pyridine-2-carboxylate
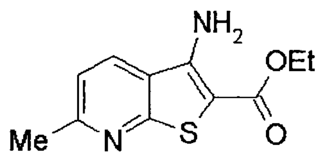

To a solution of 2-chloro-6-methylnicotinonitrile (44.0 g, 0.289 mol) and ethyl thioglycolate (35.0 mL, 0.318 mol) in DMF (500 mL) was added sodium methoxide (21.7 g, 0.318 mol), and the mixture was stirred at room temperature for 30 min. Sodium ethoxide (5.00 g, 73.5 mmol) was further added, and the mixture was stirred at room temperature for 30 min. Water was added to the reaction mixture and the precipitated solid was collected by filtration, washed with water, and dried to give the title compound (66.4 g, yield 97%) as a yellow solid.
$^1H$ NMR ($CDCl_3$) δ1.39 (3H, t, J = 7.2 Hz), 2.68 (3H, s), 4.36 (2H, q, J = 7.2 Hz), 5.89 (2H, br), 7.16 (1H, d, J = 8.3 Hz), 7.81 (1H, d, J = 8.3 Hz).

Reference Example 44

ethyl 3-bromo-6-methylthieno[2,3-b]pyridine-2-carboxylate
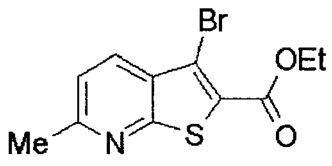

Ethyl 3-amino-6-methylthieno[2,3-b]pyridine-2-carboxylate (36.1 g, 0.153 mol) obtained in Reference Example 43 was added to a solution of copper(II) bromide (37.5 g, 0.168 mol) and tert-butyl nitrite (23.6 mL, 0.199 mol) in acetonitrile (350 mL) under water cooling over 2 hr, and the mixture was stirred for 1 hr. 1N Hydrochloric acid (700 mL) was slowly added to the reaction mixture, and the resulting precipitate was collected by filtration and washed with water. The solid was dissolved in THF, and the solution was diluted with ethyl acetate. This solution was washed with 1N hydrochloric acid and saturated brine, and dried over anhydrous sodium sulfate (solution A). The above filtrate was extracted with ethyl acetate, and the extract was washed with saturated brine, and dried over anhydrous sodium sulfate. The residue was subjected to basic silica gel column chromatography (ethyl acetate) to give a crude product (about 5 g). This was combined with solution A and the mixture was subjected to basic silica gel column chromatography (ethyl acetate) again and crystallized from hexane to give the title compound (30.5 g, yield 66%) as a pale-yellow solid.
$^1$H NMR ($CDCl_3$) δ1.35 (3H, t, J = 7.1 Hz), 2.67 (3H, s), 4.39 (2H, q, J = 7.1 Hz), 7.54 (1H, d, J = 8.3 Hz), 8.20 (1H, d, J = 8.3 Hz).

Reference Example 45

3-bromo-6-methylthieno[2,3-b]pyridine-2-carboxylic acid
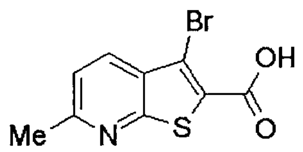

To a solution of ethyl 3-bromo-6-methylthieno[2,3-b]pyridine-2-carboxylate (79.2 g, 0.264 mol) obtained in Reference Example 44 in ethanol (250 mL) was added 2N aqueous sodium hydroxide solution (264 mL, 0.527 mol), and the mixture was stirred at room temperature for 16 hr. The reaction mixture was diluted with water (1 L), adjusted to pH 5 - 6 with 1N hydrochloric acid (530 mL) and the precipitated solid was collected and washed with water. The obtained solid was suspended in acetone, and the precipitate was collected by filtration, and washed successively with acetone and diisopropyl ether to give the title compound (68.5 g, yield 95%).
$^1$H NMR ($CDCl_3$) δ2.66 (3H, s), 7.52 (1H, d, J = 8.5 Hz), 8.18 (1H, d, J = 8.3 Hz), 14.06 (1H, br s).

Reference Example 46

tert-butyl (3-bromo-6-methylthieno[2,3-b]pyridin-2-yl)carbamate
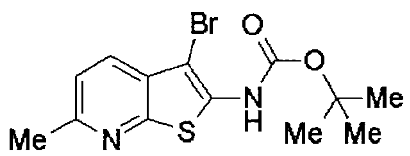

A solution of 3-bromo-6-methylthieno[2,3-b]pyridine-2-carboxylic acid (15.0 g, 55.2 mmol) obtained in Reference Example 45, diphenylphosphoryl azide (13.1 mL, 60.6 mmol) and triethylamine (11.6 mL, 82.8 mmol) in tert-butanol (100 mL) was heated at 90°C for 15 hr. The reaction mixture was diluted with ethyl acetate. This solution was washed successively with saturated aqueous sodium hydrogen carbonate solution and saturated brine, and dried over anhydrous magnesium sulfate. The solvent was evaporated under reduced pressure, and the obtained residue was purified by silica gel column chromatography (ethyl acetate:hexane=1:19 to 3:7) to give the title compound (16.3 g, yield 86%) as

a white solid.
$^1$H NMR (DMSO-$d_6$) δ1.51 (9H, s), 2.58 (3H, s), 7.32 (1H, d, J = 8.4 Hz), 7.78 (1H, d, J = 8.4 Hz), 10.12 (1H, s).

Reference Example 47

2-[(tert-butoxycarbonyl)amino]-6-methylthieno[2,3-b]pyridine-3-carboxylic acid
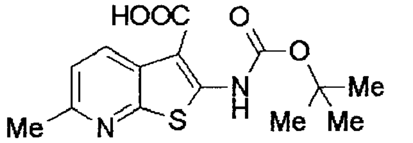

To a solution of tert-butyl (3-bromo-6-methylthieno[2,3-b]pyridin-2-yl)carbamate (9.07 g, 26.4 mmol) obtained in Reference Example 46 in dry THF (88 mL) was added dropwise 1.6M n-butyllithium hexane solution (38 mL, 60.7 mmol) under a nitrogen atmosphere at -78°C, and the mixture was stirred at the same temperature for 1 hr. A dry carbon dioxide gas vaporized from dry ice was bubbled at 0-10°C. The reaction mixture was diluted with water and ethyl acetate, 1N hydrochloric acid was added, and the precipitated solid was collected by filtration. The obtained solid was sequentially suspended and collected by filtration using water and acetonitrile/diethyl ether (1:1) repeatedly for purification to give the title compound (6.51 g, yield 80%) as white crystals.
melting point 162-163°C
EI(pos) 309 $[M+]^+$
$^1$H NMR (DMSO-$d_6$) δ1.53 (9H, s), 2.50 (3H, s), 7.29 (1H, d, J = 8.3 Hz), 8.37 (1H, d, J = 8.3 Hz), 11.26 (1H, br s).

Reference Example 48

ethyl 3-aminothieno[2,3-b]pyridine-2-carboxylate
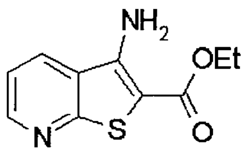

The title compound was obtained in the same manner as in Reference Example 43 from 2-chloronicotinonitrile. yield 83%.

Reference Example 49

ethyl 3-bromothieno[2,3-b]pyridine-2-carboxylate
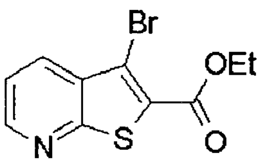

The title compound was obtained in the same manner as in Reference Example 44 from ethyl 3-aminothieno [2,3-b]pyridine-2-carboxylate obtained in Reference Example 48. yield 72%.
$^1$H NMR ($CDCl_3$) δ1.45 (3H, t, J = 7.2 Hz), 4.46 (2H, q, J = 7.2 Hz), 7.46 (1H, dd, J = 4.5 Hz, 8.3 Hz), 8.23 (1H, dd, J = 1.7 Hz, 8.3 Hz), 8.73 (1H, dd, J = 1.7 Hz, 4.5 Hz).

Reference Example 50

3-bromothieno[2,3-b]pyridine-2-carboxylic acid

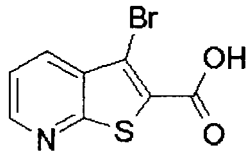

The title compound was obtained in the same manner as in Reference Example 45 from 3-bromothieno[2,3-b]pyridine-2-carboxylic acid obtained in Reference Example 49. yield 93%. $^1$H NMR (DMSO-$d_6$) δ7.66 (1H, dd, J = 4.5 Hz, 8.1 Hz), 8.32 (1H, dd, J = 1.5 Hz, 8.1 Hz), 8.81 (1H, dd, J = 1.5 Hz, 4.5 Hz), 14.19 (1H, s).

Reference Example 51

tert-butyl (3-bromothieno[2,3-b]pyridin-2-yl)carbamate

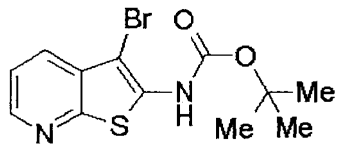

The title compound was obtained in the same manner as in Reference Example 46 from 3-bromothieno[2,3-b]pyridine-2-carboxylic acid obtained in Reference Example 50. yield 80%.
$^1$H NMR (DMSO-$d_6$) δ1.52 (9H, s), 7.47 (1H, dd, J = 4.8 Hz, 8.1 Hz), 7.90 (1H, dd, J = 1.5 Hz, 8.1 Hz), 8.47 (1H, dd, J = 1.5 Hz, 4.8 Hz), 10.28 (1H, s).

Reference Example 52

2-[(tert-butoxycarbonyl)amino]thieno[2,3-b]pyridine-3-carboxylic acid

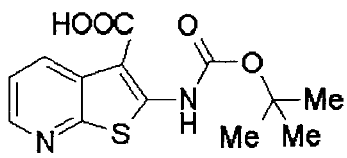

The title compound was obtained in the same manner as in Reference Example 47 from tert-butyl (3-bromothieno [2,3-b]pyridin-2-yl)carbamate obtained in Reference Example 51. yield 82%.
melting point 199-201°C
EI(pos) 295 [M+]$^+$
$^1$H NMR (DMSO-$d_6$) δ1.54 (9H, s), 7.46 (1H, dd, J = 4.9 Hz, 8.1 Hz), 8.41-8.49 (2H, m), 10.96 (1H, s), 13.86 (1H, br s).

Reference Example 53

ethyl 3-aminothieno[3,2-b]pyridine-2-carboxylate

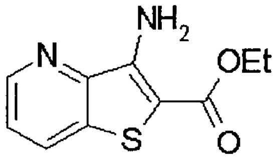

The title compound was obtained in the same manner as in Reference Example 43 from 3-chloro-2-cyanopyridine. yield 86%.

Reference Example 54

ethyl 3-bromothieno[3,2-b]pyridine-2-carboxylate

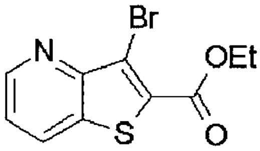

The title compound as an oil was obtained in the same manner as in Reference Example 44 from ethyl 3-aminothieno[3,2-b]pyridine-2-carboxylate obtained in Reference Example 53. yield 56%.
$^1H$ NMR ($CDCl_3$) δ1.46 (3H, t, J = 7.2 Hz), 4.48 (2H, q, J = 7.2 Hz), 7.45 (1H, dd, J = 8.3, 4.5 Hz), 8.19-8.22 (1H, m), 8.88-8.90 (1H, m).

Reference Example 55

3-bromothieno[3,2-b]pyridine-2-carboxylic acid

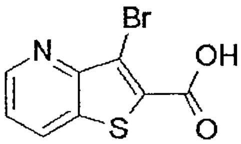

The title compound as a brown solid was obtained in the same manner as in Reference Example 45 from ethyl 3-bromothieno[3,2-b]pyridine-2-carboxylate obtained in Reference Example 54. yield 51%.
$^1H$ NMR ($CDCl_3$) δ7.62 (1H, dd, J = 8.3, 4.4 Hz), 8.61 (1H, dd, J = 8.3, 1.5 Hz), 8.86 (1H, dd, J = 4.4, 1.5 Hz).

Reference Example 56

tert-butyl (3-bromothieno[3,2-b]pyridin-2-yl)carbamate

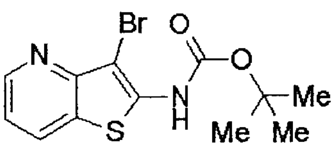

The title compound was obtained as a yellow solid in the same manner as in Reference Example 46 from 3-bromothieno[3,2-b]pyridine-2-carboxylic acid obtained in Reference Example 55. yield 81%.

$^1H$ NMR ($CDCl_3$) δ1.59 (9H, s), 7.19 (1H, dd, J = 8.0, 4.5 Hz), 7.49 (1H, br), 8.01 (1H, dd, J = 8.0, 1.1 Hz), 8.67 (1H, dd, J = 4.5, 1.5 Hz).

Reference Example 57

2-[(tert-butoxycarbonyl)amino]thieno[3,2-b]pyridine-3-carboxylic acid
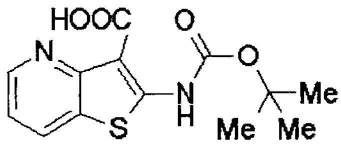

The title compound was obtained as a white solid in the same manner as in Reference Example 47 from tert-butyl (3-bromothieno[3,2-b]pyridin-2-yl)carbamate obtained in Reference Example 56. yield 61%.
$^1H$ NMR ($CDCl_3$) δ1.55 (9H, s), 7.46 (1H, dd, J = 8.2, 5.0 Hz), 8. 58 (1H, dd, J = 8.2, 1.3 Hz), 8.64 (1H, dd, J = 5.0, 1.3 Hz), 10.93 (1H, br).

Reference Example 58

ethyl 3-amino-4,6-dimethylthieno[2,3-b]pyridine-2-carboxylate
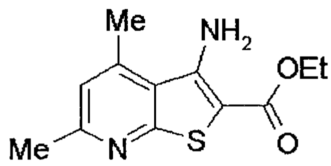

The title compound was obtained in the same manner as in Reference Example 43 from 2-chloro-3-cyano-4,6-dimethylpyridine. yield 96%.
$^1H$ NMR ($CDCl_3$) δ1.39 (3H, t, J = 7.0 Hz), 2.59 (3H, s), 2.74 (3H, s), 4.34 (2H, q, J = 7.0 Hz), 6.16 (2H, br), 6.86 (1H, s).

Reference Example 59

ethyl 3-bromo-4,6-dimethylthieno[2,3-b]pyridine-2-carboxylate
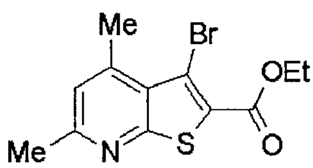

The title compound was obtained in the same manner as in Reference Example 44 from ethyl 3-amino-4,6-dimethylthieno[2,3-b]pyridine-2-carboxylate obtained in Reference Example 58. yield 53%.
$^1H$ NMR ($CDCl_3$) δ1.43 (3H, t, J = 7.2 Hz), 2.63 (3H, s), 2.95 (3H, s), 4.43 (2H, q, J = 7.2 Hz), 7.02 (1H, s).

Reference Example 60

3-bromo-4,6-dimethylthieno[2,3-b]pyridine-2-carboxylic acid
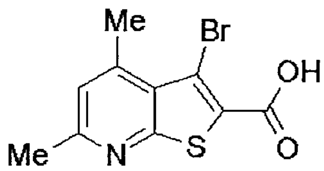

The title compound was obtained in the same manner as in Reference Example 45 from ethyl 3-bromo-4,6-dimethylthieno[2,3-b]pyridine-2-carboxylate obtained in Reference Example 59. yield 86%.

Reference Example 61

tert-butyl (3-bromo-4,6-dimethylthieno[2,3-b]pyridin-2-yl)carbamate
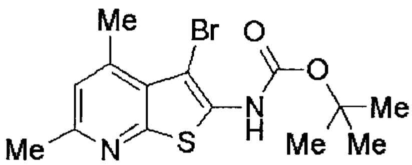

The title compound was obtained in the same manner as in Reference Example 46 from 3-bromo-4,6-dimethylthieno[2,3-b]pyridine-2-carboxylic acid obtained in Reference Example 60. yield 62%.
$^1$H NMR ($CDCl_3$) δ1.57 (9H, s), 2.57 (3H, s), 2.80 (3H, s), 7.26 (1H, s), 7.31 (1H, br).

Reference Example 62

2-[(tert-butoxycarbonyl)amino]-4,6-dimethylthieno[2,3-b]pyridine-3-carboxylic acid
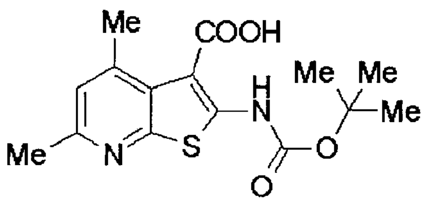

The title compound was obtained in the same manner as in Reference Example 47 from tert-butyl (3-bromo-4,6-dimethylthieno[2,3-b]pyridin-2-yl)carbamate obtained in Reference Example 61. yield 78%.
$^1$H NMR (DMSO-$d_6$) δ1.52 (9H, s), 2.48 (3H, s), 2.53 (3H, s), 7.09 (1H, s), 10.40 (1H, br).

Reference Example 63

2-chloro-3-cyano-5,6-dimethylpyridine
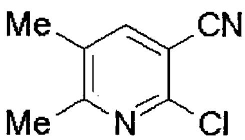

To a mixture of 28% sodium methoxide methanol solution (59 mL) and diethyl ether (380 mL) was added

dropwise a mixture of 2-butanone (20.9 g, 290 mmol) and ethyl formate (23.0 g, 310 mmol) over 45 min while maintaining an inside temperature at 4-5°C. The mixture was stirred at room temperature for 6 hr, and the resulting precipitate was collected by filtration. A solution of the solid, 2-cyanoacetamide (14.1 g, 168 mmol), piperidine (12.3 mL, 124 mmol) and acetic acid (7.50 g, 124 mmol) in water (336 mL) was heated under reflux for 17 hr. Then, acetic acid (26 mL) was added dropwise while maintaining an inside temperature at 65°C, and the mixture was cooled to room temperature. The resulting precipitate was collected by filtration and suspended in a mixed solvent of acetonitrile and diisopropyl ether, and the precipitate was collected by filtration, and dried under reduced pressure. The solid was added to phosphorus oxychloride (80 mL), and the mixture was stirred at 100°C for 6 hr. The reaction mixture was added to ice water, ethyl acetate was added thereto, and the mixture was neutralized with potassium carbonate. The aqueous layer was extracted with ethyl acetate. The combined organic layer was washed with saturated brine, and dried over magnesium sulfate. The solvent was evaporated under reduced pressure, and the residue was washed with diethyl ether and dried to give the title compound (16.1 g, yield 33%).
$^1$H NMR (DMSO-$d_6$) δ2.28 (3H, s), 2.50 (3H, s), 8.22 (1H, s).

Reference Example 64

ethyl 3-amino-5,6-dimethylthieno[2,3-b]pyridine-2-carboxylate
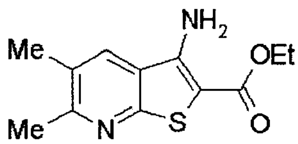

The title compound was obtained in the same manner as in Reference Example 43 from 2-chloro-3-cyano-5,6-dimethylpyridine obtained in Reference Example 63. yield 83%.
$^1$H NMR (DMSO-$d_6$) δ1.29 (3H, t, J = 7.2 Hz), 2.34 (3H, s), 2.53 (3H, s), 4.25 (2H, q, J = 7.2 Hz), 7.17 (2H, br), 8.24 (1H, s).

Reference Example 65

ethyl 3-bromo-5,6-dimethylthieno[2,3-b]pyridine-2-carboxylate
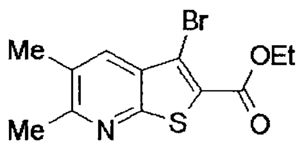

The title compound was obtained in the same manner as in Reference Example 44 from ethyl 3-amino-5,6-dimethylthieno[2,3-b]pyridine-2-carboxylate obtained in Reference Example 64. yield 48%.
$^1$H NMR (DMSO-$d_6$) δ1.35 (3H, t, J = 7.0 Hz), 2.44 (3H, s), 2.61 (3H, s), 4.38 (2H, q, J = 7.0 Hz), 8.03 (1H, s).

Reference Example 66

3-bromo-5,6-dimethylthieno[2,3-b]pyridine-2-carboxylic acid
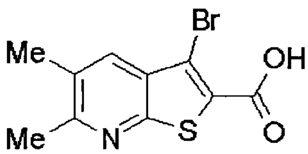

The title compound was obtained in the same manner as in Reference Example 45 from ethyl 3-bromo-5,6-dimethylthieno[2,3-b]pyridine-2-carboxylate obtained in Reference Example 65. yield 99%.
$^1$H NMR (DMSO-$d_6$) δ2.42 (3H, s), 2.57 (3H, s), 7.95 (1H, s), 14.08 (1H, br).

Reference Example 67

tert-butyl (3-bromo-5,6-dimethylthieno[2,3-b]pyridin-2-yl)carbamate
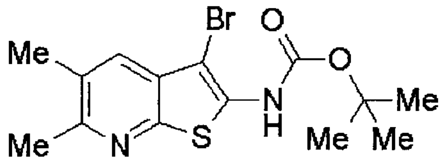

The title compound was obtained in the same manner as in Reference Example 46 from 3-bromo-5,6-dimethylthieno[2,3-b]pyridine-2-carboxylic acid obtained in Reference Example 66. yield 47%.
$^1$H NMR ($CDCl_3$) δ1.57 (9H, s), 2.39 (3H, s), 2.59 (3H, s), 7.54 (1H, s).

Reference Example 68

2-[(tert-butoxycarbonyl)amino]-5,6-dimethylthieno[2,3-b]pyridine-3-carboxylic acid
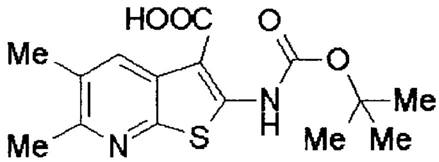

The title compound was obtained in the same manner as in Reference Example 47 from tert-butyl (3-bromo-5,6-dimethylthieno[2,3-b]pyridin-2-yl)carbamate obtained in Reference Example 67. yield 80%.
$^1$H NMR (DMSO-$d_6$) δ1.57 (9H, s), 2.34 (3H, s), 2.50 (3H, s), 8.20 (1H, s), 11.10 (1H, br), 13.80 (1H, br).

Reference Example 69

optically active form of tert-butyl 4-[2-ethyl-1-oxo-2,7-diazaspiro[4.5]dec-7-yl]piperidine-1-carboxylate
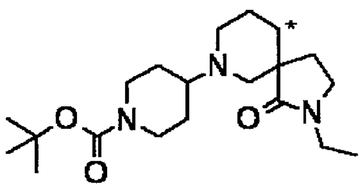

The title compound (2.38 g, yield 71%) as a yellow oil was obtained by an operation in the same manner as in Reference Example 5 and using tert-butyl 4-(1-oxo-2,7-diazaspiro[4.5]dec-7-yl)piperidine-1-carboxylate in an optically active form (long retention time) (3.08 g, 9.13 mmol) obtained in Reference Example 4.
EI(pos) 366 $[M+H]^+$

Reference Example 70

2-amino-6-methylthieno[2,3-b]pyridine-3-carboxylic acid trifluoroacetate
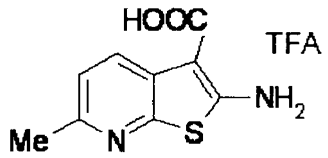

Trifluoroacetic acid (80 mL) was added to 2-[(tert-butoxycarbonyl)amino]-6-methylthieno[2,3-b]pyridine-3-carboxylic acid (10.2 g, 33.1 mmol) obtained in Reference Example 47, and the mixture was stirred for 1 hr. The insoluble material was filtered off, and the filtrate was concentrated under reduced pressure. Ethyl acetate was added to the residue, and the resulting crystals were collected, and washed with diisopropyl ether to give the title compound (6.76 g, yield 63%) as yellow crystals.
$^1$H NMR (DMSO-$d_6$) δ2.45 (3H, s), 7.12 (1H, d, J = 8.4 Hz), 8.04 (1H, d, J = 8.4 Hz).
Calculated C; 41.00, H; 2.81, N; 8.69
Found C; 41.29, H; 2.82, N; 8.75.

Reference Example 71

ethyl 3-bromo-6-methylthieno[2,3-b]pyridine-2-carboxylate 7-oxide
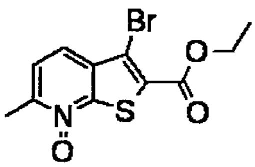

To a solution of ethyl 3-bromo-6-methylthieno[2,3-b]pyridine-2-carboxylate (2.00 g, 6.67 mmol) obtained in Reference Example 44 in dichloromethane (20 mL) was added m-chloroperbenzoic acid (2.14 g, 8.67 mmol) under ice-cooling, and the mixture was stirred at room temperature for 1 hr. The reaction mixture was concentrated under reduced pressure, and the obtained residue was purified by silica gel column chromatography (ethyl acetate:hexane=2:39 to 1:0) to give the title compound (1.97 g, yield 93%) as a white solid.
EI(pos) 318 $[M+H]^+$

Reference Example 72

ethyl 6-[(acetyloxy)methyl]-3-bromothieno[2,3-b]pyridine-2-carboxylate
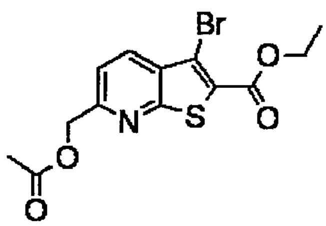

A solution of ethyl 3-bromo-6-methylthieno[2,3-b]pyridine-2-carboxylate 7-oxide (1.97 g, 6.23 mmol) obtained in Reference Example 71 in acetic anhydride (15 mL) was heated at 110°C for 1 hr. The reaction mixture was concentrated and the residue was purified by basic silica gel column chromatography (ethyl acetate) to give the title compound (1.71 g, yield 76%) as a yellow solid.

$^1$H NMR ($CDCl_3$) δ1.45 (3H, t, J = 7.1 Hz), 2.20 (3H, s), 4.46 (2H, q, J = 7.1 Hz), 5.36 (2H, s), 7.05 (1H, d, J = 8.5 Hz), 8.22 (1H, d, J = 8.5 Hz).

Reference Example 73

ethyl 3-bromo-6-(hydroxymethyl)thieno[2,3-b]pyridine-2-carboxylate
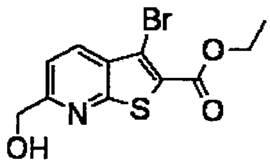

To a solution of ethyl 6-[(acetyloxy)methyl]-3-bromothieno[2,3-b]pyridine-2-carboxylate (1.71 g, 4.78 mmol) obtained in Reference Example 72 in ethanol (30 mL) was added potassium carbonate (1.32 g, 9.55 mmol), and the mixture was heated at 60°C for 1 hr. The reaction mixture was filtered, and the filtrate was concentrated. The obtained residue was purified by basic silica gel column chromatography (ethyl acetate) to give the title compound (1.46 g, yield 96%) as a yellow solid.
$^1$H NMR ($CDCl_3$) δ1.45 (3H, t, J = 7.0 Hz), 3.42-3.54 (1H, m), 4.46 (2H, q, J = 7.2 Hz), 4.94 (2H, d, J = 5.3 Hz), 7.40 (1H, d, J = 8.7 Hz), 8.20 (1H, d, J = 8.3 Hz).

Reference Example 74

ethyl 3-bromo-6-({[tert-butyl(diphenyl)silyl]oxy}methyl)thieno[2,3-b]pyridine-2-carboxylate
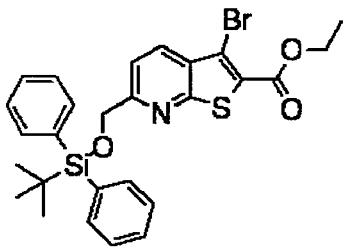

To a solution of ethyl 3-bromo-6-(hydroxymethyl)thieno[2,3-b]pyridine-2-carboxylate (1.46 g, 4.62 mmol) in DMF (10 mL) obtained in Reference Example 73 were added imidazole (0.63 g, 9.24 mmol) and tert-butyl(chloro)diphenylsilane (1.44 mL, 5.54 mmol), and the mixture was stirred at room temperature for 30 min. The reaction mixture was diluted with ethyl acetate, washed 3 times with saturated brine, and dried over anhydrous sodium sulfate. The solvent was evaporated under reduced pressure, and the obtained residue was purified by silica gel column chromatography (ethyl acetate:hexane=1:19 to 1:3) to give the title compound (2.48 g, yield 96%) as an oil.
$^1$H NMR ($CDCl_3$) δ1.15 (9H, s), 1.43 (3H, t, J = 7.2 Hz), 4.44 (2H, q, J = 7.1 Hz), 5.00 (2H, s), 7.34-7.46 (6H, m), 7.67-7.70 (4H, m), 7.86 (1H, d, J = 8.5 Hz), 8.26 (1H, d, J = 8.5 Hz).

Reference Example 75

tert-butyl [3-bromo-6-({[tert-butyl(diphenyl)silyl]oxy}methyl)thieno[2,3-b]pyridin-2-yl]carbamate
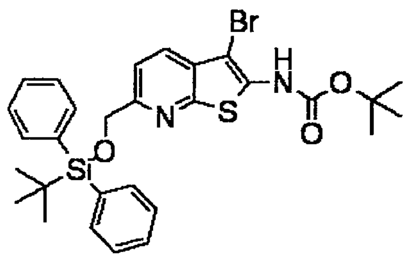

The title compound (1.98 g, yield 74%) as an oil was obtained in the same manner as in Reference Examples 45 and 46 and using ethyl 3-bromo-6-({[tert-butyl(diphenyl)silyl]oxy}methyl)thieno[2,3-b]pyridine-2-carboxylate (2.48 g, 4.48 mmol) obtained in Reference Example 74. $^1H$ NMR ($CDCl_3$) δ1.13 (9H, s), 1.57 (9H, s), 4.97 (2H, s), 7.33-7.45 (7H, m), 7.68-7.71 (5H, m), 7.86 (1H, d, J = 8.1 Hz).

Reference Example 76

2-[(tert-butoxycarbonyl)amino]-6-({[tert-butyl(diphenyl)silyl]oxy}methyl)thieno[2,3-b]pyridine-3-carboxylic acid

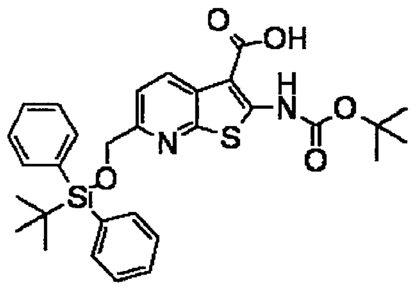

The title compound (1.68 g, yield 89%) as an oil was obtained in the same manner as in Reference Example 47 and using tert-butyl [3-bromo-6-({[tert-butyl(diphenyl)silyl]oxy}methyl)thieno[2,3-b]pyridin-2-yl]carbamate (1.98 g, 3.32 mmol) obtained in Reference Example 75.
EI(pos) 563 $[M+H]^+$

Reference Example 77

ethyl 3-amino-6-(trifluoromethyl)thieno[2,3-b]pyridine-2-carboxylate

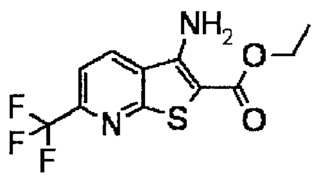

The title compound (6.29 g, yield 85%) as a yellow solid was obtained in the same manner as in Reference Example 43 and using 2-chloro-6-(trifluoromethyl)nicotinonitrile (5.25 g, 25.5 mmol).
$^1H$ NMR (DMSO-$d_6$) δ 1.32 (3H, t, J = 7.1 Hz), 4.32 (2H, q, J = 7.1 Hz), 7.44 (2H, br), 7.99 (1H, d, J = 8.5 Hz), 8.84 (1H, d, J = 8.5 Hz).

Reference Example 78

ethyl 3-bromo-6-(trifluoromethyl)thieno[2,3-b]pyridine-2-carboxylate

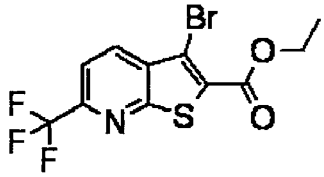

The title compound (5.75 g, yield 75%) as a yellow solid was obtained in the same manner as in Reference Example 44 and using ethyl 3-amino-6-(trifluoromethyl)thieno[2,3-b]pyridine-2-carboxylate (6.28 g, 21.7 mmol) obtained in Reference Example 77.
$^1$H NMR ($CDCl_3$) δ 1.46 (3H, t, J = 7.2 Hz), 4.49 (2H, q, J = 6. 91 Hz), 7.81 (1H, d, J = 8.3 Hz), 8.40 (1H, d, J = 8.3 Hz).

Reference Example 79

tert-butyl [3-bromo-6-(trifluoromethyl)thieno[2,3-b]pyridin-2-yl]carbamate
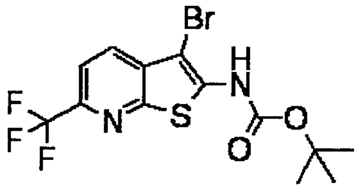

The title compound (5.38 g, yield 91%) as a yellow solid was obtained by an operation in the same manner as in Reference Examples 45 and 46 and using ethyl 3-bromo-6-(trifluoromethyl)thieno[2,3-b]pyridine-2-carboxylate (6.40 g, 18.2 mmol) obtained in Reference Example 78.
$^1$H NMR (DMSO-$d_6$) δ 7.96 (1H, d, J = 8.3 Hz), 8.36 (1H, d, J = 8.3 Hz).

Reference Example 80

2-[(tert-butoxycarbonyl)amino]-6-(trifluoromethyl)thieno[2,3-b]pyridine-3-carboxylic acid
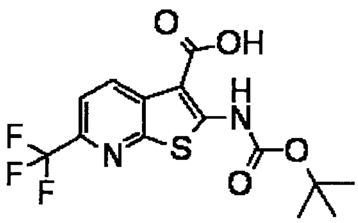

The title compound (1.11 g, yield 67%) as a yellow solid was obtained by an operation in the same manner as in Reference Example 47 and using tert-butyl [3-bromo-6-(trifluoromethyl)thieno[2,3-b]pyridin-2-yl]carbamate (1.80 g, 4.55 mmol) obtained in Reference Example 79.
$^1$H NMR (DMSO-$d_6$) δ 1.56 (9H, s), 7. 92 (1H, d, J = 8.7 Hz), 8.66 (1H, d, J = 8.5 Hz), 11.06 (1H, br).

Reference Example 81

1-benzyl 3-ethyl 3-(2-tert-butoxy-2-oxoethyl)piperidine-1,3-dicarboxylate
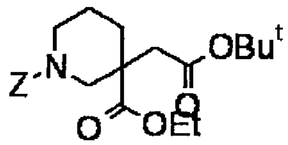

To a solution of 1-benzyl 3-ethyl piperidine-1,3-dicarboxylate (100 g, 343 mmol) in cyclopentyl methyl ether (300 mL) was added a 1.1M solution (312 mL, 343 mmol) of lithium bis(trimethylsilyl)amide in THF at -78°C over 50 min, and the mixture was stirred at the same temperature for 1 hr. To this solution was added t-butyl bromoacetate (50.7 mL, 343 mmol) at -78°C, and the mixture was stirred for 2 hr. Water (100 mL) was added thereto to quench the reaction and the mixture was separated between ethyl acetate (400 mL) and water (300 mL). The aqueous layer was extracted with ethyl acetate (100 mL), and the combined extract was washed with 1N hydrochloric acid (350 mL x 3), 10% aqueous potassium carbonate solution (300 mL x 1) and saturated brine (300 mL x 1), and dried over anhydrous sodium sulfate. The solvent was evaporated under reduced pressure, and the obtained residue was dissolved in ethanol (500 mL). At room temperature, 2N aqueous sodium hydroxide solution (172 mL) was added thereto, and the mixture was stirred for 16 hr. Ethanol was evaporated, 10% aqueous potassium carbonate solution (100 mL) was added, and the mixture was extracted with ethyl acetate (500 mL). The aqueous layer was extracted with ethyl acetate (100 mL), and the combined extract was washed with 10% aqueous potassium carbonate solution (200 mL x 2) and saturated brine (200 mL x 1), and dried over anhydrous sodium sulfate. The solution was applied to basic silica gel column chromatography (30 g, eluent; ethyl acetate) to give the title compound (74.5 g, yield 54%) as a colorless oil.
EI(pos) 306 $[M-Bu^t+H]^+$

Reference Example 82

1'-benzyl 3-ethyl 3-(2-tert-butoxy-2-oxoethyl)-1,4'-bipiperidine-1',3-dicarboxylate
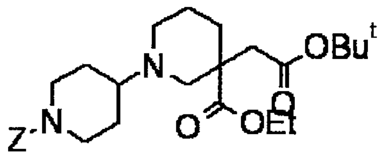

To a solution of 1-benzyl 3-ethyl 3-(2-tert-butoxy-2-oxoethyl)piperidine-1,3-dicarboxylate (74.5 g, 184 mmol) obtained in Reference Example 81 in THF (500 mL) was added 10% Pd-C (50% containig water, 10 g) under a nitrogen atmosphere, and the mixture was stirred under a hydrogen atmosphere for 4 hr. The catalyst was filtered off through celite, and the filtrate was concentrated under reduced pressure. Toluene (100 mL x 2) was added to the residue and the mixture was concentrated under reduced pressure to give an oil (49.9 g).
To a solution of the obtained amine form and Z-4-piperidone (42.9 g, 184 mmol) in THF (400 mL) was added NaBH $(OAc)_3$ (58.5 g, 276 mmol), and the mixture was stirred at room temperature for 1 day. The solvent was concentrated to about half amount, 10% aqueous potassium carbonate solution (600 mL) was slowly added and the mixture was stirred and extracted with ethyl acetate. The organic layer was washed with 10% aqueous potassium carbonate solution and saturated brine, and dried over anhydrous sodium sulfate. The solvent was evaporated under reduced pressure, and the obtained residue was purified by silica gel column chromatography (200 g, eluent; ethyl acetate:hexane=1:3→3:1) to give the title compound (82.6 g, yield 92%) as an oil.
EI(pos) 433 $[M-Bu^t+H]^+$

Reference Example 83

[1'-[(benzyloxy)carbonyl]-3-(ethoxycarbonyl)-1,4'-bipiperidin-3-yl]acetic acid hydrochloride
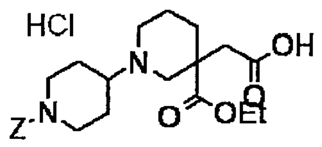

1'-Benzyl 3-ethyl 3-(2-tert-butoxy-2-oxoethyl)-1,4'-bipiperidine-1',3-dicarboxylate (31.7 g, 64.8 mmol) obtained in Reference Example 82 was dissolved in ethyl acetate (50 mL), 4N HCl-AcOEt (300 mL) was added, and the mixture was stood for 5 hr and concentrated under reduced pressure. Toluene (100 mL x 2) was added to the residue and the mixture was concentrated under reduced pressure. Diisopropyl ether (600 mL) was added, and the mixture was vigorously stirred for 16 hr. The obtained powder was collected, and washed with diisopropyl ether to give the title compound (29.9 g, yield 98%) as a powder
EI(pos) 433 $[M+H]^+$

Reference Example 84

1'-benzyl 3-ethyl 3-[2-(isopropylamino)-2-oxoethyl]-1,4'-bipiperidine-1',3-dicarboxylate
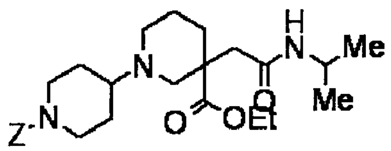

To a solution of [1'-[(benzyloxy)carbonyl]-3-(ethoxycarbonyl)-1,4'-bipiperidin-3-yl]acetic acid hydrochloride (54.4 g, 116 mmol) obtained in Reference Example 83 and HOBt (17.2 mg, 128 mmol) in DMF (110 mL) were added TEA (16.1 mL, 116 mmol) and isopropylamine (14.8 mL, 174 mmol) under ice-cooling, and then WSC (24.5 g, 128 mmol) was added, and the mixture was stirred at room temperature for 16 hr. The reaction mixture was diluted with ethyl acetate, and the mixture was washed with 10% aqueous potassium carbonate solution and saturated brine, and dried over anhydrous sodium sulfate. The solvent was evaporated under reduced pressure, and the obtained residue was purified by silica gel column chromatography (eluent; ethyl acetate:hexane=1:3 to 1:0) to give the title compound (37.7 g, yield 69%) as an oil.
EI(pos) 474 $[M+H]^+$

Reference Example 85

benzyl 4-(2-isopropyl-1,3-dioxo-2,7-diazaspiro[4.5]dec-7-yl)piperidine-1-carboxylate
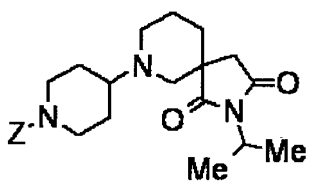

A solution of 1'-benzyl 3-ethyl 3-[2-(isopropylamino)-2-oxoethyl]-1,4'-bipiperidine-1',3-dicarboxylate (1.40 g, 2.96 mmol) obtained in Reference Example 84 in THF (2 mL) was added to a solution of 60% sodium hydride (118 mg, 2.96 mmol) in THF (5 mL) under ice-cooling. After cease of foaming (about 10 min), the mixture was stirred at room temperature for 10 min. The reaction was quenched with ice, the reaction mixture was extracted with ethyl acetate, and the extract was washed with saturated brine, and dried over anhydrous sodium sulfate. The solution was applied to basic silica gel column chromatography (5 g, eluent; ethyl acetate). The solvent was evaporated under reduced pressure,

and the obtained residue was dissolved in diisopropyl ether (2 mL). A small amount of crystal was added and the mixture was stirred for 16 hr. Hexane (6 mL) was added and the mixture was stirred. The crystals were collected, and washed with diisopropyl ether:hexane=1:3 to give the title compound (975 mg, yield 77%) as crystals.
EI(pos) 428 $[M+H]^+$

Reference Example 86

optically active forms (two kinds) of benzyl 4-(2-isopropyl-1,3-dioxo-2,7-diazaspiro[4.5]dec-7-yl)piperidine-1-carboxylate
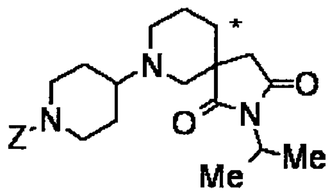

Benzyl 4-(2-isopropyl-1,3-dioxo-2,7-diazaspiro[4.5]dec-7-yl)piperidine-1-carboxylate (racemate) (42.8 g) obtained in Reference Example 85 was optically resolved by HPLC under the following conditions to give two kinds of optically active forms having "long retention time (21 g)" and "short retention time (21 g)".

<preparative HPLC conditions>

column: CHIRALCEL OJ (50 mmID×500 mmL)
mobile phase: hexane:ethanol=85:15
flow rate: 80 mL/min
column temperature: 40°C
detection: UV 220 nm
compound injection volume: 800 mg/80 mL (hexane:ethanol=85:15)

<HPLC analysis conditions>

column: CHIRALCEL OJ (4.6 mmID×250 mmL)
mobile phase: hexane:ethanol=50:50
flow rate: 0.7 mL/min
column temperature: 35°C
detection: UV 220 nm
retention time of "long retention time": 25.1 min
retention time of "short retention time": 15.5 min

Reference Example 87

optically active form of 2-isopropyl-7-(piperidin-4-yl)-2,7-diazaspiro[4.5]decane-1,3-dione
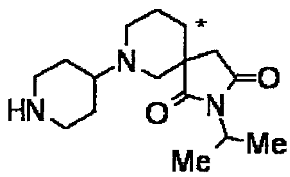

THF (250 mL) was added to benzyl 4-(2-isopropyl-1,3-dioxo-2,7-diazaspiro[4.5]dec-7-yl)piperidine-1-carboxylate in an optically active form (long retention time) (21.3 g, 49.8 mmol) obtained in Reference Example 86 and 10%

palladium carbon (50% water-containing product, 5.0 g), and the mixture was stirred under a hydrogen atmosphere for 3 hr. The reaction mixture was filtered, and the filtrate was concentrated under reduced pressure to give the title compound (14.6 g, quantitative) as an oil. The compound was used for the next step without purification.

Reference Example 88

optically active form of 2-isopropyl-7-(piperidin-4-yl)-2,7-diazaspiro[4.5]decane-1,3-dione
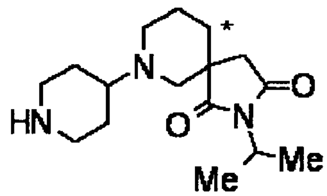

THF (15 mL) was added to benzyl 4-(2-isopropyl-1,3-dioxo-2,7-diazaspiro[4.5]dec-7-yl)piperidine-1-carboxylate in an optically active form (short retention time) (2.33 g, 5.45 mmol) obtained in Reference Example 86 and 10% palladium carbon (50% water-containing product, 500 mg), and the mixture was stirred under a hydrogen atmosphere for 2 hr. The reaction mixture was filtered, and the filtrate was concentrated under reduced pressure to give the title compound (1.60 g, quantitatively) as an oil. The compound was used for the next step without purification.

Example 1

optically active form of tert-butyl (3-{[4-(2-isopropyl-1,3-dioxo-2,7-diazaspiro[4.5]dec-7-yl)piperidin-1-yl]carbonyl}thieno[2,3-b]pyridin-2-yl)carbamate
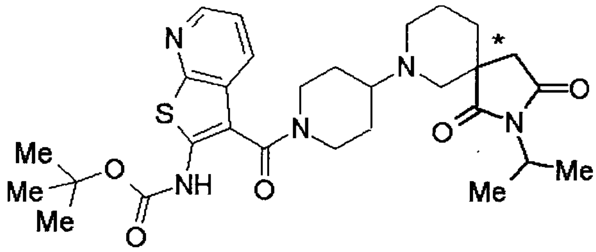

To a solution of 2-isopropyl-7-(piperidin-4-yl)-2,7-diazaspiro[4.5]decane-1,3-dione dihydrochloride in an optically active form (900 mg, 2.46 mmol) obtained in Reference Example 12, 2-[(tert-butoxycarbonyl)amino]thieno[2,3-b]pyridine-3-carboxylic acid (723 mg, 2.46 mmol) obtained in Reference Example 52, 1-hydroxybenzotriazole (332 mg, 2.46 mmol) and triethylamine (0.86 mL, 6.14 mmol) in DMF (4 mL) was added 1-ethyl-3-(3'-dimethylaminopropyl)carbodiimide hydrochloride (471 mg, 2.46 mmol), and the mixture was stirred at room temperature for 16 hr. The reaction mixture was diluted with ethyl acetate, and the mixture was washed with 10% aqueous potassium carbonate solution and saturated brine, and dried over anhydrous sodium sulfate. The solution was applied to basic silica gel column chromatography (ethyl acetate). The solvent was evaporated under reduced pressure, and the obtained residue was purified by silica gel column chromatography (hexane:ethyl acetate=3:1 to ethyl acetate), and triturated with hexane to give the title compound (910 mg, yield 65%).
EI(pos) 570.1 $[M+H]^+$

Example 2

optically active form of 7-{1-[(2-aminothieno[2,3-b]pyridin-3-yl)carbonyl]piperidin-4-yl}-2-isopropyl-2,7-diazaspiro[4.5]decane-1,3-dione
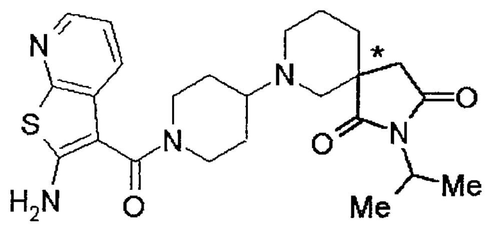

To tert-butyl (3-{[4-(2-isopropyl-1,3-dioxo-2,7-diazaspiro[4.5]dec-7-yl)piperidin-1-yl]carbonyl}thieno[2,3-b]pyridin-2-yl)carbamate in an optically active form (910 mg, 1.60 mmol) obtained in Example 1 was added trifluoroacetic acid (10 mL), and 30 min later, the mixture was concentrated under reduced pressure. The obtained oil was dissolved in ethyl acetate, and the solution was washed with 10% aqueous potassium carbonate solution and saturated brine, and dried over anhydrous sodium sulfate. The solvent was evaporated under reduced pressure, and the residue was triturated with diisopropyl ether to give the title compound (746 mg, yield 99%).
EI(pos) 470.0 $[M+H]^+$

Example 3

optically active form of N-(3-{[4-(2-isopropyl-1,3-dioxo-2,7-diazaspiro[4.5]dec-7-yl)piperidin-1-yl]carbonyl} thieno[2,3-b]pyridin-2-yl)-N'-methylurea
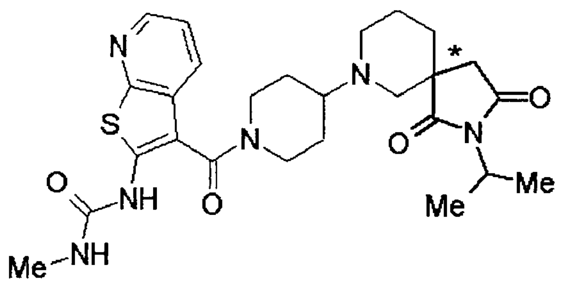

To a solution of 7-{1-[(2-aminothieno[2,3-b]pyridin-3-yl)carbonyl]piperidin-4-yl}-2-isopropyl-2,7-diazaspiro[4.5] decane-1,3-dione in an optically active form (200 mg, 0.43 mmol) obtained in Example 2 in pyridine (2 mL) was added methyl isocyanate (0.077 mL, 1.28 mmol), and the mixture was heated at 60°C for 16 hr. The solvent was evaporated under reduced pressure, and the obtained residue was purified by basic silica gel column chromatography (hexane: ethyl acetate=3:1 to ethyl acetate), and triturated with diisopropyl ether to give the title compound (160 mg, yield 72%).
EI(pos) 527.1 $[M+H]^+$

Example 4

optically active form of N-ethyl-N'-(3-{[4-(2-isopropyl-1,3-dioxo-2,7-diazaspiro[4.5]dec-7-yl)piperidin-1-yl]carbonyl}thieno[2,3-b]pyridin-2-yl)urea
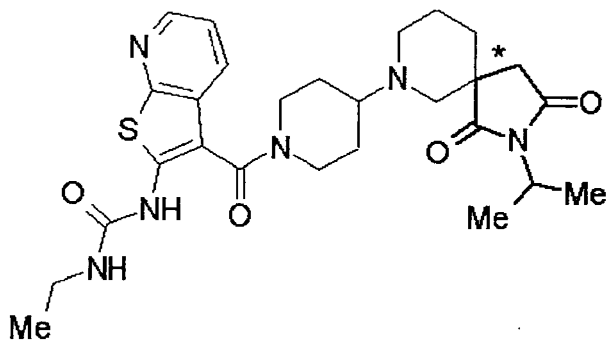

The title compound (159 mg, yield 69%) was obtained by an operation in the same manner as in Example 3 and using 7-{1-[(2-aminothieno[2,3-b]pyridin-3-yl)carbonyl]piperidin-4-yl}-2-isopropyl-2,7-diazaspiro[4.5]decane-1,3-dione in an optically active form (200 mg, 0.43 mmol) obtained in Example 2 and ethyl isocyanate, followed by trituration with diisopropyl ether. EI(pos) 541.1 $[M+H]^+$

Example 5

tert-butyl (3-{[4-(3-tert-butyl-2,4-dioxo-1-oxa-3,7-diazaspiro[4.5]dec-7-yl)piperidin-1-yl]carbonyl}thieno[2,3-b]pyridin-2-yl)carbamate

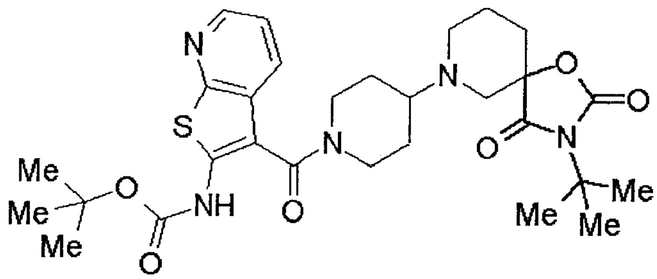

The title compound (702 mg, yield 92%) as an oil was obtained by an operation in the same manner as in Example 1 and using 3-tert-butyl-7-(piperidin-4-yl)-1-oxa-3,7-diazaspiro[4.5]decane-2,4-dione dihydrochloride (500 mg, 1.31 mmol) obtained in Reference Example 16 and 2-[(tert-butoxycarbonyl)amino]thieno[2,3-b]pyridine-3-carboxylic acid (385 mg, 1.31 mmol) obtained in Reference Example 52.
EI(pos) 586.1 $[M+H]^+$

Example 6

7-{1-[(2-aminothieno[2,3-b]pyridin-3-yl)carbonyl]piperidin-4-yl}-3-tert-butyl-1-oxa-3,7-diazaspiro[4.5]decane-2,4-dione

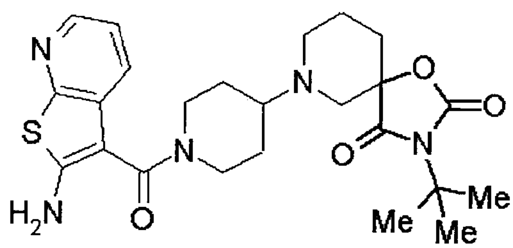

The title compound (412 mg, yield 71%) was obtained by an operation in the same manner as in Example 2

and using tert-butyl (3-{[4-(3-tert-butyl-2,4-dioxo-1-oxa-3,7-diazaspiro[4.5]dec-7-yl)piperidin-1-yl]carbonyl}thieno[2,3-b]pyridin-2-yl)carbamate (702 mg, 1.20 mmol) obtained in Example 5, followed by trituration with hexane.
EI(pos) 486.0 $[M+H]^+$

Example 7

N-(3-{[4-(3-tert-butyl-2,4-dioxo-1-oxa-3,7-diazaspiro[4.5]dec-7-yl)piperidin-1-yl]carbonyl}thieno[2,3-b]pyridin-2-yl)-N'-methylurea
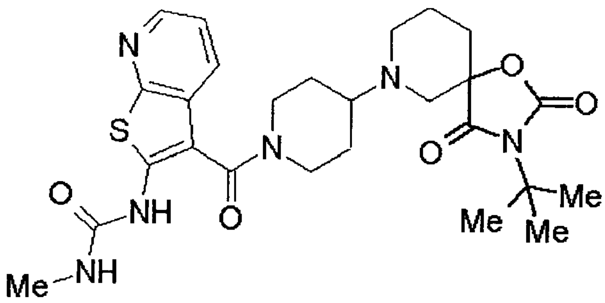

The title compound (176 mg, yield 78%) was obtained by an operation in the same manner as in Example 3 and using 7-{1-[(2-aminothieno[2,3-b]pyridin-3-yl)carbonyl]piperidin-4-yl}-3-tert-butyl-1-oxa-3,7-diazaspiro[4.5]decane-2,4-dione (200 mg, 0.41 mmol) obtained in Example 6, followed by trituration with diisopropyl ether and hexane.
EI(pos) 543.1 $[M+H]^+$

Example 8

tert-butyl (3-{[4-(3-isopropyl-2,4-dioxo-1-thia-3,7-diazaspiro[4.5]dec-7-yl)piperidin-1-yl]carbonyl}thieno[2,3-b]pyridin-2-yl)carbamate
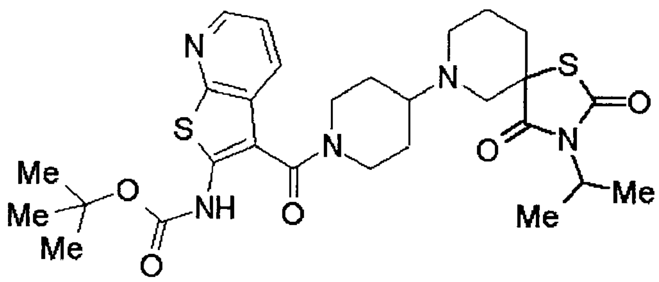

The title compound (765 mg, quantitative) as an oil was obtained by an operation in the same manner as in Example 1 and using 3-isopropyl-7-(piperidin-4-yl)-1-thia-3,7-diazaspiro[4.5]decane-2,4-dione dihydrochloride (500 mg, 1.30 mmol) obtained in Reference Example 23 and 2-[(tert-butoxycarbonyl)amino]thieno[2,3-b]pyridine-3-carboxylic acid (383 mg, 1.30 mmol) obtained in Reference Example 52.
EI(pos) 588.0 $[M+H]^+$

Example 9

7-{1-[(2-aminothieno[2,3-b]pyridin-3-yl)carbonyl]piperidin-4-yl}-3-isopropyl-1-thia-3,7-diazaspiro[4.5]decane-2,4-dione
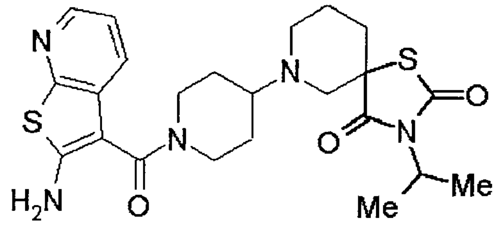

The title compound (599 mg, yield 94%) was obtained by an operation in the same manner as in Example 2 and using tert-butyl (3-{[4-(3-isopropyl-2,4-dioxo-1-thia-3,7-diazaspiro[4.5]dec-7-yl)piperidin-1-yl]carbonyl}thieno[2,3-b]pyridin-2-yl)carbamate (765 mg, 1.30 mmol) obtained in Example 8, followed by trituration with hexane.
EI(pos) 488.0 [M+H]$^+$

Example 10

N-(3-{[4-(3-isopropyl-2,4-dioxo-1-thia-3,7-diazaspiro[4.5]dec-7-yl)piperidin-1-yl]carbonyl}thieno[2,3-b]pyridin-2-yl)urea
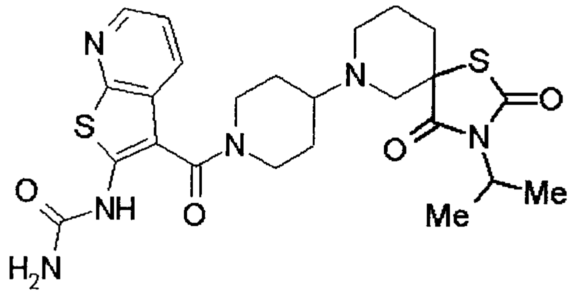

To a solution of 7-{1-[(2-aminothieno[2,3-b]pyridin-3-yl)carbonyl]piperidin-4-yl}-3-isopropyl-1-thia-3,7-diazaspiro[4.5]decane-2,4-dione (200 mg, 0.41 mmol) obtained in Example 9 in THF (2 mL) was added trichloroacetyl isocyanate (0.97 mL, 0.82 mmol) under ice-cooling, 7N ammonia-methanol (2 mL) was added 30 min later, and the mixture was stirred at room temperature for 3 hr. The reaction mixture was diluted with ethyl acetate, and the mixture was washed with 10% aqueous potassium carbonate solution and saturated brine, and dried over anhydrous sodium sulfate. This solution was purified by basic silica gel column chromatography (hexane:ethyl acetate=1:1 to ethyl acetate), and triturated with diisopropyl ether to give the title compound (176 mg, yield 81%).
EI(pos) 531.0 [M+H]$^+$

Example 11

N-(3-{[4-(3-isopropyl-2,4-dioxo-1-thia-3,7-diazaspiro[4.5]dec-7-yl)piperidin-1-yl]carbonyl}thieno[2,3-b]pyridin-2-yl)-N'-methylurea
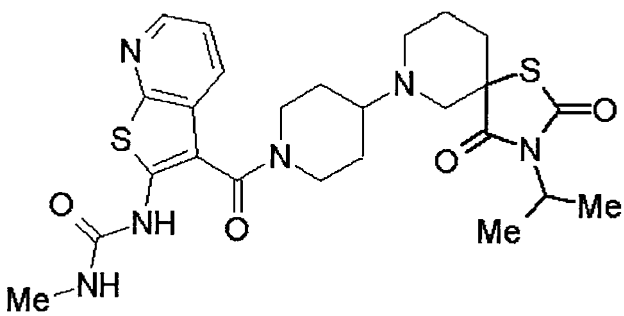

The title compound (161 mg, yield 72%) was obtained by an operation in the same manner as in Example 3 and using 7-{1-[(2-aminothieno[2,3-b]pyridin-3-yl)carbonyl]piperidin-4-yl}-3-isopropyl-1-thia-3,7-diazaspiro[4.5]decane-2,4-dione (200 mg, 0.41 mmol) obtained in Example 9, followed by trituration with hexane.
EI(pos) 545.0 [M+H]$^+$

Example 12

optically active form of tert-butyl (3-{[4-(3,3-dimethyl-1-oxo-2-oxa-7-azaspiro[4.5]dec-7-yl)piperidin-1-yl]carbonyl}thieno[2,3-b]pyridin-2-yl)carbamate
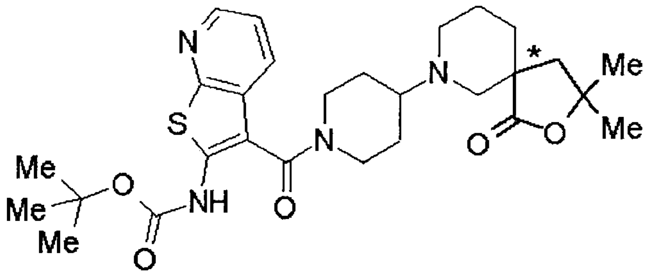

The title compound (767 mg, yield 96%) as an oil was obtained by an operation in the same manner as in Example 1 and using 3,3-dimethyl-7-(piperidin-4-yl)-2-oxa-7-azaspiro[4.5]decan-1-one dihydrochloride in an optically active form (500 mg, 1.47 mmol) obtained in Reference Example 29 and 2-[(tert-butoxycarbonyl)amino]thieno[2,3-b]pyridine-3-carboxylic acid (434 mg, 1.47 mmol) obtained in Reference Example 52.
EI(pos) 543.1 [M+H]$^+$

Example 13

optically active form of 7-{1-[(2-aminothieno[2,3-b]pyridin-3-yl)carbonyl]piperidin-4-yl}-3,3-dimethyl-2-oxa-7-azaspiro[4.5]decan-1-one
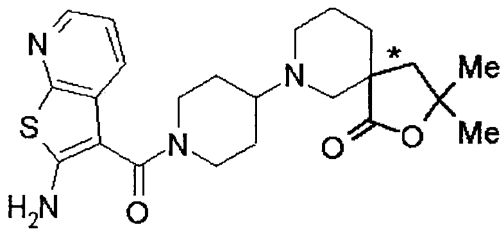

The title compound (580 mg, yield 93%) was obtained by an operation in the same manner as in Example 2 and using tert-butyl (3-{[4-(3,3-dimethyl-1-oxo-2-oxa-7-azaspiro[4.5]dec-7-yl)piperidin-1-yl]carbonyl}thieno[2,3-b]pyridin-2-yl)carbamate in an optically active form (767 mg, 1.41 mmol) obtained in Example 12, followed by trituration with diisopropyl ether and hexane.
EI(pos) 443.1 [M+H]$^+$

Example 14

optically active form of N-(3-{[4-(3,3-dimethyl-1-oxo-2-oxa-7-azaspiro[4.5]dec-7-yl)piperidin-1-yl]carbonyl}thieno[2,3-b]pyridin-2-yl)-N'-methylurea
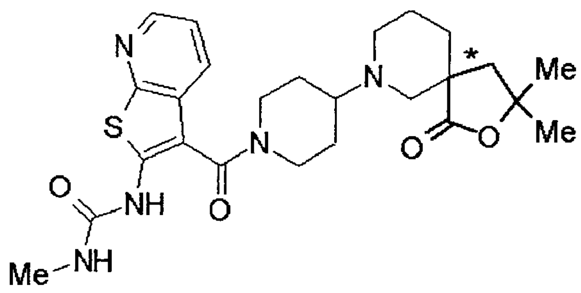

The title compound (522 mg, yield 81%) was obtained by an operation in the same manner as in Example 3 and using 7-{1-[(2-aminothieno[2,3-b]pyridin-3-yl)carbonyl]piperidin-4-yl}-3,3-dimethyl-2-oxa-7-azaspiro[4.5]decan-1-one in an optically active form (570 mg, 1.29 mmol) obtained in Example 13, followed by trituration with diisopropyl ether.
EI(pos) 500.0 $[M+H]^+$

Example 15

optically active form of tert-butyl (3-{[4-(2-isopropyl-1,3-dioxo-2,7-diazaspiro[4.5]dec-7-yl)piperidin-1-yl]carbonyl}-6-methylthieno[2,3-b]pyridin-2-yl)carbamate
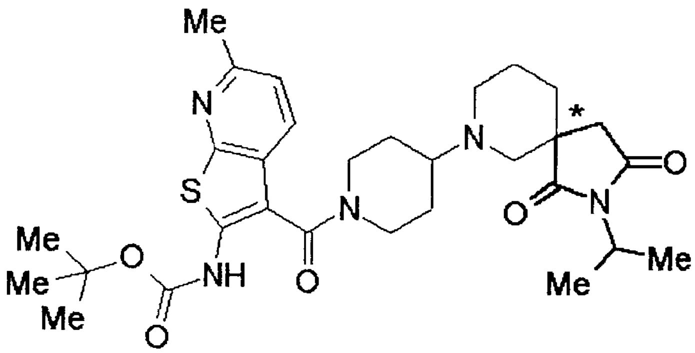

The title compound (555 mg, yield 58%) as an oil was obtained by an operation in the same manner as in Example 1 and using 2-isopropyl-7-(piperidin-4-yl)-2,7-diazaspiro[4.5]decane-1,3-dione dihydrochloride in an optically active form (600 mg, 1.64 mmol) obtained in Reference Example 12 and 2-[(tert-butoxycarbonyl)amino]-6-methylthieno [2,3-b]pyridine-3-carboxylic acid (556 mg, 1.80 mmol) obtained in Reference Example 47.
$[\alpha]_D^{20}$ -8.4°(c 0.973, methanol).
EI(pos) 584.1 $[M+H]^+$

Example 16

optically active form of 7-{1-[(2-amino-6-methylthieno[2,3-b]pyridin-3-yl)carbonyl]piperidin-4-yl}-2-isopropyl-2,7-diazaspiro[4.5]decane-1,3-dione
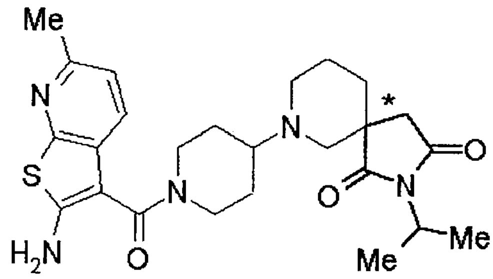

The title compound (350 mg, yield 76%) was obtained by an operation in the same manner as in Example 2 and using tert-butyl (3-{[4-(2-isopropyl-1,3-dioxo-2,7-diazaspiro[4.5]dec-7-yl)piperidin-1-yl]carbonyl}-6-methylthieno [2,3-b]pyridin-2-yl)carbamate in an optically active form (555 mg, 0.95 mmol) obtained in Example 15, followed by trituration with diisopropyl ether and hexane.
$[\alpha]_D^{20}$ -8.9°(c 0.720, methanol).
EI(pos) 484.0 $[M+H]^+$

Example 17

optically active form of N-(3-{[4-(2-isopropyl-1,3-dioxo-2,7-diazaspiro[4.5]dec-7-yl)piperidin-1-yl]carbonyl}-6-methylthieno[2,3-b]pyridin-2-yl)urea
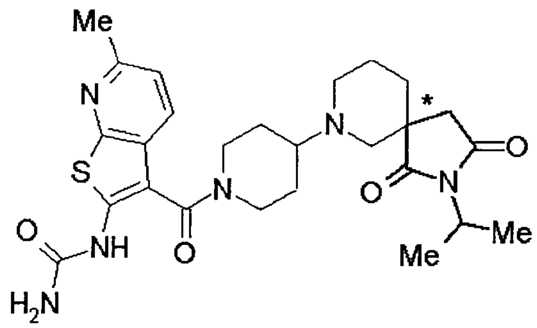

The title compound (121 mg, yield 62%) was obtained by an operation in the same manner as in Example 10 and using 7-{1-[(2-amino-6-methylthieno[2,3-b]pyridin-3-yl)carbonyl]piperidin-4-yl}-2-isopropyl-2,7-diazaspiro[4.5]decane-1,3-dione in an optically active form (180 mg, 0.37 mmol) obtained in Example 16, followed by trituration with diisopropyl ether.
EI(pos) 527.1 $[M+H]^+$

Example 18

optically active form of N-(3-{[4-(2-isopropyl-1,3-dioxo-2,7-diazaspiro[4.5]dec-7-yl)piperidin-1-yl]carbonyl}-6-methylthieno[2,3-b]pyridin-2-yl)-N'-methylurea
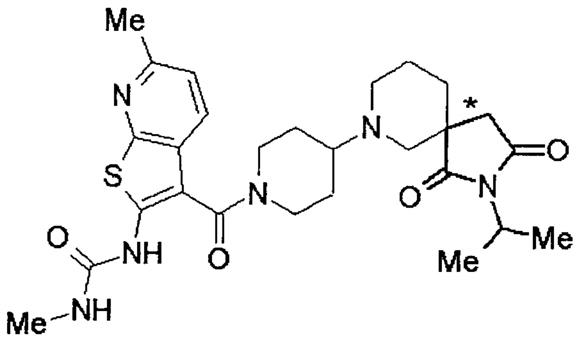

The title compound (145 mg, yield 86%) was obtained by an operation in the same manner as in Example 3 and using 7-{1-[(2-amino-6-methylthieno[2,3-b]pyridin-3-yl)carbonyl]piperidin-4-yl}-2-isopropyl-2,7-diazaspiro[4.5]decane-1,3-dione in an optically active form (150 mg, 0.31 mmol) obtained in Example 16, followed by trituration with diisopropyl ether.
EI(pos) 541.0 $[M+H]^+$
The obtained compound (100 mg) was recrystallized from ethyl acetate-hexane to give the title compound (84.4 mg) as a white solid.
melting point 205-207°C (decomposition)
$[\alpha]_D^{20}$-14.4° (c 1. 08, methanol).

Example 19

optically active form of N-ethyl-N'-(3-{[4-(2-isopropyl-1,3-dioxo-2,7-diazaspiro[4.5]dec-7-yl)piperidin-1-yl]carbonyl}-6-methylthieno[2,3-b]pyridin-2-yl)urea
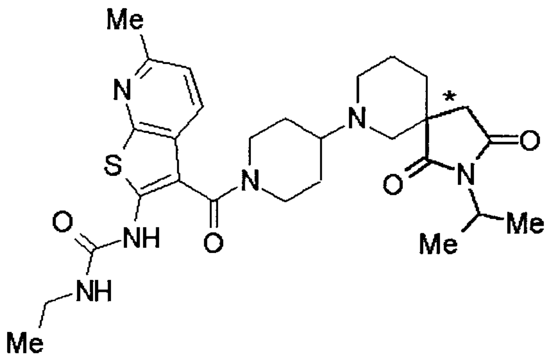

The title compound (135 mg, yield 74%) was obtained by an operation in the same manner as in Example 3 and using 7-{1-[(2-amino-6-methylthieno[2,3-b]pyridin-3-yl)carbonyl]piperidin-4-yl}-2-isopropyl-2,7-diazaspiro[4.5]decane-1,3-dione in an optically active form (159 mg, 0.33 mmol) obtained in Reference Example 16 and ethyl isocyanate, followed by trituration with diisopropyl ether and hexane.
EI(pos) 555.1 $[M+H]^+$

Example 20

optically active form of tert-butyl (3-{[4-(3-isopropyl-2,4-dioxo-1-oxa-3,7-diazaspiro[4.5]dec-7-yl)piperidin-1-yl]carbonyl}-6-methylthieno[2,3-b]pyridin-2-yl)carbamate
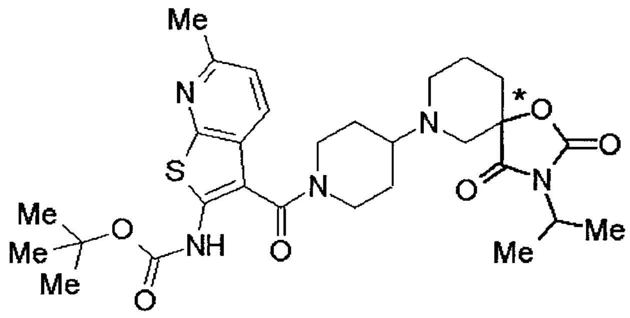

The title compound (920 mg, yield 58%) as an oil was obtained by an operation in the same manner as in Example 1 and using 3-isopropyl-7-(piperidin-4-yl)-1-oxa-3,7-diazaspiro[4.5]decane-2,4-dione dihydrochloride in an optically active form (1.00 g, 2.72 mmol) obtained in Example 18 and 2-[(tert-butoxycarbonyl)amino]-6-methylthieno[2,3-b]pyridine-3-carboxylic acid (837 mg, 2.72 mmol) obtained in Reference Example 47.
EI(pos) 586.1 $[M+H]^+$

Example 21

optically active form of 7-{1-[(2-amino-6-methylthieno[2,3-b]pyridin-3-yl)carbonyl]piperidin-4-yl}-3-isopropyl-1-oxa-3,7-diazaspiro[4.5]decane-2,4-dione
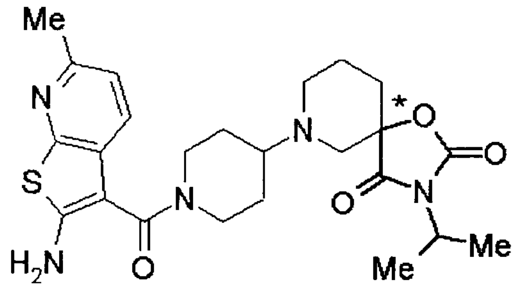

The title compound (617 mg, yield 81%) was obtained by an operation in the same manner as in Example 2 and using tert-butyl (3-{[4-(3-isopropyl-2,4-dioxo-1-oxa-3,7-diazaspiro[4.5]dec-7-yl)piperidin-1-yl]carbonyl}-6-methylthieno[2,3-b]pyridin-2-yl)carbamate in an optically active form (920 mg, 1.57 mmol) obtained in Example 20, followed by trituration with diisopropyl ether and hexane.
EI(pos) 486.0 $[M+H]^+$

Example 22

optically active form of N-(3-{[4-(3-isopropyl-2,4-dioxo-1-oxa-3,7-diazaspiro[4.5]dec-7-yl)piperidin-1-yl]carbonyl}-6-methylthieno[2,3-b]pyridin-2-yl)urea
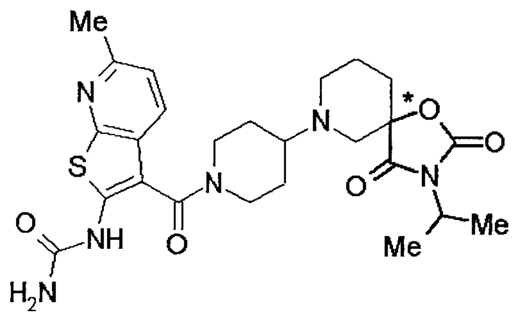

The title compound (258 mg, yield 79%) was obtained by an operation in the same manner as in Example 10 and using 7-{1-[(2-amino-6-methylthieno[2,3-b]pyridin-3-yl)carbonyl]piperidin-4-yl}-3-isopropyl-1-oxa-3,7-diazaspiro[4.5]decane-2,4-dione in an optically active form (300 mg, 0.62 mmol) obtained in Example 21, followed by trituration with diisopropyl ether.
EI(pos) 529.0 $[M+H]^+$

Example 23

optically active form of N-(3-{[4-(3-isopropyl-2,4-dioxo-1-oxa-3,7-diazaspiro[4.5]dec-7-yl)piperidin-1-yl]carbonyl}-6-methylthieno[2,3-b]pyridin-2-yl)-N'-methylurea
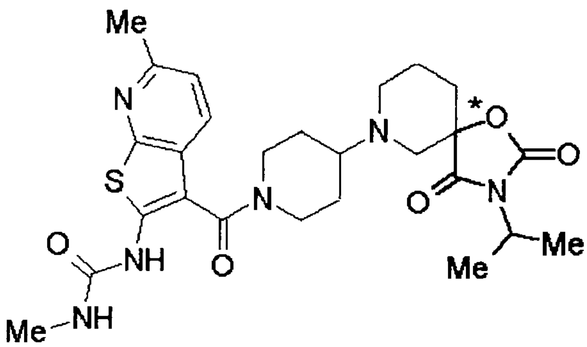

The title compound (251 mg, yield 75%) was obtained by an operation in the same manner as in Example 3 and using 7-{1-[(2-amino-6-methylthieno[2,3-b]pyridin-3-yl)carbonyl]piperidin-4-yl}-3-isopropyl-1-oxa-3,7-diazaspiro[4.5]decane-2,4-dione in an optically active form (300 mg, 0.62 mmol) obtained in Example 21, followed by trituration with diisopropyl ether.
EI(pos) 543.0 $[M+H]^+$

Example 24

optically active form of N-ethyl-N'-(3-{[4-(3-isopropyl-2,4-dioxo-1-oxa-3,7-diazaspiro[4.5]dec-7-yl)piperidin-1-yl]carbonyl}-6-methylthieno[2,3-b]pyridin-2-yl)urea
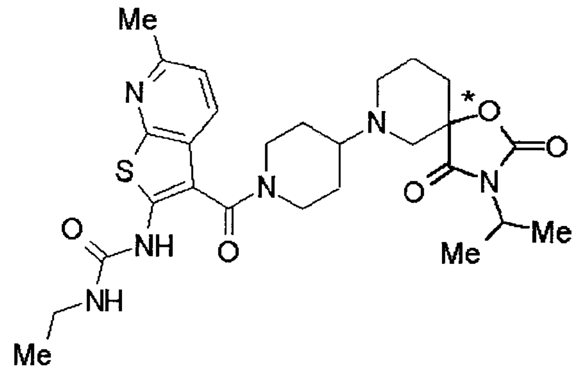

The title compound (123 mg, yield 79%) was obtained by an operation in the same manner as in Example 3 and using 7-{1-[(2-amino-6-methylthieno[2,3-b]pyridin-3-yl)carbonyl]piperidin-4-yl}-3-isopropyl-1-oxa-3,7-diazaspiro[4.5]decane-2,4-dione in an optically active form (136 mg, 0.28 mmol) obtained in Example 21 and ethyl isocyanate, followed by trituration with diisopropyl ether.
EI(pos) 557.1 $[M+H]^+$

Example 25

optically active form of tert-butyl (3-{[4-(3,3-dimethyl-1-oxo-2-oxa-7-azaspiro[4.5]dec-7-yl)piperidin-1-yl]carbonyl}-6-methylthieno[2,3-b]pyridin-2-yl)carbamate

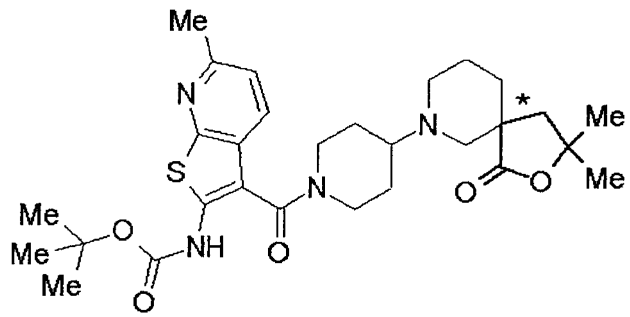

The title compound (1.19 g, yield 80%) as an oil was obtained by an operation in the same manner as in Example 1 and using 3,3-dimethyl-7-(piperidin-4-yl)-2-oxa-7-azaspiro[4.5]decan-1-one dihydrochloride in an optically active form (900 mg, 2.65 mmol) obtained in Reference Example 29 and 2-[(tert-butoxycarbonyl)amino]-6-methylthieno[2,3-b]pyridine-3-carboxylic acid (818 mg, 2.65 mmol) obtained in Reference Example 47.
EI(pos) 557.1 $[M+H]^+$

Example 26

optically active form of 7-{1-[(2-amino-6-methylthieno[2,3-b]pyridin-3-yl)carbonyl]piperidin-4-yl}-3,3-dimethyl-2-oxa-7-azaspiro[4.5]decan-1-one

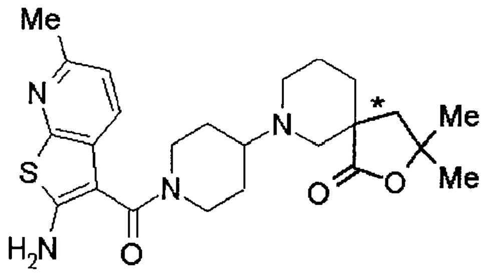

The title compound (856 mg, yield 88%) was obtained by an operation in the same manner as in Example 2 and using tert-butyl (3-{[4-(3,3-dimethyl-1-oxo-2-oxa-7-azaspiro[4.5]dec-7-yl)piperidin-1-yl]carbonyl}-6-methylthieno [2,3-b]pyridin-2-yl)carbamate in an optically active form (1.19 g, 2.14 mmol) obtained in Example 25, followed by trituration with diisopropyl ether.
EI(pos) 457.0 $[M+H]^+$

Example 27

optically active form of N-(3-{[4-(3,3-dimethyl-1-oxo-2-oxa-7-azaspiro[4.5]dec-7-yl)piperidin-1-yl]carbonyl}-6-methylthieno[2,3-b]pyridin-2-yl)-N'-methylurea
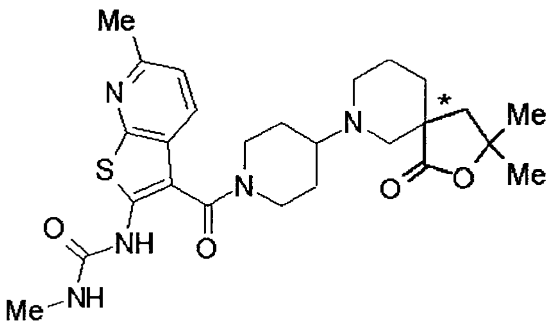

The title compound (202 mg, yield 60%) was obtained by an operation in the same manner as in Example 3 and using 7-{1-[(2-amino-6-methylthieno[2,3-b]pyridin-3-yl)carbonyl]piperidin-4-yl}-3,3-dimethyl-2-oxa-7-azaspiro[4.5] decan-1-one in an optically active form (300 mg, 0.66 mmol) obtained in Example 26, followed by trituration with diisopropyl ether and hexane.
EI(pos) 514.1 $[M+H]^+$

Example 28

optically active form of N-(3-{[4-(3,3-dimethyl-1-oxo-2-oxa-7-azaspiro[4.5]dec-7-yl)piperidin-1-yl]carbonyl}-6-methylthieno[2,3-b]pyridin-2-yl)-N'-ethylurea
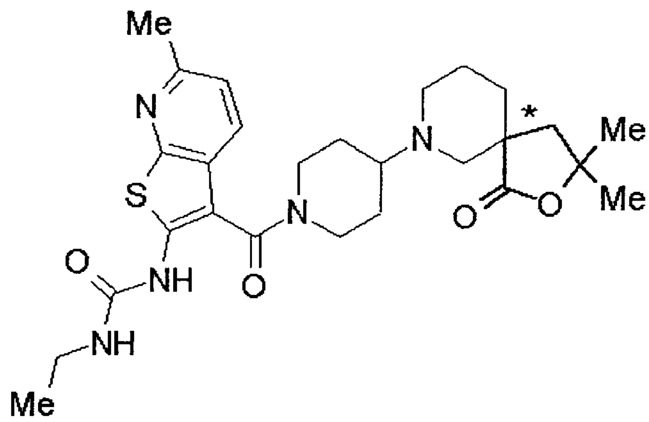

The title compound (280 mg, yield 81%) was obtained by an operation in the same manner as in Example 3 and using 7-{1-[(2-amino-6-methylthieno[2,3-b]pyridin-3-yl)carbonyl]piperidin-4-yl}-3,3-dimethyl-2-oxa-7-azaspiro[4.5]decan-1-one in an optically active form (300 mg, 0.66 mmol) obtained in Example 26 and ethyl isocyanate, followed by trituration with diisopropyl ether and hexane.
EI(pos) 528.1 $[M+H]^+$

Example 29

tert-butyl (3-{[4-(3-isopropyl-2,4-dioxo-1-thia-3,7-diazaspiro[4.5]dec-7-yl)piperidin-1-yl]carbonyl}-6-methylthieno[2,3-b]pyridin-2-yl)carbamate
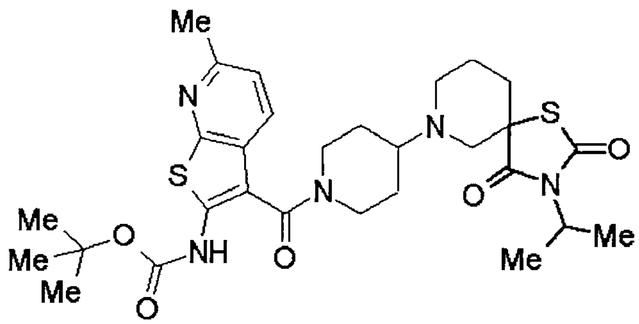

The title compound (939 mg, quantitative) as an oil was obtained by an operation in the same manner as in Example 1 and using 3-isopropyl-7-(piperidin-4-yl)-1-thia-3,7-diazaspiro[4.5]decane-2,4-dione dihydrochloride (600 mg, 1.56 mmol) obtained in Reference Example 23 and 2-[(tert-butoxycarbonyl)amino]-6-methylthieno[2,3-b]pyridine-3-carboxylic acid (481 mg, 1.56 mmol) obtained in Reference Example 47. EI(pos) 602.1 $[M+H]^+$

Example 30

7-{1-[(2-amino-6-methylthieno[2,3-b]pyridin-3-yl)carbonyl]piperidin-4-yl}-3-isopropyl-1-thia-3,7-diazaspiro[4.5]decane-2,4-dione
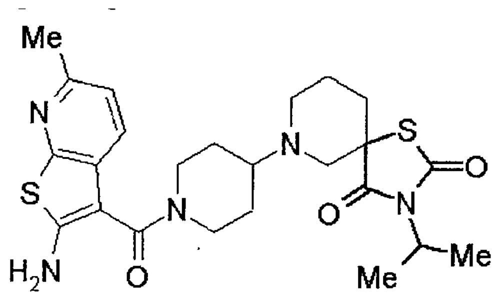

The title compound (620 mg, yield 79%) was obtained by an operation in the same manner as in Example 2 and using tert-butyl (3-{[4-(3-isopropyl-2,4-dioxo-1-thia-3,7-diazaspiro[4.5]dec-7-yl)piperidin-1-yl]carbonyl}-6-methylthieno[2,3-b]pyridin-2-yl)carbamate (939 mg, 1.56 mmol) obtained in Example 29, followed by trituration with diisopropyl ether.
EI(pos) 502.0 $[M+H]^+$

Example 31

N-(3-{[4-(3-isopropyl-2,4-dioxo-1-thia-3,7-diazaspiro[4.5]dec-7-yl)piperidin-1-yl]carbonyl}-6-methylthieno[2,3-b]pyridin-2-yl)-N'-methylurea
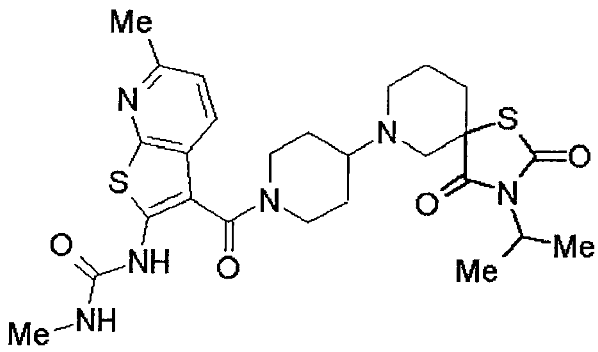

The title compound (227 mg, yield 68%) was obtained by an operation in the same manner as in Example 3 and using 7-{1-[(2-amino-6-methylthieno[2,3-b]pyridin-3-yl)carbonyl]piperidin-4-yl}-3-isopropyl-1-thia-3,7-diazaspiro[4.5]decane-2,4-dione (300 mg, 0.60 mmol) obtained in Example 30, followed by trituration with diisopropyl ether and hexane.
EI(pos) 559.0 $[M+H]^+$

Example 32

tert-butyl (3-{[4-(3,3-dimethyl-1-oxo-2-oxa-7-azaspiro[4.5]dec-7-yl)piperidin-1-yl]carbonyl}thieno[2,3-b]pyridin-2-yl)carbamate
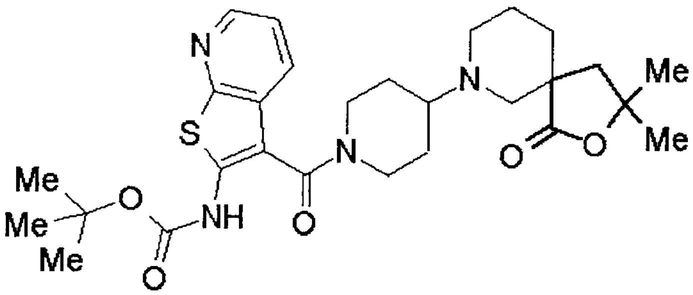

The title compound (1.05 g, yield 99%) as an oil was obtained by an operation in the same manner as in Example 1 and using 3,3-dimethyl-7-(piperidin-4-yl)-2-oxa-7-azaspiro[4.5]decan-1-one dihydrochloride (600 mg, 1.77 mmol) obtained in Reference Example 26 and 2-[(tert-butoxycarbonyl)amino]thieno[2,3-b]pyridine-3-carboxylic acid (520 mg, 1.77 mmol) obtained in Reference Example 52.
EI(pos) 543.1 $[M+H]^+$

Example 33

7-{1-[(2-aminothieno[2,3-b]pyridin-3-yl)carbonyl]piperidin-4-yl}-3,3-dimethyl-2-oxa-7-azaspiro[4.5]decan-1-one

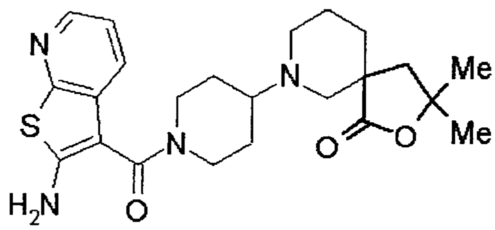

The title compound was obtained by an operation in the same manner as in Example 2 and using tert-butyl (3-{[4-(3,3-dimethyl-1-oxo-2-oxa-7-azaspiro[4.5]dec-7-yl)piperidin-1-yl]carbonyl}thieno[2,3-b]pyridin-2-yl)carbamate (1.05 g, 1.77 mmol) obtained in Example 32.

Example 34

N-(3-{[4-(3,3-dimethyl-1-oxo-2-oxa-7-azaspiro[4.5]dec-7-yl)piperidin-1-yl]carbonyl}thieno[2,3-b]pyridin-2-yl) urea

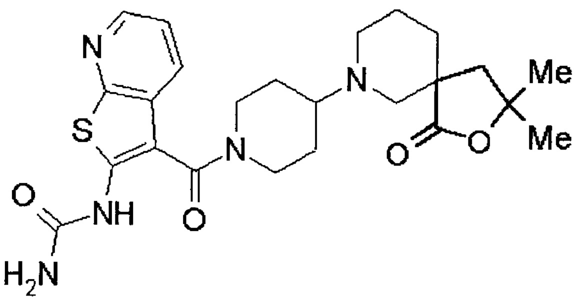

7-{1-[(2-Aminothieno[2,3-b]pyridin-3-yl)carbonyl]piperidin-4-yl}-3,3-dimethyl-2-oxa-7-azaspiro[4.5]decan-1-one obtained in Example 33 was dissolved in ethyl acetate (10 mL) and 5 mL of the solution was concentrated under reduced pressure. Using the residue and in the same manner as in Example 10, followed by trituration with diethyl ether,

the title compound (169 mg, yield from tert-butyl (3-{[4-(3,3-dimethyl-1-oxo-2-oxa-7-azaspiro[4.5]dec-7-yl)piperidin-1-yl]carbonyl}thieno[2,3-b]pyridin-2-yl)carbamate obtained in Example 32: 39%) was obtained.
EI(pos) 486.06 [M+H]$^+$

Example 35

N-(3-{[4-(3,3-dimethyl-1-oxo-2-oxa-7-azaspiro[4.5]dec-7-yl)piperidin-1-yl]carbonyl}thieno[2,3-b]pyridin-2-yl)-N'-methylurea
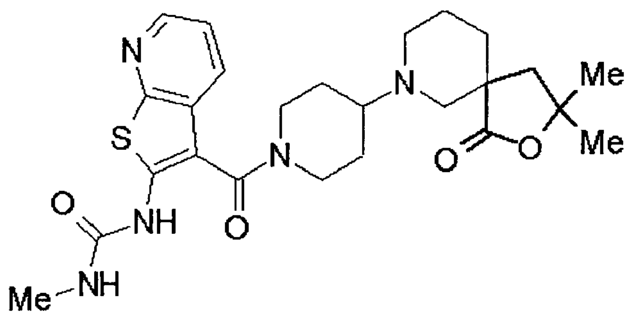

A solution (5 mL) of 7-{1-[(2-aminothieno[2,3-b]pyridin-3-yl)carbonyl]piperidin-4-yl}-3,3-dimethyl-2-oxa-7-azaspiro[4.5]decan-1-one prepared in Example 34 in ethyl acetate was concentrated under reduced pressure. Using the residue and in the same manner as in Example 3, followed by trituration with diethyl ether, the title compound (136 mg, yield from tert-butyl (3-{[4-(3,3-dimethyl-1-oxo-2-oxa-7-azaspiro[4.5]dec-7-yl)piperidin-1-yl]carbonyl}thieno[2,3-b]pyridin-2-yl)carbamate obtained in Example 32: 31%) was obtained.
EI(pos) 500.1 [M+H]$^+$

Example 36

N-(3-{[4-(3,3-dimethyl-1-oxo-2-oxa-7-azaspiro[4.5]dec-7-yl)piperidin-1-yl]carbonyl}thieno[2,3-b]pyridin-2-yl)-N'-ethylurea
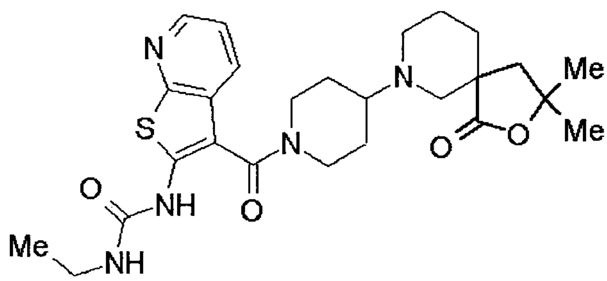

A solution of tert-butyl (3-{[4-(3,3-dimethyl-1-oxo-2-oxa-7-azaspiro[4.5]dec-7-yl)piperidin-1-yl]carbonyl}thieno[2,3-b]pyridin-2-yl)carbamate (360 mg, 0.664 mmol) obtained in Example 32 in trifluoroacetic acid (5 mL) was stirred at room temperature for 30 min. The reaction mixture was neutralized with saturated aqueous sodium hydrogen carbonate solution, extracted twice with ethyl acetate, and the extract was dried over anhydrous magnesium sulfate. The solvent was evaporated under reduced pressure, and the obtained 7-{1-[(2-aminothieno[2,3-b]pyridin-3-yl)carbonyl]piperidin-4-yl}-3,3-dimethyl-2-oxa-7-azaspiro[4.5]decan-1-one was dissolved in pyridine (5 mL). Ethyl isocyanate (0.11 mL, 1.33 mmol) was added, and the mixture was stirred at 80°C for 24 hr. The solvent was evaporated under reduced pressure, and the obtained residue was purified by basic silica gel column chromatography (ethyl acetate:hexane=7:3 to ethyl acetate) to give the title compound (178 mg, yield 54%) as an oil.
EI(pos) 514 [M+H]$^+$

Example 37

tert-butyl (3-{[4-(3-isopropyl-2,4-dioxo-1,3,7-triazaspiro[4.5]dec-7-yl)piperidin-1-yl]carbonyl}thieno[2,3-b]pyridin-2-yl)carbamate

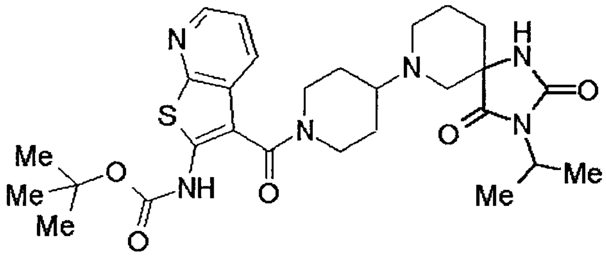

The title compound (1.48 g, yield 97%) as an oil was obtained by an operation in the same manner as in Example 1 and using 3-isopropyl-7-(piperidin-4-yl)-1,3,7-triazaspiro[4.5]decane-2,4-dione dihydrochloride (800 mg, 2.70 mmol) obtained in Reference Example 32 and 2-[(tert-butoxycarbonyl)amino]thieno[2,3-b]pyridine-3-carboxylic acid (1.00 g, 2.70 mmol) obtained in Reference Example 52.
EI(pos) 571.1 $[M+H]^+$

Example 38

7-{1-[(2-aminothieno[2,3-b]pyridin-3-yl)carbonyl]piperidin-4-yl}-3-isopropyl-1,3,7-triazaspiro[4.5]decane-2,4-dione

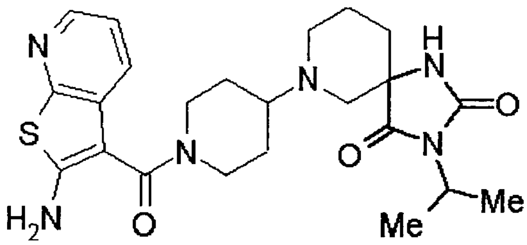

The title compound (853 mg, yield 70%) was obtained by an operation in the same manner as in Example 2 and using tert-butyl (3-{[4-(3-isopropyl-2,4-dioxo-1,3,7-triazaspiro[4.5]dec-7-yl)piperidin-1-yl]carbonyl}thieno[2,3-b]pyridin-2-yl)carbamate (1.48 g, 2.59 mmol) obtained in Example 37, followed by trituration with diisopropyl ether and hexane.
EI(pos) 471.1 $[M+H]^+$

Example 39

N-(3-{[4-(3-isopropyl-2,4-dioxo-1,3,7-triazaspiro[4.5]dec-7-yl)piperidin-1-yl]carbonyl}thieno[2,3-b]pyridin-2-yl)-N'-methylurea

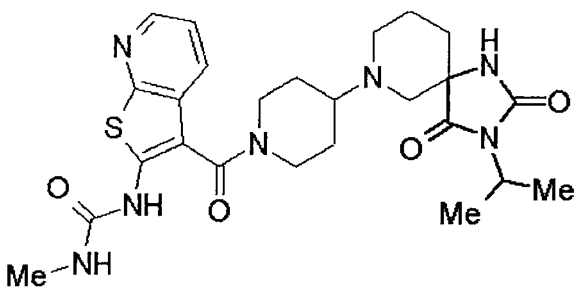

The title compound (166 mg, yield 50%) was obtained by an operation in the same manner as in Example 3 and using 7-{1-[(2-aminothieno[2,3-b]pyridin-3-yl)carbonyl]piperidin-4-yl}-3-isopropyl-1,3,7-triazaspiro[4.5]decane-2,4-dione (300 mg, 0.638 mmol) obtained in Example 38, followed by trituration with diisopropyl ether.
EI(pos) 528.1 [M+H]+

Example 40

N-ethyl-N'-(3-{[4-(3-isopropyl-2,4-dioxo-1,3,7-triazaspiro[4.5]dec-7-yl)piperidin-1-yl]carbonyl}thieno[2,3-b]pyridin-2-yl)urea
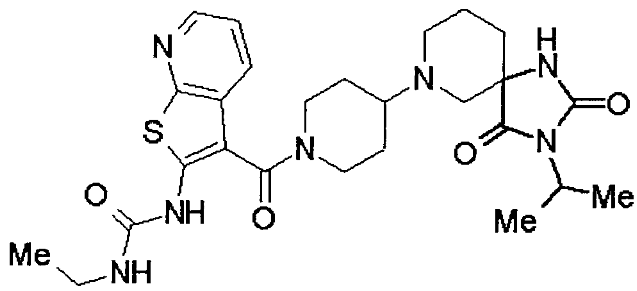

The title compound (222 mg, yield 64%) was obtained by an operation in the same manner as in Example 3 and using 7-{1-[(2-aminothieno[2,3-b]pyridin-3-yl)carbonyl]piperidin-4-yl}-3-isopropyl-1,3,7-triazaspiro[4.5]decane-2,4-dione (300 mg, 0.638 mmol) obtained in Example 38 and ethyl isocyanate, followed by trituration with diisopropyl ether.
EI(pos) 542.1 [M+H]+

Example 41

tert-butyl (3-{[4-(3-ethyl-2-methyl-4-oxo-1,3,7-triazaspiro[4.5]dec-1-en-7-yl)piperidin-1-yl]carbonyl}thieno[2,3-b]pyridin-2-yl)carbamate
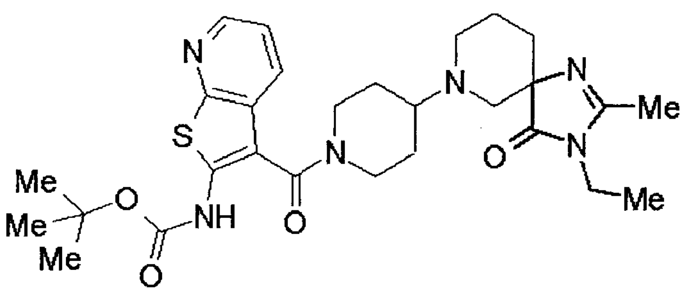

tert-Butyl 4-(3-ethyl-2-methyl-4-oxo-1,3,7-triazaspiro[4.5]dec-1-en-7-yl)piperidine-1-carboxylate (280 mg, 0.74

mmol) obtained in Reference Example 38 was dissolved in ethyl acetate (2 mL), 4N hydrogen chloride-ethyl acetate solution (3 mL) was added, and the mixture was stirred at room temperature for 10 min. The solvent was evaporated under reduced pressure and the obtained residue was dissolved in DMF (2.5 mL). 2-[(tert-Butoxycarbonyl)amino]thieno [2,3-b]pyridine-3-carboxylic acid (218 mg, 0.74 mmol) obtained in Reference Example 52, 1-hydroxybenzotriazole (148 mg, 1.1 mmol), 1-ethyl-3-(3'-dimethylaminopropyl)carbodiimide hydrochloride (210 mg, 1.1 mmol) and triethylamine (0.307 mL, 2.2 mmol) were added, and the mixture was stirred at room temperature for 20 hr. Water and saturated aqueous sodium hydrogen carbonate solution were added to the reaction mixture, and the mixture was extracted with ethyl acetate. The extract was dried over anhydrous magnesium sulfate. The solvent was evaporated under reduced pressure and the residue was purified by basic silica gel column chromatography (ethyl acetate to ethyl acetate:methanol=9:1) to give the title compound (139 mg, yield 33%) as an oil.
EI(pos) 555.1 $[M+H]^+$

Example 42

7-{1-[(2-aminothieno[2,3-b]pyridin-3-yl)carbonyl]piperidin-4-yl}-3-ethyl-2-methyl-1,3,7-triazaspiro[4.5]dec-1-en-4-one

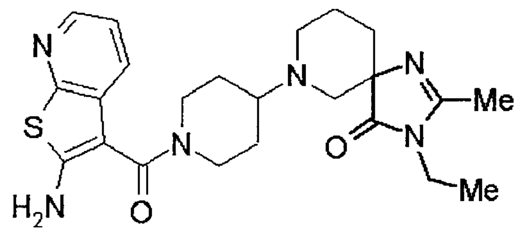

The title compound (84 mg, yield 74%) was obtained by an operation in the same manner as in Example 2 and using tert-butyl (3-{[4-(3-ethyl-2-methyl-4-oxo-1,3,7-triazaspiro[4.5]dec-1-en-7-yl)piperidin-1-yl]carbonyl}thieno[2,3-b]pyridin-2-yl)carbamate (138 mg, 0.249 mmol) obtained in Example 41, followed by trituration with diisopropyl ether and hexane.

Example 43

N-ethyl-N'-(3-{[4-(3-ethyl-2-methyl-4-oxo-1,3,7-triazaspiro[4.5]dec-1-en-7-yl)piperidin-1-yl]carbonyl}thieno [2,3-b]pyridin-2-yl)urea

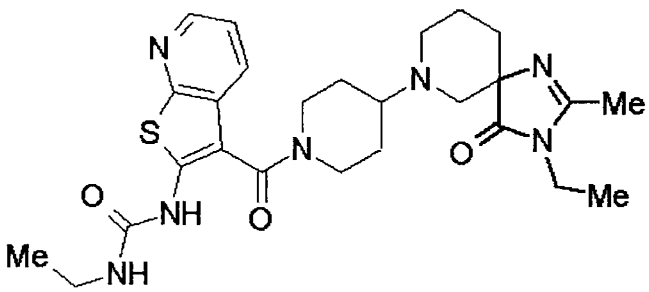

The title compound (12.3 mg, yield 13%) was obtained by an operation in the same manner as in Example 3 and using 7-{1-[(2-aminothieno[2,3-b]pyridin-3-yl)carbonyl]piperidin-4-yl}-3-ethyl-2-methyl-1,3,7-triazaspiro[4.5]dec-1-en-4-one (84.0 mg, 0.185 mmol) obtained in Example 42 and ethyl isocyanate, followed by trituration with diisopropyl ether.
EI(pos) 526.1 $[M+H]^+$

Example 44

7-{1-[(2-amino-6-methylthieno[2,3-b]pyridin-3-yl)carbonyl]piperidin-4-yl}-3-isopropyl-1,3,7-triazaspiro[4.5]decane-2,4-dione

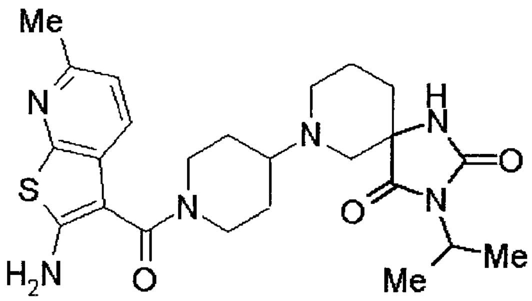

To a solution of 3-isopropyl-7-(piperidin-4-yl)-1,3,7-triazaspiro[4.5]decane-2,4-dione dihydrochloride (990 mg, 2.70 mmol) obtained in Reference Example 32, 2-[(tert-butoxycarbonyl)amino]-6-methylthieno[2,3-b]pyridine-3-carboxylic acid (831 mg, 2.70 mmol) obtained in Reference Example 47, 1-hydroxybenzotriazole (553 mg, 4.10 mmol) and triethylamine (1.10 mL, 8.10 mmol) in DMF (9 mL) was added 1-ethyl-3-(3'-dimethylaminopropyl)carbodiimide hydrochloride (773 mg, 4.10 mmol), and the mixture was stirred at room temperature for 16 hr. Saturated aqueous sodium hydrogen carbonate solution was added to the reaction mixture, and the mixture was extracted with ethyl acetate. The extract was washed with saturated brine, dried over anhydrous sodium sulfate and concentrated under reduced pressure. The residue was purified by basic silica gel column chromatography (hexane:ethyl acetate=3:7 to ethyl acetate) to give tert-butyl (3-{[4-(3-isopropyl-2,4-dioxo-1,3,7-triazaspiro[4.5]dec-7-yl)piperidin-1-yl]carbonyl}-6-methylthieno[2,3-b]pyridin-2-yl)carbamate. This was dissolved in trifluoroacetic acid (8 mL), and the solution was stirred at room temperature for 30 min. The reaction mixture was concentrated under reduced pressure, and the residue was dissolved in ethyl acetate. The solution was washed with saturated aqueous sodium hydrogen carbonate solution and passed through a basic silica gel. The solvent was evaporated under reduced pressure and the residue was triturated with diisopropyl ether to give the title compound (874 mg, yield 67%).
EI(pos) 485.0 $[M+H]^+$

Example 45

N-(3-{[4-(3-isopropyl-2,4-dioxo-1,3,7-triazaspiro[4.5]dec-7-yl)piperidin-1-yl]carbonyl}-6-methylthieno[2,3-b]pyridin-2-yl)-N'-methylurea

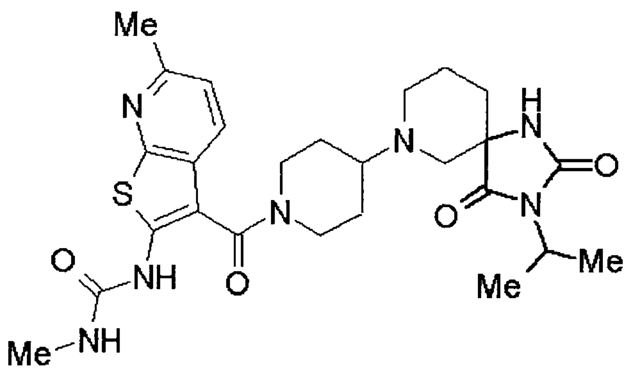

The title compound (202 mg, yield 60%) was obtained by an operation in the same manner as in Example 3 and using 7-{1-[(2-amino-6-methylthieno[2,3-b]pyridin-3-yl)carbonyl]piperidin-4-yl}-3-isopropyl-1,3,7-triazaspiro[4.5]decane-2,4-dione (300 mg, 0.62 mmol) obtained in Example 44, followed by trituration with diisopropyl ether.
EI(pos) 542.1 $[M+H]^+$

Example 46

N-ethyl-N'-(3-{[4-(3-isopropyl-2,4-dioxo-1,3,7-triazaspiro[4.5]dec-7-yl)piperidin-1-yl]carbonyl}-6-methylthieno[2,3-b]pyridin-2-yl)urea

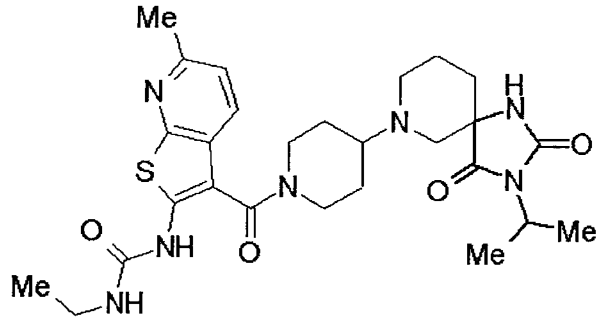

The title compound (176 mg, yield 51%) was obtained by an operation in the same manner as in Example 3 and using 7-{1-[(2-amino-6-methylthieno[2,3-b]pyridin-3-yl)carbonyl]piperidin-4-yll-3-isopropyl-1,3,7-triazaspiro[4.5]decane-2,4-dione (300 mg, 0.62 mmol) obtained in Example 44 and ethyl isocyanate, followed by trituration with diisopropyl ether.

EI(pos) 556.1 $[M+H]^+$

Example 47

tert-butyl (3-{[4-(3-isopropyl-2-methyl-4-oxo-1,3,7-triazaspiro[4.5]dec-1-en-7-yl)piperidin-1-yl]carbonyl}-6-methylthieno[2,3-b]pyridin-2-yl)carbamate

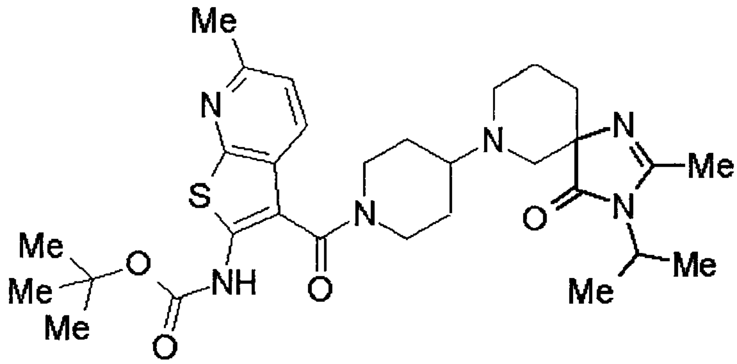

The title compound (237 mg, yield 37%) as an oil was obtained by an operation in the same manner as in Example 1 and using 3-isopropyl-2-methyl-7-(piperidin-4-yl)-1,3,7-triazaspiro[4.5]dec-1-en-4-one dihydrochloride (400 mg, 1.09 mmol) obtained in Reference Example 41 and 2-[(tert-butoxycarbonyl)amino]-6-methylthieno[2,3-b]pyridine-3-carboxylic acid (436 mg, 1.42 mmol) obtained in Reference Example 47 with diisopropyl ether.

EI(pos) 583.2 $[M+H]^+$

Example 48

7-{1-[(2-amino-6-methylthieno[2,3-b]pyridin-3-yl)carbonyl]piperidin-4-yl}-3-isopropyl-2-methyl-1,3,7-triazaspiro[4.5]dec-1-en-4-one

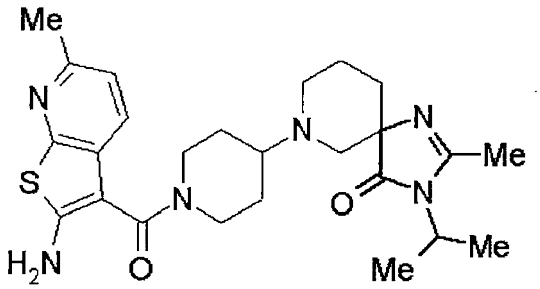

The title compound (153 mg, yield 78%) was obtained by an operation in the same manner as in Example 2 and using tert-butyl (3-{[4-(3-isopropyl-2-methyl-4-oxo-1,3,7-triazaspiro[4.5]dec-1-en-7-yl)piperidin-1-yl]carbonyl}-6-methylthieno[2,3-b]pyridin-2-yl)carbamate (237 mg, 0.407 mmol) obtained in Example 47, followed by trituration with diisopropyl ether and hexane.

Example 49

N-ethyl-N'-(3-{[4-(3-ethyl-2-methyl-4-oxo-1,3,7-triazaspiro[4.5]dec-1-en-7-yl)piperidin-1-yl]carbonyl}thieno [2,3-b]pyridin-2-yl)urea

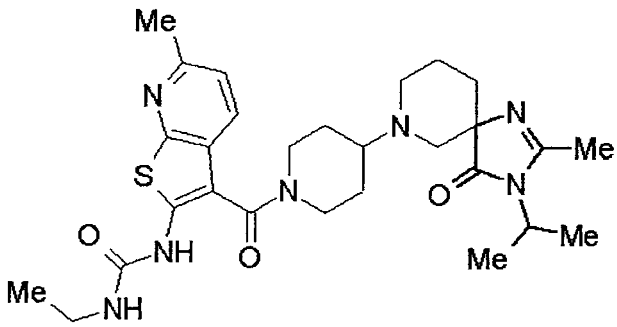

The title compound (89.1 mg, yield 51%) was obtained by an operation in the same manner as in Example 3 and using 7-{1-[(2-amino-6-methylthieno[2,3-b]pyridin-3-yl)carbonyl]piperidin-4-yl}-3-isopropyl-2-methyl-1,3,7-triazaspiro[4.5]dec-1-en-4-one (153 mg, 0.317 mmol) obtained in Example 48 and ethyl isocyanate, followed by trituration with diisopropyl ether.

EI(pos) 554.1 $[M+H]^+$

Example 50

optically active form of tert-butyl (3-{[4-(3-isopropyl-2-methyl-4-oxo-1,3,7-triazaspiro[4.5]dec-1-en-7-yl)piperidin-1-yl]carbonyl}-6-methylthieno[2,3-b]pyridin-2-yl)carbamate

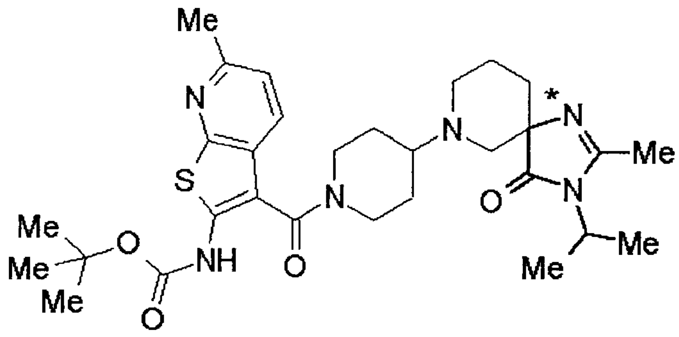

The title compound (1.04 g, yield 70%) as an oil was obtained by an operation in the same manner as in Example 1 and using 3-isopropyl-2-methyl-7-(piperidin-4-yl)-1,3,7-triazaspiro[4.5]dec-1-en-4-one dihydrochloride in an optically active form (1.02 g, 2.55 mmol) obtained in Reference Example 42 and 2-[(tert-butoxycarbonyl)amino]-6-methylthieno[2,3-b]pyridine-3-carboxylic acid (785 mg, 2.55 mmol) obtained in Reference Example 47.
EI(pos) 583.1 $[M+H]^+$

Example 51

optically active form of 7-{1-[(2-amino-6-methylthieno[2,3-b]pyridin-3-yl)carbonyl]piperidin-4-yl}-3-isopropyl-2-methyl-1,3,7-triazaspiro[4.5]dec-1-en-4-one
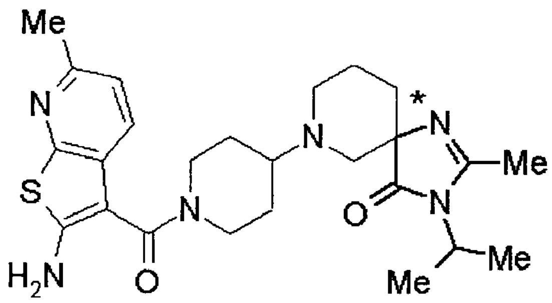

The title compound (704 mg, yield 73%) was obtained by an operation in the same manner as in Example 2 and using tert-butyl (3-{[4-(3-isopropyl-2-methyl-4-oxo-1,3,7-triazaspiro[4.5]dec-1-en-7-yl)piperidin-1-yl]carbonyl}-6-methylthieno[2,3-b]pyridin-2-yl)carbamate in an optically active form (1.04 g, 1.79 mmol) obtained in Example 50, followed by trituration with diisopropyl ether and hexane.
EI(pos) 483.0 $[M+H]^+$

Example 52

optically active form of N-ethyl-N'-(3-{[4-(3-isopropyl-2-methyl-4-oxo-1,3,7-triazaspiro[4.5]dec-1-en-7-yl)piperidin-1-yl]carbonyl}thieno[2,3-b]pyridin-2-yl)urea
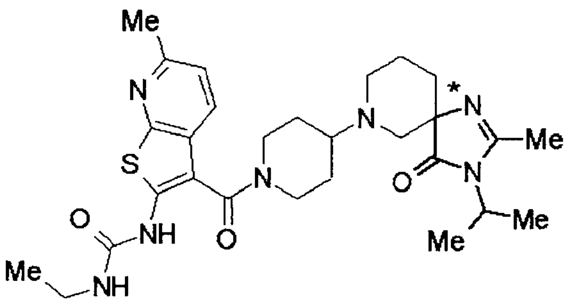

The title compound (260 mg, yield 32%) was obtained by an operation in the same manner as in Example 3 and using 7-{1-[(2-amino-6-methylthieno[2,3-b]pyridin-3-yl)carbonyl]piperidin-4-yl}-3-isopropyl-2-methyl-1,3,7-triazaspiro[4.5]dec-1-en-4-one in an optically active form (704 mg, 1.46 mmol) obtained in Example 51 and ethyl isocyanate, followed by trituration with diisopropyl ether.
EI(pos) 554.2 $[M+H]^+$

Example 53

7-{1-[(2-amino-4,6-dimethylthieno[2,3-b]pyridin-3-yl)carbonyl]piperidin-4-yl}-3-isopropyl-1,3,7-triazaspiro[4.5]decane-2,4-dione
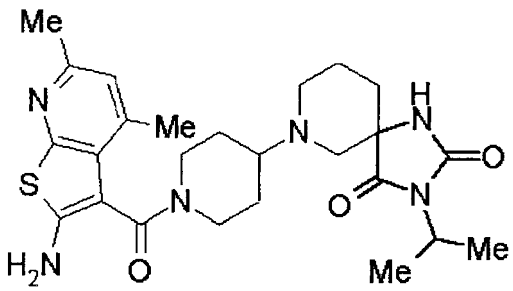

The title compound (190 mg, yield 38%) was obtained by an operation in the same manner as in Example 44 and using 3-isopropyl-7-(piperidin-4-yl)-1,3,7-triazaspiro[4.5]decane-2,4-dione dihydrochloride (367 mg, 1.00 mmol) obtained in Reference Example 32 and 2-[(tert-butoxycarbonyl)amino]-4,6-dimethylthieno[2,3-b]pyridine-3-carboxylic acid (322 mg, 1.00 mmol) obtained in Reference Example 62.
EI(pos) 499.1 $[M+H]^+$

Example 54

N-ethyl-N'-(3-{[4-(3-isopropyl-2,4-dioxo-1,3,7-triazaspiro[4.5]dec-7-yl)piperidin-1-yl]carbonyl}-6-methylthieno[2,3-b]pyridin-2-yl)urea
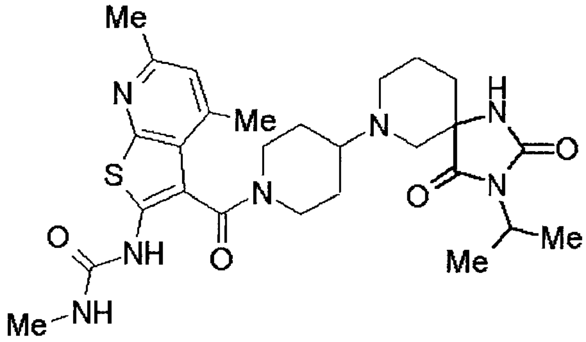

The title compound (44.6 mg, yield 20%) was obtained by an operation in the same manner as in Example 3 and using 7-{1-[(2-amino-4,6-dimethylthieno[2,3-b]pyridin-3-yl)carbonyl]piperidin-4-yl}-3-isopropyl-1,3,7-triazaspiro [4.5]decane-2,4-dione (190 mg, 0.380 mmol) obtained in Example 53 and ethyl isocyanate, followed by trituration with diisopropyl ether.
EI(pos) 570.1 $[M+H]^+$

Example 55

optically active form of 7-{1-[(2-amino-5,6-dimethylthieno[2,3-b]pyridin-3-yl)carbonyl]piperidin-4-yl}-3-isopropyl-2-methyl-1,3,7-triazaspiro[4.5]dec-1-en-4-one
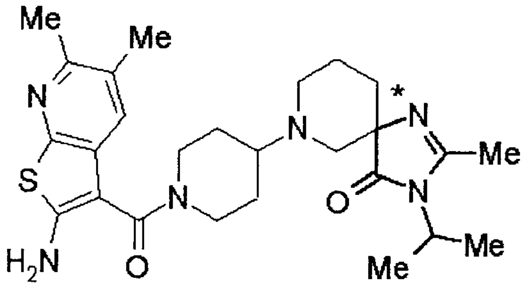

The title compound (308 mg, yield 45%) was obtained by an operation in the same manner as in Example 44 and using 3-isopropyl-2-methyl-7-(piperidin-4-yl)-1,3,7-triazaspiro[4.5]dec-1-en-4-one dihydrochloride in an optically active form (557 mg, 1.39 mmol) obtained in Reference Example 42 and 2-[(tert-butoxycarbonyl)amino]-5,6-dimethylthieno [2,3-b]pyridine-3-carboxylic acid (450 mg, 1.39 mmol) obtained in Reference Example 68.
EI(pos) 497.1 $[M+H]^+$

Example 56

optically active form of N-ethyl-N'-(3-{[4-(3-isopropyl-2-methyl-4-oxo-1,3,7-triazaspiro[4.5]dec-1-en-7-yl)piperidin-1-yl]carbonyl}thieno[2,3-b]pyridin-2-yl)urea
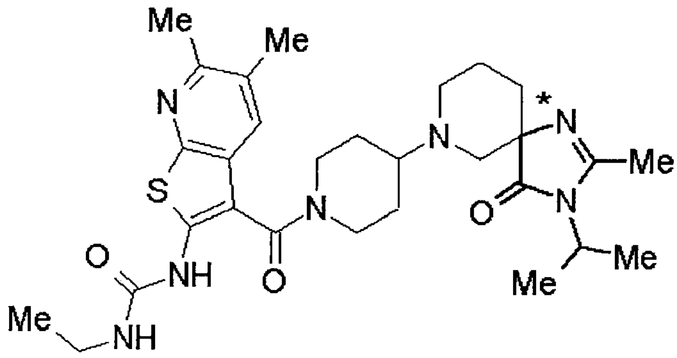

The title compound (188 mg, yield 37%) was obtained by an operation in the same manner as in Example 3 and using 7-{1-[(2-amino-5,6-dimethylthieno[2,3-b]pyridin-3-yl)carbonyl]piperidin-4-yl}-3-isopropyl-2-methyl-1,3,7-triazaspiro[4.5]dec-1-en-4-one in an optically active form (308 mg, 0.89 mmol) obtained in Example 55 and ethyl isocyanate, followed by trituration with diisopropyl ether.
EI(pos) 568.2 $[M+H]^+$

Example 57

optically active form of 7-{1-[(2-amino-5,6-dimethylthieno[2,3-b]pyridin-3-yl)carbonyl]piperidin-4-yl}-3-isopropyl-1-oxa-3,7-diazaspiro[4.5]decane-2,4-dione
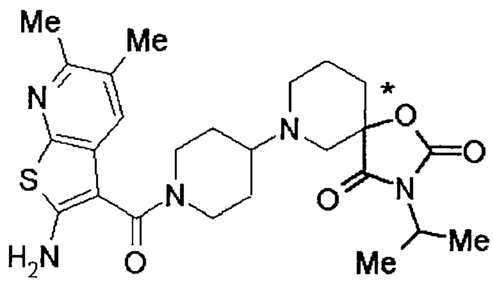

The title compound (390 mg, yield 48%) was obtained by an operation in the same manner as in Example 44 and using 3-isopropyl-7-(piperidin-4-yl)-1-oxa-3,7-diazaspiro[4.5]decane-2,4-dione dihydrochloride in an optically active form (600 mg, 1.63 mmol) obtained in Reference Example 18 and 2-[(tert-butoxycarbonyl)amino]-5,6-dimethylthieno[2,3-b]pyridine-3-carboxylic acid (524 mg, 1.63 mmol) obtained in Reference Example 68.
EI(pos) 500.1 $[M+H]^+$

Example 58

optically active form of N-(3-{[4-(3-isopropyl-2,4-dioxo-1-oxa-3,7-diazaspiro[4.5]dec-7-yl)piperidin-1-yl]carbonyl}-5,6-dimethylthieno[2,3-b]pyridin-2-yl)-N'-methylurea
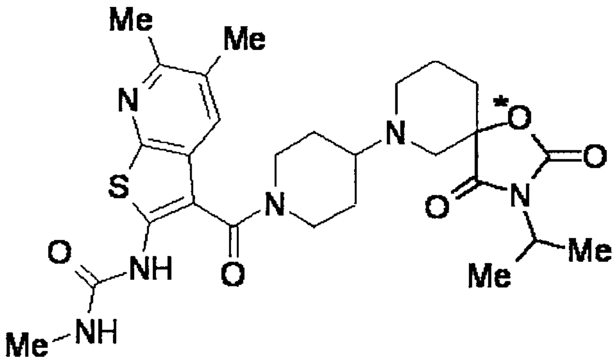

The title compound (60.5 mg, yield 28%) was obtained by an operation in the same manner as in Example 3 and using 7-{1-[(2-amino-5,6-dimethylthieno[2,3-b]pyridin-3-yl)carbonyl]piperidin-4-yl}-3-isopropyl-1-oxa-3,7-diazaspiro[4.5]decane-2,4-dione in an optically active form (195 mg, 0.390 mmol) obtained in Example 57, followed by trituration with diisopropyl ether. EI(pos) 557.1 $[M+H]^+$

Example 59

optically active form of N-ethyl-N'-(3-{[4-(3-isopropyl-2,4-dioxo-1-oxa-3,7-diazaspiro[4.5]dec-7-yl)piperidin-1-yl]carbonyl}-5,6-dimethylthieno[2,3-b]pyridin-2-yl)urea

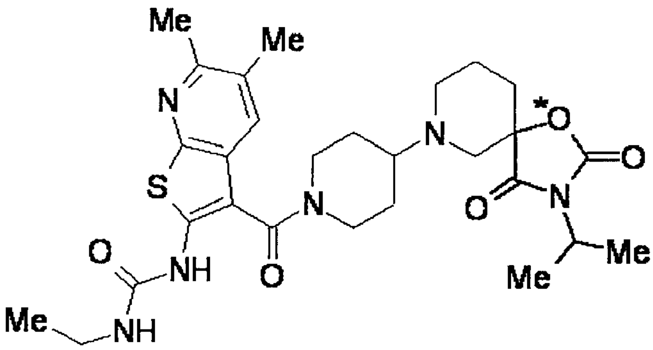

The title compound (35.5 mg, yield 16%) was obtained by an operation in the same manner as in Example 3 and using 7-{1-[(2-amino-5,6-dimethylthieno[2,3-b]pyridin-3-yl)carbonyl]piperidin-4-yl}-3-isopropyl-1-oxa-3,7-diazaspiro[4.5]decane-2,4-dione in an optically active form (195 mg, 0.390 mmol) obtained in Example 57 and ethyl isocyanate, followed by trituration with diisopropyl ether.
EI(pos) 571.1 $[M+H]^+$

Example 60

7-{1-[(2-amino-5,6-dimethylthieno[2,3-b]pyridin-3-yl)carbonyl]piperidin-4-yl}-3-isopropyl-1,3,7-triazaspiro[4.5]decane-2,4-dione

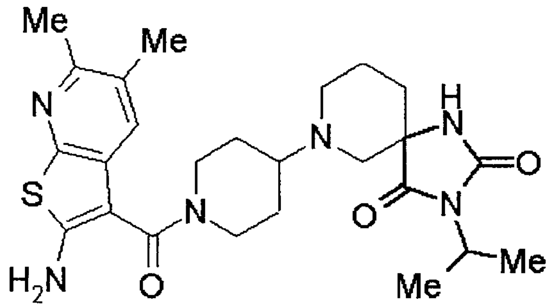

The title compound (594 mg, yield 61%) was obtained by an operation in the same manner as in Example 44 and using 3-isopropyl-7-(piperidin-4-yl)-1,3,7-triazaspiro[4.5]decane-2,4-dione dihydrochloride (720 mg, 1.96 mmol) obtained in Reference Example 32 and 2-[(tert-butoxycarbonyl)amino]-5,6-dimethylthieno[2,3-b]pyridine-3-carboxylic acid (615 mg, 1.96 mmol) obtained in Reference Example 68.
EI(pos) 499.1 $[M+H]^+$

Example 61

N-(3-{[4-(3-isopropyl-2,4-dioxo-1,3,7-triazaspiro[4.5]dec-7-yl)piperidin-1-yl]carbonyl}-5,6-dimethylthieno[2,3-b]pyridin-2-yl)-N'-methylurea

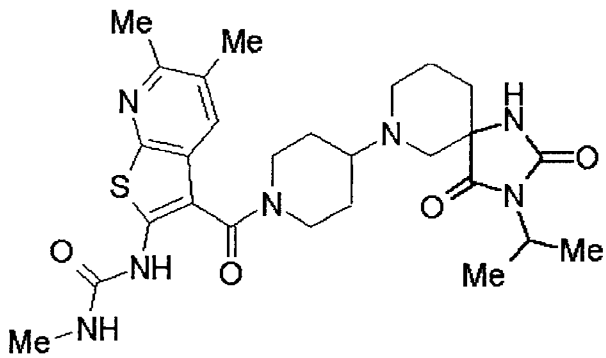

The title compound (190.3 mg, yield 57%) was obtained by an operation in the same manner as in Example 3 and using 7-{1-[(2-amino-5,6-dimethylthieno[2,3-b]pyridin-3-yl)carbonyl]piperidin-4-yl}-3-isopropyl-1,3,7-triazaspiro[4.5]decane-2,4-dione (299 mg, 0.600 mmol) obtained in Example 60, followed by trituration with diisopropyl ether.
EI(pos) 556.1 $[M+H]^+$

Example 62

N-ethyl-N'-(3-{[4-(3-isopropyl-2,4-dioxo-1,3,7-triazaspiro[4.5]dec-7-yl)piperidin-1-yl]carbonyl}-5,6-dimethylthieno[2,3-b]pyridin-2-yl)urea

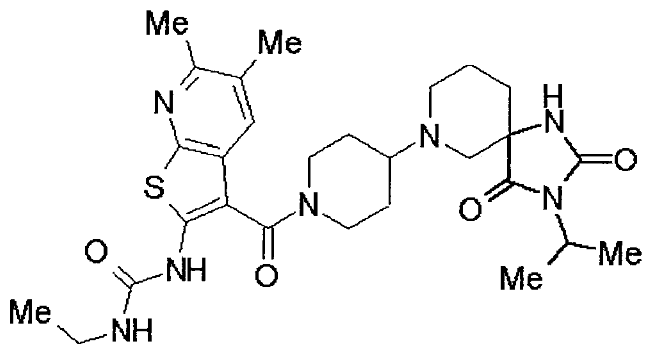

The title compound (164 mg, yield 48%) was obtained by an operation in the same manner as in Example 3 and using 7-{1-[(2-amino-5,6-dimethylthieno[2,3-b]pyridin-3-yl)carbonyl]piperidin-4-yl}-3-isopropyl-1,3,7-triazaspiro[4.5]decane-2,4-dione (299 mg, 0.60 mmol) obtained in Example 60 and ethyl isocyanate, followed by trituration with diisopropyl ether.
EI(pos) 570.1 $[M+H]^+$

Example 63

optically active form of tert-butyl (3-{[4-(2-isopropyl-1-oxo-2,7-diazaspiro[4.5]dec-7-yl)piperidin-1-yl]carbonyl}-6-methylthieno[2,3-b]pyridin-2-yl)carbamate
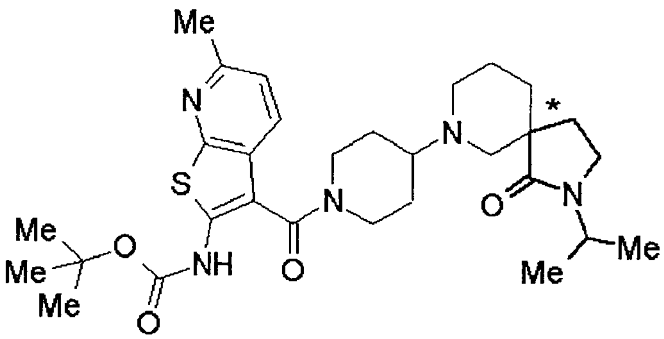

To tert-butyl 4-(2-isopropyl-1-oxo-2,7-diazaspiro[4.5]dec-7-yl)piperidine-1-carboxylate in an optically active form (1.00 g, 2.64 mmol) obtained in Reference Example 5 was added 4N hydrogen chloride-dioxane solution (10 mL), and the mixture was stirred at room temperature for 30 min. The solvent was evaporated under reduced pressure, and to a solution of the obtained residue in DMF (10 mL) were added 2-[(tert-butoxycarbonyl)amino]-6-methylthieno[2,3-b]pyridine-3-carboxylic acid (813 mg, 2.64 mmol) obtained in Reference Example 47, 1-hydroxybenzotriazole (535 mg, 3.96 mmol), 1-ethyl-3-(3'-dimethylaminopropyl)carbodiimide hydrochloride (760 mg, 3.96 mmol) and triethylamine (1.1 mL, 7.92 mmol), and the mixture was stirred at room temperature for 16 hr. The reaction mixture was diluted with ethyl acetate, washed 3 times with saturated brine, and dried over anhydrous magnesium sulfate. The solvent was evaporated under reduced pressure, and the obtained residue was purified by basic silica gel column chromatography (ethyl acetate: hexane=1:1 to ethyl acetate) to give the title compound (1.39 g, yield 92%) as an oil.
$[\alpha]_D^{20}$ -13.6°(c 1.07, methanol) .
EI(pos) 570 $[M+H]^+$

Example 64

optically active form of N-(3-{[4-(2-isopropyl-1-oxo-2,7-diazaspiro[4.5]dec-7-yl)piperidin-1-yl]carbonyl}-6-methylthieno[2,3-b]pyridin-2-yl)-N'-methylurea
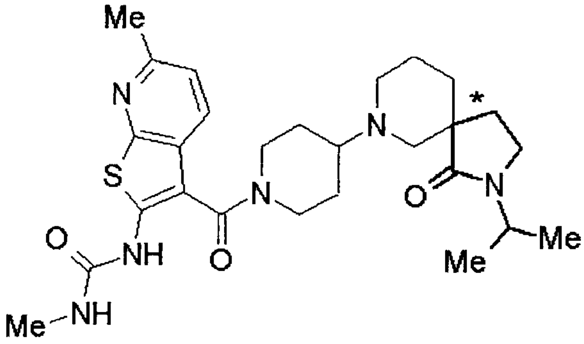

A solution of tert-butyl (3-{[4-(2-isopropyl-1-oxo-2,7-diazaspiro[4.5]dec-7-yl)piperidin-1-yl]carbonyl}-6-methylthieno[2,3-b]pyridin-2-yl)carbamate in an optically active form (1.39 g, 2.44 mmol) obtained in Example 63 in trifluoroacetic acid (10 mL) was stirred at room temperature for 30 min. The reaction mixture was neutralized with saturated aqueous sodium hydrogen carbonate solution, and extracted twice with ethyl acetate, and the organic layer was dried over anhydrous magnesium sulfate. The solvent was evaporated under reduced pressure and the obtained crude purification product was dissolved in pyridine (5 mL). Methyl isocyanate (0.29 mL, 4.88 mmol) was added and the mixture was stirred at 70°C for 6 hr. The solvent was evaporated under reduced pressure, and the obtained residue was purified by basic silica gel column chromatography (ethyl acetate to methanol:ethyl acetate=1:9) to give the title compound (578 mg, yield 44%). melting point 195-198°C (crystallized from ethyl acetate-hexane).
$[\alpha]_D^{20}$ -20.1°(c 1.17, methanol).
EI(pos) 527 $[M+H]^+$

Example 65

optically active form of N-ethyl-N'-(3-{[4-(2-isopropyl-1-oxo-2,7-diazaspiro[4.5]dec-7-yl)piperidin-1-yl]carbonyl}-6-methylthieno[2,3-b]pyridin-2-yl)urea
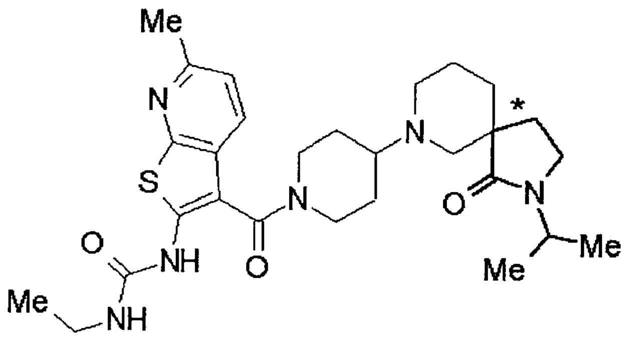

A solution of tert-butyl (3-{[4-(2-isopropyl-1-oxo-2,7-diazaspiro[4.5]dec-7-yl)piperidin-1-yl]carbonyl}-6-methylthieno[2,3-b]pyridin-2-yl)carbamate in an optically active form (1.01 g, 1.77 mmol) obtained in Example 63 in trifluoroacetic acid (5 mL) was stirred at room temperature for 30 min. The reaction mixture was neutralized with saturated aqueous sodium hydrogen carbonate solution, and extracted twice with ethyl acetate, and the organic layer was dried over anhydrous magnesium sulfate. The solvent was evaporated under reduced pressure and the obtained crude purification product was dissolved in pyridine (5 mL). Ethyl isocyanate (0.28 mL, 3.54 mmol) was added and the mixture was stirred at 70°C for 7 hr. The solvent was evaporated under reduced pressure, and the obtained residue was purified by basic silica gel column chromatography (ethyl acetate to methanol:ethyl acetate=1:9) to give the title compound (615 mg, yield 64%) as an oil. The oil (190 mg, 0.351 mmol) was crystallized from ethyl acetate-hexane to give 112 mg (yield 59%) as a white solid.
melting point 191-193°C.
$[\alpha]_D^{20}$ -21.2°(c 1.04, methanol).
EI(pos) 541 $[M+H]^+$

Example 66

optically active form of N-ethyl-N'-(3-{[4-(2-isopropyl-1-oxo-2,7-diazaspiro[4.5]dec-7-yl)piperidin-1-yl]carbonyl}-6-methylthieno[2,3-b]pyridin-2-yl)urea dihydrochloride

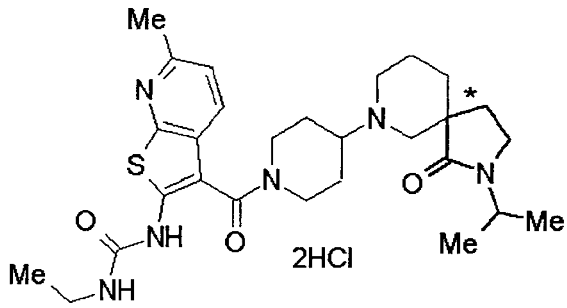

N-Ethyl-N'-(3-{[4-(2-isopropyl-1-oxo-2,7-diazaspiro[4.5]dec-7-yl)piperidin-1-yl]carbonyl}-6-methylthieno[2,3-b]pyridin-2-yl)urea in an optically active form (425 mg, 0.787 mmol) obtained in Example 65 was dissolved in 4N hydrogen chloride-ethyl acetate solution (10 mL). The precipitate was collected by filtration and washed with diethyl ether to give the title compound (383 mg, yield 79%) as a white solid.
elemental analysis: for $C_{28}H_{40}N_6O_3S \cdot 2HCl \cdot 2.5H_2O$
Calculated: C,51.06;H,7.19;N,12.76;C1,10.76.
Found: C,51.27;H,7.43;N,12.68;Cl,10.77.

Example 67

optically active form of tert-butyl (3-{[4-(2-isopropyl-1-oxo-2,7-diazaspiro[4.5]dec-7-yl)piperidin-1-yl]carbonyl}thieno[2,3-b]pyridin-2-yl)carbamate

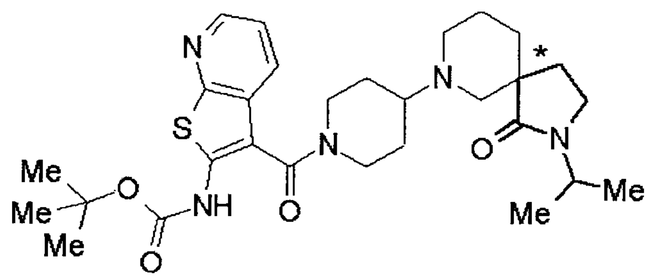

The title compound (1.46 g, yield 99%) as an oil was obtained by an operation in the same manner as in Example 63 and using tert-butyl 4-(2-isopropyl-1-oxo-2,7-diazaspiro[4.5]dec-7-yl)piperidine-1-carboxylate in an optically active form (1.00 g, 2.64 mmol) obtained in Reference Example 5 and 2-[(tert-butoxycarbonyl)amino]thieno[2,3-b]pyridine-3-carboxylic acid (776 mg, 2.64 mmol) obtained in Reference Example 52.
EI(pos) 556 $[M+H]^+$

Example 68

optically active form of N-(3-{[4-(2-isopropyl-1-oxo-2,7-diazaspiro[4.5]dec-7-yl)piperidin-1-yl]carbonyl}thieno[2,3-b]pyridin-2-yl)-N'-methylurea

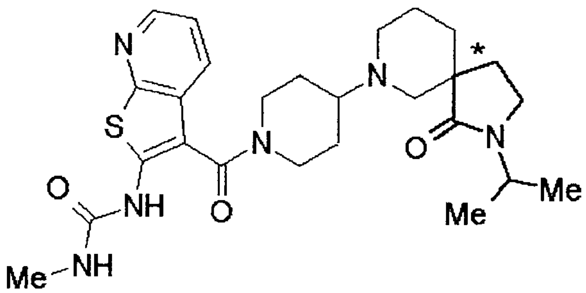

The title compound (122 mg, yield 18%) was obtained by an operation in the same manner as in Example 36 and using tert-butyl (3-{[4-(2-isopropyl-1-oxo-2,7-diazaspiro[4.5]dec-7-yl)piperidin-1-yl]carbonyl}thieno[2,3-b]pyridin-2-yl)carbamate in an optically active form (1.46 g, 2.62 mmol) obtained in Example 67 and methyl isocyanate.
EI(pos) 513 $[M+H]^+$

Example 69

optically active form of N-ethyl-N'-(3-{[4-(2-isopropyl-1-oxo-2,7-diazaspiro[4.5]dec-7-yl)piperidin-1-yl]carbonyl} thieno[2,3-b]pyridin-2-yl)urea

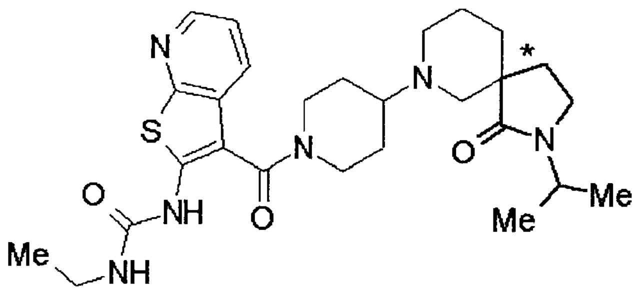

The title compound (111 mg, yield 16%) was obtained by an operation in the same manner as in Example 36 and using tert-butyl (3-{[4-(2-isopropyl-1-oxo-2,7-diazaspiro[4.5]dec-7-yl)piperidin-1-yl]carbonyl}thieno[2,3-b]pyridin-2-yl)carbamate in an optically active form (1.46 g, 2.62 mmol) obtained in Example 67.
EI(pos) 527 $[M+H]^+$

Example 70

tert-butyl (3-{[4-(3,3-dimethyl-1-oxo-2-oxa-7-azaspiro[4.5]dec-7-yl)piperidin-1-yl]carbonyl}thieno[3,2-b]pyridin-2-yl)carbamate

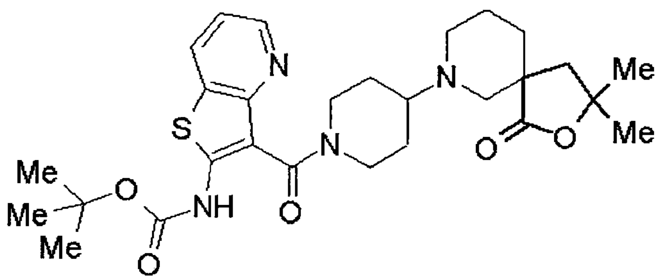

The title compound (910 mg, yield 70%) as an oil was obtained by an operation in the same manner as in

Example 1 and using 3,3-dimethyl-7-(piperidin-4-yl)-2-oxa-7-azaspiro[4.5]decan-1-one dihydrochloride (807 mg, 2.38 mmol) obtained in Reference Example 26 and 2-[(tert-butoxycarbonyl)amino]thieno[3,2-b]pyridine-3-carboxylic acid (700 mg, 2.38 mmol) obtained in Reference Example 57.
EI(pos) 543 $[M+H]^+$

Example 71

N-(3-{[4-(3,3-dimethyl-1-oxo-2-oxa-7-azaspiro[4.5]dec-7-yl)piperidin-1-yl]carbonyl}thieno[3,2-b]pyridin-2-yl)-N'-ethylurea

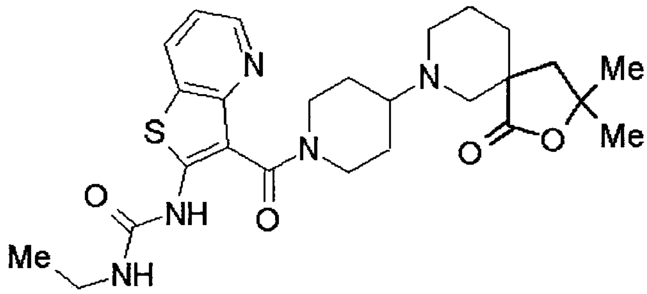

The title compound (698 mg, yield 81%) as an oil was obtained by an operation in the same manner as in Example 36 and using tert-butyl (3-{[4-(3,3-dimethyl-1-oxo-2-oxa-7-azaspiro[4.5]dec-7-yl)piperidin-1-yl]carbonyl}thieno [3,2-b]pyridin-2-yl)carbamate (910 mg, 1.68 mmol) obtained in Example 70.
EI(pos) 514 $[M+H]^+$

Example 72

tert-butyl (3-{[4-(3,3-dimethyl-1-oxo-2-oxa-7-azaspiro[4.5]dec-7-yl)piperidin-1-yl]carbonyl}-5,6-dimethylthieno [2,3-b]pyridin-2-yl)carbamate

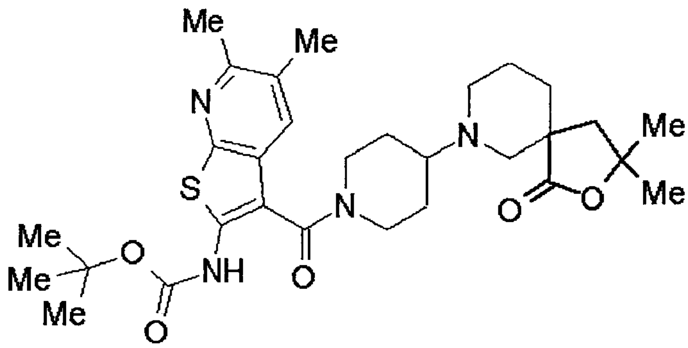

The title compound (507 mg, yield 64%) as an oil was obtained by an operation in the same manner as in Example 1 and using 3,3-dimethyl-7-(piperidin-4-yl)-2-oxa-7-azaspiro[4.5]decan-1-one dihydrochloride (471 mg, 1.39 mmol) obtained in Reference Example 26 and 2-[(tert-butoxycarbonyl)amino]-5,6-dimethylthieno[2,3-b]pyridine-3-carboxylic acid (450 mg, 1.39 mmol) obtained in Reference Example 68.
EI(pos) 571.1 $[M+H]^+$

Example 73

7-{1-[(2-amino-5,6-dimethylthieno[2,3-b]pyridin-3-yl)carbonyl]piperidin-4-yl}-3,3-dimethyl-2-oxa-7-azaspiro [4.5]decan-1-one

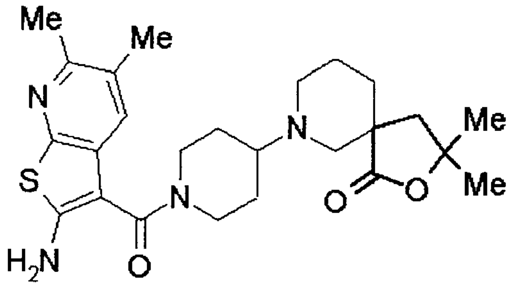

The title compound (345 mg, yield 83%) was obtained by an operation in the same manner as in Example 2 and using tert-butyl (3-{[4-(3,3-dimethyl-1-oxo-2-oxa-7-azaspiro[4.5]dec-7-yl)piperidin-1-yl]carbonyl}-5,6-dimethylthieno[2,3-b]pyridin-2-yl)carbamate (507 mg, 0.889 mmol) obtained in Example 72, followed by trituration with diisopropyl ether and hexane. EI(pos) 471.0 $[M+H]^+$

Example 74

N-(3-{[4-(3,3-dimethyl-1-oxo-2-oxa-7-azaspiro[4.5]dec-7-yl)piperidin-1-yl]carbonyl}thieno[2,3-b]pyridin-2-yl)-N'-methylurea

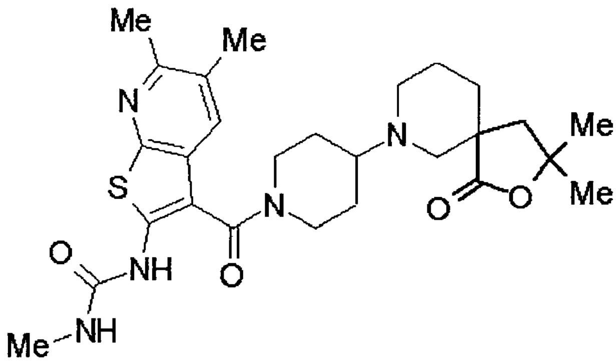

The title compound (51.1 mg, yield 26%) was obtained by an operation in the same manner as in Example 3 and using 7-{1-[(2-amino-5,6-dimethylthieno[2,3-b]pyridin-3-yl)carbonyl]piperidin-4-yl}-3,3-dimethyl-2-oxa-7-azaspiro[4.5]decan-1-one (172 mg, 0.367 mmol) obtained in Example 73, followed by trituration with diisopropyl ether and hexane. EI(pos) 528.1 $[M+H]^+$

Example 75

N-ethyl-N'-(3-{[4-(3,3-dimethyl-1-oxo-2-oxa-7-azaspiro[4.5]dec-7-yl)piperidin-1-yl]carbonyl}thieno[2,3-b]pyridin-2-yl)urea

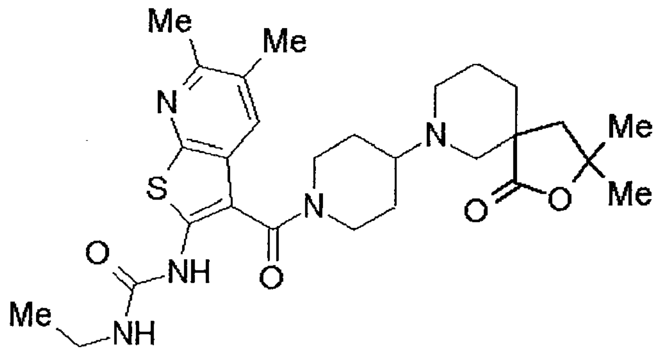

The title compound (49.9 mg, yield 25%) was obtained by an operation in the same manner as in Example 3 and using 7-{1-[(2-amino-5,6-dimethylthieno[2,3-b]pyridin-3-yl)carbonyl]piperidin-4-yl}-3,3-dimethyl-2-oxa-7-azaspiro[4.5]decan-1-one (172 mg, 0.367 mmol) obtained in Example 73 and ethyl isocyanate, followed by trituration with diisopropyl ether and hexane.
EI(pos) 542.1 [M+H] $^+$

Example 76

tert-butyl (3-{[4-(3,3-dimethyl-1-oxo-2-oxa-7-azaspiro[4.5]dec-7-yl)piperidin-1-yl]carbonyl}-6-methylthieno[2,3-b]pyridin-2-yl)carbamate
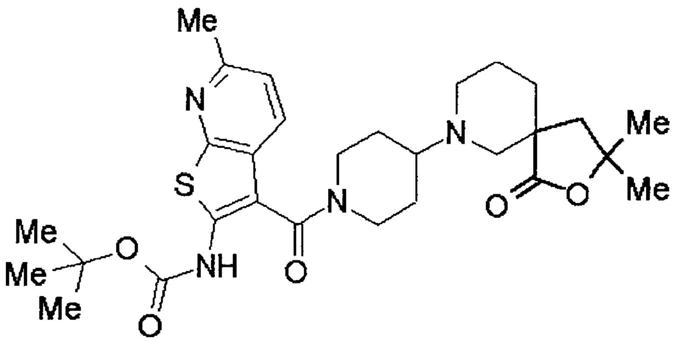

The title compound (557 mg, yield 78%) as an oil was obtained by an operation in the same manner as in Example 1 and using 3,3-dimethyl-7-(piperidin-4-yl)-2-oxa-7-azaspiro[4.5]decan-1-one dihydrochloride (434 mg, 1.28 mmol) obtained in Reference Example 26 and 2-[(tert-butoxycarbonyl)amino]-6-methylthieno[2,3-b]pyridine-3-carboxylic acid (394 mg, 1.28 mmol) obtained in Reference Example 47. EI(pos) 557.5 [M+H]$^+$

Example 77

7-{1-[(2-amino-6-methylthieno[2,3-b]pyridin-3-yl)carbonyl]piperidin-4-yl}-3,3-dimethyl-2-oxa-7-azaspiro[4.5]decan-1-one
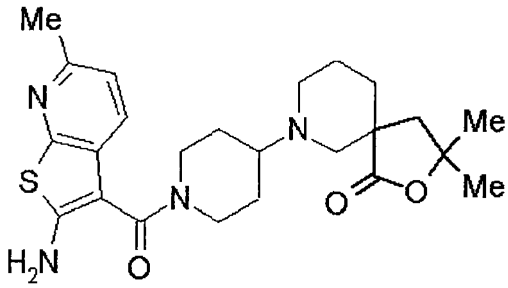

The title compound (350 mg, yield 77%) was obtained by an operation in the same manner as in Example 2 and using tert-butyl (3-{[4-(3,3-dimethyl-1-oxo-2-oxa-7-azaspiro[4.5]dec-7-yl)piperidin-1-yl]carbonyl}-6-methylthieno[2,3-b]pyridin-2-yl)carbamate (557 mg, 1.00 mmol) obtained in Example 76, followed by trituration with diisopropyl ether.
EI(pos) 457.4 [M+H]$^+$

Example 78

N-(3-{[4-(3,3-dimethyl-1-oxo-2-oxa-7-azaspiro[4.5]dec-7-yl)piperidin-1-yl]carbonyl}-6-methylthieno[2,3-b]pyridin-2-yl)-N'-methylurea
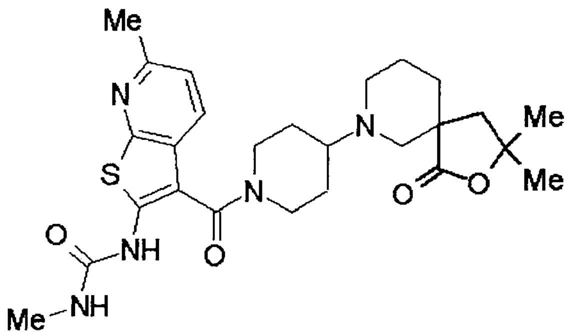

The title compound (104 mg, yield 69%) was obtained by an operation in the same manner as in Example 3 and using 7-{1-[(2-amino-6-methylthieno[2,3-b]pyridin-3-yl)carbonyl]piperidin-4-yl}-3,3-dimethyl-2-oxa-7-azaspiro[4.5]decan-1-one (134 mg, 0.293 mmol) obtained in Example 77, followed by trituration with diisopropyl ether.
EI(pos) 514.5 [M+H]$^+$

Example 79

N-(3-{[4-(3,3-dimethyl-1-oxo-2-oxa-7-azaspiro[4.5]dec-7-yl)piperidin-1-yl]carbonyl}-6-methylthieno[2,3-b]pyridin-2-yl)-N'-ethylurea
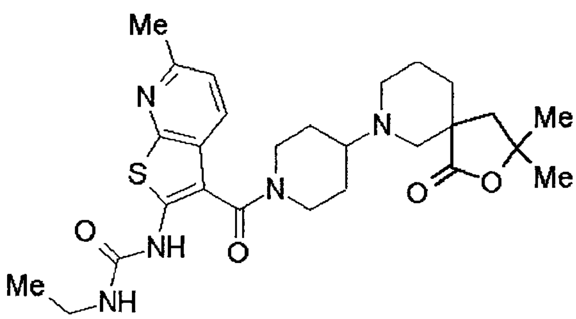

The title compound (175 mg, yield 76%) was obtained by an operation in the same manner as in Example 3 and using 7-{1-[(2-amino-6-methylthieno[2,3-b]pyridin-3-yl)carbonyl]piperidin-4-yl}-3,3-dimethyl-2-oxa-7-azaspiro[4.5] decan-1-one (200 mg, 0.438 mmol) obtained in Example 77 and ethyl isocyanate, followed by trituration with diisopropyl ether.
EI(pos) 528.5 $[M+H]^+$

Example 80

optically active form of tert-butyl (3-{[4-(2-isopropyl-1,3-dioxo-2,7-diazaspiro[4.5]dec-7-yl)piperidin-1-yl]carbonyl}-5,6-dimethylthieno[2,3-b]pyridin-2-yl)carbamate
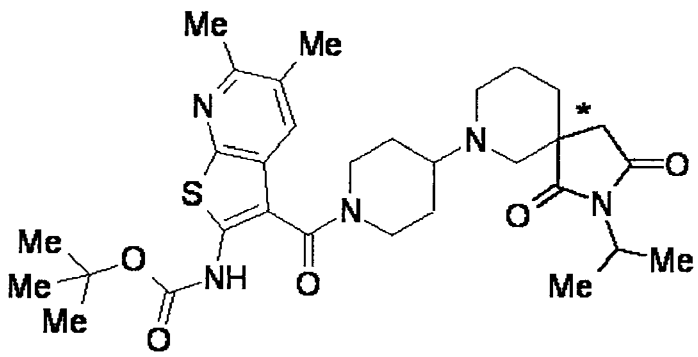

The title compound (409 mg, yield 50%) as an oil was obtained by an operation in the same manner as in Example 1 and using 2-isopropyl-7-(piperidin-4-yl)-2,7-diazaspiro[4.5]decane-1,3-dione dihydrochloride in an optically active form (500 mg, 1.37 mmol) obtained in Reference Example 12, 2-[(tert-butoxycarbonyl)amino]-5,6-dimethylthieno [2,3-b]pyridine-3-carboxylic acid (442 mg, 1.37 mmol) obtained in Reference Example 68.
EI(pos) 598.2 $[M+H]^+$

Example 81

7-{1-[(2-amino-5,6-dimethylthieno[2,3-b]pyridin-3-yl)carbonyl]piperidin-4-yl}-2-isopropyl-2,7-diazaspiro[4.5] decane-1,3-dione
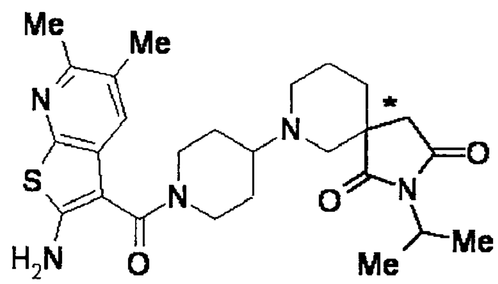

The title compound (246 mg, yield 72%) was obtained by an operation in the same manner as in Example 2 and using tert-butyl (3-{[4-(2-isopropyl-1,3-dioxo-2,7-diazaspiro[4.5]dec-7-yl)piperidin-1-yl]carbonyl}-5,6-dimethylthieno [2,3-b]pyridin-2-yl)carbamate in an optically active form (409 mg, 0.685 mmol) obtained in Example 80, followed by trituration with diisopropyl ether and hexane.
EI(pos) 498.1 $[M+H]^+$

Example 82

N-ethyl-N'-(3-{[4-(2-isopropyl-1,3-dioxo-2,7-diazaspiro[4.5]dec-7-yl)piperidin-1-yl]carbonyl}-5,6-dimethylthieno[2,3-b]pyridin-2-yl)urea

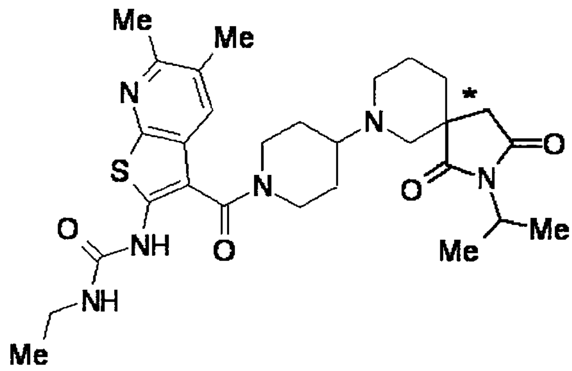

The title compound (79.6 mg, yield 57%) was obtained by an operation in the same manner as in Example 4 and using 7-{1-[(2-amino-5,6-dimethylthieno[2,3-b]pyridin-3-yl)carbonyl]piperidin-4-yl}-2-isopropyl-2,7-diazaspiro[4.5] decane-1,3-dione (123 mg, 0.247 mmol) obtained in Example 81, followed by trituration with diisopropyl ether and hexane. EI(pos) 569.1 $[M+H]^+$

Example 83

N-methyl-N'-(3-{[4-(2-isopropyl-1,3-dioxo-2,7-diazaspiro[4.5]dec-7-yl)piperidin-1-yl]carbonyl}-5,6-dimethylthieno[2,3-b]pyridin-2-yl)urea

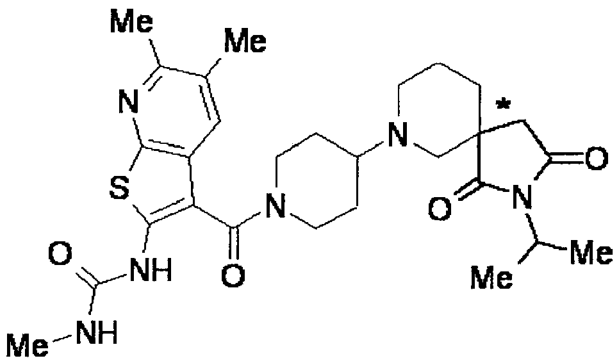

The title compound (56.1 mg, yield 41%) was obtained by an operation in the same manner as in Example 3 and using 7-{1-[(2-amino-5,6-dimethylthieno[2,3-b]pyridin-3-yl)carbonyl]piperidin-4-yll-2-isopropyl-2,7-diazaspiro[4.5] decane-1,3-dione (123 mg, 0.247 mmol) obtained in Example 81, followed by trituration with diisopropyl ether and hexane. EI(pos) 555.1 $[M+H]^+$

Example 84

tert-butyl (3-{[4-(2-ethyl-1-oxo-2,7-diazaspiro[4.5]dec-7-yl)piperidin-1-yl]carbonyl}-6-methylthieno[2,3-b]pyridin-2-yl)carbamate

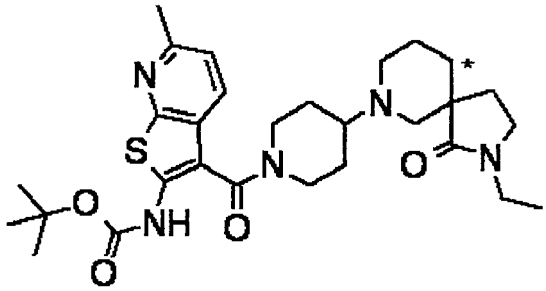

The title compound (0.95 g, yield 48%) as an oil was obtained by an operation in the same manner as in Example 63 and using tert-butyl 4-[2-ethyl-1-oxo-2,7-diazaspiro[4.5]dec-7-yl]piperidine-1-carboxylate in an optically active form (1.19 g, 3.53 mmol) obtained in Reference Example 69.
EI(pos) 556 $[M+H]^+$

Example 85

7-{1-[(2-amino-6-methylthieno[2,3-b]pyridin-3-yl)carbonyl]piperidin-4-yl}-2-ethyl-2,7-diazaspiro[4.5]decan-1-one
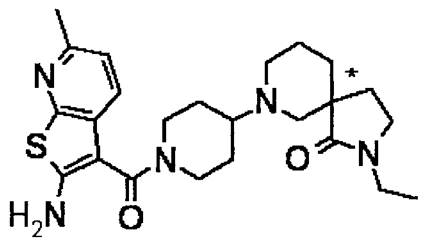

A solution of tert-butyl (3-{[4-(2-ethyl-1-oxo-2,7-diazaspiro[4.5]dec-7-yl)piperidin-1-yl]carbonyl}-6-methylthieno[2,3-b]pyridin-2-yl)carbamate (0.95 g, 1.71 mmol) obtained in Example 84 in trifluoroacetic acid (5 mL) was stirred at room temperature for 30 min. The reaction mixture was neutralized with saturated aqueous sodium hydrogen carbonate solution, and extracted twice with ethyl acetate, and the organic layer was dried over anhydrous magnesium sulfate. The solvent was evaporated under reduced pressure and the obtained residue was purified by basic silica gel column chromatography (methanol:ethyl acetate=0:1 to 1:9) to give the title compound (0.78 g, quantitatively) as an oil.

Example 86

N-ethyl-N'-(3-{[4-(2-ethyl-1-oxo-2,7-diazaspiro[4.5]dec-7-yl)piperidin-1-yl]carbonyl}-6-methylthieno[2,3-b]pyridin-2-yl)urea
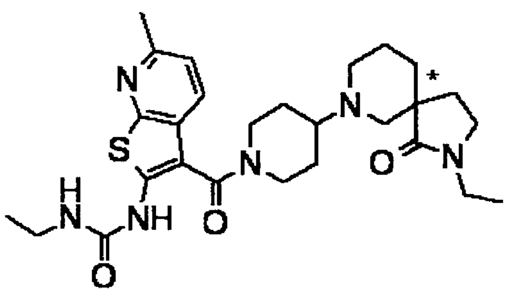

The title compound (0.30 g, yield 66%) as an oil was obtained by an operation in the same manner as in Example 4 and using 7-{1-[(2-amino-6-methylthieno[2,3-b]pyridin-3-yl)carbonyl]piperidin-4-yl}-2-ethyl-2,7-diazaspiro [4.5]decan-1-one (0.39 g, 0.855 mmol) obtained in Example 85.
EI(pos) 527 $[M+H]^+$

Example 87

N-(3-{[4-(2-ethyl-1-oxo-2,7-diazaspiro[4.5]dec-7-yl)piperidin-1-yl]carbonyl}-6-methylthieno[2,3-b]pyridin-2-yl)-N'-methylurea
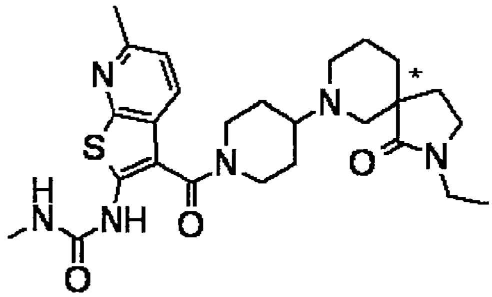

The title compound (248 mg, yield 56%) as an oil was obtained by an operation in the same manner as in Example 3 and using 7-{1-[(2-amino-6-methylthieno[2,3-b]pyridin-3-yl)carbonyl]piperidin-4-yl}-2-ethyl-2,7-diazaspiro [4.5]decan-1-one (0.39 g, 0.835 mmol) obtained in Example 85.
EI(pos) 513 $[M+H]^+$

Example 88

optically active form of 7-{1-[(2-amino-6-methylthieno[2,3-b]pyridin-3-yl)carbonyl]piperidin-4-yl}-2-isopropyl-2,7-diazaspiro[4.5]decane-1,3-dione
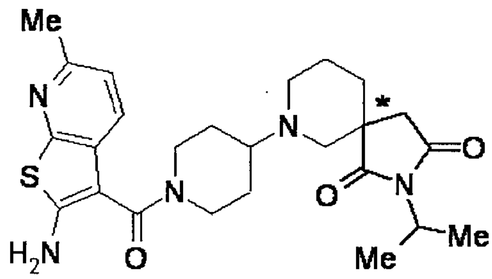

The title compound (2.38 g, yield 90%) as an oil was obtained by an operation in the same manner as in Example 1 and using 2-isopropyl-7-(piperidin-4-yl)-2,7-diazaspiro[4.5]decane-1,3-dione in an optically active form (1.60 g, 5.45 mmol) obtained in Reference Example 88 and 2-amino-6-methylthieno[2,3-b]pyridine-3-carboxylic acid trifluoroacetate (1.85 g, 5.72 mmol) obtained in Reference Example 70.
EI(pos) 484.0 $[M+H]^+$

Example 89

optically active form of N-(3-{[4-(2-isopropyl-1,3-dioxo-2,7-diazaspiro[4.5]dec-7-yl)piperidin-1-yl]carbonyl}-6-methylthieno[2,3-b]pyridin-2-yl)-N'-methylurea
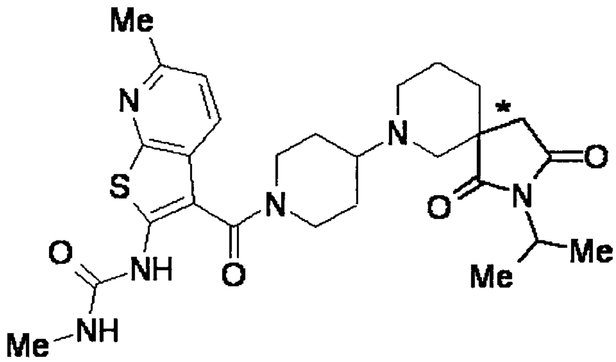

The title compound (1.81 g, yield 69%) was obtained by an operation in the same manner as in Example 3 and using 7-{1-[(2-amino-6-methylthieno[2,3-b]pyridin-3-yl)carbonyl]piperidin-4-yl}-2-isopropyl-2,7-diazaspiro[4.5]decane-1,3-dione in an optically active form (2.37 g, 4.90 mmol) obtained in Example 88, followed by recrystallization from ethyl acetate and hexane. EI(pos) 541.7 $[M+H]^+$
melting point 199°C (decomposition)
$[\alpha]_D^{20}$ +14.8° (c 1.02, methanol).
Calculated C; 59.98, H; 6.71, N; 15.54
Found C; 59.84, H; 6.69, N; 15.41.

Example 90

optically active form of tert-butyl (6-({[tert-butyl(diphenyl)silyl]oxy}methyl)-3-{[4-(2-isopropyl-1,3-dioxo-2,7-diazaspiro[4.5]dec-7-yl)piperidin-1-yl]carbonyl}thieno[2,3-b]pyridin-2-yl)carbamate
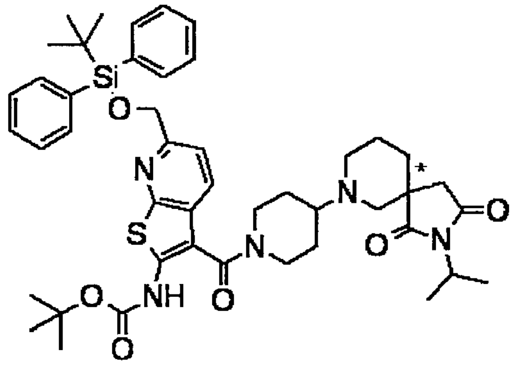

The title compound (0.68 g, yield 27%) was obtained in the same manner as in Example 1 and using 2-isopropyl-7-(piperidin-4-yl)-2,7-diazaspiro[4.5]decane-1,3-dione in an optically active form (876 mg, 2.98 mmol) obtained in Reference Example 87 and 2-[(tert-butoxycarbonyl)amino]-6-({[tert-butyl(diphenyl)silyl]oxy}methyl)thieno[2,3-b]pyridine-3-carboxylic acid (1.68 g, 2.98 mmol) obtained in Reference Example 76.
EI(pos) 839 $[M+H]^+$

Example 91

optically active form of 7-(1-{[2-amino-6-({[tert-butyl(diphenyl)silyl]oxy}methyl)thieno[2,3-b]pyridin-3-yl]carbonyl}piperidin-4-yl)-2-isopropyl-2,7-diazaspiro[4.5]decane-1,3-dione
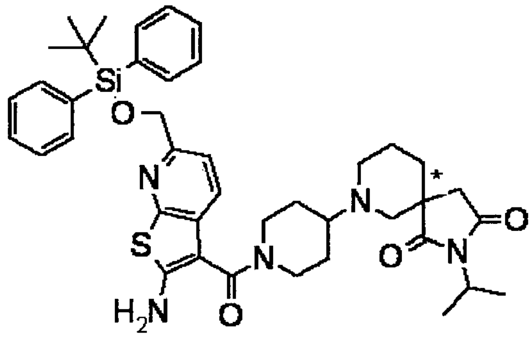

To tert-butyl (6-({[tert-butyl(diphenyl)silyl]oxy}methyl)-3-{[4-(2-isopropyl-1,3-dioxo-2,7-diazaspiro[4.5]dec-7-yl)piperidin-1-yl]carbonyl}thieno[2,3-b]pyridin-2-yl)carbamate in an optically active form (0.68 g, 0.811 mmol) obtained in Example 90 was added 4N hydrogen chloride-ethyl acetate (10 mL), and the mixture was stirred for 15 min. Saturated aqueous sodium hydrogen carbonate solution was added to the reaction mixture, and the mixture was extracted with ethyl acetate. The organic layer was washed with saturated brine, and dried over anhydrous magnesium sulfate. The solvent was evaporated under reduced pressure and the obtained residue was purified by silica gel column chromatography (ethyl acetate:hexane=3:2 to ethyl acetate) to give the title compound (523 mg, yield 87%) as an oil.
EI(pos) 738 $[M+H]^+$

Example 92

optically active form of N-(6-({[tert-butyl(diphenyl)silyl]oxy}methyl)-3-{[4-(2-isopropyl-1,3-dioxo-2,7-diazaspiro[4.5]dec-7-yl)piperidin-1-yl]carbonyl}thieno[2,3-b]pyridin-2-yl)-N'-methylurea
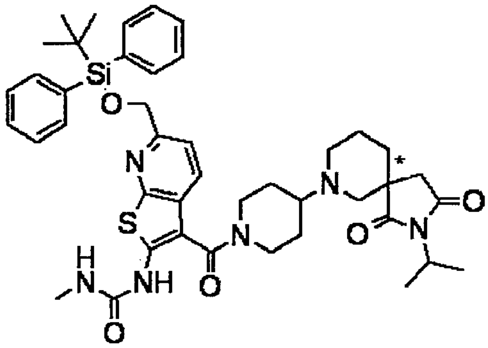

The title compound (303 mg, yield 53%) as an oil was obtained by an operation in the same manner as in Example 3 and using 7-(1-{[2-amino-6-({[tert-butyl(diphenyl)silyl]oxy}methyl)thieno[2,3-b]pyridin-3-yl]carbonyl}piperidin-4-yl)-2-isopropyl-2,7-diazaspiro[4.5]decane-1,3-dione in an optically active form (523 mg, 0.709 mmol) obtained in Example 91.
EI(pos) 795 $[M+H]^+$

Example 93

optically active form of N-(6-(hydroxymethyl)-3-{[4-(2-isopropyl-1,3-dioxo-2,7-diazaspiro[4.5]dec-7-yl)piperidin-1-yl]carbonyl}thieno[2,3-b]pyridin-2-yl)-N'-methylurea
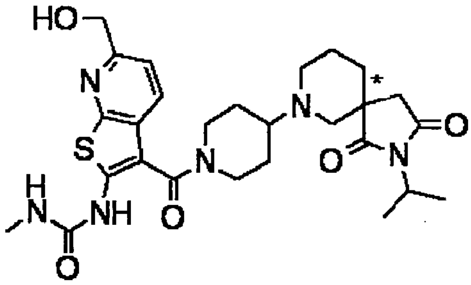

To a solution of N-(6-({[tert-butyl(diphenyl)silyl]oxy}methyl)-3-{[4-(2-isopropyl-1,3-dioxo-2,7-diazaspiro[4.5]dec-7-yl)piperidin-1-yl]carbonyl}thieno[2,3-b]pyridin-2-yl)-N'-methylurea in an optically active form (303 mg, 0.381 mmol) obtained in Example 92 in THF (5 mL) was added tetrabutylammonium fluoride (1M in THF, 0.58 mL, 0.580 mmol), and the mixture was stirred at room temperature for 2 hr. The reaction mixture was diluted with water, and extracted twice with ethyl acetate. The extract was washed with saturated brine, and dried over anhydrous magnesium sulfate. The solvent was evaporated under reduced pressure and the obtained residue was purified by silica gel column chromatography (ethyl acetate to methanol:ethyl acetate=1:9) to give the title compound (162 mg, yield 76%) as an oil.
EI(pos) 557 $[M+H]^+$

Example 94

tert-butyl [3-{[4-(2-isopropyl-1,3-dioxo-2,7-diazaspiro[4.5]dec-7-yl)piperidin-1-yl]carbonyl}-6-(trifluoromethyl)thieno[2,3-b]pyridin-2-yl]carbamate

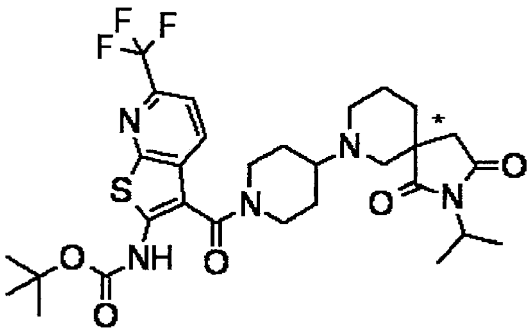

The title compound (0.73 g, yield 79%) was obtained in the same manner as in Example 1 and using 2-isopropyl-7-(piperidin-4-yl)-2,7-diazaspiro[4.5]decane-1,3-dione in an optically active form (426 mg, 1.45 mmol) obtained in Reference Example 87 and 2-[(tert-butoxycarbonyl)amino]-6-(trifluoromethyl)thieno[2,3-b]pyridine-3-carboxylic acid (524 mg, 1.45 mmol) obtained in Reference Example 80.
EI(pos) 638 $[M+H]^+$

Example 95

7-(1-{[2-amino-6-(trifluoromethyl)thieno[2,3-b]pyridin-3-yl]carbonyl}piperidin-4-yl)-2-isopropyl-2,7-diazaspiro[4.5]decane-1,3-dione

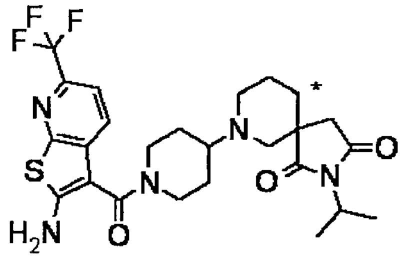

The title compound (0.61 g, yield 98%) as an oil was obtained by an operation in the same manner as in Example 85 and using tert-butyl [3-{[4-(2-isopropyl-1,3-dioxo-2,7-diazaspiro[4.5]dec-7-yl)piperidin-1-yl]carbonyl}-6-(trifluoromethyl)thieno[2,3-b]pyridin-2-yl]carbamate (0.73 g, 1.15 mmol) obtained in Example 94.
EI(pos) 538 $[M+H]^+$

Example 96

N-ethyl-N'-[3-{[4-(2-isopropyl-1,3-dioxo-2,7-diazaspiro[4.5]dec-7-yl)piperidin-1-yl]carbonyl}-6-(trifluoromethyl) thieno[2,3-b]pyridin-2-yl]urea
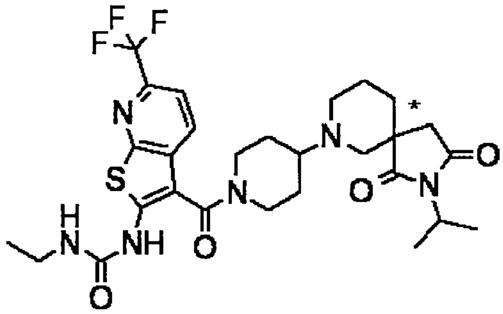

The title compound (51 mg, yield 51%) as an oil was obtained in the same manner as in Example 4 and using 7-(1-{[2-amino-6-(trifluoromethyl)thieno[2,3-b]pyridin-3-yl]carbonyl}piperidin-4-yl)-2-isopropyl-2,7-diazaspiro[4.5]decane-1,3-dione (0.61 g, 1.13 mmol) obtained in Example 95.
EI(pos) 609 $[M+H]^+$

Example 97

tert-butyl [3-{[4-(3-isopropyl-2,4-dioxo-1-oxa-3,7-diazaspiro[4.5]dec-7-yl)piperidin-1-yl]carbonyl}-6-(trifluoromethyl)thieno[2,3-b]pyridin-2-yl]carbamate
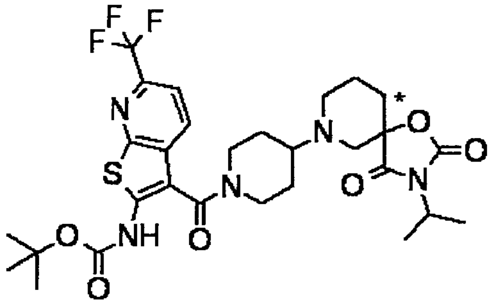

The title compound (1.06 g, yield 99%) was obtained in the same manner as in Example 1 and using 3-isopropyl-7-(piperidin-4-yl)-1-oxa-3,7-diazaspiro[4.5]decane-2,4-dione dihydrochloride in an optically active form (612 mg, 1.66

mmol) obtained in Reference Example 18 and 2-[(tert-butoxycarbonyl)amino]-6-(trifluoromethyl)thieno[2,3-b]pyridine-3-carboxylic acid (600 mg, 1.66 mmol) obtained in Reference Example 80.
EI(pos) 640 $[M+H]^+$

Example 98

7-(1-{[2-amino-6-(trifluoromethyl)thieno[2,3-b]pyridin-3-yl]carbonyl}piperidin-4-yl)-3-isopropyl-1-oxa-3,7-diazaspiro[4.5]decane-2,4-dione

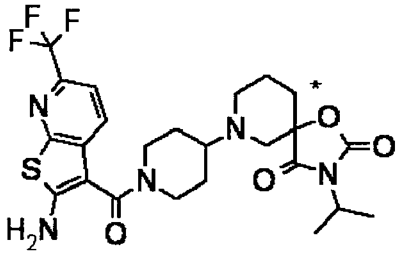

The title compound (451 mg, yield 50%) as an oil was obtained by an operation in the same manner as in Example 85 and using tert-butyl [3-{[4-(3-isopropyl-2,4-dioxo-1-oxa-3,7-diazaspiro[4.5]dec-7-yl)piperidin-1-yl]carbonyl}-6-(trifluoromethyl)thieno[2,3-b]pyridin-2-yl]carbamate (1.06 g, 1.65 mmol) obtained in Example 97.
EI(pos) 540 $[M+H]^+$

Example 99

N-[3-{[4-(3-isopropyl-2,4-dioxo-1-oxa-3,7-diazaspiro[4.5]dec-7-yl)piperidin-1-yl]carbonyl}-6-(trifluoromethyl) thieno[2,3-b]pyridin-2-yl]-N'-methylurea

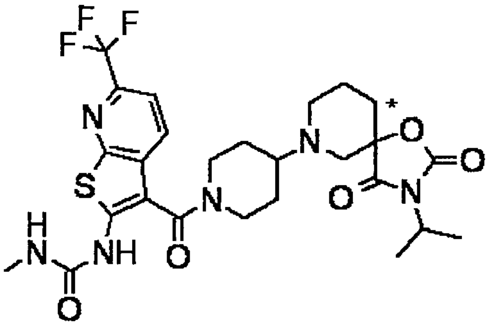

The title compound (190 mg, yield 45%) as an oil was obtained by an operation in the same manner as in Example 3 and using 7-(1-{[2-amino-6-(trifluoromethyl)thieno[2,3-b]pyridin-3-yl]carbonyl}piperidin-4-yl)-3-isopropyl-1-oxa-3,7-diazaspiro[4.5]decane-2,4-dione (451 mg, 0.705 mmol) obtained in Example 98.
EI(pos) 597 $[M+H]^+$

Experimental Example 1

The ACC1 inhibitory action of the compound of the present invention was evaluated by the following method.

(1) Cloning of human ACC1 gene and preparation of recombinant Baculovirus

Human ACC1 gene was cloned by PCR using a human liver cDNA library (Clontech) as a template and Primer 1 and Primer 2 shown below. Primer 1 and Primer 2 were prepared by adding SalI, NotI restriction enzyme recognition sequence based on the information of the base sequence (Genbank Accession U19822) of human ACC1 gene.
Primer 1 5'AAAAGTCGACCCACCATGGATGAACCTTCTCCCTTGGCCC (SEQ ID NO:1)

Primer 2 5'AAAAGCGGCCGCCTACGTAGAAGGGGAGTCCATAGTG (SEQ ID NO:2)
PCR was performed using a Pyrobest DNA polymerase (TAKARA BIO INC.) The obtained PCR product was cloned to pT7 Blue vector (Novagen) and, after confirmation of the base sequence, digested with restriction enzymes SalI and NotI. The obtained DNA fragment was inserted to pFAST-BacHTc (Invitrogen) digested with restriction enzymes SalI and NotI to give expression plasmid ACCl/pFAST-BacHTc.
Using the expression plasmid and BAC-TO-BAC Baculovirus Expression System (Invitrogen), virus stock BAC-ACC1 of recombinant Baculovirus was prepared.

(2) Preparation of ACC1 protein

SF-9 cells (Invitrogen) were inoculated to a medium (1 L) for insect cells (Sf-900IISFM medium (Invitrogen) containing 10% fetal bovine serum (Trace), 50 mg/L Gentamicin (Invitrogen) and 0.1% Pluronic F-68 (Invitrogen)) at $1\times10^6$ cells/mL, and cultured with shaking at 27°C, 100 rpm in a 2 L Erlenmeyer flask.
After 24 hr of the culture, recombinant Baculovirus BAC-ACC1 (10 mL) was added, and the cells were further cultured for 3 days. The culture medium was centrifuged at 1000xg for 5 min to give virus-infected cells. The cells were washed with phosphate buffered saline (Invitrogen), and centrifuged under the same conditions, and the obtained cells were cryopreserved at -80°C.
The cryopreserved cells were thawed in ice and suspended in 100 mL of 25 mM HEPES buffer (pH 7.5) containing 10% Glycerol, 0.13 M NaCl, 1 mM EDTA, 25 mM Sodium β-Glycerophosphate and 1 mM Sodium Orthovanadate, and supplemented with Complete Protease Inhibitor (Nippon Boehringer Ingelheim Co., Ltd.). The obtained suspension was homogenized 3 times in a polytron homogenizer (Kinematica) at 20,000 rpm for 30 sec. The obtained cell disruption solution was clarified by centrifugation at 185700xg for 50 min, and filtered through a 0.45 μm filter. The filtrate was passed through a column packed with 12 mL of Ni-NTA Super Flow Gel (QUIAGEN) at a flow rate of about 5 mL/min. The column was washed with buffer A (50 mM HEPES (pH 7.5) containing 0.3 M NaCl), further washed with buffer A containing 20 mM Imidazole, and eluted with buffer A containing 100 mM Imidazole. The eluate was concentrated with Vivaspin 20 (Vivascience) with a molecular weight cut off of 30K. The obtained concentrate was dialyzed against Sephadex G-25 (Amersham Biosciences, 358 mL) equilibrated with 50 mM HEPES (pH 7.5) containing 10 mM $MgCl_2$, 2 mM Dithiothreitol, 10 mM Tripotassium Citrate and 0.3 M NaCl. The inner dialysate was concentrated with Vivaspin 20 (Vivascience) with a molecular weight cut off of 30K, and the concentrate was filtered through a 0.22 μm filter to give ACC1. The obtained ACC1 was cryopreserved at -80°C.

(3) Measurement of ACC1 inhibitory activity

ACC1 (0.93 mg/ml) obtained in the above-mentioned (2) was diluted with an enzyme reaction buffer (50 mM HEPES (pH 7.5), 10 mM $MgCl_2$, 10 mM Tripottasium Citrate, 2 mM Dithiothreitol, 0.75 mg/ml Fatty acid free BSA) to concentration of 8 μg/ml, and the mixture was added to each well of 384 well assay plate (Nunc 265196) by 10 μl. Thereafter, ACC1 inhibitory rate (%) was measured in the same manner as in the below-mentioned Experimental Example 2-(3), and $IC_{50}$ value was calculated. As a result, the compounds of Examples 11, 14, 18, 19, 27, 28, 31, 52, 56, 65, 66, 78, 79 and 96 showed an $IC_{50}$ value of 10 to 100 nM.
As shown above, the compound of the present invention has a superior ACC1 inhibitory action.

Experimental Example 2

The ACC2 inhibitory action of the compound of the present invention was evaluated by the following method.

(1) Cloning of human ACC2 gene and preparation of recombinant Baculovirus

Human ACC2 gene was cloned by PCR using a human skeletal muscle cDNA library (Clontech) as a template and Primer 1 and Primer 2 shown below. Primer 1 and Primer 2 were prepared by adding SalI, XbaI restriction enzyme recognition sequences based on the information of the base sequence (Genbank Accession U89344) of human ACC2 gene.
Primer 1 5'AAAAGTCGACCCACCATGGTCTTGCTTCTTTGTCTATCTTG (SEQ ID NO:3)
Primer 2 5'TTTTTCTAGATCAGGTAGAGGCCGGGCTGTCCATG (SEQ ID NO:4)
PCR was performed using Pyrobest DNA polymerase (TAKARA BIO INC.). The obtained PCR product was cloned to pT7 Blue vector (Novagen) and, after confirmation of the base sequence, digested with restriction enzymes SalI and XbaI. The obtained DNA fragment was inserted into pFAST-BacHTa (Invitrogen) digested with restriction enzymes SalI and XbaI to give expression plasmid ACC2/pFAST-BacHTa.
PCR was performed using the expression plasmid as a template and Primer 3 and Primer 4 shown below to prepare a

plasmid to be used for expression of ACC2 free of mitochondrial targeting sequence.
Primer 3 5'CCAGGTCGACCCGCCAACGGGACTGGGACACAAGG (SEQ ID NO:5)
Primer 4 5'CGCACTCTCAGTTTCCCGGATTCCC (SEQ ID NO:6)
PCR was performed using Pyrobest-DNA polymerase (TAKARA BIO INC.). The obtained PCR product was cloned to pT7 Blue vector (Novagen) and, after confirmation of the base sequence, digested with restriction enzymes SalI and AflII. The obtained DNA fragment was inserted into pFAST-BacHTa (Invitrogen) digested with restriction enzymes SalI and AflII to give expression plasmid ACC2mito7/pFAST-BacHTa.
Using the expression plasmid and BAC-TO-BAC Baculovirus Expression System (Invitrogen), virus stock BAC-ACC2 (N terminal deleted (hereinafter Nd)) of recombinant Baculovirus was prepared.

(2) Preparation of ACC2 (Nd) protein

SF-9 cells (Invitrogen) were inoculated to a medium (2 L) for insect cells (Sf-900IISFM medium (Invitrogen) containing 10% fetal bovine serum (Trace), 50 mg/L Gentamicin (Invitrogen), 0.1% Pluronic F-68 (Invitrogen)) at $0.5\times10^6$ cells/mL, and cultured with shaking in Wave Bioreactor (Wave) at 27°C, 20 rpm, rocking angle 6°, oxygen concentration 30%.
On day 4 of the culture, 3 L of the medium for insect cells was added, the rocking angle was set to 8°, and the cells were cultured. On day 5 of the culture, 100 mL of recombinant Baculovirus BAC-ACC2 (Nd) was added, 5 L of the medium for insect cells was further added, the rocking angle was set to 11°, and the cells were cultured for 3 days. The culture medium was centrifuged at 1000×g for 10 min to give virus-infected cells. The cells were washed with phosphate buffered saline (Invitrogen) and centrifuged under the same conditions. The obtained cells were cryopreserved at -80°C.
The cryopreserved cells were thawed in ice and suspended in 900 mL of 25 mM HEPES buffer (pH 7.5) containing 10% Glycerol, 0.13 M NaCl, 1 mM EDTA, 25 mM Sodium β-Glycerophosphate and 1 mM Sodium Orthovanadate, and supplemented with Complete Protease Inhibitor (Nippon Boehringer Ingelheim Co., Ltd.). The obtained suspension was homogenized 3 times in a polytron homogenizer (Kinematica) at 20,000 rpm for 30 sec. The obtained cell disruption solution was clarified by centrifugation at 31000×g for 60 min, and filtered through a 0.45 μm filter. The filtrate was passed through a column packed with 60 mL of Ni-NTA Super Flow Gel (QUIAGEN) at a flow rate of about 5 mL/min. The column was washed with buffer A (50 mM HEPES (pH 7.5) containing 0.3 M NaCl), further washed with buffer A containing 20 mM Imidazole, and eluted with buffer A containing 100 mM Imidazole. The eluate was concentrated with Vivaspin 20 (Vivascience) with a molecular weight cut off of 30K. The obtained concentrate was dialyzed against 50 mM HEPES (pH 7.5) containing 10 mM $MgCl_2$, 2 mM Dithiothreitol, 10 mM Tripotassium Citrate and 0.3 M NaCl. The inner dialysate was filtered through a 0.22 μm filter to give ACC2 (Nd). The obtained ACC2 (Nd) was cryopreserved at - 80°C.

(3) Measurement of ACC2 inhibitory activity

ACC2 (Nd) (1.1 mg/ml) obtained in the above-mentioned (2) was diluted with an enzyme reaction buffer (50 mM HEPES (pH 7.5), 10 mM $MgCl_2$, 10 mM Tripottasium Citrate, 2 mM Dithiothreitol, 0.75 mg/ml Fatty acid free BSA) to a concentration of 6.4 μg/ml, and the mixture was added to each well of a 384 well assay plate (Nunc 265196) by 10 μl. A test compound was dissolved in dimethyl sulfoxide (DMSO) and the mixture was diluted with an enzyme reaction buffer and the resulting solution (5 μl) was added to each well. The mixture was incubated at 30°C for 60 min. Then, a substrate solution (50 mM $KHCO_3$, 200 μM ATP, 200 μM Acetyl-CoA, 5 μl) was added to each well, and the mixture was reacted at 30°C for 20 min (test compound addition group).
In addition, a reaction was performed in the same manner as above and without adding the test compound (test compound non-addition group).
Furthermore, a reaction was performed in the same manner as above and without adding the test compound and Acetyl-CoA (control group).
The reaction was quenched by adding a malachite green solution to each of the obtained reaction mixtures by 5 μl and stirring the mixtures. The obtained reaction mixture was left standing at room temperature for 20 min, and absorbance (620 nm) was measured using wallac1420 (PerkinElmer Japan Co., Ltd.). The above-mentioned malachite green solution was prepared by mixing Solution A (0.12% malachite green solution, prepared with 5N $H_2SO_4$, preserved at 4°C in shading), Solution B (7.5% aqueous ammonium molybdate solution, prepared when in use) and Solution C (11% aqueous Tween 20 solution, preserved at room temperature) at a ratio of Solution A: Solution B: Solution C=100:25:2 (volume ratio).
Then, ACC2 inhibitory rate (%) was measured from the calculation formula:

(1-(absorbance of test compound addition group - absorbance of control group)÷(absorbance of test compound non-addition group - absorbance of control group))×100, and

the $IC_{50}$ value vas calculated. As a result, the compounds of Examples 14, 18, 27, 28, 56, 65, 66, 74, 75, 78 and 79 showed an $IC_{50}$ value of 1 to 10 nM.
As shown above, the compound of the present invention has a superior ACC2 inhibitory action.

Formulation Example 1 (production of capsules)

| | | |
|---|---|---|
| 1) | compound of Example 1 | 30 mg |
| 2) | finely divided powder cellulose | 10 mg |
| 3) | lactose | 19 mg |
| 4) | magnesium stearate | 1 mg |
| | | total 60 mg |

1), 2), 3) and 4) are mixed and filled in a gelatin capsule.

Formulation Example 2 (production of tablets)

| | | |
|---|---|---|
| 1) | compound of Example 1 | 30 g |
| 2) | lactose | 50 g |
| 3) | cornstarch | 15 g |
| 4) | calcium carboxymethylcellulose | 44 g |
| 5) | magnesium stearate | 1 g |
| | 1000 tablets | total 140 g |

The total amount of 1), 2) and 3) and 4) (30 g) is kneaded with water, vacuum dried, and sieved.
The sieved powder is mixed with 4) (14 g) and 5) (1 g), and punched by a tableting machine, whereby 1000 tablets containing 30 mg of the compound of Example 1 per tablet are obtained.

## Industrial Applicability

The compound of the present invention has ACC (acetyl-CoA carboxylase) inhibitory action, and is useful for the prophylaxis or treatment of obesity, diabetes, hypertension, hyperlipidemia, cardiac failure, diabetic complications, metabolic syndrome, sarcopenia, cancer and the like.
This application is based on patent application No. 015593/2007 filed in Japan, the contents of which are hereby incorporated by reference.

## Claims

1. A compound represented by the formula (I):

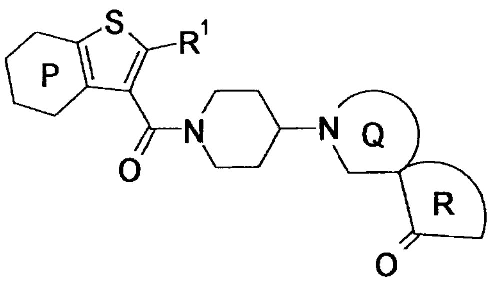

(I)

wherein
$R^1$ is a hydrogen atom or a substituent;
ring P is an optionally substituted 6-membered nitrogen-containing aromatic heterocycle;
ring Q is an optionally further substituted 5- to 7-membered nitrogen-containing non-aromatic heterocycle; and
ring R is an optionally fused 5- to 7-membered non-aromatic ring, which is further optionally substituted,
or a salt thereof.

2. The compound of claim 1, wherein $R^1$ is an optionally substituted amino group.

3. The compound of claim 1, wherein ring P is an optionally substituted pyridine ring.

4. The compound of claim 1, wherein ring Q is an optionally further substituted 6-membered monocyclic nitrogen-containing non-aromatic heterocycle.

5. The compound of claim 1, wherein ring R is an optionally further substituted 5-membered monocyclic non-aromatic heterocycle.

6. N-(3-{[4-(3,3-dimethyl-1-oxo-2-oxa-7-azaspiro[4.5]dec-7-yl)piperidin-1-yl]carbonyl}thieno[2,3-b]pyridin-2-yl)-N'-methylurea,

N-(3-{[4-(2-isopropyl-1,3-dioxo-2,7-diazaspiro[4.5]dec-7-yl)piperidin-1-yl]carbonyl}-6-methylthieno[2,3-b]pyridin-2-yl)-N'-methylurea,
N-ethyl-N'-(3-{[4-(3-isopropyl-2-methyl-4-oxo-1,3,7-triazaspiro[4.5]dec-1-en-7-yl)piperidin-1-yl]carbonyl}thieno[2,3-b]pyridin-2-yl)urea, or
N-ethyl-N'-(3-{[4-(2-isopropyl-1-oxo-2,7-diazaspiro[4.5]dec-7-yl)piperidin-1-yl]carbonyl}-6-methylthieno[2,3-b]pyridin-2-yl)urea,
or a salt thereof.

**7.** A prodrug of the compound of claim 1.

**8.** A pharmaceutical agent comprising the compound of claim 1 or a prodrug thereof.

**9.** The pharmaceutical agent of claim 8, which is an acetyl-CoA carboxylase inhibitor.

**10.** The pharmaceutical agent of claim 8, which is an agent for the prophylaxis or treatment of obesity, diabetes, hypertension, hyperlipidemia, cardiac failure, diabetic complications, metabolic syndrome, sarcopenia or cancer.

**11.** A method of inhibiting acetyl-CoA carboxylase in a mammal, which comprises administering the compound of claim 1 or a prodrug thereof to the mammal.

**12.** A method for the prophylaxis or treatment of obesity, diabetes, hypertension, hyperlipidemia, cardiac failure, diabetic complications, metabolic syndrome, sarcopenia or cancer in a mammal, which comprises administering the compound of claim 1 or a prodrug thereof to the mammal.

**13.** Use of the compound of claim 1 or a prodrug thereof, for the production of an acetyl-CoA carboxylase inhibitor.

**14.** Use of the compound of claim 1 or a prodrug thereof, for the production of an agent for the prophylaxis or treatment of obesity, diabetes, hypertension, hyperlipidemia, cardiac failure, diabetic complications, metabolic syndrome, sarcopenia or cancer.

**INTERNATIONAL SEARCH REPORT**

International application No. PCT/JP2008/050949

A. CLASSIFICATION OF SUBJECT MATTER
*C07D519/00*(2006.01)i, *A61K31/4545*(2006.01)i, *A61K31/695*(2006.01)i, *A61P1/00*(2006.01)i, *A61P1/04*(2006.01)i, *A61P1/12*(2006.01)i, *A61P1/14*(2006.01)i, *A61P1/16*(2006.01)i, *A61P1/18*(2006.01)i, *A61P3/04*(2006.01)i,

According to International Patent Classification (IPC) or to both national classification and IPC

B. FIELDS SEARCHED

Minimum documentation searched (classification system followed by classification symbols)
C07D519/00, A61K31/4545, A61K31/695, A61P1/00, A61P1/04, A61P1/12, A61P1/14, A61P1/16, A61P1/18, A61P3/04, A61P3/06, A61P3/10, A61P7/00, A61P7/02, A61P7/10, A61P9/04, A61P9/10, A61P9/12, A61P11/00, A61P11/04, A61P13/10,

Documentation searched other than minimum documentation to the extent that such documents are included in the fields searched

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | JP 2006-131559 A (Takeda Chemical Industries, Ltd.), 25 May, 2006 (25.05.06), (Family: none) | 1-10,13,14 |
| A | US 2003/0187254 A1 (PFIZER PRODUCTS INC., USA), 02 October, 2003 (02.10.03), & WO 2003/072197 A1 & AU 2003248354 A1 & EP 1478437 A1 & EP 1478437 B1 & CN 1642599 A & AT 303178 T & ES 2246481 T3 & NZ 534582 A & US 6979741 B2 & ZA 2004006332 A & NO 2004004034 A | 1-10,13,14 |
| A | JP 2005-119987 A (Ajinomoto Co., Inc.), 12 May, 2005 (12.05.05), (Family: none) | 1-10,13,14 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 April, 2008 (07.04.08) | 22 April, 2008 (22.04.08) |
| Name and mailing address of the ISA/ Japanese Patent Office | Authorized officer |
| Facsimile No. | Telephone No. |

Form PCT/ISA/210 (second sheet) (April 2007)

**INTERNATIONAL SEARCH REPORT**

International application No.
PCT/JP2008/050949

C (Continuation). DOCUMENTS CONSIDERED TO BE RELEVANT

| Category* | Citation of document, with indication, where appropriate, of the relevant passages | Relevant to claim No. |
|---|---|---|
| A | JP 2003-533509 A (Merck Sharp & Dohme Ltd.), 11 November, 2003 (11.11.03), & WO 2001/87838 A1 & AU 200156509 A & EP 1286967 A1 & US 2003/236250 A1 & US 7105507 B2 & EP 1286967 B1 & DE 60123420 E & ES 2273837 T3 & DE 60123420 T2 | 1-10,13,14 |
| A | JP 2005-510476 A (Bristol-Myers Squibb Co.), 21 April, 2005 (21.04.05), & WO 2003/029245 A1 & US 2004/009998 A1 & EP 1432700 A1 & KR 2004041649 A & BR 200213025 A & US 2004/248920 A1 & US 2004/259897 A1 & US 2005/004153 A1 & HU 200402338 A2 & NO 200401339 A & ZA 200402553 A & NZ 531870 A & CN 1596253 A & US 6977267 B2 & US 7078420 B2 & IN 200400871 P1 | 1-10,13,14 |
| A | WO 2005/075484 A2 (ROCHE PALO ALTO L.L.C., USA), 18 August, 2005 (18.08.05), & US 2005/0176703 A1 & US 7332500 B2 & AU 2005211499 A1 & CA 2554671 A1 & EP 1716156 A2 & CN 1918171 A & BR 2005007586 A & JP 2007-522160 A & NO 2006003495 A | 1-10,13,14 |
| A | WO 2005/097795 A1 (JANSSEN PHARMACEUTICA N. V., BELG.), 20 October, 2005 (20.10.05), & AU 2005231985 A1 & CA 2561944 A1 & EP 1735312 A1 & CN 1942471 A & JP 2007-531787 A & US 2007/0232636 A1 | 1-10,13,14 |
| A | WO 2004/026873 A1 (Ono Pharmaceutical Co., Ltd.), 01 April, 2004 (01.04.04), & CA 2497903 A1 & AU 2003272879 A1 & EP 1541574 A1 & BR 2003014304 A & CN 1688577 A & US 2005/0267114 A1 & NO 2005001379 A & ZA 2005002222 A | 1-10,13,14 |
| P,A | WO 2007/013691 A1 (Takeda Chemical Industries, Ltd.), 01 February, 2007 (01.02.07), (Family: none) | 1-10,13,14 |
| P,A | WO 2007/119833 A1 (Takeda Chemical Industries, Ltd.), 25 October, 2007 (25.10.07), (Family: none) | 1-10,13,14 |

Form PCT/ISA/210 (continuation of second sheet) (April 2007)

**INTERNATIONAL SEARCH REPORT**

International application No.
PCT/JP2008/050949

Continuation of A. CLASSIFICATION OF SUBJECT MATTER
(International Patent Classification (IPC))

*A61P3/06*(2006.01)i, *A61P3/10*(2006.01)i, *A61P7/00*(2006.01)i, *A61P7/02*(2006.01)i, *A61P7/10*(2006.01)i, *A61P9/04*(2006.01)i, *A61P9/10*(2006.01)i, *A61P9/12*(2006.01)i, *A61P11/00*(2006.01)i, *A61P11/04*(2006.01)i, *A61P13/10*(2006.01)i, *A61P13/12*(2006.01)i, *A61P15/00*(2006.01)i, *A61P15/08*(2006.01)i, *A61P19/02*(2006.01)i, *A61P19/06*(2006.01)i, *A61P19/10*(2006.01)i, *A61P21/00*(2006.01)i, *A61P21/04*(2006.01)i, *A61P25/02*(2006.01)i, *A61P25/16*(2006.01)i, *A61P25/22*(2006.01)i, *A61P25/28*(2006.01)i, *A61P29/00*(2006.01)i, *A61P35/00*(2006.01)i, *A61P43/00*(2006.01)i, *C07F7/18*(2006.01)i, *C07K5/02*(2006.01)i

(According to International Patent Classification (IPC) or to both national classification and IPC)

Continuation of B. FIELDS SEARCHED
Minimum documentation searched (International Patent Classification (IPC))

A61P13/12, A61P15/00, A61P15/08, A61P19/02, A61P19/06, A61P19/10, A61P21/00, A61P21/04, A61P25/02, A61P25/16, A61P25/22, A61P25/28, A61P29/00, A61P35/00, A61P43/00, C07F7/18, C07K5/02

Minimum documentation searched (classification system followed by classification symbols)

Form PCT/ISA/210 (extra sheet) (April 2007)

**INTERNATIONAL SEARCH REPORT**

International application No.
PCT/JP2008/050949

**Box No. II Observations where certain claims were found unsearchable (Continuation of item 2 of first sheet)**

This international search report has not been established in respect of certain claims under Article 17(2)(a) for the following reasons:

1. [X] Claims Nos.: 11, 12
because they relate to subject matter not required to be searched by this Authority, namely:
Claims 11 and 12 pertain to methods for treatment of the human body by therapy and thus relate to a subject matter which this International Searching Authority is not required, under the provisions of the PCT Rule 39.1(iv), to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying additional fees, this Authority did not invite payment of additional fees.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the

[ ] The additional search fees were accompanied by the applicant's protest and, where applicable, payment of a protest fee.

[ ] The additional search fees were accompanied by the applicant's protest but the applicable protest fee was not paid within the time limit specified in the invitation.

Form PCT/ISA/210 (continuation of first sheet (2)) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03029245 A
- WO 03072197 A
- JP 2006131559 A
- WO 0114372 A
- WO 9710224 A
- WO 0182925 A
- WO 0187834 A
- WO 2004000846 A
- WO 2006053024 A2
- JP 2007015593 A


### Non-patent literature cited in the description


- IYAKUHIN no KAIHATSU, Development of Pharmaceuticals. Design of Molecules. HIROKAWA SHOTEN, 1990, vol. 7, 163-198
- ORGANIC FUNCTIONAL GROUP PREPARATIONS. ACADEMIC PRESS, INC, 1989
- Comprehensive Organic Transformations. VCH Publishers Inc, 1989
- Protective Groups in Organic Synthesis. John Wiley and Sons, 1980
- *Journal of Medicinal Chemistry,* 1998, 2439-2441
- *Heterocycles,* 1992, 2263-2267
- Bioorganic and Medicinal Chemistry. 1999, 2945-2952
- *Journal of Medicinal Chemistry,* 2004, 2441-2450
- *Tetrahedron Letters,* 2001, 8345-8349
- *Journal of Medicinal Chemistry,* 1998, 4118-4129
- *Journal of Medicinal Chemistry,* 1990, 820-826
- *Helvetica Chimica Acta,* 1983, 450-465
- *Journal of Organic Chemistry,* 2004, 2441-2450
- *Tetrahedron Letters,* 1986, 3285-3288
- *Journal of Medicinal Chemistry,* 1988, 486-491
- Jikken Kagaku Kouza. vol. 20, 304, 477-479

SEQUENCE LISTING

<110> Takeda Pharmaceutical Company Limited

<120> SPIRO-RING COMPOUND

<130> 091173

<150> JP 2007-015593

<151> 2007-01-25

<160> 6

<170> PatentIn version 3.1

<210> 1
<211> 40
<212> DNA
<213> Artificial

<220>
<223> primer for cloning human ACC1 gene

<400> 1
aaaagtcgac ccaccatgga tgaaccttct cccttggccc 40

<210> 2
<211> 37
<212> DNA
<213> Artificial

<220>
<223> primer for cloning human ACC1 gene

<400> 2
aaaagcggcc gcctacgtag aaggggagtc catagtg 37

<210> 3
<211> 41
<212> DNA
<213> Artificial

<220>
<223> primer for cloning human ACC2 gene

<400> 3
aaaagtcgac ccaccatggt cttgcttctt tgtctatctt g 41

<210> 4
<211> 35
<212> DNA
<213> Artificial

<220>
<223> primer for cloning human ACC2 gene

<400> 4
tttttctaga tcaggtagag gccgggctgt ccatg 35

<210> 5
<211> 35
<212> DNA

<213> Artificial

<220>
<223> primer for cloning human ACC2 gene

<400> 5
ccaggtcgac ccgccaacgg gactgggaca caagg 35

<210> 6
<211> 25
<212> DNA
<213> Artificial

<220>
<223> primer for cloning human ACC2 gene

<400> 6
cgcactctca gtttcccgga ttccc 25